(19) Europäisches Patentamt
European Patent Office
Office européen des brevets

(11) **EP 2 971 105 B1**

(12) **EUROPEAN PATENT SPECIFICATION**

(45) Date of publication and mention
of the grant of the patent:
**01.08.2018 Bulletin 2018/31**

(51) Int Cl.:
***C12Q 1/6844*** (2018.01)   ***C12Q 1/6881*** (2018.01)

(21) Application number: **14722474.5**

(22) Date of filing: **17.03.2014**

(86) International application number:
**PCT/US2014/030859**

(87) International publication number:
**WO 2014/145992 (18.09.2014 Gazette 2014/38)**

(54) **METHOD OF IDENTIFYING ADAPTIVE IMMUNE RECEPTOR PAIRS**

VERFAHREN ZUR IDENTIFIZIERUNG VON ERWORBENEN
IMMUNSYSTEMSREZEPTORPAAREN

MÉTHODE D'IDENTIFICATION DE PAIRES DE RECEPTEURS IMMUNITAIRES ADAPTATIFS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **15.03.2013 US 201361789408 P
14.06.2013 PCT/US2013/045994**

(43) Date of publication of application:
**20.01.2016 Bulletin 2016/03**

(73) Proprietors:
• **Adaptive Biotechnologies Corporation
Seattle, Washington 98102 (US)**
• **Fred Hutchinson Cancer Research Center
Seattle, WA 98109 (US)**

(72) Inventor: **ROBINS, Harlan, S.
Seattle, WA 98102 (US)**

(74) Representative: **Boult Wade Tennant
Verulam Gardens
70 Gray's Inn Road
London WC1X 8BT (GB)**

(56) References cited:
WO-A1-2011/139371     WO-A2-2006/138284
WO-A2-2012/083069     WO-A2-2013/134162
US-A1- 2012 058 902     US-A1- 2012 135 409

• **Douglas Curran-Everett: "Multiple comparisons:
philosophies and illustrations", American
Journal of Physiology - Regulatory, Integrative
and Comparative Physiology, 1 July 2000
(2000-07-01), pages R1-R8, XP55127700, UNITED
STATES Retrieved from the Internet:
URL:http://ajpregu.physiology.org/content/
279/1/R1 [retrieved on 2014-07-09]**
• **PRADYOT DASH ET AL: "Paired analysis of
TCR[alpha] and TCR[beta] chains at the
single-cell level in mice", JOURNAL OF CLINICAL
INVESTIGATION, vol. 121, no. 1, 4 January 2011
(2011-01-04), pages 288-295, XP055083657, ISSN:
0021-9738, DOI: 10.1172/JCI44752**
• **SCOTT D BOYD ET AL: "Measurement and
clinical monitoring of human lymphocyte
clonality by massively parallel VDJ
pyrosequencing", SCIENCE TRANSLATIONAL
MEDICINE, vol. 1, no. 12, 23 December 2009
(2009-12-23), XP055076874, -& Scott D Boyd ET
AL: "Measurement and clinical monitoring of
human lymphocyte clonality by massively
parallel VDJ pyrosequencing: supplementary
materials", Science translational medicine, 23
December 2009 (2009-12-23), XP055083655,
United States Retrieved from the Internet:
URL:http://www.ncbi.nlm.nih.gov/pmc/articl
es/PMC2819115/ [retrieved on 2013-10-11]**
• **JENNIFER BENICHOU ET AL: "Rep-Seq:
uncovering the immunological repertoire
through next-generation sequencing",
IMMUNOLOGY, vol. 135, no. 3, 24 March 2012
(2012-03-24), pages 183-191, XP055083677, ISSN:
0019-2805, DOI:
10.1111/j.1365-2567.2011.03527.x**

Remarks:
•The complete document including Reference Tables and the Sequence Listing can be downloaded from the EPO website

•The file contains technical information submitted after the application was filed and not included in this specification

## Description

### RELATED APPLICATIONS

This application claims the benefit of U.S. Provisional Application No. 61/789,408, filed on March 15, 2013, and International Application No. PCT/US2013/045994, filed on Jun. 14, 2013.

### SEQUENCE LISTING

The instant application contains a Sequence Listing which has been submitted via EFS-Web. Said ASCII copy, created on March 13, 2014, is named 26371PCT_CRF_sequencelisting.txt, and is 4,382,702 bytes in size.

### BACKGROUND OF THE INVENTION

### Technical Field

The present disclosure relates generally to quantitative high-throughput sequencing of adaptive immune receptor encoding DNA or RNA (*e.g.,* DNA or RNA encoding T cell receptors and immunoglobulins) in multiplexed nucleic acid amplification reactions. In particular, the compositions and methods described herein permit quantitative sequencing of DNA sequences encoding both chains of an adaptive immune receptor heterodimer in a single cell. Also disclosed herein are embodiments that overcome undesirable distortions in the quantification of adaptive immune receptor encoding sequences that can result from biased over-utilization and/or under-utilization of specific oligonucleotide primers in multiplexed DNA amplification.

### Description of the Related Art

The adaptive immune system employs several strategies to generate a repertoire of T- and B-cell antigen receptors, *i.e.,* adaptive immune receptors, with sufficient diversity to recognize the universe of potential pathogens. The ability of T cells to recognize the universe of antigens associated with various cancers or infectious organisms is conferred by its T cell antigen receptor (TCR), which is a heterodimer of an $\alpha$ (alpha) chain from the TCRA locus and a $\beta$ (beta) chain from the TCRB locus, or a heterodimer of a $\gamma$ (gamma) chain from the TCRG locus and a $\delta$ (delta) chain from the TCRD locus. The proteins which make up these chains are encoded by DNA, which in lymphoid cells employs a unique rearrangement mechanism for generating the tremendous diversity of the TCR. This multi-subunit immune recognition receptor associates with the CD3 complex and binds to peptides presented by the major histocompatibility complex (MHC) class I and II proteins on the surface of antigen-presenting cells (APCs). Binding of TCR to the antigenic peptide on the APC is the central event in T cell activation, which occurs at an immunological synapse at the point of contact between the T cell and the APC.

Each TCR peptide contains variable complementarity determining regions (CDRs), as well as framework regions (FRs) and a constant region. The sequence diversity of $\alpha\beta$ T cells is largely determined by the amino acid sequence of the third complementarity-determining region (CDR3) loops of the $\alpha$ and $\beta$ chain variable domains, which diversity is a result of recombination between variable ($V_{\beta}$), diversity ($D_{\beta}$), and joining ($J_{\beta}$) gene segments in the $\beta$ chain locus, and between analogous $V_{\alpha}$ and $J_{\alpha}$ gene segments in the $\alpha$ chain locus, respectively. The existence of multiple such gene segments in the TCR $\alpha$ and $\beta$ chain loci allows for a large number of distinct CDR3 sequences to be encoded. CDR3 sequence diversity is further increased by independent addition and deletion of nucleotides at the $V_{\beta}$-$D_{\beta}$, $D_{\beta}$-$J_{\beta}$, and $V_{\alpha}$-$J_{\alpha}$ junctions during the process of TCR gene rearrangement. In this respect, immunocompetence is reflected in the diversity of TCRs.

The $\gamma\delta$ TCR is distinctive from the $\alpha\beta$ TCR in that it encodes a receptor that interacts closely with the innate immune system, and recognizes antigen in a non-HLA-dependent manner. TCR$\gamma\delta$ is expressed early in development, and has specialized anatomical distribution, unique pathogen and small-molecule specificities, and a broad spectrum of innate and adaptive cellular interactions. A biased pattern of TCR$\gamma$ V and J segment expression is established early in ontogeny. Consequently, the diverse TCR$\gamma$ repertoire in adult tissues is the result of extensive peripheral expansion following stimulation by environmental exposure to pathogens and toxic molecules.

Immunoglobulins (Igs or IG) expressed by B cells, also referred to herein as B cell receptors (BCR), are proteins consisting of four polypeptide chains, two heavy chains (H chains) from the IGH locus and two light chains (L chains) from either the IGK (kappa) or the IGL (lambda) locus, forming an $H_2L_2$ structure. Both H and L chains contain complementarity determining regions (CDR) involved in antigen recognition, and a constant domain. The H chains of IGs are initially expressed as membrane-bound isoforms using either the IgM or IgD constant region isoform, but after antigen recognition the H chain constant region can class switch to several additional isotypes, including IgG, IgE and IgA. As with TCR, the diversity of naive Igs within an individual is mainly determined by the hypervariable complementarity determining regions (CDR). Similar to the TCR, the CDR3 domain of IGH chains is created by the combinatorial joining of the $V_H$, $D_H$, and $J_H$ gene segments. Hypervariable domain sequence diversity is further increased by independent addition and deletion of nucleotides at the $V_H$-$D_H$, $D_H$-$J_H$, and $V_H$-$J_H$ junctions during the process of Ig gene rearrangement. Distinct from TCR, Ig sequence diversity is further augmented by somatic hypermutation (SHM) throughout the rearranged IG gene after a naive B cell initially recognizes an antigen. The process of SHM is not restricted to CDR3, and therefore can introduce changes in the germline sequence in framework regions, CDR1 and CDR2, as well as in the somatically rearranged CDR3.

As the adaptive immune system functions in part by clonal expansion of cells expressing unique TCRs or BCRs, accurately measuring the changes in total abundance of each clone is important to understanding the dynamics of an adaptive immune response. For instance, a healthy human has a few million unique TCRβ chains, each carried in hundreds to thousands of clonal T-cells out of the roughly trillion T cells in a healthy individual. Utilizing advances in high-throughput sequencing, a new field of molecular immunology has recently emerged to profile the vast TCR and BCR repertoires. Compositions and methods for the sequencing of rearranged adaptive immune receptor gene sequences and for adaptive immune receptor clonotype determination are described, for example, in Robins et al., 2009 Blood 114, 4099; Robins et al., 2010 Sci. Translat. Med. 2:47ra64; Robins et al., 2011 J. Immunol. Meth. doi:10.1016/j.jim.2011.09. 001; Sherwood et al. 2011 Sci. Translat. Med. 3:90ra61; U.S.A.N. 13/217,126 (US Pub. No. 2012/0058902), U.S.A.N. 12/794,507 (US Pub. No. 2010/0330571), WO/2010/151416, WO/2011/106738 (PCT/US2011/026373), and WO2012/027503 (PCT/US2011/049012).

To date, several different strategies have been employed to sequence nucleic acids encoding adaptive immune receptors quantitatively at high throughput, and these strategies may be distinguished, for example, by the approach that is used to amplify the CDR3-encoding regions, and by the choice of sequencing genomic DNA (gDNA) or messenger RNA (mRNA).

Sequencing mRNA is a potentially easier method than sequencing gDNA, because mRNA splicing events remove the intron between J and C segments. This allows for the amplification of adaptive immune receptors (*e.g.*, TCRs or Igs) having different V regions and J regions using a common 3' polymerase chain reaction (PCR) amplification primer in the C region. For each TCRβ, for example, the thirteen J segments are all less than 60 base pairs (bp) long. Therefore, splicing events bring identical polynucleotide sequences encoding TCRβ constant regions (regardless of which V and J sequences are used) to within less than 100 bp of the rearranged VDJ junction. The spliced mRNA can then be reverse transcribed into complementary DNA (cDNA) using poly-dT primers complementary to the poly-A tail of the mRNA, random small primers (usually hexamers or nonamers) or C-segment-specific oligonucleotides. This reverse transcription should produce an unbiased library of TCR cDNA (because all cDNAs are primed with the same oligonucleotide, whether poly-dT, random hexamer, or C segment-specific oligo) that may then be sequenced to obtain information on the V and J segment used in each rearrangement, as well as the specific sequence of the CDR3. Such sequencing could use single, long reads spanning CDR3 ("long read") technology, or could instead involve fractionating many copies of the longer sequences and using higher throughput shorter sequence reads.

Efforts to quantify the number of cells in a sample that express a particular rearranged TCR (or Ig) based on mRNA sequencing are difficult to interpret, however, because each cell potentially expresses different quantities of TCR mRNA. For example, T cells activated in vitro have 10-100 times as much mRNA per cell than quiescent T cells. To date, there is very limited information on the relative amount of TCR mRNA in T cells of different functional states, and therefore quantitation of mRNA in bulk does not necessarily accurately measure the number of cells carrying each clonal TCR.

Most T cells, on the other hand, have one productively rearranged TCRα and one productively rearranged TCRβ gene (or two rearranged TCRγ and TCRδ), and most B cells have one productively rearranged Ig heavy-chain gene and one productively rearranged Ig light-chain gene (either IGK or IGL) so quantification in a sample of genomic DNA encoding TCRs or BCRs should directly correlate with, respectively, the number of T or B cells in the sample. Genomic sequencing of polynucleotides encoding any one or more of the adaptive immune receptor chains, for instance, using the human TCRβ chain as a representative example, desirably entails amplifying with equal efficiency all of the many possible rearranged TCRβ encoding sequences that are present in a sample containing DNA from lymphoid cells of a subject, followed by quantitative sequencing, such that a quantitative measure of the relative abundance of each clonotype can be obtained.

Difficulties are encountered with such approaches, however, in that equal amplification and sequencing efficiencies may not be achieved readily, for example, for each rearranged TCRβ encoding clone, where each clone employs one of 54 possible germline V region-encoding genes and one of 13 possible J region-encoding genes. The specific sequences of the highly diverse V and J segments in the TCRβ genomic locus vary widely among the large number of possible rearrangements that result from using different V or J genes, due to diversity-generating mechanisms such as those summarized above.

This sequence diversity yields complex DNA samples in which accurate determination of the multiple distinct sequences contained therein is hindered by technical limitations on the ability to quantify a plurality of molecular species simultaneously using multiplexed amplification and high throughput sequencing. In addition, it is difficult from existing methodologies to sequence quantitatively DNA or RNA encoding both chains of a TCR or IG heterodimer in a manner that permits determination that both chains originated from the same lymphoid cell.

One or more factors can give rise to artifacts that skew sequencing data outputs, compromising the ability to obtain reliable quantitative data from sequencing strategies that are based on multiplexed amplification of a highly diverse collection of TCR or IG gene templates. These artifacts often result from unequal use of diverse primers during the multiplexed amplification step. Such biased utilization of one or more oligonucleotide primers in a multiplexed reaction that uses diverse amplification templates may arise as a function of one or more of differences in the nucleotide base composition of templates and/or oligonucleotide primers, differences in template and/or primer length, the particular polymerase that is used, the amplification reaction temperatures (*e.g.*, annealing, elongation and/or denaturation temperatures), and/or other factors (*e.g.,* Kanagawa, 2003 J. Biosci. Bioeng. 96:317; Day et al., 1996 Hum. Mol. Genet. 5:2039; Ogino et al., 2002 J. Mol. Diagnost. 4:185; Barnard et al., 1998 Biotechniques 25:684; Aird et al., 2011 Genome Biol. 12:R18).

WO2013/134162 teaches a method for determining paired immune receptor chains comprising partitioning a sample into a plurality of subsets, determining nucleotide sequences of a first chain and a second chain of each pair of immune receptor chains, and identifying as paired immune receptor chains those pairs of first chains and second chains (i) that, for every subset of the portion, either occur together or do not occur and (ii) that occur together in at least one subset of the portion and do not occur in at least one subset of the portion.

Clearly there remains a need for improved compositions and methods that will permit accurate quantification of adaptive immune receptor-encoding DNA and RNA sequence diversity in complex samples, in a manner that avoids skewed results such as misleading over- or underrepresentation of individual sequences due to biases in the utilization of one or more oligonucleotide primers in an oligonucleotide primer set used for multiplexed amplification of a complex template DNA population, and in a manner that permits determination of the coding sequences for both chains of a TCR or IG heterodimer that originate from the same lymphoid cell. The presently described embodiments address this need and provide other related advantages.

## SUMMARY OF THE INVENTION

The present invention provides a method of identifying a plurality of adaptive immune receptor (AIR) cognate pairs as defined in the appended claims.

The disclosure provides a method of identifying a plurality of cognate pairs comprising a first polypeptide and a second polypeptide that form an adaptive immune receptor heterodimer, said adaptive immune receptor heterodimer comprising a T cell receptor (TCR) or Immunoglobulin (IG) from a single clone in a sample, said sample comprising a plurality of lymphoid cells from a mammalian subject, said method comprising: distributing a plurality of lymphoid cells among a plurality of containers, each container comprising a plurality of lymphoid cells; generating a library of amplicons in said plurality of containers by performing multiplex PCR of cDNA molecules that have been reverse-transcribed from mRNA molecules obtained from said plurality of lymphoid cells, said library of amplicons comprising: i) a plurality of first adaptive immune receptor amplicons encoding said first polypeptide, each comprising a unique variable (V) region encoding sequence, a unique J region encoding sequence or both a unique J region encoding sequence and a unique C region encoding sequence, at least one barcode sequence, at least one universal adaptor sequence, and a sequencing platform tag sequence, and ii) a plurality of second adaptive immune receptor amplicons encoding said second polypeptide, each comprising a unique V region encoding sequence, a unique J region encoding sequence or both a unique J region encoding sequence and a unique C region encoding sequence, at least one barcode sequence, at least one universal adaptor sequence, and a sequencing platform tag sequence; performing high throughput sequencing of said library of amplicons to obtain a data set of a plurality of first and second adaptive immune receptor amplicon sequences.

The method includes determining a container occupancy pattern for each unique first adaptor immune receptor amplicon sequence by assigning each unique first adaptor immune receptor amplicon sequence to one or more containers, and a container occupancy pattern for each unique second adaptor immune receptor amplicon sequence by assigning each unique second adaptor immune receptor amplicon sequence to one or more containers, wherein each barcode sequence in said unique first or second adaptor immune receptor amplicon sequences is associated with a particular container. The method also includes for each possible pairing of a unique first and second adaptive immune receptor amplicon sequence to form a putative cognate pair, calculating a statistical probability of observing said container occupancy patterns, or observing any larger proportion of shared containers than expected by chance, given that said first and second adaptor immune receptor amplicon sequences do not originate from the same clonal population of lymphoid cells, and identifying a plurality of a putative cognate pairs based on said statistical probability having a score lower than a predetermined likelihood cutoff.

The method includes for each identified putative cognate pair, determining a false discovery rate estimation for a possible false pairing of said unique first adaptor immune receptor amplicon sequence and said unique second adaptor immune receptor amplicon sequence; and identifying a plurality of cognate pairs of unique first and second adaptive immune receptor sequences as true cognate pairs that encode said adaptive immune receptors in said sample based on said statistical probability and said false discovery rate estimation.

In some embodiments, the statistical score comprises a p-value calculated for pairing each putative cognate pair of unique first and second adaptive immune receptor amplicon sequences. In one embodiment, calculating the statistical score comprises calculating a probability that said unique first and second adaptive immune receptor amplicon sequences should jointly occupy as many or more containers than they are observed to jointly occupy, assuming no true cognate pairing and given the number of containers occupied by said unique first adaptive immune receptor amplicon sequence and the number of containers occupied by said unique second adaptive immune receptor amplicon sequence.

In another embodiment, identifying a plurality of a putative cognate pairs that have a high likelihood of pairing based on said statistical probability comprises for each unique first adaptor immune receptor amplicon sequence identifying the unique second adaptor immune receptor amplicon sequence that has the lowest p-value score of matching, or for each unique second adaptor immune receptor amplicon sequence finding the unique first adaptor immune receptor amplicon sequence that has the lowest p-value score of matching.

In other embodiments, determining a false discovery rate estimation comprises: calculating p-values for each of said plurality of putative cognate pairs identified in said sample; comparing the p-values for all of said plurality of putative cognate pairs with an expected p-value distribution, said expected p-value distribution calculated to represent an experiment where no true cognate pairs are present; and determining for each putative cognate pair, an expected proportion of false positive results such that all p-values at or below the p-value of said putative cognate pair are determined to represent a true cognate pairing.

In certain embodiments, calculating said expected p-value distribution comprises: permuting the containers in which each first and second adaptive immune receptor sequence has been observed in an otherwise-identical experiment with no true cognate pairs, and calculating the distribution of p-values associated with each putative cognate pair.

In some embodiments, the method includes identifying a plurality of cognate pairs of unique first and second adaptive immune receptor sequences as true cognate pairs by selecting a plurality of putative cognate pairs that have p-values below a threshold calculated based on said false discovery rate estimation.

In one embodiment, the identified cognate pair of unique first and second adaptive immune receptor amplicon sequences has a false discovery rate estimation of less than 1%.

In another embodiment, contacting each of said plurality of containers, under conditions and for a time sufficient to promote reverse transcription of mRNA molecules obtained from said plurality of lymphoid cells, with a first reverse transcription primer set. In certain embodiments, the (A) first oligonucleotide reverse transcription primer set comprises primers capable of reverse transcribing a plurality of mRNA sequences encoding said plurality of first and second adaptive immune receptor polypeptides for generating a plurality of first and second reverse-transcribed adaptive immune receptor cDNA amplicons, wherein said plurality of first reverse-transcribed adaptive immune receptor cDNA amplicons encoding said first adaptive immune receptor polypeptide comprise 1) a unique V region encoding gene sequence, and 2) a unique J region encoding gene sequence or both a unique J region encoding gene sequence and a unique C region encoding gene sequence, and wherein said plurality of second reverse-transcribed adaptive immune receptor cDNA amplicons encoding said second adaptive immune receptor polypeptide comprise 1) a unique V region encoding gene sequence, and 2) a unique J region encoding gene sequence or both a unique J region encoding gene sequence and a unique C region encoding gene sequence.

In certain aspects, the method includes contacting each of said plurality of containers, under conditions and for a time sufficient to promote a multiplex PCR amplification of said first and second reverse-transcribed adaptive immune receptor cDNA amplicons with a second (B) and third (C) oligonucleotide primer sets. In some aspects, the (B) second oligonucleotide primer set comprises forward and reverse primers capable of amplifying said plurality of first reverse-transcribed adaptor immune receptor cDNA amplicons, wherein said forward and reverse primers each are capable of hybridizing to said first reverse-transcribed adaptive immune receptor cDNA amplicons. In one aspect, each pair of forward and reverse primers in said second oligonucleotide primer set is capable of amplifying said first reverse-transcribed adaptive immune receptor cDNA amplicons. In another aspect, the forward primers in said second oligonucleotide primer set comprise a first universal adaptor sequence and a region complementary to said V region encoding gene sequence. In other aspects, the reverse primers in said second oligonucleotide primer set comprise a second universal adaptor sequence and a region complementary to said J region encoding gene sequence or said C region encoding gene sequence. In another aspect, the (C) third oligonucleotide primer set comprises forward and reverse primers capable of amplifying said plurality of reverse-transcribed second adaptive immune receptor cDNA amplicons. In one embodiment, each pair of forward and reverse primers in said third oligonucleotide primer set is capable of amplifying said second reverse-transcribed adaptive immune receptor cDNA amplicons. In one aspect, the forward primers in said third oligonucleotide primer set comprise a first universal adaptor sequence and a region complementary to said V region encoding gene sequence. In some aspects, the reverse primers in said third oligonucleotide primer set comprise a second universal adaptor sequence and a region complementary to said J region encoding gene sequence or complementary to said C region encoding gene sequence.

The method also includes generating i) a plurality of third adaptive immune receptor amplicons each comprising a unique V region encoding gene sequence, or complement thereof, a unique J region encoding gene sequence or both a unique J region encoding gene sequence and a unique C region encoding gene sequence, or complement thereof, and said first and second universal adaptor sequences, and ii) a plurality of fourth adaptive immune receptor amplicons each comprising a unique V region encoding gene sequence, or complement thereof, a unique J region encoding gene sequence or both a unique J region encoding gene sequence and a unique C region encoding gene sequence, or complement thereof, and said first and second universal adaptor sequences.

In another embodiment, the method includes contacting each of said plurality of containers, under conditions and for a time sufficient to promote a second multiplex PCR amplification of said plurality of third and fourth adaptive immune receptor amplicons with a fourth (D) oligonucleotide primer set and fifth (E) oligonucleotide primer set. In one embodiment, the (D) fourth oligonucleotide primer set comprises forward and reverse primers capable of amplifying said plurality of third adaptor immune receptor amplicons, wherein said forward and reverse primers each are capable of hybridizing to said third adaptive immune receptor amplicons. In one aspect, each pair of forward and reverse primers in said fourth oligonucleotide primer set is capable of amplifying said third adaptor immune receptor amplicons. In another aspect, the forward primer in said fourth oligonucleotide primer set comprises a sequencing platform tag sequence and a region complementary to said first universal adaptor sequence in said plurality of third adaptive immune receptor amplicon and said reverse primer comprises a sequencing platform tag sequence and a region complementary to said second universal adaptor sequence in said plurality of third adaptive immune receptor amplicons. In another embodiment, either one or both of said forward and reverse primers in said fourth oligonucleotide primer set comprises a unique barcode sequence associated with said container in which said fourth oligonucleotide primer set is introduced.

In one embodiment, the (E) fifth oligonucleotide primer set comprises forward and reverse primers capable of amplifying said plurality of fourth adaptor immune receptor amplicons, wherein said forward and reverse primers each are capable of hybridizing to said fourth adaptive immune receptor amplicons. In one embodiment, each pair of forward and reverse primers in said fourth oligonucleotide primer set is capable of amplifying said plurality of fourth adaptor immune receptor amplicons. In another embodiment, the forward primer in said fifth oligonucleotide primer set comprises a sequencing platform tag sequence and a region complementary to said first universal adaptor sequence in said plurality of fourth adaptive immune receptor amplicons, and said reverse primer in said fifth oligonucleotide primer set comprises a sequencing platform tag sequence and a region complementary to said second universal adaptor sequence in said plurality of fourth adaptive immune receptor amplicons. In other embodiments, either one or both of said forward and reverse primers of said fourth oligonucleotide primer set comprises a unique barcode sequence associated with said container in which said fourth oligonucleotide primer set is introduced, thereby generating said library of amplicons comprising said plurality of first adaptive immune receptor amplicons and said plurality of second adaptive immune receptor amplicons.

In one aspect, the method includes combining said library of amplicons from said plurality of containers into a mixture for sequencing. In another aspect, the plurality of first adaptive immune receptor amplicons comprise a C region encoding sequence. In some aspects, the plurality of second adaptive immune receptor amplicons comprise a C region encoding sequence.

In one embodiment, the sample comprises a blood sample. In another embodiment, the sample comprises a tissue sample. In certain embodiments, the sample comprises a sample purified or cultured human lymphoid cells. In other embodiments, the container comprises at least $10^4$ lymphoid cells. In another embodiment, the sample comprises at least $10^4$ cells.

In certain aspects, the first polypeptide of said adaptive immune receptor heterodimer is a TCR alpha (TCRA) chain and the second polypeptide of said adaptive immune receptor heterodimer is a TCR beta (TCRB) chain. In one aspect, first polypeptide of the adaptive immune receptor heterodimer is a TCR gamma (TCRG) chain and said second polypeptide of said adaptive immune receptor heterodimer is a TCR delta (TCRD) chain. In another aspect, the first polypeptide of said adaptive immune receptor heterodimer is an immunoglobulin heavy (IGH) chain and said second polypeptide of the adaptive immune receptor heterodimer is selected from an immunoglobulin light IGL or an IGK chain. In yet another aspect, if the first polypeptide of the adaptive immune receptor heterodimer is an IGH chain and the second polypeptide of the adaptive immune receptor heterodimer is both IGL and IGK, then three different amplification primer sets are used comprising: a first oligonucleotide amplification primer set for IGH, a second oligonucleotide amplification primer set for IGK, and a third oligonucleotide amplification primer set for IGL.

The disclosure provides a method of identifying a plurality of cognate pairs comprising a first polypeptide and a second polypeptide that form an adaptive immune receptor heterodimer, said adaptive immune receptor heterodimer comprising a T cell receptor (TCR) or Immunoglobulin (IG) from a single clone in a sample, said sample comprising a plurality of lymphoid cells from a mammalian subject. The method includes distributing a plurality of lymphoid cells among a plurality of containers, each container comprising a plurality of lymphoid cells and generating a library of amplicons in said plurality of containers by performing multiplex PCR of genomic molecules obtained from said plurality of lymphoid cells. The library of amplicons comprises i) a plurality of first adaptive immune receptor amplicons encoding said first polypeptide, each comprising a unique variable (V) region encoding sequence, a unique J region encoding sequence, at least one barcode sequence, at least one universal adaptor sequence, and a sequencing platform tag sequence, and ii) a plurality of second adaptive immune receptor amplicons encoding said second polypeptide, each comprising a unique V region encoding sequence, a unique J region encoding, at least one barcode sequence, at least one universal adaptor sequence, and a sequencing platform tag sequence.

The method includes performing high throughput sequencing of said library of amplicons to obtain a data set of a plurality of first and second adaptive immune receptor amplicon sequences and determining a container occupancy pattern for each unique first adaptor immune receptor amplicon sequence by assigning each unique first adaptor immune receptor amplicon sequence to one or more containers, and a container occupancy pattern for each unique second adaptor immune receptor amplicon sequence by assigning each unique second adaptor immune receptor amplicon sequence to one or more containers, wherein each barcode sequence in said unique first or second adaptor immune receptor amplicon sequences is associated with a particular container.

In some embodiments, the method includes for each possible pairing of a unique first and second adaptive immune receptor amplicon sequence to form a putative cognate pair, calculating a statistical probability of observing said container occupancy patterns, or observing any larger proportion of shared containers than expected by chance, given that said first and second adaptor immune receptor amplicon sequences do not originate from the same clonal population of lymphoid cells. In some embodiments, the method includes identifying a plurality of a putative cognate pairs based on said statistical probability having a score lower than a predetermined likelihood cutoff.

In other embodiments, the method includes for each identified putative cognate pair, determining a false discovery rate estimation for a possible false pairing of said unique first adaptor immune receptor amplicon sequence and said unique second adaptor immune receptor amplicon sequence.

In one embodiment, the method includes identifying a plurality of cognate pairs of unique first and second adaptive immune receptor sequences as true cognate pairs that encode said adaptive immune receptors in said sample based on said statistical probability and said false discovery rate estimation.

In other embodiments, the statistical score comprises a p-value calculated for pairing each putative cognate pair of unique first and second adaptive immune receptor amplicon sequences. In other embodiments, the step of calculating said statistical score comprises calculating a probability that said unique first and second adaptive immune receptor amplicon sequences should jointly occupy as many or more containers than they are observed to jointly occupy, assuming no true cognate pairing and given the number of containers occupied by said unique first adaptive immune receptor amplicon sequence and the number of containers occupied by said unique second adaptive immune receptor amplicon sequence.

In another embodiment, the method includes identifying a plurality of a putative cognate pairs that have a high likelihood of pairing based on said statistical probability comprises for each unique first adaptor immune receptor amplicon sequence identifying the unique second adaptor immune receptor amplicon sequence that has the lowest p-value score of matching, or for each unique second adaptor immune receptor amplicon sequence finding the unique first adaptor immune receptor amplicon sequence that has the lowest p-value score of matching.

In other embodiments, the step of determining a false discovery rate estimation comprises: calculating p-values for each of said plurality of putative cognate pairs identified in said sample; comparing the p-values for all of said plurality of putative cognate pairs with an expected p-value distribution, said expected p-value distribution calculated to represent an experiment where no true cognate pairs are present; and determining for each putative cognate pair, an expected proportion of false positive results such that all p-values at or below the p-value of said putative cognate pair are determined to represent a true cognate pairing.

In one embodiment, the step of calculating said expected p-value distribution comprises: permuting the containers in which each first and second adaptive immune receptor sequence has been observed in an otherwise-identical experiment with no true cognate pairs, and calculating the distribution of p-values associated with each putative cognate pair.

In another aspect, the method includes identifying a plurality of cognate pairs of unique first and second adaptive immune receptor sequences as true cognate pairs by selecting a plurality of putative cognate pairs that have p-values below a threshold calculated based on said false discovery rate estimation.

In one embodiment, an identified cognate pair of unique first and second adaptive immune receptor amplicon sequences has a false discovery rate estimation of less than 1%.

In another embodiment, the method includes contacting each of said plurality of containers, under conditions and for a time sufficient to promote a multiplex PCR amplification of said first and second adaptive immune receptor cDNA amplicons with a first (A) and second (B) oligonucleotide primer sets. In one embodiment, the (A) first oligonucleotide primer set comprises forward and reverse primers capable of amplifying said plurality of first adaptor immune receptor amplicons, wherein said forward and reverse primers each are capable of hybridizing to said first adaptive immune receptor amplicons. In one aspect, each pair of forward and reverse primers in said first oligonucleotide primer set is capable of amplifying said first adaptive immune receptor amplicons. In another aspect, the forward primers in said first oligonucleotide primer set comprise a first universal adaptor sequence and a region complementary to said V region encoding gene sequence. In one aspect, the reverse primers in said second oligonucleotide primer set comprise a second universal adaptor sequence and a region complementary to said J region encoding gene sequence.

In another aspect, the (B) second oligonucleotide primer set comprises forward and reverse primers capable of amplifying said plurality of second adaptive immune receptor amplicons. In one embodiment, each pair of forward and reverse primers in said second oligonucleotide primer set is capable of amplifying said second adaptive immune receptor amplicons. In another embodiment, the forward primers in said second oligonucleotide primer set comprise a first universal adaptor sequence and a region complementary to said V region encoding gene sequence. In another aspect, the reverse primers in said second oligonucleotide primer set comprise a second universal adaptor sequence and a region complementary to said J region encoding gene sequence.

In some embodiments, the method also includes generating i) a plurality of third adaptive immune receptor amplicons each comprising a unique V region encoding gene sequence, or complement thereof, a unique J region encoding gene sequence, or complement thereof, and said first and second universal adaptor sequences, and ii) a plurality of fourth adaptive immune receptor amplicons each comprising a unique V region encoding gene sequence, or complement thereof, a unique J region encoding gene sequence, or complement thereof, and said first and second universal adaptor sequences.

The method also includes the step of contacting each of said plurality of containers, under conditions and for a time sufficient to promote a second multiplex PCR amplification of said plurality of third and fourth adaptive immune receptor amplicons with a third (C) oligonucleotide primer set and fourth (D) oligonucleotide primer set. In one embodiment, the (C) third oligonucleotide primer set comprises forward and reverse primers capable of amplifying said plurality of third adaptor immune receptor amplicons, wherein said forward and reverse primers each are capable of hybridizing to said third adaptive immune receptor amplicons. In another embodiment, each pair of forward and reverse primers in said third oligonucleotide primer set is capable of amplifying said second adaptor immune receptor amplicons. In yet another embodiment, the forward primer in said third oligonucleotide primer set comprises a sequencing platform tag sequence and a region complementary to said first universal adaptor sequence in said plurality of third adaptive immune receptor amplicon and said reverse primer in said third oligonucleotide primer set comprises a sequencing platform tag sequence and a region complementary to said second universal adaptor sequence in said plurality of second adaptive immune receptor amplicons. In one aspect, either one or both of said forward and reverse primers in said fourth oligonucleotide primer set comprises a unique barcode sequence associated with said container in which said third oligonucleotide primer set is introduced.

In another aspect, the (D) fourth oligonucleotide primer set comprises forward and reverse primers capable of amplifying said plurality of fourth adaptor immune receptor amplicons, wherein said forward and reverse primers each are capable of hybridizing to said fourth adaptive immune receptor amplicons. In one embodiment, each pair of forward and reverse primers in said fourth oligonucleotide primer set is capable of amplifying said plurality of fourth adaptor immune receptor amplicons. In some embodiments, the forward primer in said fourth oligonucleotide primer set comprises a sequencing platform tag sequence and a region complementary to said first universal adaptor sequence in said plurality of fourth adaptive immune receptor amplicons, and said reverse primer in said fourth oligonucleotide primer set comprises a sequencing platform tag sequence and a region complementary to said second universal adaptor sequence in said plurality of fourth adaptive immune receptor amplicons. In yet another embodiment, either one or both of said forward and reverse primers of said fourth oligonucleotide primer set comprises a unique barcode sequence associated with said container in which said fourth oligonucleotide primer set is introduced, thereby generating said library of amplicons comprising said plurality of first adaptive immune receptor amplicons and said plurality of second adaptive immune receptor amplicons.

In other embodiments, the method includes combining said library of amplicons from said plurality of containers into a mixture for sequencing.

In some embodiments, the sample comprises a blood sample. In one embodiment, the sample comprises a tissue sample. In another embodiment, the sample comprises a sample purified or cultured human lymphoid cells. In some embodiments, each container comprises at least $10^4$ lymphoid cells. In other embodiments, the sample comprises at least $10^4$ cells.

In other embodiments, the first polypeptide of said adaptive immune receptor heterodimer is a TCR alpha (TCRA) chain and the second polypeptide of said adaptive immune receptor heterodimer is a TCR beta (TCRB) chain. In some embodiments, the first polypeptide of the adaptive immune receptor heterodimer is a TCR gamma (TCRG) chain and said second polypeptide of said adaptive immune receptor heterodimer is a TCR delta (TCRD) chain. In another embodiment, the first polypeptide of said adaptive immune receptor heterodimer is an immunoglobulin heavy (IGH) chain and said second polypeptide of the adaptive immune receptor heterodimer is selected from an immunoglobulin light IGL or an IGK chain. In other embodiments, if the first polypeptide of the adaptive immune receptor heterodimer is an IGH chain and the second polypeptide of the adaptive immune receptor heterodimer is both IGL and IGK, then three different amplification primer sets are used comprising: a first oligonucleotide amplification primer set for IGH, a second oligonucleotide amplification primer set for IGK, and a third oligonucleotide amplification primer set for IGL.

**BRIEF DESCRIPTION OF THE SEVERAL VIEWS OF THE DRAWINGS**

These and other features, aspects, and advantages of the present invention will become better understood with regard to the following description, and accompanying drawings, where:

Figure (FIG.) 1 depicts a schematic representation of certain herein described compositions and methods. U1 and U2 represent universal adaptor oligonucleotides. BC 1 and BC2 represent barcode oligonucleotides. J represents an adaptive immune receptor joining (J) region gene and Jpr represents a region of such a gene to which a J-specific oligonucleotide primer specifically anneals. V represents an adaptive immune receptor variable (V) region gene and Vpr represents a region of such a gene to which a V-specific oligonucleotide primer specifically anneals. NDN represents the diversity (D) region found in some adaptive immune receptor encoding genes, flanked on either side by junctional nucleotides (N) which may include non-templated nucleotides. Adap1 and Adap2 represent sequencing platform-specific adapters. The segment shown as "n6" represents a spacer nucleotide segment of any nucleotide sequence, in this case, a spacer of six randomly selected nucleotides.

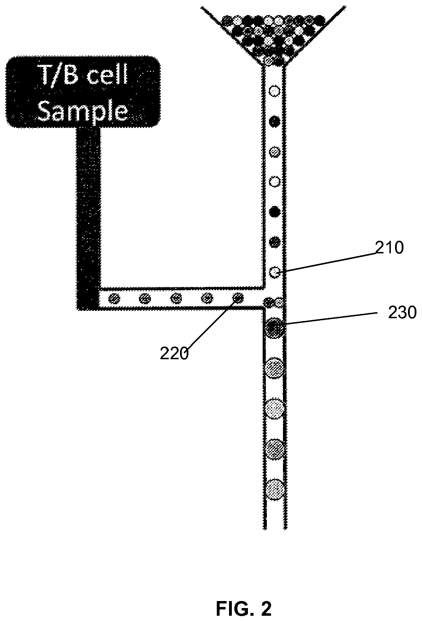

FIG. 2 depicts a schematic representation of certain herein described compositions and methods in which individual first and second microdroplets are contacted to permit fusion events between single first and second microdroplets, by which fusion events DNA from individual lymphoid cells (*e.g.,* T or B cells) is introduced, within a fused microdroplet, to first and second oligonucleotide amplification primer sets that are capable of amplifying, respectively, DNA encoding sequences (*e.g.,* CDR3 encoding DNA) of first and second adaptive immune receptor polypeptide encoding genes from the same cell. Amplification and oligonucleotide barcode labeling of at least two rearranged DNA loci from the same cell are thus contemplated as described herein, *e.g.,* [IGH + IGL], [IGH + IGK], [IGH + IGK + IGL], [TCRA + TCRB], [TCRG + TCRG], *etc.*

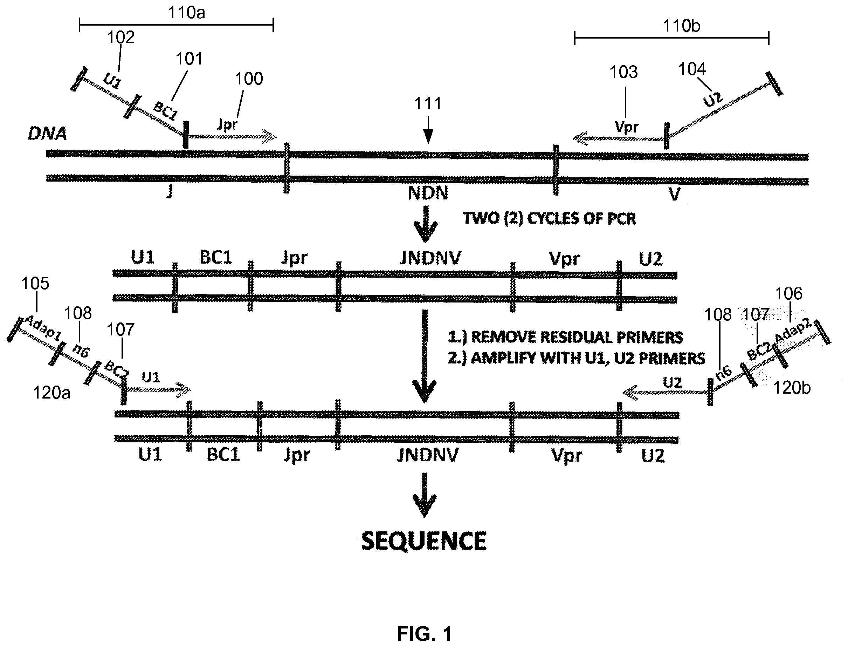

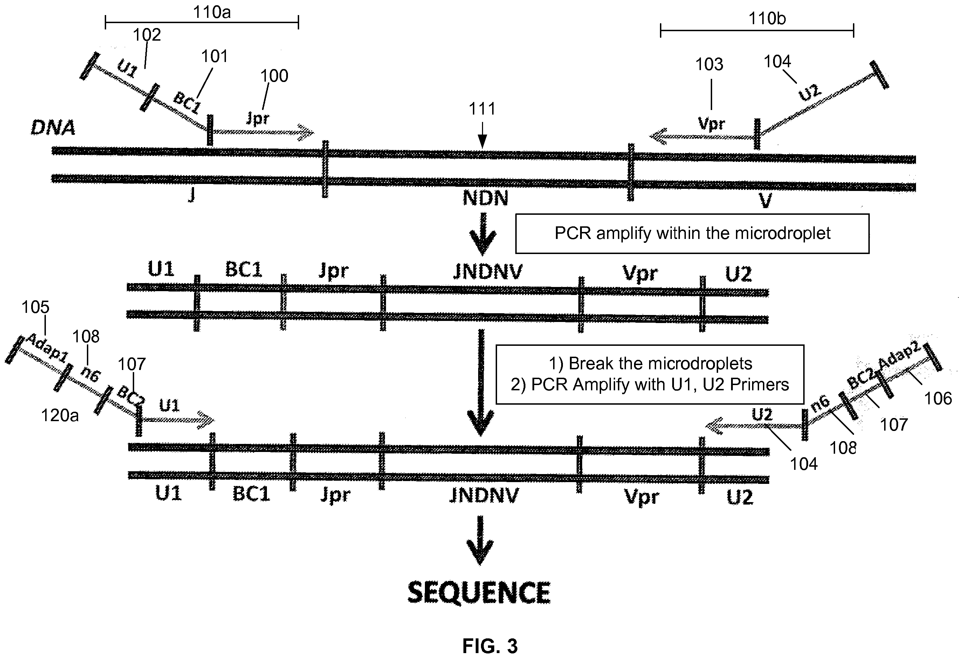

FIG. 3 depicts an exemplary schematic representation of certain herein described compositions and methods according to which, for example, DNA from individual lymphoid cells (*e.g.,* T or B cells), or cDNA that has been reverse transcribed from mRNA of single lymphoid cells, is introduced, within a fused microdroplet, to first and second oligonucleotide amplification primer sets that are capable of amplifying, respectively, DNA encoding sequences (*e.g.,* CDR3 encoding DNA) of first and second adaptive immune receptor polypeptide encoding genes from the same cell, after which the individual microdroplets are disrupted (*e.g.,* by chemical, physical and/or mechanical dissolution, dissociation, breakage, etc.) and the released bar-coded double-stranded DNAs are amplified with universal oligonucleotide primers and sequencing platform-specific adapters to permit large-scale multiplexed quantitative sequencing. See Brief Description of Fig. 1 for abbreviations.

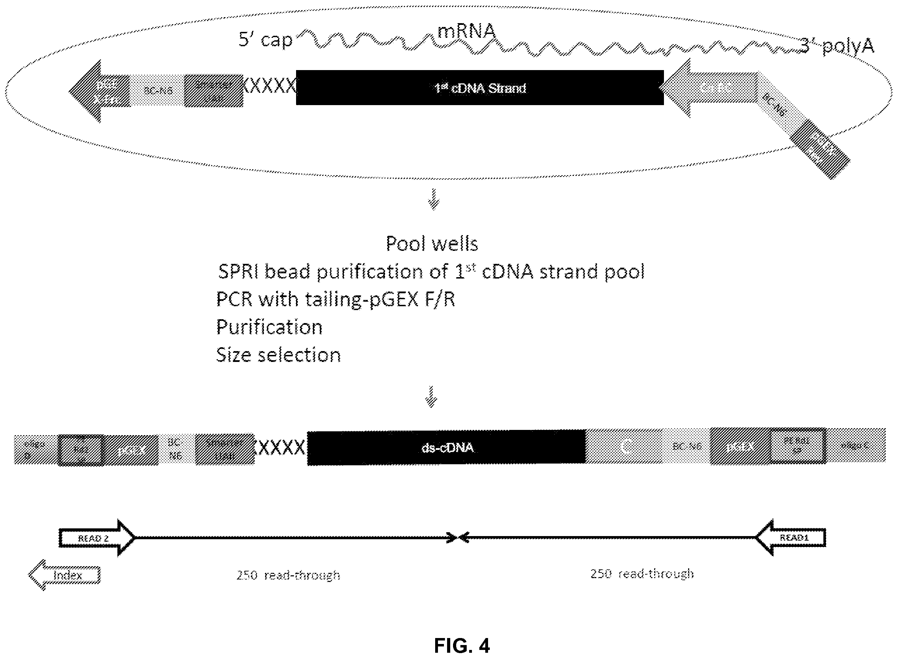

FIG. 4 depicts a schematic representation of labeling adaptive immune receptor polypeptide encoding cDNA during reverse transcription by using an oligonucleotide reverse transcription primer that directs incorporation of oligonucleotide barcode and universal adaptor oligonucleotide sequences into cDNA.

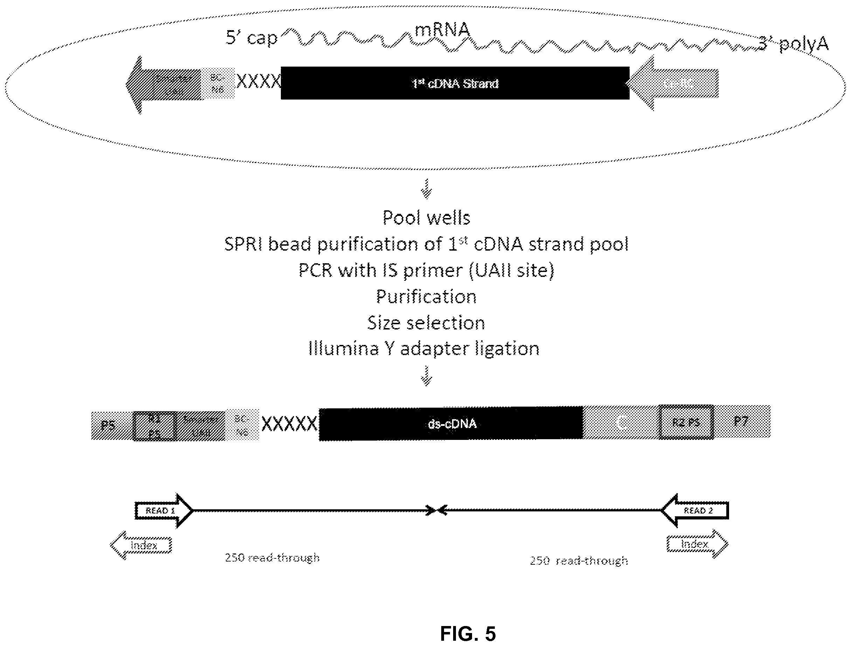

FIG. 5 depicts a schematic representation of labeling adaptive immune receptor polypeptide encoding cDNA during reverse transcription by using an oligonucleotide reverse transcription primer that directs incorporation of oligonucleotide barcode and universal adaptor oligonucleotide sequences into cDNA.

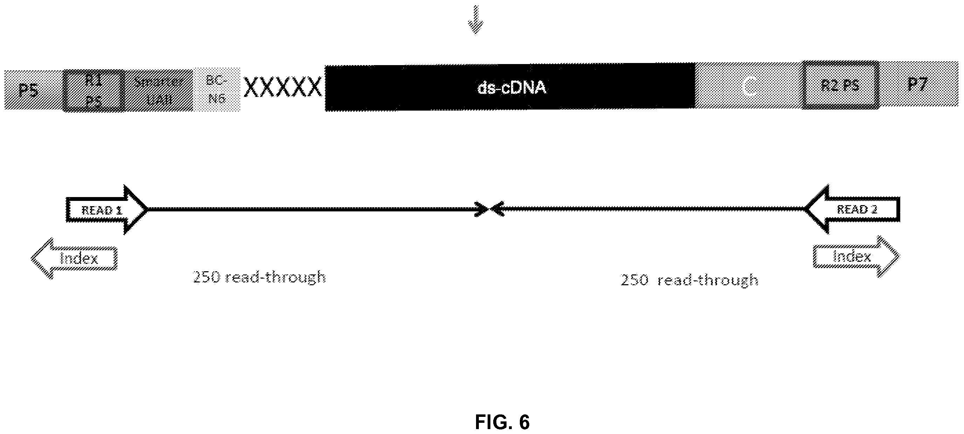

FIG. 6 presents a schematic representation of a DNA product that is amenable to sequencing following modification with Illumina sequencing adapters of amplified adaptive immune receptor polypeptide encoding cDNA that has been labeled during reverse transcription by using an oligonucleotide reverse transcription primer that directs incorporation of oligonucleotide barcode and universal adaptor oligonucleotide sequences.

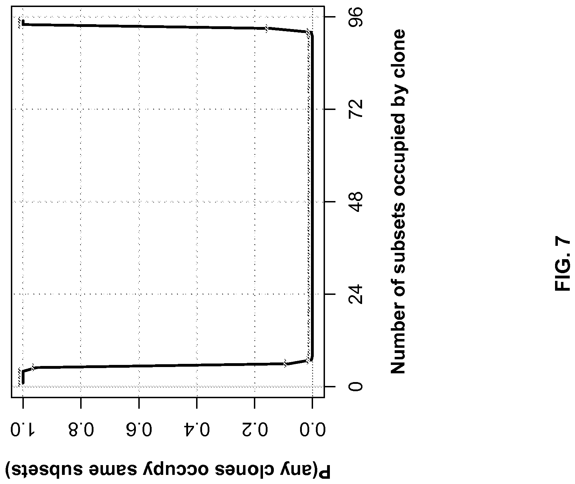

FIG. 7 shows the number of occupied T cell subsets vs. probability of shared subsets. For a simulated experiment using the methods of the invention, in which T cells are divided into 96 subsets containing 70,000 T cells each, this plot gives the probability (y-axis) that *any* clones present in a given number of subsets (x-axis) will occur in exactly the same subsets.

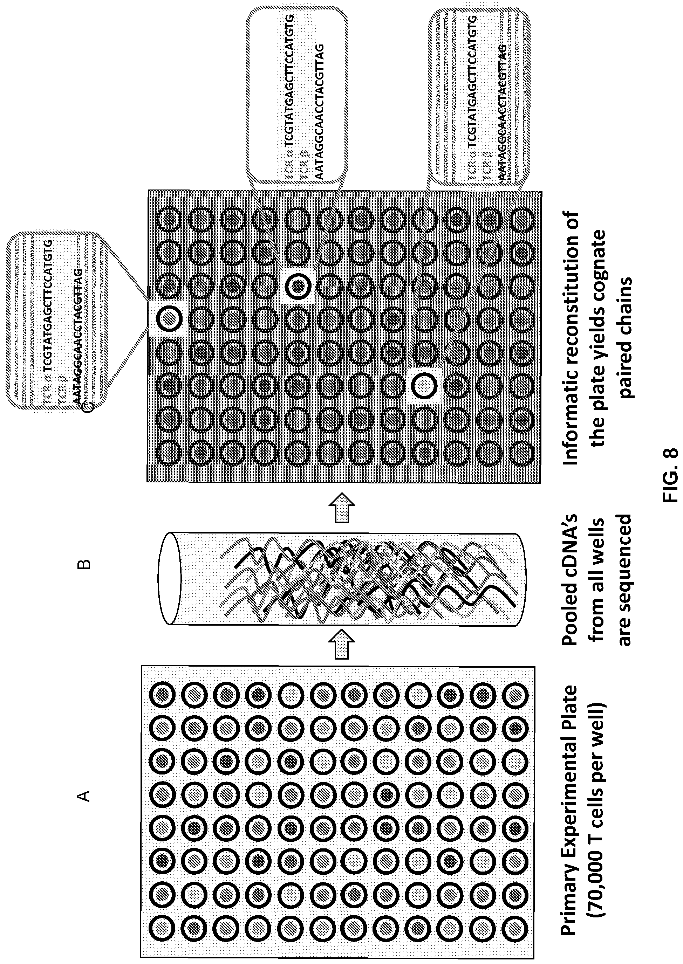

FIG. 8 shows a fixed number of T cells (e.g., 70,000 T cells) that are randomly allocated to each well on a 96-well plate, where the mRNA is extracted, converted to cDNA, and amplified by TCR-specific primers. Well-specific bar codes are attached and the TCR molecules are pooled for sequencing, followed by computational de-multiplexing to map each TCR sequence back to the wells in which it originated. The immune repertoire is highly diverse, and the probability that two clones will share a well pattern is miniscule, so any TCRA/TCRB pair that shares a well pattern can be inferred to have come from the same clone.

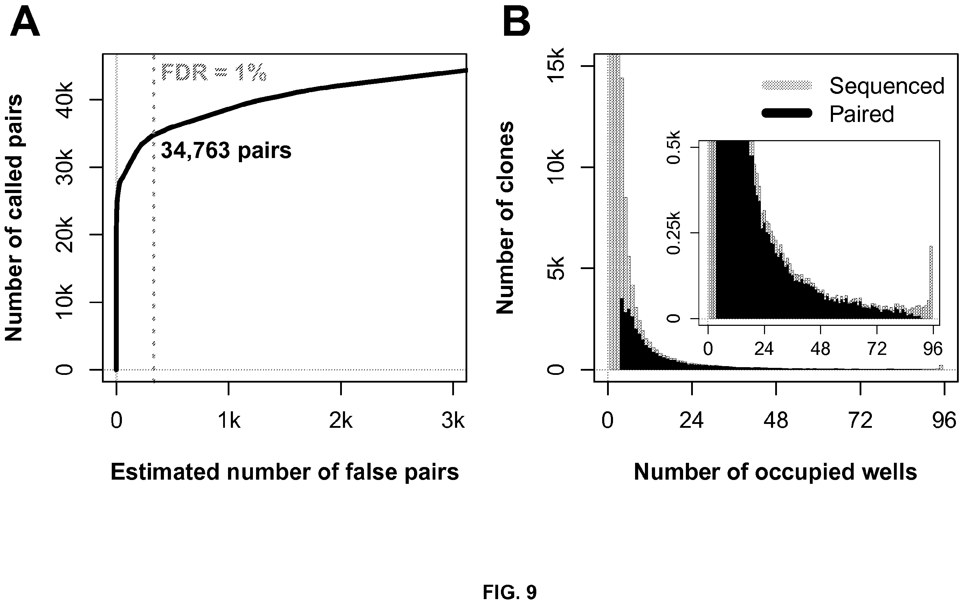

FIGs. 9A and 9B show the result of a high-throughput pairing experiment, according to an embodiment of the invention. FIG. 9A shows false discovery rate curve for the high-throughput pairing experiment with 96 wells and 70,000 T cells per well. A total of 34,763 pairs of TCR sequences were called with FDR<1% (red dotted line). FIG.

9B shows numbers of sequenced (light grey) and paired (dark) TCRB clones as a function of well occupancy. The inset provides a zoomed-in view of clones seen in >20 wells.

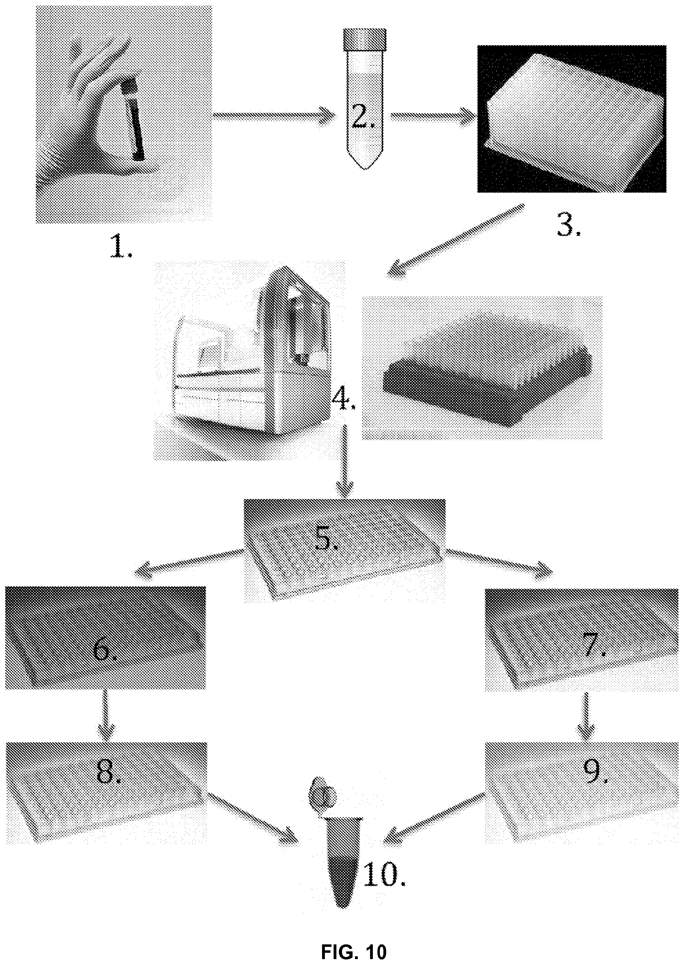

FIG. 10 shows an exemplary workflow for a high-throughput pairing experiment, according to an embodiment of the invention.

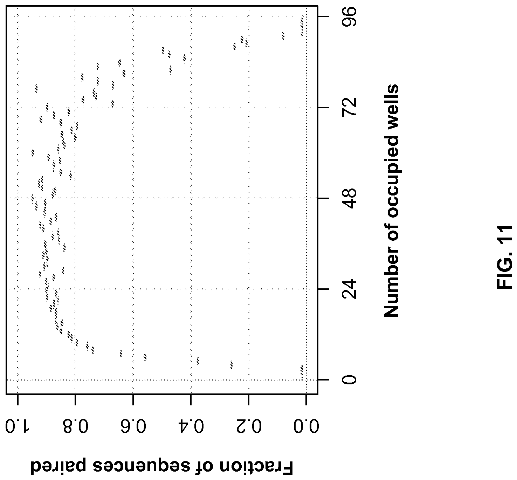

FIG. 11 shows the well occupancy vs. fraction of paired TCRB sequences, according to an embodiment of the invention. Pairing yield (fraction of paired sequences) for TCRB clones from a high-throughput pairing experiment with 96 wells and 70,000 T cells per well. TCRB clones were grouped by the number of wells in which they were observed (x-axis), and the fraction of these sequences that were paired at FDR<1% were computed (y-axis).

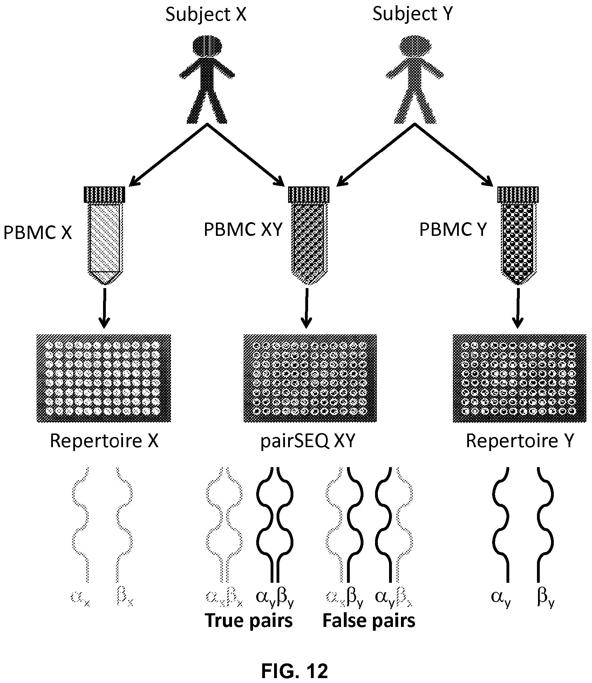

FIG. 12 shows a schematic of two-subject validation experiment, according to an embodiment of the invention. Peripheral blood is collected from two subjects, X and Y. Deep immunosequencing is used to characterize the TCRA and TCRB repertoire of each subject. PBMCs from the two subjects are then mixed, and the resulting mix is used to perform a high-throughput pairing experiment of the invention. True-positive pairs must include a TCRA and a TCRB from the same subject, while approximately half of false-positive results will be cross-subject TCRA/TCRB pairs.

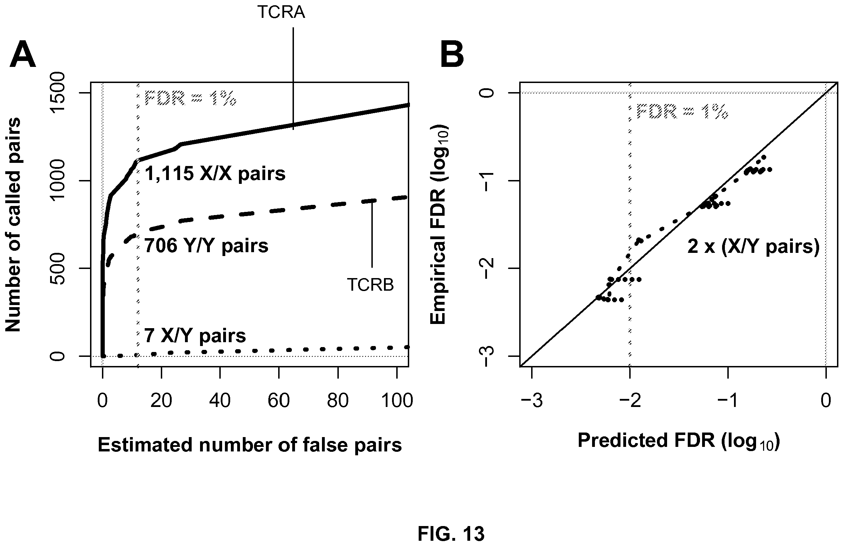

FIGs. 13A and 13B show validation of the high-throughput pairing approach using two donors. In FIG. 13A, false discovery rate curves for an experiment are shown in which PBMCs from two subjects ('X' and 'Y') were mixed and 25,000 cells were dispensed to each of 96 wells. Pairs are split into groups named 'X/X' (both members of a pair seen only in Subject X), 'Y/Y' (both members of a pair seen only in Subject Y), and 'X/Y' (one member of a pair seen only in Subject X and one member seen only in Subject Y). The dotted vertical line shows the cutoff for an estimated FDR<1%; below this threshold, there are 1,115 X/X pairs, 706 Y/Y pairs, and 7 X/Y pairs. In FIG. 13B, the predicted versus empirical $\log_{10}$ FDR from the sample-mixing experiment are shown. For each possible p-value cutoff, a predicted FDR value was provided by statistical methods of the disclosure and an empirical FDR value was computed as twice the number of X/Y pairs divided by the number of called pairs, under the assumption that X/Y pairs represent half the total number of errors. The vertical dotted line corresponds to the cutoff used in FIG. 13A.

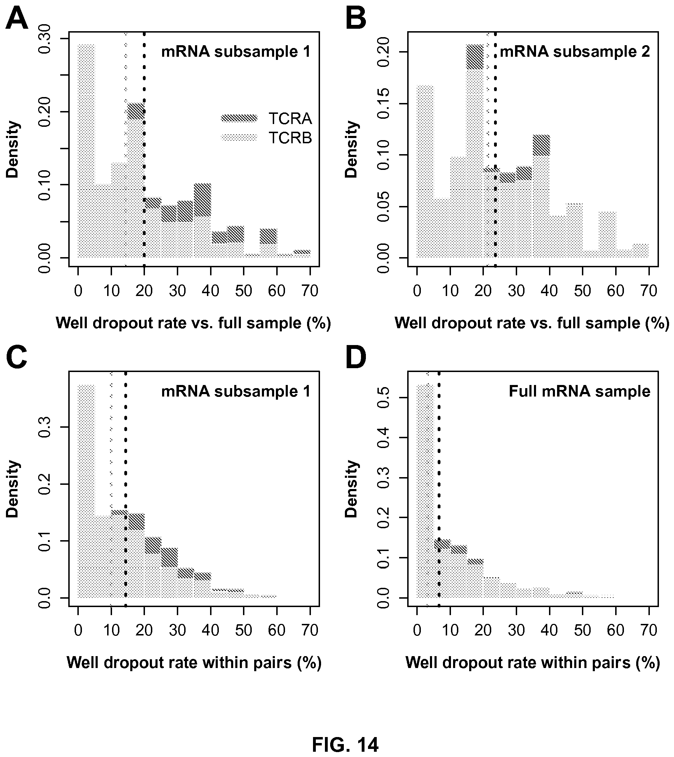

FIGs. 14A-14D show the well dropout rates in mRNA subsamples and within pairs. FIG. 14A shows well dropout rates in the first subsample of mRNA from a high-throughput pairing method of the invention with 70,000 T cells per well. Median dropout rates (dotted vertical lines) are 20% for TCRA and 14% for TCRB. FIG. 14B shows the well dropout rates in the second subsample of mRNA from a high-throughput pairing method of the invention. Median dropout rates are 24% for TCRA and 21% for TCRB. FIG. 14C shows the well dropout rates estimated from confidently paired sequences (FDR<1%) in the first subsample of mRNA (same data as in panel A). Median dropout rates are 14% for TCRA and 10% for TCRB, which are biased downward from the estimates in panel A. FIG. 14D shows well dropout rates estimated from confidently paired sequences (FDR<1%) in the full combined sample of mRNA, i.e., the data used in our discovery of ~35,000 pairs. Median dropout rates are 7% for TCRA and 3% for TCRB; after accounting for bias, we expect that the true median dropout rates are 10% and 5%, respectively.

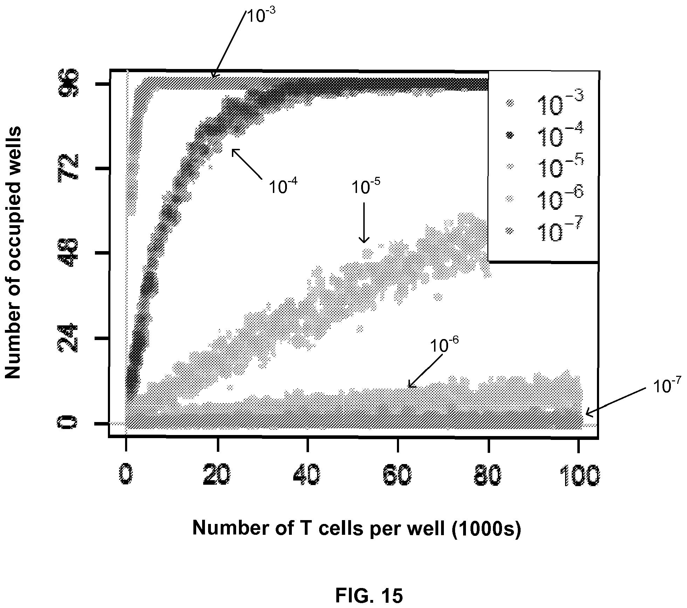

FIG. 15 shows simulated well occupancy as a function of clone frequency and number of input T cells. A key design parameter in a high-throughput pairing method of the invention is the number of T cells allocated to each well. FIG. 15 shows the outcomes of simulated experiments of the invention for a 96-well plate and T cell inputs ranging from 100-100,000 cells per well (x-axis). Different colors depict clones with different repertoire frequencies, and the plot shows the number of wells they occupied in our simulations. To capture clones from different frequency bands, one can simply change the number of input T cells in an experiment. It is also possible to capture multiple frequency bands in a single experiment by varying the number of input T cells across the wells on a plate.

## DETAILED DESCRIPTION OF THE INVENTION

The present disclosure provides, in certain embodiments and as described herein, compositions and methods that are useful for reliably quantifying and determining the sequences of large and structurally diverse populations of rearranged genes encoding adaptive immune receptors, such as immunoglobulins (IG) and/or T cell receptors (TCR). These rearranged genes may be present in a biological sample containing DNA from lymphoid cells of a subject or biological source, including a human subject, and/or mRNA transcripts of these rearranged genes may be present in such a sample and used as templates for cDNA synthesis by reverse transcription.

Disclosed herein are unexpectedly advantageous approaches for uniquely and unambiguously labeling individual, sequence-distinct IG and TCR encoding gene segments or mRNA transcripts thereof, or cDNA that has been reverse transcribed from such mRNA transcripts, by performing such labeling prior to conventional steps of expanding a population of such gene segments or transcripts thereof (including reverse transcripts) through established nucleic acid amplification techniques. Without wishing to be bound by theory, by labeling individual TCR and IG encoding gene segments or transcripts thereof (including complementary DNA generated by reverse transcription) as described herein, prior to commonly practiced amplification steps which are employed to generate DNA copies in sufficient quantities for sequencing, the present embodiments offer unprecedented sensitivity in the detection and quantification of diverse TCR and IG encoding sequences, while at the same time avoiding misleading, inaccurate or incomplete results that may occur due to biases in oligonucleotide primer utilization during multiple rounds of nucleic acid amplification from an original sample, using a sequence-diverse set of amplification primers.

Also described herein, in certain embodiments, are unprecedented compositions and methods that permit quantitative determination of the sequences encoding both polypeptides in an adaptive immune receptor heterodimer from a single cell, such as both TCRA and TCRB from a T cell, or both IgH and IgL from a B cell. By providing the ability to obtain such information from a complex sample such as a sample containing a heterogeneous mixture of T and/or B cells from a subject, these and related embodiments permit more accurate determination of the relative representation in a sample of particular T and/or B cell clonal populations than has previously been possible.

Certain embodiments contemplate modifications as described herein to oligonucleotide primer sets that are used in multiplexed nucleic acid amplification reactions to generate a population of amplified rearranged DNA molecules from a biological sample containing rearranged genes encoding adaptive immune receptors, prior to quantitative high throughput sequencing of such amplified products. Multiplexed amplification and high throughput sequencing of rearranged TCR and BCR encoding DNA sequences are described, for example, in Robins et al., 2009 Blood 114:4099; Robins et al., 2010 Sci. Translat. Med. 2:47ra64; Robins et al., 2011 J. Immunol. Meth. doi:10.1016/j.jim.2011.09. 001; Sherwood et al. 2011 Sci. Translat. Med. 3:90ra61; U.S.A.N. 13/217,126 (US Pub. No. 2012/0058902), U.S.A.N. 12/794,507 (US Pub. No. 2010/0330571), WO/2010/151416, WO/2011/106738 (PCT/US2011/026373), and WO2012/027503 (PCT/US2011/049012).

According to certain embodiments, in a sample containing a plurality of sequence-diverse TCR or IG encoding gene segments, such as a sample comprising DNA (or mRNA transcribed therefrom or cDNA reverse-transcribed from such mRNA) from lymphoid cells in which DNA rearrangements have taken place to encode functional TCR and/or IG heterodimers (or in which non-functional TCR or IG pseudogenes have been involved in DNA rearrangements), a plurality of individual TCR or IG encoding sequences may each be uniquely tagged with a specific oligonucleotide barcode sequence as described herein, through a single round of nucleic acid amplification (e.g., polymerase chain reaction PCR). The population of tagged polynucleotides can then be amplified to obtain a library of tagged molecules, which can then be quantitatively sequenced by existing procedures such as those described, for example, in U.S.A.N. 13/217,126 (US Pub. No. 2012/0058902), U.S.A.N. 12/794,507 (US Pub. No. 2010/0330571), WO/2010/151416, WO/2011/106738 (PCT/US2011/026373), and WO2012/027503 (PCT/US2011/049012).

In the course of these sequence reads, the incorporated barcode tag sequence is sequenced and can be used as an identifier in the course of compiling and analyzing the sequence data so obtained. In certain embodiments, it is contemplated that for each barcode tag sequence, a consensus sequence for the associated TCR or IG sequences may be determined. A clustering algorithm can then be applied to identify molecules generated from the same original clonal cell population. By such an approach, sequence data of high quality can be obtained in a manner that overcomes inaccuracies associated with sequencing artifacts.

An exemplary embodiment is depicted in Figure 1, according to which from a starting template population of genomic DNA or cDNA from a lymphoid cell-containing population, two or more cycles of PCR are performed using an oligonucleotide primer composition that contains primers having the general formula $U1\text{-}B1_n\text{-}X$ as described herein. As shown in Figure (Fig.) 1, the J-specific primer 110a contains a J primer sequence 100 that is complementary to a portion of the J segment, a barcode tag (BC1) 101 in Fig. 1, or $B1_n$ in the generic formula) and also includes a first external universal adaptor sequence (U1) 102, while the V-specific primer 110b includes a V primer sequence 103 that is complementary to a portion of the V segment and a second external universal adaptor sequence (U2) 104. The disclosed method need not be so limited, however, and also contemplates related embodiments, such as those where the barcode may instead or may in addition be present as part of the V-specific primer and is situated between the V-sequence and the second universal adaptor. It will be appreciated that based on the present disclosure, those skilled in the art can design other suitable primers by which to introduce the herein described barcode tags to uniquely label individual TCR and/or IG encoding gene segments.

As described herein, a large number (up to $4^n$, where n is the length of the barcode sequence) of different barcode sequences are present in the oligonucleotide primer composition that contains primers having the general formula $U1\text{-}B1_n\text{-}X$ as described herein, such that the PCR products of the large number of different amplification events following specific annealing of appropriate V- and J-specific primers are differentially labeled. In some embodiments, the number of barcode sequences is up to or smaller than $4^n$. In one embodiment, a set of 192 different barcode sequences are used based on a barcode of length n=8. The length of the barcode "n" determines the possible number of barcodes ($4^n$ as described herein), but in some embodiments, a smaller subset is used to avoid closely related barcodes or barcodes with different annealing temperatures. In other embodiments, as described herein, sets of m and n barcode sequences are used in subsequent amplification steps (e.g., to individually label each rearranged TCR or IG sequence and then to uniformly label ("tailing") a set of sequences obtained from the same source, or sample In preferred embodiments, the V and J primers 100 and 103 are capable of promoting the amplification of a TCR or Ig encoding sequence that includes the CDR3 encoding sequence, which in Fig. 1 includes the NDN region 111. As also indicated in Fig. 1, following no more than two amplification cycles, the first amplification primer set 110a, 110b is separated from the double-stranded DNA product. By such a step, it is believed according to non-limiting theory that contamination of the product preparation by subsequent rounds of amplification is avoided, where contaminants could otherwise be produced by amplifying newly formed double-stranded DNA molecules with amplification primers that are present in the complex reaction but which are primers other than those used to generate the double-stranded DNA in the first one or two amplification cycles. A variety of chemical and biochemical techniques are known in the art for separating double-stranded DNA from oligonucleotide amplification primers.

Once the first amplification primer set 110a, 110b is removed, by which the unique barcode tag sequences have been introduced, the tagged double-stranded DNA (dsDNA) products can be amplified using a second amplification primer set 120a, 120b as described herein and depicted in Fig. 1, to obtain a DNA library suitable for sequencing. The second amplification primer set advantageously exploits the introduction, during the preceding step, of the universal adaptor sequences 102, 104 (*e.g.,* U1 and U2 in Fig. 1) into the dsDNA products. Accordingly, because these universal adaptor sequences have been situated external to the unique barcode tags (BC1) 101 in Fig. 1, the amplification products that comprise the DNA library to be sequenced retain the unique barcode identifier sequences linked to each particular rearranged V-J gene segment combination, whilst being amenable to amplification via the universal adaptors. An exemplary set of such a second primer set, also known as "tailing" primers, is shown in Table 7.

In preferred embodiments and as also depicted in Fig. 1, the second amplification primer set 120a, 120b may introduce sequencing platform-specific oligonucleotide sequences (Adap1 105 and Adap2 106 in Fig. 1), however these are not necessary in certain other related embodiments. The second amplification primer set 120a, 120b may also optionally introduce a second oligonucleotide barcode identifier tag (BC2 107 in Fig. 1), such as a single barcode sequence that may desirably identify all products of the amplification from a particular sample (*e.g.,* as a source subject-identifying code) and ease multiplexing multiple samples to allow for higher throughput. The barcode (BC2; 107 in Fig. 1) is a modification that increases the throughput of the assay (e.g., allows samples to be multiplexed on the sequencer), but is not required. Alternatively, a universal primer without adaptors can be used to amplify the tagged molecules. After amplification, the molecules can be additionally tagged with platform specific oligonucleotide sequences. Such inclusion of a second, sample-identifying barcode, may beneficially aid in the identification of sample origins when samples from several different subjects are mixed, or in the identification of inadvertent contamination of one sample preparation with material from another sample preparation. The second amplification primer set may also, as shown in Fig. 1, optionally include a spacer nucleotide ("n6"; 108 in Fig. 1), which may facilitate the operation of the sequencing platform-specific sequences. The spacer improves the quality of the sequencing data, but is not required or present in certain embodiments. The spacer is specifically added to increase the number of random base pairs during the first 12 cycles of the sequencing step of the method. By increasing the diversity of the first 12 cycles, cluster definition and basecalling is improved. The spacer nucleotide 108 may be 0, 1, 2, 3, 4, 5, 6, 7, 8, 9, 10, 11-20, 21-30 or more nucleotides of any sequence, typically a randomly generated sequence. Where it may be of concern that the presence of such random sequences will result in uneven annealing rates amongst the oligonucleotide primers containing such sequences, it may be preferred to perform a relatively small number of amplification cycles, typically three, four or five cycles, or optionally 1-6 or no more than eight cycles, to reduce the potential for unevenness in amplification that could skew downstream results.

The resulting DNA library can then be sequenced according to standard methodologies and using available instrumentation as provided herein and known in the art. Where a second, sample-identifying barcode (BC2 107 in Fig. 1) is present, sequencing that includes reading both such barcodes is performed, with the sequence information (V-J junction including CDR3 encoding sequence, along with the first oligonucleotide barcode BC1 101 that uniquely tags each distinct sequence) between the two occurrences of the sample-identifying barcode 107 also being read. Sequencing primers may include, for instance, and with reference to Fig. 1, the universal primer 102 on the J side of NDN 111 for the first read, followed by a barcode sequence BC1 101, a J primer sequence 100 and CDR3 sequences. The second set of amplification primers include a forward primer comprising the platform-specific primer (Adap1 105) on the J side, a spacer sequence comprising random nucleotides (labeled "n6"; 108 in Fig. 1), and BC2 sample-identifying barcodes 107. The reverse primer in the second set of amplification primers includes the universal primer 104 on the V side of NDN 111, a spacer sequence 108 comprising random nucleotides, and a BC2 sample-identifying barcode sequence 107, and optionally a paired-end read using the reverse second sequencing platform-specific primer (Adap2 106). The second sequencing platform-specific primer (Adap2 106) is used to sequence and "read" the spacer sequence 108, the sample-identifying barcode sequence BC2 107, the universal adaptor sequence 104, the V sequence 103, and NDN 111. To capture the CDR3 sequence, one can use J amplification primers, C amplification primers or the V amplification primers.

Sequence data may be sorted using the BC2 sample-identifying barcodes 107 and then further sorted according to sequences that contain a common first barcode BC1 101. Within such sorted sequences, CDR3 sequences may be clustered to determine whether more than one sequence cluster is present using any of a known variety of algorithms for clustering (*e.g.,* BLASTClust, UCLUST, CD-HIT, or others, or as described in Robins et al., 2009 *Blood* 114:4099). Additionally or alternatively, sequence data may be sorted and selected on the basis of those sequences that are found at least twice. Consensus sequences may then be determined by sequence comparisons, for example, to correct for sequencing errors. Where multiple unique identifier barcode tags (BC1 101) are detected among sequences that otherwise share a common consensus sequence, the number of such barcode tags that is identified may be regarded as reflective of the number of molecules in the sample from the same T cell or B cell clone.

**Identifying Both Chains of a TCR or IG Heterodimer From a Single Adaptive Immune Cell**

As also noted above, in certain other embodiments there is provided herein a method for determining rearranged DNA sequences (or mRNA sequences transcribed therefrom or cDNA that has been reverse transcribed from such mRNA) encoding first and second polypeptide sequences of an adaptive immune receptor heterodimer in a single lymphoid cell. The method includes uniquely labeling each rearranged DNA sequence with a unique barcode sequence for identifying a particular cell and/or sample.

Briefly, and by way of illustration and not limitation, these and related embodiments comprise a method comprising steps of (1) in each of a plurality of parallel reactions, contacting first and second microdroplets and permitting them to fuse under conditions permissive for nucleic acid amplification, to generate double-stranded DNA products (or single-stranded cDNA products) that all contain an identical barcode oligonucleotide sequence and that correspond to the two chains of an adaptive immune receptor heterodimer; (2) disrupting the fused microdroplets to obtain a heterogeneous mixture of double-stranded (or single-stranded) DNA products; (3) amplifying the heterogeneous mixture of double-stranded DNA (or single-stranded) products to obtain a DNA library for sequencing; and (4) sequencing the library to obtain a data set of DNA sequences encoding the first and second polypeptides of the heterodimer.

The method comprises contacting and permitting to fuse in pairwise fashion (A) individual first microdroplets that each (or in every n$^{th}$ droplet) contain a single lymphoid cell or genomic DNA isolated therefrom, or cDNA has been reverse transcribed from mRNA, with (B) individual second microdroplets from a plurality of second liquid microdroplets that each contain two oligonucleotide amplification primer sets, the first set for amplifying any rearranged DNA that encodes the first chain of an adaptive immune receptor heterodimer (*e.g.,* an IGH chain, or a TCRA chain), and the second set for amplifying any rearranged DNA that encodes the second chain of the heterodimer (*e.g.,* an IGL chain, or a TCRB chain). Significantly, in a given second microdroplet, all oligonucleotide amplification primers will comprise the same barcode oligonucleotide, but within different second microdroplets, the primer sets will comprise different barcode sequences. The step of contacting is controlled so that in each of a plurality of events, a single first microdroplet fuses with a single second microdroplet to obtain a fused microdroplet. The contents of each of the first and second microdroplets come into contact with one another in the fused microdroplet. Oligonucleotide amplification primer sets capable of amplifying any rearranged DNA encoding a given TCR or IG polypeptide are described elsewhere herein.

Those familiar with the art will be aware of any of a number of microfluidics apparatus and devices by which microdroplet compositions that have defined contents and properties (such as the ability to controllably undergo fusion) may be prepared, such as the RainDance™ microdroplet digital PCR system (RainDance Technologies, Lexington, MA) or any of the systems described, for example, in Pekin et al., 2011 Lab Chip 11:2156; Miller et al., 2012 Proc. Nat. Acad. Sci. USA 109:378; Brouzes et al., 2009 Proc. Nat. Acad. Sci. USA 106:14195; Joensson et al., 2009 Angew. Chem. Int. Ed. 81:4813; Baret et al., 2009 Lab Chip 9:1850; Frenz et al., 2009 Lab Chip 9:1344; Kiss et al., 2008 Anal. Chem. 80:8975; Leamon et al., 2006 Nat. Meths. 3:541; which may be adapted to a particular method such as those described herein through modifications that are routine in view of the present disclosure.

As a non-limiting example, certain embodiments may exploit the properties of aqueous phase microdroplets dispersed in an oil phase using microfluidic channels. Microdroplets may be water-in-oil emulsions, oil-in-water emulsions, or similar aqueous and non-aqueous emulsion compositions. Microdroplets may also be called microdroplets or micellar microdroplets. Conventional water-in-oil (WO) emulsions have found many applications in biology, including next-generation sequencing (Margulies et al., Nature 2005, 437, 376-380), rare mutation detection (Diehl, F. et al. Proc. Natl. Acad. Sci. U.S.A. 2005, 102, 16368-16373; Li, M. et al., Nat.Methods 2006, 3, 95-97; Diehl, F. et al., Nat. Med. 2008, 14, 985-990) and quantitative detection of DNA methylation (Li, M. et al., Nat. Biotechnol. 2009, 27, 858-U118), but these emulsions suffer from droplet polydispersity and shearing stresses which can disrupt cells during mechanical agitation used to form the emulsions. The use of microfluidics overcomes these limitations and leads to an improved performance of biochemical and cell based assays (Zeng, Y. et al., Anal. Chem. 2010, 82, 3183-3190). Microfluidic chips with channel diameters of 10-100 $\mu$m are typically fabricated from quartz, silicon, glass, or polydimethylsiloxane (PDMS) using standard soft photolithography techniques (A. Manz, N. Graber and H.M. Widmer: Miniaturized total Chemical Analysis systems: A Novel Concept for Chemical Sensing, Sensors and Actuators, B Chemical (1990) 244-248). Droplets are typically generated at rates of ~ 1-10Hz by flowing an aqueous solution in one channel into a stream of oil. The use of flow focusing nozzles enables generation of controlled size droplets of aqueous phase. The droplet size and rate of droplet generation are controlled by the ratio of oil and aqueous phase flow rates, for a given nozzle geometry. The chip channel surface is usually modified to be hydrophobic, for instance, by one of the many published silanization chemistries (Zeng, Y. et al., Anal. Chem. 2010, 82, 3183-3190). For droplets to be fully functional microvessels, the use of hydrophobic and lipophobic oils may be beneficial, since the molecular diffusion between droplets is minimized, the oils have low solubility for biological reagents contained in the aqueous phase and have good gas solubility, which ensures viability of encapsulated cells in certain applications. In addition, surfactants may desirably, according to certain embodiments, be mixed into the oil phase, since droplets tend to coalesce. Surfactants may also inhibit adsorption of biomolecules at the microdroplet interfaces. A novel class of block copolymer surfactants, comprising perfluorinated polyethers (PFPE) coupled to polyethyleneglycol (PEG), has been described for use with fluorocarbon oils, for example, the fluorinated oil FC-40 (Sigma), a mix of perfluoro tri-n-butyl amine with di(perfluoro(n-butyl))perfluoromethyl amine (Holtze, C. et al., Lab Chip, 2008, DOI: 10.1039/b806706f). These compositions have led to very stable, biocompatible emulsions (Brouzes, E., et al., PNAS 2009, 106(34), 14195-14200).

Droplets traveling in microfluidic channels may be maintained as discrete microdroplets by means of their surface tension. Various methods have also been proposed to overcome the surface tension and allow droplets to merge when desired, thus allowing reagent mixing, *e.g.*, by micro fabrication of passive, flow reducing elements in channels (Niu, X. et al., Lab Chip 2008, 8, 1837-1841), by the use of electrostatic charge (electrocoalescence) (Zagnoni, M. et al., Langmuir, 2010, 26(18), 14443-14449), or by manipulating microchannel geometry (Dolomite Merger chip; *see also* WO/2012/083225). A method of adding reagents to droplets in microfluidic channels via picoinjectors (pressurized reagent filled channels, perpendicular to the droplet channel, operated by electric fields), has recently been published (Abate, A.R. et al., PNAS 2010, 107(45), 19163-19166) and may also be adapted according to certain presently contemplated embodiments as described herein.

The microdroplet contents and the step of contacting are selected to be permissive for nucleic acid amplification interactions between the genomic DNA and the amplification primers. Nucleic acid amplification (*e.g.,* PCR) reagents and conditions are well known. Such amplification is permitted to proceed at least to obtain first and second double-stranded DNA products that include the nucleotide sequences of the first and second oligonucleotide amplification primers as provided herein, and the complementary sequences thereto. Thus, for example, any single fused microdroplet may contain (i) a first double-stranded DNA product that comprises at least a first universal adaptor sequence, the barcode sequence, a V region and a J or C region sequence that encode a portion of the first adaptive immune receptor polypeptide of the heterodimer, and a second universal adaptor sequence, and (ii) a second double-stranded DNA product that comprises at least a third universal adaptor sequence, the same barcode sequence as in (i), a V region and a J or C region sequence that encode a portion of the second adaptive immune receptor polypeptide of the heterodimer, and a fourth universal adaptor sequence.

Conditions for the amplification step in the fused microdroplets are stopped prior to the next step. This can be achieved by changing the temperature of the environment in which the microdroplets are contained (e.g., in a container or well) to stop the amplification process.

In some embodiments, the method comprises disrupting the plurality of fused microdroplets to obtain a heterogeneous mixture of the first and second double-stranded products. Disruption may be selected on the basis of the chemical properties and composition of the microdroplets, and may be achieved, for instance, by chemical, biochemical and/or physical manipulations, such as the introduction of a diluent, detergent, chaotrope, surfactant, osmotic agent, or other chemical agent, or by the use of sonication, pressure, electrical field or other disruptive conditions. It will be appreciated that preferred conditions will involve the use of aqueous solvents for the included volumes within the microdroplets and/or for the heterogeneous mixture that is obtained by the step of disrupting. By using microdroplets instead of individual cells as an assay format, one can analyze data on the number of input cells in the sample. One can correct for PCR and sequencing errors, and in the case of IG molecules differentiate between non-germline sequences due to somatic hypermutation (SHM) from non-germline sequences introduced due to PCR error.

In some embodiments, the method comprises an ensuing step for contacting the mixture of first and second double-stranded DNA products with the herein described third and fourth amplification primer sets. Conditions for this step may similarly be achieved using accepted methodologies for DNA amplification to obtain a DNA library for sequencing, which may also be achieved according to any of a number of established DNA sequencing technologies. In certain related embodiments, instead of using first liquid microdroplets that each contain a single lymphoid cell or genomic DNA isolated therefrom, each of the first liquid microdroplets contains complementary DNA (cDNA) that has been reverse transcribed from the mRNA of a single lymphoid cell, such as a first cDNA that encodes the first chain of the adaptive immune receptor heterodimer and a second cDNA that encodes the second chain of the heterodimer.

In certain related embodiments, the individual second microdroplets may each contain a third oligonucleotide primer set that is capable of amplifying additional cDNA sequences that encode a lymphocyte status indicator molecule or molecules, The third primer set is labeled with the same barcode sequence that is present in the first and second primer sets that are in the microdroplet. In such embodiments, the biological status can be determined for the single source cell from which a given TCR or IG heterodimeric sequence is identified. The biological status can be activated vs. quiescent, maturational stage, naive vs. memory, regulatory vs. effector, etc. Exemplary lymphocyte status indicator molecules include, *e.g.*, lck, fyn, FoxP3, CD4, CD8, CD11a, CD18, CD25, CD28, CD29, CD44, CD45, CD49d, CD62, CD69, CD71, CD103, CD137 (4-1BB), HLA-DR, etc.

Certain embodiments include a third oligonucleotide primer set that is capable of amplifying a third cDNA sequence that encodes a lymphocyte status indicator molecule, where the third oligonucleotide primer set is labeled with the same barcode sequence that is present in the first and second primer sets, and where the lymphocyte status indicator molecule comprises one or more of the following: FoxP3, CD4, CD8, CD11a, CD18, CD21, CD25, CD29, CCD30, CD38, CD44, CD45, CD45RA, CD45RO, CD49d, CD62, CD62L, CD69, CD71, CD103, CD137 (4-1BB), CD138, CD161, CD294, CCR5, CXCR4, IgG1-4 H-chain constant region, IgA H-chain constant region, IgE H-chain constant region, IgD H-chain constant region, IgM H-chain constant region, HLA-DR, IL-2, IL-5, IL-6, IL-9, IL-10, IL-12, IL-13, IL-15, IL-21, TGF-β, TLR1, TLR2, TLR3, TLR4, TLR5, TLR6, TLR7, TLR8, TLR9 and TLR10.

*Table 1: EXEMPLARY LYMPHOCYTE STATUS INDICATORS*

| Gene Name | Status Marker for: | Human Transcript Sequence Accession # |
|---|---|---|
| FOXP3 | Treg cells | NM_014009, NM_001114377 |
| IL9 | Th9 cells | NM_ 000590 |
| CD21 | EBV receptor on B cells | NM_001006658, NM_001877 |
| CD30 | Activated T and B cells, NK cells monocytes, and Reed-Sternburg cells (Hodgkin's Lymphoma) | NM_001243, NM_152942 |
| CD38 | Plasma cells, activated B and T cells | NM_001775 |
| CD138 | Plasma cells | NM_ 001006946, NM_002997 |
| CD45RA | Naïve T cells | NM_002838, NM_080921, NM_001267798 |
| CD45RO | Memory T cells | NM_002838, NM_080921, NM_001267798 |
| CD62L | Homing of naive cells to peripheral lymph nodes | NM_ 000655 |
| CD294 | TH2 cells | NM_004778 |
| Helios | Thymic Treg cells | NM_001079526, NM_016260 |
| CD161 | NK cells | NM_002258 |
| IL2 | CD4$^+$ T cells and some CD8$^+$ T cells | NM_ 000586 |
| IL5 | TH2 cells | NM_000879 |
| IL6 | Macrophages, endothelial cells, and T cells | NM_ 000600 |
| IL10 | Macrophages and TH2 cells | NM_000572 |
| TGF-β | T cells and macrophages | NM_ 000660 |
| IL12B | Macrophages and dendritic cells | NM_002187 |
| IL12A | Macrophages and dendritic cells | NM_000882 |
| IL13 | TH2 cells | NM_002188 |
| IL15 | Macrophages | NM_0172175, NM_000585 |
| IL21 | Activated T cells (mainly TH2, TH17, and NKT cells) | NM_021803, NM_ 001207006 |
| CCR5 | T cells and macrophages | NM_000579, NM_ 001100168 |
| CXCR4 | T cells | NM_003467, NM_ 001008540 |
| IGHG1 | IgG1 heavy chain constant region | AJ294730, J00228 |
| IGHG2 | IgG2 heavy chain constant region | AJ294731, J00230 |
| IGHG3 | IgG3 heavy chain constant region | D78345 |
| IGHG4 | IgG4 heavy chain constant region | AJ294733, K01316 |
| IGHA1 | IgA1 heavy chain constant region | J00220 |
| IGHA2 | IgA2 heavy chain constant region | M60192, J00221 |
| IGHE | IgGE1 heavy chain constant region | L00022, J00222 |

(continued)

| Gene Name | Status Marker for: | Human Transcript Sequence Accession # |
|---|---|---|
| IGHD | IgD heavy chain constant region | K02875, K02876, K02877, K02878, K02879, K02880, K02881, K02992, X57331 |
| IGHM | IgM heavy chain constant region | J00260, K01310, X14939, X14940, X57331 |
| TLR1 | B cells | NM_003263 |
| TLR2 | T and B cells | NM_003264 |
| TLR3 | T cells | NM_003265 |
| TLR4 | T cells | NM_003266, NM_138554, NM_138557 |
| TLR5 | Treg and naive T cells | NM_003268 |

These and related embodiments need not be so limited, however, such that there are also contemplated embodiments according to which, additionally or alternatively, there may be included a third oligonucleotide primer set that is capable of amplifying a third cDNA sequence that encodes a lymphocyte status indicator molecule, where the third primer set is labeled with the same barcode sequence that is present in the first and second primer sets, and where the lymphocyte status indicator molecule comprises a cell surface receptor.

Examples of cell surface receptors include the following, or the like: CD2 (*e.g.,* GenBank Acc. Nos. Y00023, SEG_HUMCD2, M16336, M16445, SEG_MUSCD2, M14362), 4-1BB (CDw137, Kwon et al., 1989 Proc. Nat. Acad. Sci. USA 86:1963, 4-1BB ligand (Goodwin et al., 1993 Eur. J. Immunol. 23:2361; Melero et al., 1998 Eur. J. Immunol. 3:116), CD5 (*e.g.,* GenBank Acc. Nos. X78985, X89405), CD10 (*e.g.,* GenBank Acc. Nos. M81591, X76732) CD27 (*e.g.,* GenBank Acc. Nos. M63928, L24495, L08096), CD28 (June et al., 1990 Immunol. Today 11:211; see also, *e.g.,* GenBank Acc. Nos. J02988, SEG_HUMCD28, M34563), CD152/CTLA-4 (*e.g.,* GenBank Acc. Nos. L15006, X05719, SEG_HUMIGCTL), CD40 (*e.g.,* GenBank Acc. Nos. M83312, SEG_MUSC040A0, Y10507, X67878, X96710, U15637, L07414), interferon-γ (IFN-γ; see, *e.g.,* Farrar et al. 1993 Ann. Rev. Immunol. 11:571 and references cited therein, Gray et al. 1982 Nature 295:503, Rinderknecht et al. 1984 J. Biol. Chem. 259:6790, DeGrado et al. 1982 Nature 300:379), interleukin-4 (IL-4; see, e.g., 53rd Forum in Immunology, 1993 Research in Immunol. 144:553-643; Banchereau et al., 1994 in The Cytokine Handbook, 2nd ed., A. Thomson, ed., Academic Press, NY, p. 99; Keegan et al., 1994 J Leukocyt. Biol.. 55:272, and references cited therein), interleukin-17 (IL-17) (*e.g.,* GenBank Acc. Nos. U32659, U43088) and interleukin-17 receptor (IL-17R) (*e.g.,* GenBank Acc. Nos. U31993, U58917).

Additional cell surface receptors include the following or the like: CD59 *(e.g.,* GenBank Acc. Nos. SEG_HUMCD590, M95708, M34671), CD48 (*e.g.,* GenBank Acc. Nos. M59904), CD58/LFA-3 (*e.g.,* GenBank Acc. No. A25933, Y00636, E12817; see also JP 1997075090-A), CD72 (*e.g.,* GenBank Acc. Nos. AA311036, S40777, L35772), CD70 (*e.g.,* GenBank Acc. Nos. Y13636, S69339), CD80/B7.1 (Freeman et al., 1989 J. Immunol. 43:2714; Freeman et al., 1991 J. Exp. Med. 174:625; see also *e.g.,* GenBank Acc. Nos. U33208, 1683379), CD86/B7.2 (Freeman et al., 1993 J. Exp. Med. 178:2185, Boriello et al., 1995 J. Immunol. 155:5490; see also, *e.g.,* GenBank Acc. Nos. AF099105, SEG_MMB72G, U39466, U04343, SEG_HSB725, L25606, L25259), B7-H1/B7-DC (*e.g.,* Genbank Acc. Nos. NM_014143, AF177937, AF317088; Dong et al., 2002 Nat. Med. Jun 24 [epub ahead of print], PMID 12091876; Tseng et al., 2001 J. Exp. Med. 193:839; Tamura et al., 2001 Blood 97:1809; Dong et al., 1999 Nat. Med. 5:1365), CD40 ligand (*e.g.,* GenBank Acc. Nos. SEG_HUMCD40L, X67878, X65453, L07414), IL-17 (*e.g.,* GenBank Acc. Nos. U32659, U43088), CD43 (*e.g.,* GenBank Acc. Nos. X52075, J04536), ICOS (*e.g.,* Genbank Acc. No. AH011568), CD3 (*e.g.,* Genbank Acc. Nos. NM_000073 (gamma subunit), NM_000733 (epsilon subunit), X73617 (delta subunit)), CD4 (*e.g.,* Genbank Acc. No. NM_000616), CD25 *(e.g.,* Genbank Acc. No. NM_000417), CD8 (*e.g.,* Genbank Acc. No.M12828), CDllb (*e.g.,* Genbank Acc. No. J03925), CD14 (*e.g.,* Genbank Acc. No. XM_039364), CD56 (*e.g.,* Genbank Acc. No.U63041), CD69 (*e.g.,* Genbank Acc. No.NM_001781) and VLA-4 ($\alpha_4\beta_7$) (*e.g.,* GenBank Acc. Nos. L12002, X16983, L20788, U97031, L24913, M68892, M95632).

The following cell surface receptors are typically associated with B cells: CD19 (*e.g.,* GenBank Acc. Nos. SEG_HUMCD19W0, M84371, SEG_MUSCD19W, M62542), CD20 (*e.g.,* GenBank Acc. Nos. SEG_HUMCD20, M62541), CD22 (*e.g.,* GenBank Acc. Nos. 1680629, Y10210, X59350, U62631, X52782, L16928), CD30 (*e.g.,* Genbank Acc. Nos. M83554, D86042), CD153 (CD30 ligand, *e.g.,* GenBank Acc. Nos. L09753, M83554), CD37 (*e.g.,* GenBank Acc. Nos. SEG_MMCD37X, X14046, X53517), CD50 (ICAM-3, *e.g.,* GenBank Acc. No. NM_002162), CD106 (VCAM-1) (*e.g.,* GenBank Acc. Nos. X53051, X67783, SEG_MMVCAM1C, *see also* U.S. Patent No. 5,596,090), CD54 (ICAM-1) (*e.g.,* GenBank Acc. Nos. X84737, S82847, X06990, J03132, SEG_MUSICAM0), interleukin-12 (see, *e.g.,* Reiter et al, 1993 Crit. Rev. Immunol. 13:1, and references cited therein), CD134 (OX40, *e.g.,* GenBank Acc. No. AJ277151), CD137 (41BB, *e.g.*, GenBank Acc. No. L12964, NM_001561), CD83 (*e.g.,* GenBank Acc. Nos. AF001036, AL021918), DEC-205 (*e.g.,* GenBank Acc. Nos. AF011333, U19271).

Examples of other cell surface receptors include the following, or the like: HER1 (e.g., GenBank Accession Nos. U48722, SEG_HEGFREXS, KO3193), HER2 (Yoshino et al., 1994 J. Immunol. 152:2393; Disis et al., 1994 Canc. Res. 54:16; see also, *e.g.,* GenBank Acc. Nos. X03363, M17730, SEG_HUMHER20), HER3 (*e.g.,* GenBank Acc. Nos. U29339, M34309), HER4 (Plowman et al., 1993 Nature 366:473; see also *e.g.,* GenBank Acc. Nos. L07868, T64105), epidermal growth factor receptor (EGFR) (*e.g.,* GenBank Acc. Nos. U48722, SEG_HEGFREXS, KO3193), vascular endothelial cell growth factor (*e.g.*, GenBank No. M32977), vascular endothelial cell growth factor receptor (*e.g.,* GenBank Acc. Nos. AF022375, 1680143, U48801, X62568), insulin-like growth factor-I (*e.g.,* GenBank Acc. Nos. X00173, X56774, X56773, X06043, *see also* European Patent No. GB 2241703), insulin-like growth factor-II (*e.g.,* GenBank Acc. Nos. X03562, X00910, SEG_HUMGFIA, SEG_HUMGFI2, M17863, M17862), transferrin receptor (Trowbridge and Omary, 1981 Proc. Nat. Acad. USA 78:3039; see also *e.g.,* GenBank Acc. Nos. X01060, M11507), estrogen receptor (*e.g.,* GenBank Acc. Nos. M38651, X03635, X99101, U47678, M12674), progesterone receptor (*e.g.,* GenBank Acc. Nos. X51730, X69068, M15716), follicle stimulating hormone receptor (FSH-R) (*e.g.,* GenBank Acc. Nos. Z34260, M65085), retinoic acid receptor (*e.g.,* GenBank Acc. Nos. L12060, M60909, X77664, X57280, X07282, X06538), MUC-1 (Barnes et al., 1989 Proc. Nat. Acad. Sci. USA 86:7159; see also *e.g.,* GenBank Acc. Nos. SEG_MUSMUCIO, M65132, M64928) NY-ESO-1 (*e.g.,* GenBank Acc. Nos. AJ003149, U87459), NA 17-A (*e.g.,* European Patent No. WO 96/40039), Melan-A/MART-1 (Kawakami et al., 1994 Proc. Nat. Acad. Sci. USA 91:3515; see also *e.g.,* GenBank Acc. Nos. U06654, U06452), tyrosinase (Topalian et al., 1994 Proc. Nat. Acad. Sci. USA 91:9461; see also *e.g.,* GenBank Acc. Nos. M26729, SEG_HUMTYR0, *see also* Weber et al., J. Clin. Invest (1998) 102:1258), Gp-100 (Kawakami et al., 1994 Proc. Nat. Acad. Sci. USA 91:3515; see also *e.g.,* GenBank Acc. No. S73003, *see also* European Patent No. EP 668350; Adema et al., 1994 J. Biol. Chem. 269:20126), MAGE (van den Bruggen et al., 1991 Science 254:1643; see also *e.g,* GenBank Acc. Nos. U93163, AF064589, U66083, D32077, D32076, D32075, U10694, U10693, U10691, U10690, U10689, U10688, U10687, U10686, U10685, L18877, U10340, U10339, L18920, U03735, M77481), BAGE (*e.g.,* GenBank Acc. No. U19180, *see also* U.S. Patent Nos. 5,683,886 and 5,571,711), GAGE (*e.g.,* GenBank Acc. Nos. AF055475, AF055474, AF055473, U19147, U19146, U19145, U19144, U19143, U19142), any of the CTA class of receptors including in particular HOM-MEL-40 antigen encoded by the SSX2 gene (*e.g.,* GenBank Acc. Nos. X86175, U90842, U90841, X86174), carcinoembyonic antigen (CEA, Gold and Freedman, 1985 J. Exp. Med. 121:439; see also *e.g.*, GenBank Acc. Nos. SEG_HUMCEA, M59710, M59255, M29540), and PyLT (*e.g.,* GenBank Acc. Nos. J02289, J02038).

A lymphocyte status indicator may also include one or more apoptosis signaling polypeptides, sequences of which are known to the art, as reviewed, for example, in When Cells Die: A Comprehensive Evaluation of Apoptosis and Programmed Cell Death (R.A. Lockshin et al., Eds., 1998 John Wiley & Sons, New York; see also, *e.g.,* Green et al., 1998 Science 281:1309 and references cited therein; Ferreira et al., 2002 Clin. Canc. Res. 8:2024; Gurumurthy et al., 2001 Cancer Metastas. Rev. 20:225; Kanduc et al., 2002 Int. J. Oncol. 21:165). Typically, an apoptosis signaling polypeptide sequence comprises all or a portion of, or is derived from, a receptor death domain polypeptide, for instance, FADD (*e.g.,* Genbank Acc. Nos. U24231, U43184, AF009616, AF009617, NM_012115), TRADD (*e.g.,* Genbank Acc. No. NM_003789), RAIDD (*e.g.,* Genbank Acc. No. U87229), CD95 (FAS/Apo-1; *e.g.*, Genbank Acc. Nos. X89101, NM_003824, AF344850, AF344856), TNF-$\alpha$-receptor-1 (TNFR1, *e.g.,* Genbank Acc. Nos. S63368, AF040257), DR5 *(e.g.,* Genbank Acc. No. AF020501, AF016268, AF012535), an ITIM domain (*e.g.,* Genbank Acc. Nos. AF081675, BC015731, NM_006840, NM_006844, NM_006847, XM_017977; see, *e.g.*, Billadeau et al., 2002 J. Clin. Invest. 109:161), an ITAM domain *(e.g.,* Genbank Acc. Nos. NM_005843, NM_003473, BC030586; see, *e.g.*, Billadeau et al., 2002), or other apoptosis-associated receptor death domain polypeptides known to the art, for example, TNFR2 (*e.g.,* Genbank Acc. No. L49431, L49432), caspase/procaspase-3 (*e.g.,* Genbank Acc. No. XM_54686), caspase/procaspase-8 (*e.g.,* AF380342, NM_004208, NM_001228, NM_033355, NM_033356, NM_033357, NM_033358), caspase/procaspase-2 (*e.g.,* Genbank Acc. No. AF314174, AF314175), etc. Cells in a biological sample that are suspected of undergoing apoptosis may be examined for morphological, permeability, biochemical, molecular genetic, or other changes that will be apparent to those familiar with the art.

These and related methods for the first time permit rapid determination of the rearranged DNA sequences that encode both chains of a TCR or IG heterodimer from a single cell. Such embodiments will find uses for diagnostic and prognostic purposes, by permitting high-throughput sequencing of adaptive immune receptor encoding sequences from each of a plurality of single cells, and will also usefully inform immunological investigations into TCR or IG heterodimeric pairings and their underlying molecular mechanisms. The rapid and large-scale availability of DNA sequence information for both subunits of a large number of TCR and/or IG heterodimers will accelerate development of synthetic antibody technologies and related arts, for example, where antibodies or complete or partial TCR or IG antigen-binding regions may be usefully engineered into diagnostic, therapeutic, biomimetic, enzymatic or catalytic (*e.g.,* Abzymes) or other industrially useful compositions. By virtue of the quantitative nature of the high throughput TCR and/or IG sequencing afforded by the present disclosure, high precision in the quantitative characterization of TCR and/or IG heterodimer sequences that are present in a sample will advantageously improve the ability to determine the number of cells that belong to a specific T cell or B cell clone.

As noted above, according to these embodiments for identifying both chains of a TCR or IG heterodimer from a single adaptive immune cell, in any given second microdroplet, all oligonucleotide amplification primers will comprise the same barcode oligonucleotide, but within different second microdroplets the primer sets will comprise different barcode sequences. Accordingly, after sequencing the DNA library obtained as described above to obtain a data set of sequences, the sequences in the data set can be sorted into groups of sequences that have identical barcode sequences, and such barcode groups can be further sorted into those having X1 or X2 sequences (which include portions of V and J or C regions) that will indicate whether a given sequence reflects the amplification product of a first TCR or IG encoding chain (*e.g.,* a TCRA or IGH chain) or a second TCR or IG encoding chain (*e.g.,* a TCRB or IGL chain).

Sequences that have been so sorted by barcode and by TCR or IG chain may be further subject to cluster analysis using any of a known variety of algorithms for clustering (*e.g.,* BLASTClust, UCLUST, CD-HIT, *see also* IEEE Rev Biomed Eng. 2010;3:120-54. doi: 10.1109/RBME.2010.2083647; Clustering algorithms in biomedical research: a review, Xu R, Wunsch DC 2nd; Mol Biotechnol. 2005 Sep;31(1):55-80; Data clustering in life sciences. Zhao Y, Karypis G; Methods Mol Biol. 2010;593:81-107. doi: 10.1007/978-1-60327-194-3_5; Overview on techniques in cluster analysis. Frades I, Matthiesen R, and error correction in the case of sequences that fail to cluster with other sequences having shared barcode sequences but which instead would cluster with sequences having a barcode that differs by a single nucleotide. *See, e.g.,* Proc Natl Acad Sci USA. 2012 Jan 24;109(4):1347-52. doi: 10.1073/pnas.1118018109. Epub 2012 Jan 9. Digital RNA sequencing minimizes sequence-dependent bias and amplification noise with optimized single-molecule barcodes. Shiroguchi K, Jia TZ, Sims PA, Xie XS; Proc Natl Acad Sci USA. 2012 Sep 4;109(36):14508-13. doi: 10.1073/pnas.1208715109. Epub 2012 Aug 1. Detection of ultra-rare mutations by next-generation sequencing. Schmitt MW, Kennedy SR, Salk JJ, Fox EJ, Hiatt JB, Loeb LA.

Accordingly, certain embodiments comprise a method including steps of (a) sorting the data set of sequences (obtained as described above) according to oligonucleotide barcode sequences identified therein to obtain a plurality of barcode sequence sets each having a unique barcode; (b) sorting each barcode sequence set of (a) into an X1 sequence-containing subset and an X2 sequence-containing subset; (c) clustering members of each of the X1 and X2 sequence-containing subsets according to X1 and X2 sequences to obtain one or a plurality of X1 sequence cluster sets and one or a plurality of X2 sequence cluster sets, respectively, and error-correcting single nucleotide barcode sequence mismatches within any one or more of said X1 and X2 sequence cluster sets; and (d) identifying as originating from the same cell sequences that are members of an X1 and an X2 sequence cluster set that belong to the same one or more barcode sequence sets.

It will be appreciated that according to non-limiting theory, first and second adaptive immune receptor chain encoding sequences that occur with the same set of barcode sequences have an extremely high probability of having originated from the same fused microdroplet, and thus from the same source cell. For example, where $10^4$ different barcodes are used in the construction of the first and second oligonucleotide amplification primers, the probability that two independent (*i.e.,* originating from different cells) double-stranded first and second products would be obtained having the same barcode sequence is one in $10^8$. Hence, if according to the methods described herein, three or more copies of a given set of first and second adaptive immune receptor polypeptide encoding sequences (*e.g.,* X1 and X2) share common barcode sequences (*e.g.,* belong to the same barcode sequence set), the probability that the sequences are of independent cellular origin approaches zero.

Similarly, it will be appreciated that analysis of the data set of sequences obtained according to the present methods may also be used to characterize the biological status of the lymphoid cell source of genomic DNA. For example, because in B cells IGH gene rearrangement is known to precede IGL gene rearrangement, barcode sequence analysis as described herein may reveal multiple single lymphoid cell genomes having the same rearranged IGH sequence but different IGL sequences, indicating origins of these sequences in immunologically naive cells.

Alternatively, the analysis may exploit the observation that T cells express proteins that are specific to their functions, such as lymphocyte status indicator molecules as described herein. For example, regulatory T cells express the protein FOXP3. If a cDNA that has been reverse transcribed from T cell mRNA is subsequently amplified, co-amplification products may include cDNA species that reflect other mRNAs encoding phenotypic specific proteins such as FOXP3, along with cDNAs encoding the TCRB and TCRA molecules. This approach may permit identification of the adaptive immune receptors that are expressed by T cells having specific phenotypes, such as T regulatory cells or effector T cells.

Thus, there is provided herein a method for determining rearranged DNA sequences encoding first and second polypeptide sequences of an adaptive immune receptor heterodimer in a single lymphoid cell, comprising (1) contacting (A) individual first microdroplets that each contain a single lymphoid cell or genomic DNA isolated therefrom, with (B) individual second microdroplets from a plurality of second liquid microdroplets that each contain (i) a first oligonucleotide amplification primer set that is capable of amplifying a rearranged DNA sequence encoding a first complementarity determining region-3 (CDR3) of a first polypeptide of an adaptive immune receptor heterodimer, and (ii) a second oligonucleotide amplification primer set that is capable of amplifying a rearranged DNA sequence encoding a second complementarity determining region-3 (CDR3) of a second polypeptide of the adaptive immune receptor heterodimer. The first oligonucleotide amplification primer set comprises a composition comprising a plurality of oligonucleotides having a plurality of oligonucleotide sequences of general formula: U1/2-B1-X1, in which U1/2 comprises an oligonucleotide which comprises a first universal adaptor oligonucleotide sequence when B1 is present or a second universal adaptor oligonucleotide sequence when B1 is nothing. In some embodiments, B1 comprises an oligonucleotide that comprises either nothing or a first oligonucleotide barcode sequence of 6, 7, 8, 9, 10, 11, 12, 13, 14, 15, 16, 17, 18, 19 or 20 contiguous nucleotides, and X1 comprises an oligonucleotide that is one of: (a) a polynucleotide comprising at least 20, 30, 40 or 50 and not more than 100, 90, 80, 70 or 60 contiguous nucleotides of an adaptive immune receptor variable (V) region encoding gene sequence for said first polypeptide of an adaptive immune receptor heterodimer, or the complement thereof, and in each of the plurality of oligonucleotide sequences of general formula U1/2-B1-X1, X1 comprises a unique oligonucleotide sequence, and (b) a polynucleotide comprising at least 15-30 or 31-50 and not more than 80, 70, 60 or 55 contiguous nucleotides of either (i) an adaptive immune receptor joining (J) region encoding gene sequence for said first polypeptide of an adaptive immune receptor heterodimer, or the complement thereof, or (ii) an adaptive immune receptor constant (C) region encoding gene sequence for said first polypeptide of an adaptive immune receptor heterodimer, or the complement thereof, and in each of the plurality of oligonucleotide sequences of general formula U1/2-B1-X1, X1 comprises a unique oligonucleotide sequence. The second oligonucleotide amplification primer set can comprise a composition comprising a plurality of oligonucleotides having a plurality of oligonucleotide sequences of general formula: U3/4-B2-X2 in which U3/4 comprises an oligonucleotide which comprises a third universal adaptor oligonucleotide sequence when B2 is present or a fourth universal adaptor oligonucleotide sequence when B2 is nothing, B2 comprises an oligonucleotide that comprises either nothing or a second oligonucleotide barcode sequence of 6, 7, 8, 9, 10, 11, 12, 13, 14, 15, 16, 17, 18, 19 or 20 contiguous nucleotides that is from the same as B1, and X2 comprises an oligonucleotide that is one of: (a) a polynucleotide comprising at least 20, 30, 40 or 50 and not more than 100, 90, 80, 70 or 60 contiguous nucleotides of an adaptive immune receptor variable (V) region encoding gene sequence for said second polypeptide of an adaptive immune receptor heterodimer, or the complement thereof, and in each of the plurality of oligonucleotide sequences of general formula U3/4-B2-X2, X2 comprises a unique oligonucleotide sequence, and (b) a polynucleotide comprising at least 15-30 or 31-50 and not more than 80, 70, 60 or 55 contiguous nucleotides of either (i) an adaptive immune receptor joining (J) region encoding gene sequence for said second polypeptide of an adaptive immune receptor heterodimer, or the complement thereof, or (ii) an adaptive immune receptor constant (C) region encoding gene sequence for said second polypeptide of an adaptive immune receptor heterodimer, or the complement thereof, and in each of the plurality of oligonucleotide sequences of general formula U3/4-B2-X2, X2 comprises a unique oligonucleotide sequence. The step of contacting can take place under conditions and for a time sufficient for a plurality of fusion events between one of the first microdroplets and one of the second microdroplets to produce a plurality of fused microdroplets in which nucleic acid amplification interactions occur between the genomic DNA and the first and second oligonucleotide amplification primer sets, to obtain in each of one or more of said plurality of fused microdroplets: a first double-stranded DNA product that comprises at least one first universal adaptor oligonucleotide sequence, at least one first oligonucleotide barcode sequence, at least one X1 oligonucleotide V region encoding gene sequence of said first polypeptide of the adaptive immune receptor heterodimer, at least one second universal adaptor oligonucleotide sequence, and at least one X1 oligonucleotide J region or C region encoding gene sequence of said first polypeptide of the adaptive immune receptor heterodimer. The conditions also permit obtaining in each of one or more of said plurality of fused microdroplets: a second double-stranded DNA product that comprises at least one third universal adaptor oligonucleotide sequence, at least one second oligonucleotide barcode sequence, at least one X2 oligonucleotide V region encoding gene sequence of said second polypeptide of the adaptive immune receptor heterodimer, at least one fourth universal adaptor oligonucleotide sequence, and at least one X2 oligonucleotide J region or C region encoding gene sequence of said second polypeptide of the adaptive immune receptor heterodimer.

The method also includes disrupting the plurality of fused microdroplets to obtain a heterogeneous mixture of said first and second double-stranded DNA products and contacting the mixture of first and second double-stranded DNA products with a third amplification primer set and a fourth amplification primer set. In some embodiments, the third amplification primer set comprises (i) a plurality of first sequencing platform tag-containing oligonucleotides that each comprise an oligonucleotide sequence that is capable of specifically hybridizing to the first universal adaptor oligonucleotide and a first sequencing platform-specific oligonucleotide sequence that is linked to and positioned 5' to the first universal adaptor oligonucleotide sequence, and (ii) a plurality of second sequencing platform tag-containing oligonucleotides that each comprise an oligonucleotide sequence that is capable of specifically hybridizing to the second universal adaptor oligonucleotide sequence and a second sequencing platform-specific oligonucleotide sequence that is linked to and positioned 5' to the second universal adaptor oligonucleotide sequence. In other embodiments, the fourth amplification primer set comprises (i) a plurality of third sequencing platform tag-containing oligonucleotides that each comprise an oligonucleotide sequence that is capable of specifically hybridizing to the third universal adaptor oligonucleotide and a third sequencing platform-specific oligonucleotide sequence that is linked to and positioned 5' to the third universal adaptor oligonucleotide sequence, and (ii) a plurality of fourth sequencing platform tag-containing oligonucleotides that each comprise an oligonucleotide sequence that is capable of specifically hybridizing to the fourth universal adaptor oligonucleotide sequence and a fourth sequencing platform-specific oligonucleotide sequence that is linked to and positioned 5' to the fourth universal adaptor oligonucleotide sequence. The contacting step can take place under conditions and for a time sufficient to amplify both strands of the first and second double-stranded DNA products of (2), to obtain a DNA library for sequencing. The method also includes sequencing the DNA library obtained in (3) to obtain a data set of sequences encoding the first and second polypeptide sequences of the adaptive immune receptor heterodimer.

FIG. 2 illustrates one method by which a plurality of first microdroplets 210 that contain a single lymphoid cell or genomic DNA fuse with a plurality of individual second microdroplets 220 to form a plurality of fused microdroplets 230. The second plurality of droplets may comprise amplification primer sets, as described herein, and the fused droplets can be placed under conditions where the amplification primers can amplify the DNA found in the single lymphoid cell or the genomic DNA (or cDNA) within the microdroplet.

These and related embodiments permit high throughput sequencing of rearranged genes encoding both chains from the same cell of an adaptive immune receptor heterodimer, such as IGH plus IGL, or IGH plus IGK, or TCRA plus TCRB, or TCRG plus TCRD. Advantageously, this approach also permits quantifying the number of cells having a given TCR or IG. A schematic depiction of an exemplary embodiment is shown in FIG. 3, according to which steps highly similar to those described above are carried out, significantly, however, with the step of contacting DNA from a single lymphoid cell with first and second amplification primer sets as described herein to effect the first amplification reaction by which the unique molecular-tagging barcode is incorporated taking place within a single microdroplet, such as those that are formed from emulsions for use in the RainDance™ microdroplet digital PCR system (RainDance Technologies, Lexington, MA) (e.g., Pekin et al., 2011 Lab. Chip 11(13):2156; Zhong et al., 2011 Lab. Chip 11(13):2167; Tewhey et al., 2009 Nature Biotechnol. 27:1025; 2010 Nature Biotechnol. 28:178) or other comparable systems, any of which may be adapted by the skilled person for use with the herein described compositions and methods. Subsequent to the incorporation into a plurality of distinct dsDNA products of the plurality of unique molecular-tagging barcodes, the microdroplets may be disrupted and the ensuing steps that include amplifying and introducing sequencing platform-specific oligonucleotides may be carried out as described herein and shown in FIG. 3.

In these and related embodiments, a single tagging barcode (BC1) may be shared by all J primers (or in certain embodiments by all V primers) and it may be desirable to produce such primers with a finite set of specific and pre-identified barcode sequences. Only a single tagging barcode sequence (BC1) will be present within any given microdroplet during the first step, however. Hence, even after a large and diverse set of sequence information is obtained following the sequencing step when practiced starting with a sample that comprises a plurality of heterogeneous lymphoid cells as provided herein, analysis of such information may include determination of first and second TCR or Ig heterodimeric polypeptide chain encoding sequences that contain the same tagging barcode (BC1), from which a probabilistic basis would indicate an extremely high likelihood that both chains are the products of the same cell. Accordingly, the present disclosure for the first time provides compositions and methods for determining and quantifying the relative representation in a sample of both chains of a TCR or Ig heterodimer that are expressed in the same cell.

### Clonal Heterodimer Sequence Determination Without MicroDroplets

According to certain other embodiments, determination of rearranged DNA sequences encoding first and second adaptive immune receptor heterodimer polypeptide sequences in a single cell may be achieved without first preparing separate populations of first and second microdroplets that contain, respectively, single lymphoid cell genomic DNA (or cDNA that has been reverse transcribed from mRNA therefrom) and oligonucleotide amplification primer sets.

Instead, these alternative embodiments contemplate separating the cells of a lymphoid cell-containing cell suspension (e.g., a blood cell preparation from a subject or a cell subpopulation thereof) into subpopulations by distributing the cells to a plurality of containers, such as multiple wells of a multi-well cell culture plate or assay plate (e.g., 96-, 384- or 1536-well formats). Persons familiar with the art will be aware of a number of devices and methodologies for distributing a cell suspension into such multiple containers, for instance, using fluorescence activated cell sorting (FACS) or with automated low-volume dispensing equipment or by limiting dilution, to obtain a desired number of cells per well, container, tube, compartment or the like. In certain embodiments it may be preferred to distribute substantially the same number of cells to each container, although certain other contemplated embodiments need not be so limited.

Briefly, according to these and related embodiments, separated lymphoid cell subpopulations may provide mRNA molecules that are used as templates for reverse transcription to produce cDNA molecules that are concomitantly labeled during the reverse transcription (RT) step (see Figures 4 and 5). FIG. 4 depicts a schematic representation of labeling adaptive immune receptor polypeptide encoding cDNA during reverse transcription by using an oligonucleotide reverse transcription primer that directs incorporation of oligonucleotide barcode and universal adaptor oligonucleotide sequences into cDNA. The cDNA strand is amplified with primers comprising a pGEX-Rev sequence, a barcode BC and N6 spacer sequence (BC-N6) and a "Cn-RC" sequence. The 3' end of the amplified cDNA strand includes a pGEX-FRC sequence, a barcode BC-N6 spacer sequence, and a "Smarter UAII" sequence. The wells or containers of amplified cDNA are pooled, and SPRI bead purification is performed of the first cDNA strand pool. PCR amplification is performed using a tailing-pGEX F/R sequence. The amplicons are purified and selected based on size. The resulting cDNA amplicon is shown in FIG. 4.

FIG. 5 depicts a schematic representation of labeling adaptive immune receptor polypeptide encoding cDNA during reverse transcription by using an oligonucleotide reverse transcription primer that directs incorporation of oligonucleotide barcode and universal adaptor oligonucleotide sequences into cDNA. Figure 6 presents a schematic representation of a DNA product that is amenable to sequencing following modification with Illumina sequencing adapters of amplified adaptive immune receptor polypeptide encoding cDNA that has been labeled during reverse transcription by using an oligonucleotide reverse transcription primer that directs incorporation of oligonucleotide barcode and universal adaptor oligonucleotide sequences.

As provided herein, oligonucleotide RT primers in such embodiments include oligonucleotide sequences that specifically hybridize to target adaptive immune receptor encoding regions such as V, J or C region sequences, and also include oligonucleotide barcode sequences as molecular labels, along with universal adaptor oligonucleotide sequences as described herein. The process of reverse transcription from adaptive immune receptor encoding mRNA may thus be accompanied by incorporation into cDNA products of (i) oligonucleotide barcode sequences as source identifiers, and (ii) universal adaptors to facilitate automated high throughput sequencing as described herein. By way of illustration and not limitation, in certain of these embodiments all RT primers in the oligonucleotide RT primer sets that are contacted with the contents of a single particular container (e.g., one well of a multi-well plate) share a common barcode oligonucleotide sequence (B), and a different barcode oligonucleotide sequence (B) is present in each separate container (such as each well of a multi-well plate).

For instance, a cell suspension (e.g., blood cells or a fraction thereof, such as nucleated cells, lymphoid cells, etc.) may be divided by random distribution among different wells of a multi-well plate to physically separate the cells into subsets. The subset of cells in each well may then be lysed or otherwise processed according to any of a number of conventional procedures to liberate mRNA present within the cells, which may include mRNA encoding both chains of TCR (e.g., TCRA and TCRB, or TCRG and TCRG) or IG (e.g., IGH and IGL) heterodimers expressed by the cells, and which may also include mRNA encoding one or more lymphocyte status indicator molecules.

The mRNA may then be used as a template for cDNA synthesis by modification of established reverse transcription (RT) protocols, using oligonucleotide reverse transcription primer sets as described herein that are capable of introducing into the cDNA products, in each separate well, a unique oligonucleotide barcode sequence that is linked to the TCR or IG encoding sequence or complement thereof (see, e.g., Figs. 4-5). External to the barcode (e.g., distal from the TCR or IG encoding sequence, relative to the barcode), the oligonucleotide reverse transcription primer sets may also be designed to introduce a universal adaptor oligonucleotide sequence as described herein and/or other known oligonucleotide sequence features such as those that may facilitate downstream amplification, processing and/or other manipulation steps such as those that will be compatible with automated high throughput quantitative sequencing.

Following DNA amplification of the reverse transcription cDNA products, each amplified DNA molecule within a given well of the multi-well plate will have the same oligonucleotide barcode sequence, while the barcode sequences of the amplification products in each different well will be distinct from one another. In this manner within each well, all DNA molecules that encode either chain of an adaptive immune receptor heterodimer (e.g., IGH and IGL, TCRA and TCRB, TCRG and TCRD) will have the same oligonucleotide barcode sequence.

The amplification products may be pooled and quantitatively sequenced using automated high throughput DNA sequencing as described elsewhere herein to obtain a data set of sequences, which include TCR and/or IG sequences along with associated oligonucleotide barcode sequences. As disclosed herein, in certain preferred embodiments the data set of sequences may be analyzed by a combinatorics approach, which permits matching particular pairs of adaptive immune receptor heterodimer subunit encoding sequences to identify them as having originated from the same lymphoid cell.

As a non-limiting illustrative example, a hypothetical data set of sequences may be obtained from a set of 100 wells into which a lymphoid cell suspension is distributed. In each well, the cells' mRNA cDNA is reverse transcribed using first and second oligonucleotide reverse transcription primer sets that are specific, respectively, for portions of TCRA and TCRB encoding sequences. The oligonucleotide reverse transcription primer sets also introduce a different oligonucleotide barcode sequence into the cDNA products in each distinct well. If, hypothetically, T cells having a single, common clonal origin (e.g., T cells that express the identical TCRA/B sequences) are randomly distributed into five different wells of the 100 wells, then the sequence data set will include five separate instances in which the unique pair of TCRA and TCRB sequences occurs in DNA amplification products that share an identical barcode sequence. In other words, in each of the five separate wells, the oligonucleotide reverse transcription primer set promotes the generation of cDNAs having identical rearranged TCRA and TCRB sequences, but the cDNA products of each well include a distinct, well-specific barcode sequence. According to non-limiting theory, on a probabilistic basis the likelihood would be extremely high that the unique TCRA/TCRB sequence pair originates in the same T cell clone, members of which would have been randomly distributed into the five different wells.

According to certain embodiments, a more detailed description of this high throughput method for determining rearranged DNA sequences encoding first and second polypeptide sequences of an adaptive immune receptor heterodimer in a single lymphoid cell is as follows:

Lymphoid cells are isolated from an anti-coagulated whole blood sample using either density gradient centrifugation (*e.g.,* FicollPaque®, GE Healthcare Bio-Sciences, Piscataway, NJ), or by binding to antibody-coated magnetic beads, such as CD45 beads from Miltenyi Biotec (Auburn, CA). Alternatively, T lymphocytes may be purified from a whole blood sample by binding to CD3+ magnetic beads, and B lymphocytes may be purified from a whole blood sample by binding to CD19+ magnetic beads. Isolated cell populations may then be checked for viability. Dead cells may be removed from the sample with a filter, for example, using a Miltenyi Biotec Dead Cell Removal kit. Depending on the application, isolated viable lymphoid cells (*e.g.,* as may be present in unsorted peripheral blood mononuclear cells (PBMC), or as preparations of specific cell sub-sets) may be cultured in short-tem cell culture, and in certain embodiments cells may be activated by any of a number of known activation paradigms, such as by exposure to one or more of cytokines, chemokines, specific antibodies, mitogens, polyclonal activators, etc. The final cell sample may be prepared by resuspending the cells in culture media (*e.g.,* RPMI with 10% fetal bovine serum) or appropriate isotonic buffered solutions (*e.g.,* phosphate buffered saline, PBS), supplemented with agents which prevent cell clumping (*e.g.,* 0.1% BSA, 1% Pluronic® F-68). Alternatively, whole blood or PBMCs may be utilized without sorting. As the most general case, any set of cells present as a suspension in an aqueous solution that contains B or T cells may be used.

The cell preparation comprising a plurality of lymphoid cells is divided into a plurality of physically separated subsets, for example, by distributing the suspension of cells amongst a plurality of containers or compartments that are capable of containing the cells to obtain a plurality of containers or compartments that each contain a subpopulation of the lymphoid cells, wherein each subpopulation comprises one lymphoid cell or a plurality of lymphoid cells, and wherein each container or compartment is physically separate so that the contents are not in fluid communication with one another. Preferably the cells are distributed or divided into the plurality of containers so that each container contains a substantially equivalent number of cells, which may result in there being the same number of cells in each container, or in there being in each container a number of cells that is within 1, 2, 3, 4, 5, 6, 7, 8, 9, 10, 11, 12, 13, 14, 15, 16, 17, 18, 19, 20, 21-30, 31-50, 51-70, 71-80, or 81-100 percent of the number of cells in any other container. Exemplary containers may be wells of multi-well culture or assay plates such as 6-, 12-, 24-, 48-, 96-, 384- or 1536-well multi-well plates or any other multi-well plate format; arrays of tubes, filters, microfabricated well arrays, laser-generated matrices or any other suitable containers that are capable of containing the cells are also contemplated. In certain exemplary embodiments, cells may be distributed amongst the plurality of containers by fluorescence activated cell sorting (FACS): A predetermined number of cells may be isolated, sorted, and deposited into a multi-well (*e.g.,* 96, 384 or 1536) reaction plate using FACS. Any of a number of methodologies and instrumentation may be employed using flow cytometers that are capable of preparative sorting of cells onto multi-well plates (*e.g.,* Beckton Dickinson FACSAria® III, Beckman MoFlo™ XDP, etc.). FACS allows for specific subsets of cells to be isolated by antibody staining, viability staining or multicolor combination of specific cell staining reagents. Cell sorters may be employed to count target cells and deposit specified numbers of cells into each well of a collection multi-well plate (10-20%CV). Alternatively, automated low volume (nl to µl volumes per well) dispensers, capable of preferably non-contact dispensing of uniform cell suspensions onto high density micro-well plates (384, 1536, 3456 wells), such as Beckman Coulter BioRAPTR FRD™, LambdaJet™ IIIMT (Thermo Fisher Scientific), CyBi™ Drop (Jena Analytik), Furukawa Perflow™, or similar instruments, may be used to deposit specified numbers of cells into each well of a collection multi-well plate with high precision and reproducibility (10-20%CV).

The adaptive immune receptor encoding polynucleotide sequences are then amplified from each well, with a unique, well-specific, barcode oligonucleotide attached to all samples. One way to do this is to convert cellular mRNA to cDNA by reverse transcription, and to add to the cDNA products a molecular label in the form of an oligonucleotide barcode during the reverse transcription step. The same barcode may be added to cDNAs that are complementary to mRNAs encoding both chains of each heterodimeric adaptive immune receptor molecule within the well, for instance, the immunoglobulin heavy and light chains, the TCRA and TCRB chains, and the TCRG and TCRD chains. In this and related embodiments, antigen receptor encoding sequences are amplified from cDNA made by reverse transcription from mRNA; genomic DNA (gDNA) is not amplified. To do this, each well of a microwell plate may contain a medium containing an RNase inhibitor, and a medium designed either to protect RNA in cells (such as Qiagen RNAlater™, Qiagen, Valencia, CA), or to lyse cells and isolate RNA (Trizol, guanidium isothiocyanate - Qiagen RNeasy™ etc.). Extracted total cellular RNA may then be transferred into another multi-well plate for the reverse transcription reaction using robotic liquid handlers. Alternatively, sorted cells may be lysed directly in a reverse-transcription reaction mix containing an RNase inhibitor. Reverse transcription reaction (RT) may be initiated by exposing cellular RNA to a reaction mix containing an appropriate buffer, dNTPs, an enzyme (reverse transcriptase) and a set of oligonucleotide reverse transcription primers. These primers will generally comprise a multiplicity of subsets of primers that may anneal to IgG, IgM, IgA, IgD, IgE, Ig kappa, Ig lambda, TCR alpha, beta, gamma and delta constant region (C-segment) gene-specific oligonucleotide sequences, as well as a universal template switching oligonucleotide (*e.g.,* Clontech Smarter™ UAII oligonucleotide, Clontech, Mountain View, CA). For instance, either the C-segment gene specific primers, or the Smarter™ UAII oligonucleotide, or both, will be uniquely tagged with a DNA barcode, which will be a unique sequence 6, 7, 8, 9, 10, 11, 12, 13, 14, 15, ... etc. base pairs long. Each well of the RT reaction plate will contain the same multiplicity of primers, where each primer in the mix will be tagged with the same DNA barcode, but a different barcode will be used in each well. Thus, upon completion of the reverse transcription reaction, each first strand cDNA molecule in a given well will be barcoded with an identical DNA barcode sequence.

***List of BCR/ TCR C-segment primers for 1st cDNA strand synthesis:***

| Name | Sequence | SEQ ID NO: |
|------|----------|------------|
| Ck | GATGAAGACAGATGGTGCAGC | 5579 |
| Cl-1 | GGCGGGAACAGAGTGAC | 5580 |
| Cl-2 | AGGGTGGGAACAGAGTGAC | 5581 |
| Cl-3 | GCTTGAAGCTCCTCAGAGG | 5582 |
| Cl-4 | GGCGGGAACAGAGTGAC | 5583 |
| IgA | AGGCTCAGCGGGAAGAC | 5584 |
| IgD | GAACACATCCGGAGCCTTG | 5585 |
| IgE | GGTGGCATTGGAGGGAATG | 5586 |
| IgG-1 | AAGACCGATGGGCCCTTG | 5587 |
| IgG-2 | CTCTCGGAGGTGCTCCTG | 5588 |
| IgM | AATTCTCACAGGAGACGAGGG | 5589 |
| TCRa | TGGTACACGGCAGGGTC | 5590 |
| TCRA_RACE_ JB2 | AGTCTCTCAGCTGGT ACACGGCAGGGTC | 5591 |
| | 5'-AGTCTCTCAGCTGGTACACGGCAGGGTC-3' | 5591 |
| TCRA_50 | 5'- ACA GAC TTG TCA CTG GAT TTA GAG TCT CTC AGC TGG TAC ACG GCA GGG TC -3' | 5592 |
| TCRB_50 | 5'- GAG ATC TCT GCT TCT GAT GGC TCA AAC ACA GCG ACC TCG GGT GGG AAC AC -3' | 5593 |
| TCRb-1 | CAAACACAGCGACCTCGG | 5594 |
| TCRb-2 | ATGGCTCAAACACAGCGAC | 5595 |
| TRCd-1 | GATGGTTTGGTATGAGGCTGAC | 5596 |
| TCRd-2 | CCTTCACCAGACAAGCGAC | 5597 |
| TCRg-1 | GAAAAATAGTGGGCTTGGGGG | 5598 |
| | Primers from Bolotin et al., Eur. J. Immunol. 2012 | |
| TCRb_BC1R | CAGTATCTGGAGTCATTGA | 5599 |
| TCRb_BC2R | TGCTTCTGATGGCTCAAACAC | 5600 |
| | Primers from Glanville et al., PNAS 2011 | |
| IgM_RACE | 5'-GATGGAGTCGGGAAGGAAGTCCTGTGCGAG-3' | 5601 |
| IgG_RACE | 5'-GGGAAGACSGATGGGCCCTTGGTGG-3' | 5602 |

(continued)

| Name | Sequence | SEQ ID NO: |
|---|---|---|
| IgA_RACE | 5'-CAGGCAKGCGAYGACCACGTTCCCATC-3' | 5603 |
| Igκ_RACE | 5'-CAT C AG A TGGCGGGAAGA TGAAGACAGA TGGTGC-3' | 5604 |
| Igλ_RACE | 5'-CCTCAGAGGAGGGTGGGAACAGAGTGAC-3' | 5605 |
| TCRB_RACE | 5'-GCTCAAACACAGCGACCTCGGGTGGGAACAC-3' | 5606 |
| | Clontech Smarter primers | |
| Smarter UAII | 5'-AAGCAGTGGTATCAACGCAGAGTACrGrGrGrGrG-P-3 | 5607 |
| Islam UAII | 5'–AAGCAGTGGTATCAACGCAGAGTGCAGUGCUXXXXXXXrGrGrG–3' | 5608 |
| Smarter CDS | 5'-Bio-AAGCAGTGGTATCAACGCAGAGTACT(30)N-1N-3' | 5609 |
| Smarter IS PCR | 5'-Bio-AAGCAGTGGTATCAACGCAGAGT-3' | 5610 |
| 5'RACE long | 5'-CTAATACGACTCACTATAGGGCAAGCAGTGGTATCAACGCAGAGT-3' | 5611 |
| 5'RACE short | 5'-CTAATACGACTCACTATAGGGC-3' | 5612 |

Accordingly, following the step of distributing cells to a plurality of containers, each of the containers is contacted, under conditions and for a time sufficient to promote reverse transcription of mRNA in the lymphoid cells in the plurality of containers, with a first and a second oligonucleotide reverse transcription primer set, wherein (A) the first oligonucleotide reverse transcription primer set is capable of reverse transcribing a plurality of first mRNA sequences encoding a plurality of first polypeptides of an adaptive immune receptor heterodimer, and (B) the second oligonucleotide reverse transcription primer set is capable of reverse transcribing a plurality of second mRNA sequences encoding a plurality of second polypeptides of the adaptive immune receptor heterodimer, and wherein: (I) the first oligonucleotide reverse transcription primer set comprises a composition comprising a plurality of oligonucleotides having a plurality of oligonucleotide sequences of general formula:

**U1/2-B1-X1**

in which U1/2 comprises an oligonucleotide which comprises a first universal adaptor oligonucleotide sequence when B1 is present or a second universal adaptor oligonucleotide sequence when B1 is nothing, B1 comprises an oligonucleotide that comprises either nothing or a first oligonucleotide barcode sequence of 6, 7, 8, 9, 10, 11, 12, 13, 14, 15, 16, 17, 18, 19 or 20 contiguous nucleotides, and X1 comprises an oligonucleotide that is one of: (a) a polynucleotide comprising at least 20, 30, 40 or 50 and not more than 100, 90, 80, 70 or 60 contiguous nucleotides of an adaptive immune receptor variable (V) region encoding gene sequence for said first polypeptide of an adaptive immune receptor heterodimer, or the complement thereof, and in each of the plurality of oligonucleotide sequences of general formula U1/2-B1-X1, X1 comprises a unique oligonucleotide sequence, and (b) a polynucleotide comprising at least 15-30 or 31-50 and not more than 80, 70, 60 or 55 contiguous nucleotides of either (i) an adaptive immune receptor joining (J) region encoding gene sequence for said first polypeptide of an adaptive immune receptor heterodimer, or the complement thereof, or (ii) an adaptive immune receptor constant (C) region encoding gene sequence for said first polypeptide of an adaptive immune receptor heterodimer, or the complement thereof, and in each of the plurality of oligonucleotide sequences of general formula U1/2-B1-X1, X1 comprises a unique oligonucleotide sequence, and (II) the second oligonucleotide reverse transcription primer set comprises a composition comprising a plurality of oligonucleotides having a plurality of oligonucleotide sequences of general formula:

**U3/4-B2-X2**

in which U3/4 comprises an oligonucleotide which comprises a third universal adaptor oligonucleotide sequence when B2 is present or a fourth universal adaptor oligonucleotide sequence when B2 is nothing, B2 comprises an oligonucleotide that comprises either nothing or a second oligonucleotide barcode sequence of 6, 7, 8, 9, 10, 11, 12, 13, 14, 15, 16, 17, 18, 19 or 20 contiguous nucleotides that is, for each of the first and second reverse transcription primer sets that are contacted with a single one of the plurality of containers, the same as B1, and X2 comprises an oligonucleotide that is one of: (a) a polynucleotide comprising at least 20, 30, 40 or 50 and not more than 100, 90, 80, 70 or 60 contiguous nucleotides of an adaptive immune receptor variable (V) region encoding gene sequence for said second polypeptide of an adaptive immune receptor heterodimer, or the complement thereof, and in each of the plurality of oligonucleotide sequences of general formula U3/4-B2-X2, X2 comprises a unique oligonucleotide sequence, and (b) a polynucleotide comprising at least 15-30 or 31-50 and not more than 80, 70, 60 or 55 contiguous nucleotides of either (i) an adaptive immune receptor joining (J) region encoding gene sequence for said second polypeptide of an adaptive immune receptor heterodimer, or the complement thereof, or (ii) an adaptive immune receptor constant (C) region encoding gene sequence for said second polypeptide of an adaptive immune receptor heterodimer, or the complement thereof, and in each of the plurality of oligonucleotide sequences of general formula U3/4-B2-X2, X2 comprises a unique oligonucleotide sequence, said step of contacting taking place under conditions and for a time sufficient to obtain in each of one or more of said plurality of containers: a first reverse-transcribed complementary DNA (cDNA) product that comprises at least one first universal adaptor oligonucleotide sequence, at least one first oligonucleotide barcode sequence, at least one X1 oligonucleotide V region encoding gene sequence of said first polypeptide of the adaptive immune receptor heterodimer, at least one second universal adaptor oligonucleotide sequence, and at least one X1 oligonucleotide J region or C region encoding gene sequence of said first polypeptide of the adaptive immune receptor heterodimer, and also to obtain in each of one or more of said plurality of containers: a second reverse-transcribed cDNA product that comprises at least one third universal adaptor oligonucleotide sequence, at least one second oligonucleotide barcode sequence, at least one X2 oligonucleotide V region encoding gene sequence of said second polypeptide of the adaptive immune receptor heterodimer, at least one fourth universal adaptor oligonucleotide sequence, and at least one X2 oligonucleotide J region or C region encoding gene sequence of said second polypeptide of the adaptive immune receptor heterodimer.

After the step of contacting, there is performed a step of combining the first and second reverse-transcribed cDNA products from the plurality of containers to obtain a mixture of reverse-transcribed cDNA products.

The combining step is followed by contacting the mixture of first and second reverse-transcribed cDNA products with a first oligonucleotide amplification primer set and a second oligonucleotide amplification primer set, wherein the first amplification primer set comprises (i) a plurality of first sequencing platform tag-containing oligonucleotides that each comprise an oligonucleotide sequence that is capable of specifically hybridizing to the first universal adaptor oligonucleotide and a first sequencing platform-specific oligonucleotide sequence that is linked to and positioned 5' to the first universal adaptor oligonucleotide sequence, and (ii) a plurality of second sequencing platform tag-containing oligonucleotides that each comprise an oligonucleotide sequence that is capable of specifically hybridizing to the second universal adaptor oligonucleotide sequence and a second sequencing platform-specific oligonucleotide sequence that is linked to and positioned 5' to the second universal adaptor oligonucleotide sequence, and wherein the second oligonucleotide amplification primer set comprises (i) a plurality of third sequencing platform tag-containing oligonucleotides that each comprise an oligonucleotide sequence that is capable of specifically hybridizing to the third universal adaptor oligonucleotide and a third sequencing platform-specific oligonucleotide sequence that is linked to and positioned 5' to the third universal adaptor oligonucleotide sequence, and (ii) a plurality of fourth sequencing platform tag-containing oligonucleotides that each comprise an oligonucleotide sequence that is capable of specifically hybridizing to the fourth universal adaptor oligonucleotide sequence and a fourth sequencing platform-specific oligonucleotide sequence that is linked to and positioned 5' to the fourth universal adaptor oligonucleotide sequence, said step of contacting taking place under conditions and for a time sufficient to amplify both of the first and second reverse-transcribed cDNA products, to obtain a DNA library for sequencing.

Once the DNA library for sequencing has been so obtained, in a step which follows there takes place the sequencing of the DNA library, to obtain a data set of sequences encoding the first and second polypeptide sequences of the adaptive immune receptor heterodimer.

Analysis of the data set of sequences may then proceed essentially as described elsewhere herein, to determine rearranged DNA sequences encoding first and second polypeptides of an adaptive immune receptor heterodimer that originate in a single (*i.e.,* the same) lymphoid cell. Briefly, the method may further comprise the steps of: (a) sorting the data set of sequences according to oligonucleotide barcode sequences identified therein to obtain a plurality of barcode sequence sets each having a unique barcode; (b) sorting each barcode sequence set of (a) into an X1 sequence-containing subset and an X2 sequence-containing subset; (c) clustering members of each of the X1 and X2 sequence-containing subsets according to X1 and X2 sequences to obtain one or a plurality of X1 sequence cluster sets and one or a plurality of X2 sequence cluster sets, respectively, and error-correcting single nucleotide barcode sequence mismatches within any one or more of said X1 and X2 sequence cluster sets; (d) identifying each first and second adaptive immune receptor heterodimer polypeptide encoding sequence based on known X1 and X2 sequences, wherein each X1 sequence and each X2 sequence is associated with one or a plurality of unique B sequences to identify the container from which each B sequence-associated X1 sequence and each B sequence-associated X2 sequence originated; and (e) combinatorically matching B sequence-associated X1 and X2 sequences of (d) as being of common clonal origin based on a probability of B sequences that are coincident with common first and second adaptive immune receptor heterodimer polypeptide encoding sequences, and therefrom determining that rearranged DNA sequences encoding first and second polypeptide sequences of the adaptive immune receptor heterodimer originated in a single lymphoid cell.

Accordingly and in summary, in certain of the herein disclosed embodiments, sequencing adapters may be put onto each end of all reverse transcribed/ amplified TCR and/or IG encoding segments, for instance, by synthesizing universal adaptor sequences onto each end of each cDNA molecule outside of the well-specific barcode. Then, the adapters can be synthesized onto each molecule in a tailing PCR reaction. In such embodiments, fusion RT primers may be synthesized and used for the first cDNA strand synthesis. These primers will all contain the same unique DNA barcode, as well as universal (e.g., pGEX) priming sites. Upon completion of the first cDNA strand synthesis by reverse transcription, the contents of all plate wells will be recovered in a quantitative manner and pooled (*e.g.,* by an inverted centrifugation onto a trough), purified and consequently split into a multiplicity of wells for PCR with universal adapter primers (pGEX) containing "tail" sequences designed to incorporate sequences to be used for amplification and sequencing using a next-generation sequence analysis system (*e.g.,* Illumina, San Diego, CA). Alternatively, the sequencing platform specific adapters can be ligated onto the ends of tagged molecules (*e.g.*, Illumina TrueSeq™ sample preparation method). The molecules from all the wells are pooled thus generating a high-complexity sequencing library of uniquely tagged BCR or TCR ds-cDNA products. The molecules are all sequenced using high-throughput sequencing.

Universal sequencing primers, complementary to the sequencing platform-specific adapters may desirably be used. This will allow sample indexing of multiple samples, where a sample specific index will be used for each pool of uniquely tagged IGH / TCR products, originating from 96, 384, 1536 etc. original RT reaction wells. Or, a multiplex PCR with a mix of a universal UAII-Forward/ multiplex V, J or C reverse primers may be used to amplify specific target fragments while preserving the original cell transcripts barcoding. If the Illumina sequencing platform (MiSeq™) is used, a paired end sequencing of 2x 250bp would span the majority of the whole BCR / TCR heavy and light (alpha / beta; gamma / delta) chain sequences, thus allowing recovery of the whole coding sequence of each receptor domain. Alternatively, sequencing platforms with extended read length (Roche 454, Life Ion Torrent, OGT etc.) may be used to read through all library fragments in a single sequencing read in one direction. After sequencing, the reads from each sample may be demultiplexed, provided that more than one sample were in the same sequencing lane. Demultiplexing may be performed by assigning sequencing reads to one of multiple indexes used as part of the universal sequencing adapters. For each sample demultiplexed sequence reads, all reads may be divided by the well specific barcodes. Each set of reads with a specific barcode may be clustered separately to correct PCR and sequencing errors and determine the unique sequences for each barcode:

Sequences that have been so sorted by barcode and by TCR or IG chain may be further subject to cluster analysis using any of a known variety of algorithms for clustering (*e.g.*, BLASTClust, UCLUST, CD-HIT) and error correction in the case of sequences that fail to cluster with other sequences having shared barcode sequences but which instead would cluster with sequences having a barcode that differs by a single nucleotide. The unique sequences can be identified as IG heavy or light (kappa or lambda) chain, or as TCR (alpha or beta; gamma or delta) chains, by sequence match to known receptor sequences. Each heavy and light chain sequence may thus be associated with a list of barcodes corresponding to an original sample well position. The data can then be reordered by sequence. Associated to each unique sequence will be the set of multi-well plate well-specific barcodes within which set that sequence is found. For every B or T cell clone, the heavy and light chain sequences may be associated with the barcodes from all the wells for which one or more copies of the clone is present. Combinatorics may then be used to match heavy and light chains from the same clone. For example, in a 96 well plate, if particular heavy and light chain sequences are both associated with the same 12 barcodes, this particular pair of heavy and light chains may be assumed to have originated from the same clone, insofar as the probability of two sequences randomly having the exact same 12 barcodes out of 96 is infinitesimally small.

### *Exemplary Algorithm*

It will be appreciated that according to non-limiting theory, first and second adaptive immune receptor chain encoding sequences that occur with the same set of barcode sequences have a high probability of having originated from the same plate well, and thus from the same source cell. For example, where $10^3$ different barcodes are used in the construction of the first and second oligonucleotide reverse transcription primer sets, the probability that two independent (*i.e.,* originating from different cells) double-stranded cDNA first and second products would be obtained having the same barcode sequence is one in $10^6$, if one cell per each plate well were sorted.

Hence, if according to the methods described herein, three or more copies of a given set of first and second adaptive immune receptor polypeptide encoding sequences (*e.g.*, XI and X2) share common barcode sequences (*e.g.*, belong to the same barcode sequence set), the probability that the sequences are of independent cellular origin approaches zero.

In certain embodiments, barcode oligonucleotides B (B1, B2) may optionally comprise a first and a second oligonucleotide barcode sequence, wherein the first barcode sequence is selected to identify uniquely a particular V oligonucleotide sequence and the second barcode sequence is selected to identify uniquely a particular J oligonucleotide sequence. The relative positioning of the barcode oligonucleotides B1 and B2 and universal adaptors (U) advantageously permits rapid identification and quantification of the amplification products of a given unique template oligonucleotide by short sequence reads and paired-end sequencing on automated DNA sequencers (*e.g.*, Illumina HiSeq™ or Illumina MiSEQ®, or GeneAnalyzer™-2, Illumina Corp., San Diego, CA). In particular, these and related embodiments permit rapid high-throughput determination of specific combinations of a V and a J sequence that are present in an amplification product, thereby to characterize the relative representation of annealing targets for each combination of a V-specific primer and a J-specific primer that may be present in a sample such as a sample comprising rearranged TCR or BCR encoding DNA. Verification of the identities and/or quantities of the amplification products may be accomplished by longer sequence reads.

A large number of adaptive immune receptor variable (V) region and joining (J) region gene sequences are known as nucleotide and/or amino acid sequences, including non-rearranged genomic DNA sequences of TCR and Ig loci, and productively rearranged DNA sequences at such loci and their encoded products. See, *e.g.,* US201070330571; WO2011106738; WO2012027503. These and other sequences known to the art may be used according to the present disclosure for the design and production of oligonucleotides to be included in the presently provided compositions and methods.

V region-specific oligonucleotides may include a polynucleotide sequence of at least 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400 or 450 and not more than 1000, 900, 800, 700, 600 or 500 contiguous nucleotides of an adaptive immune receptor *(e.g.,* TCR or BCR) variable (V) region gene sequence, or the complement thereof, and in each of the plurality of oligonucleotide sequences V comprises a unique oligonucleotide sequence. Genomic sequences for TCR and BCR V region genes of humans and other species are known and available from public databases such as Genbank; V region gene sequences include polynucleotide sequences that encode the products of expressed, rearranged TCR and BCR genes and also include polynucleotide sequences of pseudogenes that have been identified in the V region loci. The diverse V polynucleotide sequences that may be incorporated into the presently disclosed oligonucleotides may vary widely in length, in nucleotide composition (*e.g.*, GC content), and in actual linear polynucleotide sequence, and are known, for example, to include "hot spots" or hypervariable regions that exhibit particular sequence diversity.

The polynucleotide V may thus includes sequences to which members of oligonucleotide primer sets specific for TCR or BCR genes can specifically anneal. Primer sets that are capable of amplifying rearranged DNA encoding a plurality of TCR or BCR are described, for example, in US201070330571; WO2011106738; or WO2012027503; or the like; or as described therein may be designed to include oligonucleotide sequences that can specifically hybridize to each unique V gene and to each J gene in a particular TCR or BCR gene locus (*e.g.,* TCRA, TCRB, TCRG, TCRD, IGH, IGK or IGL). For example by way of illustration and not limitation, an oligonucleotide primer of an oligonucleotide primer amplification set that is capable of amplifying rearranged DNA encoding one or a plurality of TCR or BCR may typically include a nucleotide sequence of 15, 16, 17, 18, 19, 20, 21, 22, 23, 24, 25, 26, 27, 28, 29, 30, 31, 32, 33, 34, 35, 36, 37, 38, 39 or 40 contiguous nucleotides, or more, and may specifically anneal to a complementary sequence of 15, 16, 17, 18, 19, 20, 21, 22, 23, 24, 25, 26, 27, 28, 29, 30, 31, 32, 33, 34, 35, 36, 37, 38, 39 or 40 contiguous nucleotides of a V or a J polynucleotide as provided herein. In certain embodiments the primers may comprise at least 19, 20, 21, 22, 23, 24, 25, 26, 27, 28, 29 or 30 nucleotides, and in certain embodiment the primers may comprise sequences of no more than 15, 16, 17, 18, 19, 20, 21, 22, 23, 24, 25, 26, 27, 28, 29, 30, 31, 32, 33, 34, 35, 36, 37, 38, 39 or 40 contiguous nucleotides. Primers and primer annealing sites of other lengths are also expressly contemplated, as disclosed herein.

The V polynucleotide may thus, in certain embodiments, comprise a nucleotide sequence having a length that is less than, the same or similar to that of the length of a typical V gene from its start codon to its CDR3 encoding region and may, but need not, include a nucleotide sequence that encodes the CDR3 region. In certain preferred embodiments the V polynucleotide includes all or a portion of a CDR3 encoding nucleotide sequence or the complement thereto and CDR3 sequence lengths may vary considerably and have been characterized by several different numbering schemes *(e.g.,* Lefranc, 1999 The Immunologist 7:132; Kabat et al., 1991 In: Sequences of Proteins of Immunological Interest, NIH Publication 91-3242; Chothia et al., 1987 J. Mol. Biol. 196:901; Chothia et al., 1989 Nature 342:877; Al-Lazikani et al., 1997 J. Mol. Biol. 273:927; see also, *e.g.,* Rock et al., 1994 J. Exp. Med. 179:323; Saada et al., 2007 Immunol. Cell Biol. 85:323).

Briefly, the CDR3 region typically spans the polypeptide portion extending from a highly conserved cysteine residue (encoded by the trinucleotide codon TGY; Y = T or C) in the V segment to a highly conserved phenylalanine residue (encoded by TTY) in the J segment of TCRs, or to a highly conserved tryptophan (encoded by TGG) in IGH. More than 90% of natural, productive rearrangements in the TCRB locus have a CDR3 encoding length by this criterion of between 24 and 54 nucleotides, corresponding to between 9 and 17 encoded amino acids. The numbering schemes for CDR3 encoding regions described above denote the positions of the conserved cysteine, phenylalanine and tryptophan codons, and these numbering schemes may also be applied to pseudogenes in which one or more codons encoding these conserved amino acids may have been replaced with a codon encoding a different amino acid. For pseudogenes which do not use these conserved amino acids, the CDR3 length may be defined relative to the corresponding position at which the conserved residue would have been observed absent the substitution, according to one of the established CDR3 sequence position numbering schemes referenced above.

The polynucleotide J may comprise a polynucleotide comprising at least 15-30, 31-50, 51-60, 61-90, 91-120, or 120-150, and not more than 600, 500, 400, 300 or 200 contiguous nucleotides of an adaptive immune receptor joining (J) region encoding gene sequence, or the complement thereof, and in each of the plurality of oligonucleotide sequences J comprises a unique oligonucleotide sequence. The polynucleotide J (or its complement) includes sequences to which members of oligonucleotide primer sets specific for TCR or BCR genes can specifically anneal. Primer sets that are capable of amplifying rearranged DNA encoding a plurality of TCR or BCR are described, for example, in US201070330571; WO2011106738; or WO2012027503 or the like; or as described therein may be designed to include oligonucleotide sequences that can specifically hybridize to each unique V gene and to each unique J gene in a particular TCR or BCR gene locus *(e.g.,* TCR α, β, γ or δ, or IgH μ, γ, δ, α or ε, or IgL κ or λ).

It may be preferred in certain embodiments that the plurality of J polynucleotides that are present in the herein described primer compositions have lengths that simulate the overall lengths of known, naturally occurring J gene nucleotide sequences. The J region lengths in the herein described templates may differ from the lengths of naturally occurring J gene sequences by no more than 1, 2, 3, 4, 5, 6, 7, 8, 9, 10, 11, 12, 13, 14, 15, 16, 17, 18, 19 or 20 percent. The J polynucleotide may thus, in certain embodiments, comprise a nucleotide sequence having a length that is the same or similar to that of the length of a typical naturally occurring J gene and may, but need not, include a nucleotide sequence that encodes the CDR3 region, as discussed above.

Genomic sequences for TCR and BCR J region genes of humans and other species are known and available from public databases such as Genbank; J region gene sequences include polynucleotide sequences that encode the products of expressed and unexpressed rearranged TCR and BCR genes. The diverse J polynucleotide sequences that may be incorporated into the presently disclosed primers may vary widely in length, in nucleotide composition (*e.g.*, GC content), and in actual linear polynucleotide sequence.

Alternatives to the V and J sequences described herein, for use in construction of the herein described V-segment and J-segment oligonucleotide primers, may be selected by a skilled person based on the present disclosure using knowledge in the art regarding published gene sequences for the V- and J-encoding regions of the genes for each TCR and Ig subunit. Reference Genbank entries for human adaptive immune receptor sequences include: TCRα: (TCRA/D): NC_000014.8 (chr14:22090057..23021075); TCRβ: (TCRB): NC_000007.13 (chr7:141998851..142510972); TCRγ: (TCRG): NC_000007.13 (chr7:38279625..38407656); immunoglobulin heavy chain, IgH (IGH): NC_000014.8 (chr14: 106032614..107288051); immunoglobulin light chain-kappa, IgLκ (IGK): NC_000002.11 (chr2: 89156874..90274235); and immunoglobulin light chain-lambda, IgLλ (IGL): NC_000022.10 (chr22: 22380474..23265085). Reference Genbank entries for mouse adaptive immune receptor loci sequences include: TCRβ: (TCRB): NC_000072.5 (chr6: 40841295..41508370), and immunoglobulin heavy chain, IgH (IGH): NC_000078.5 (chr12:114496979..117248165).

Primer design analyses and target site selection considerations can be performed, for example, using the OLIGO primer analysis software and/or the BLASTN 2.0.5 algorithm software (Altschul et al., Nucleic Acids Res. 1997, 25(17):3389-402), or other similar programs available in the art.

Accordingly, based on the present disclosure and in view of these known adaptive immune receptor gene sequences and oligonucleotide design methodologies, for inclusion in the instant oligonucleotides those skilled in the art can design a plurality of V region-specific and J region-specific polynucleotide sequences that each independently contain oligonucleotide sequences that are unique to a given V and J gene, respectively. Similarly, from the present disclosure and in view of known adaptive immune receptor sequences, those skilled in the art can also design a primer set comprising a plurality of V region-specific and J region-specific oligonucleotide primers that are each independently capable of annealing to a specific sequence that is unique to a given V and J gene, respectively, whereby the plurality of primers is capable of amplifying substantially all V genes and substantially all J genes in a given adaptive immune receptor-encoding locus (*e.g.,* a human TCR or IGH locus). Such primer sets permit generation, in multiplexed (*e.g.,* using multiple forward and reverse primer pairs) PCR, of amplification products that have a first end that is encoded by a rearranged V region-encoding gene segment and a second end that is encoded by a J region-encoding gene segment.

Typically and in certain embodiments, such amplification products may include a CDR3-encoding sequence although the disclosed method is not intended to be so limited and contemplates amplification products that do not include a CDR3-encoding sequence. The primers may be preferably designed to yield amplification products having sufficient portions of V and J sequences and in certain preferred embodiments also of barcode (B) sequences as described herein, such that by sequencing the products (amplicons), it is possible to identify on the basis of sequences that are unique to each gene segment (i) the particular V gene, and (ii) the particular J gene in the proximity of which the V gene underwent rearrangement to yield a rearranged adaptive immune receptor-encoding gene. Typically, and in preferred embodiments, the PCR amplification products will not be more than 600 base pairs in size, which according to non-limiting theory will exclude amplification products from non-rearranged adaptive immune receptor genes. In certain other preferred embodiments the amplification products will not be more than 500, 400, 300, 250, 200, 150, 125, 100, 90, 80, 70, 60, 50, 40, 30 or 20 base pairs in size, such as may advantageously provide rapid, high-throughput quantification of sequence-distinct amplicons by short sequence reads.

## *Primers*

According to the present disclosure, oligonucleotide primers are provided in an oligonucleotide primer set that comprises a plurality of V-segment primers and a plurality of J-segment primers, where the primer set is capable of amplifying rearranged DNA encoding adaptive immune receptors in a biological sample that comprises lymphoid cell DNA. Suitable primer sets are known in the art and disclosed herein, for example, the primer sets in US 2012/0058902, US201070330571, WO2011106738, or WO2012027503 or the like; or those shown in Table 1. In certain embodiments the primer set is designed to include a plurality of V sequence-specific primers that includes, for each unique V region gene (including pseudogenes) in a sample, at least one primer that can specifically anneal to a unique V region sequence; and for each unique J region gene in the sample, at least one primer that can specifically anneal to a unique J region sequence.

Primer design may be achieved by routine methodologies in view of known TCR and BCR genomic sequences. Accordingly, the primer set is preferably capable of amplifying every possible V-J combination that may result from DNA rearrangements in the TCR or BCR locus. As also described below, certain embodiments contemplate primer sets in which one or more V primers may be capable of specifically annealing to a "unique" sequence that may be shared by two or more V regions but that is not common to all V regions, and/or in which in which one or more J primers may be capable of specifically annealing to a "unique" sequence that may be shared by two or more J regions but that is not common to all J regions.

In particular embodiments, oligonucleotide primers for use in the compositions and methods described herein may comprise or consist of a nucleic acid of at least about 15 nucleotides long that has the same sequence as, or is complementary to, a 15 nucleotide long contiguous sequence of the target V- or J- segment (*i.e.,* portion of genomic polynucleotide encoding a V-region or J-region polypeptide). Longer primers, *e.g.,* those of about 16, 17, 18, 19, 20, 21, 22, 23, 24, 25, 26, 27, 28, 29, 30, 31, 32, 33, 34, 35, 36, 37, 38, 39, 40, 45, or 50, nucleotides long that have the same sequence as, or sequence complementary to, a contiguous sequence of the target V- or J- region encoding polynucleotide segment, will also be of use in certain embodiments. All intermediate lengths of the presently described oligonucleotide primers are contemplated for use herein. As would be recognized by the skilled person, the primers may have additional sequence added (*e.g.,* nucleotides that may not be the same as or complementary to the target V- or J-region encoding polynucleotide segment), such as restriction enzyme recognition sites, adaptor sequences for sequencing, barcode sequences, and the like (see *e.g.,* primer sequences provided in the Tables and sequence listing herein). Therefore, the length of the primers may be longer, such as about 55, 56, 57, 58, 59, 60, 61, 62, 63, 64, 65, 66, 67, 68, 69, 70, 71, 72, 73, 74, 75, 80, 85, 90, 95, 100 or more nucleotides in length or more, depending on the specific use or need.

Also contemplated for use in certain embodiments are adaptive immune receptor V-segment or J-segment oligonucleotide primer variants that may share a high degree of sequence identity to the oligonucleotide primers for which nucleotide sequences are presented herein, including those set forth in the Sequence Listing. Thus, in these and related embodiments, adaptive immune receptor V-segment or J-segment oligonucleotide primer variants may have substantial identity to the adaptive immune receptor V-segment or J-segment oligonucleotide primer sequences disclosed herein, for example, such oligonucleotide primer variants may comprise at least 70% sequence identity, preferably at least 75%, 80%, 85%, 90%, 91%, 92%, 93%, 94%, 95%, 96%, 97%, 98%, or 99% or higher sequence identity compared to a reference polynucleotide sequence such as the oligonucleotide primer sequences disclosed herein, using the methods described herein (*e.g.,* BLAST analysis using standard parameters). One skilled in this art will recognize that these values can be appropriately adjusted to determine corresponding ability of an oligonucleotide primer variant to anneal to an adaptive immune receptor segment-encoding polynucleotide by taking into account codon degeneracy, reading frame positioning and the like.

Typically, oligonucleotide primer variants will contain one or more substitutions, additions, deletions and/or insertions, preferably such that the annealing ability of the variant oligonucleotide is not substantially diminished relative to that of an adaptive immune receptor V-segment or J-segment oligonucleotide primer sequence that is specifically set forth herein.

Table 2 presents as a non-limiting example an oligonucleotide primer set that is capable of amplifying productively rearranged DNA encoding TCR β-chains (TCRB) in a biological sample that comprises DNA from lymphoid cells of a subject. In this primer set the J segment primers share substantial sequence homology, and therefore may cross-prime amongst more than one target J polynucleotide sequence, but the V segment primers are designed to anneal specifically to target sequences within the CDR2 region of V and are therefore unique to each V segment. An exception, however, is present in the case of several V primers where the within-family sequences of the closely related target genes are identical (e.g., V6-2 and V6-3 are identical at the nucleotide level throughout the coding sequence of the V segment, and therefore may have a single primer, TRB2V6-2/3).

**Table 2. Exemplary Oligonucleotide Primer Set (hsTCRB PCR Primers)**

| Name | Sequence | SEQ ID NO: |
|---|---|---|
| TRBJ1-1 | TTACCTACAACTGTGAGTCTGGTGCCTTGTCCAAA | 1631 |
| TRBJ1-2 | ACCTACAACGGTTAACCTGGTCCCCGAACCGAA | 1632 |
| TRBJ1-3 | ACCTACAACAGTGAGCCAACTTCCCTCTCCAAA | 1633 |
| TRBJ1-4 | CCAAGACAGAGAGCTGGGTTCCACTGCCAAA | 1634 |
| TRBJ1-5 | ACCTAGGATGGAGAGTCGAGTCCCATCACCAAA | 1635 |
| TRBJ1-6 | CTGTCACAGTGAGCCTGGTCCCGTTCCCAAA | 1636 |
| TRBJ2-1 | CGGTGAGCCGTGTCCCTGGCCCGAA | 1637 |
| TRBJ2-2 | CCAGTACGGTCAGCCTAGAGCCTTCTCCAAA | 1638 |
| TRBJ2-3 | ACTGTCAGCCGGGTGCCTGGGCCAAA | 1639 |
| TRBJ2-4 | AGAGCCGGGTCCCGGCGCCGAA | 1640 |
| TRBJ2-5 | GGAGCCGCGTGCCTGGCCCGAA | 1641 |
| TRBJ2-6 | GTCAGCCTGCTGCCGGCCCCGAA | 1642 |
| TRBJ2-7 | GTGAGCCTGGTGCCCGGCCCGAA | 1643 |
| TRB2V10-1 | AACAAAGGAGAAGTCTCAGATGGCTACAG | 1644 |
| TRB2V10-2 | GATAAAGGAGAAGTCCCCGATGGCTATGT | 1645 |
| TRB2V10-3 | GACAAAGGAGAAGTCTCAGATGGCTATAG | 1646 |
| TRB2V6-2/3 | GCCAAAGGAGAGGTCCCTGATGGCTACAA | 1647 |
| TRB2V6-8 | CTCTAGATTAAACACAGAGGATTTCCCAC | 1648 |
| TRB2V6-9 | AAGGAGAAGTCCCCGATGGCTACAATGTA | 1649 |
| TRB2V6-5 | AAGGAGAAGTCCCCAATGGCTACAATGTC | 1650 |
| TRB2V6-6 | GACAAAGGAGAAGTCCCGAATGGCTACAAC | 1651 |
| TRB2V6-7 | GTTCCCAATGGCTACAATGTCTCCAGATC | 1652 |
| TRB2V6-1 | GTCCCCAATGGCTACAATGTCTCCAGATT | 1653 |
| TRB2V6-4 | GTCCCTGATGGTTATAGTGTCTCCAGAGC | 1654 |
| TRB2V24-1 | ATCTCTGATGGATACAGTGTCTCTCGACA | 1655 |
| TRB2V25-1 | TTTCCTCTGAGTCAACAGTCTCCAGAATA | 1656 |
| TRB2V27 | TCCTGAAGGGTACAAAGTCTCTCGAAAAG | 1657 |
| TRB2V26 | CTCTGAGAGGTATCATGTTTCTTGAAATA | 1658 |
| TRB2V28 | TCCTGAGGGGT ACAGTGTCTCT AGAGAGA | 1659 |
| TRB2V19 | TATAGCTGAAGGGTACAGCGTCTCTCGGG | 1660 |
| TRB2V4-1 | CTGAATGCCCCAACAGCTCTCTCTTAAAC | 1661 |
| TRB2V4-2/3 | CTGAATGCCCCAACAGCTCTCACTTATTC | 1662 |

(continued)

| Name | Sequence | SEQ ID NO: |
|------|----------|------------|
| TRB2V2P | CCTGAATGCCCTGACAGCTCTCGCTTATA | 1663 |
| TRB2V3-1 | CCTAAATCTCCAGACAAAGCTCACTTAAA | 1664 |
| TRB2V3-2 | CTCACCTGACTCTCCAGACAAAGCTCAT | 1665 |
| TRB2V16 | TTCAGCTAAGTGCCTCCCAAATTCACCCT | 1666 |
| TRB2V23-1 | GATTCTCATCTCAATGCCCCAAGAACGC | 1667 |
| TRB2V18 | ATTTTCTGCTGAATTTCCCAAAGAGGGCC | 1668 |
| TRB2V17 | ATTCACAGCTGAAAGACCTAACGGAACGT | 1669 |
| TRB2V14 | TCTTAGCTGAAAGGACTGGAGGGACGTAT | 1670 |
| TRB2V2 | TTCGATGATCAATTCTCAGTTGAAAGGCC | 1671 |
| TRB2V12-1 | TTGATTCTCAGCACAGATGCCTGATGT | 1672 |
| TRB2V12-2 | GCGATTCTCAGCTGAGAGGCCTGATGG | 1673 |
| TRB2V12-3/4 | TCGATTCTCAGCTAAGATGCCTAATGC | 1674 |
| TRB2V12-5 | TTCTCAGCAGAGATGCCTGATGCAACTTTA | 1675 |
| TRB2V7-9 | GGTTCTCTGCAGAGAGGCCTAAGGGATCT | 1676 |
| TRB2V7-8 | GCTGCCCAGTGATCGCTTCTTTGCAGAAA | 1677 |
| TRB2V7-4 | GGCGGCCCAGTGGTCGGTTCTCTGCAGAG | 1678 |
| TRB2V7-6/7 | AT G A TCGGTTCTCTGCAGAGAGGCCTGAGG | 1679 |
| TRB2V7-2 | AGTGATCGCTTCTCTGCAGAGAGGACTGG | 1680 |
| TRB2V7-3 | GGCTGCCCAACGATCGGTTCTTTGCAGT | 1681 |
| TRB2V7-1 | TCCCCGTGA TCGGTTCTCTGCACAGAGGT | 1682 |
| TRB2V11-123 | CTAAGGATCGATTTTCTGCAGAGAGGCTC | 1683 |
| TRB2V13 | CTGATCGATTCTCAGCTCAACAGTTCAGT | 1684 |
| TRB2V5-1 | TGGTCGATTCTCAGGGCGCCAGTTCTCTA | 1685 |
| TRB2V5-3 | TAATCGATTCTCAGGGCGCCAGTTCCATG | 1686 |
| TRB2V5-4 | TCCTAGATTCTCAGGTCTCCAGTTCCCTA | 1687 |
| TRB2V5-8 | GGAAACTTCCCTCCTAGATTTTCAGGTCG | 1688 |
| TRB2V5-5 | AAGAGGAAACTTCCCTGATCGATTCTCAGC | 1689 |
| TRB2V5-6 | GGCAACTTCCCTGATCGATTCTCAGGTCA | 1690 |
| TRB2V9 | GTTCCCTGACTTGCACTCTGAACTAAAC | 1691 |
| TRB2V15 | GCCGAACACTTCTTTCTGCTTTCTTGAC | 1692 |
| TRB2V30 | GACCCCAGGACCGGCAGTTCATCCTGAGT | 1693 |
| TRB2V20-1 | ATGCAAGCCTGACCTTGTCCACTCTGACA | 1694 |
| TRB2V29-1 | CA TCAGCCGCCCAAACCT AACA TTCTCAA | 1695 |

In certain preferred embodiments, the V-segment and J-segment oligonucleotide primers as described herein are designed to include nucleotide sequences such that adequate information is present within the sequence of an amplification product of a rearranged adaptive immune receptor (TCR or Ig) gene to identify uniquely both the specific V and the specific J genes that give rise to the amplification product in the rearranged adaptive immune receptor locus (*e.g.,* at least 1, 2, 3, 4, 5, 6, 7, 8, 9, 10, 11, 12, 13, 14, 15, 16, 17, 18, 19 or 20 base pairs of sequence upstream of the V gene recombination signal sequence (RSS), preferably at least about 22, 24, 26, 28, 30, 32, 34, 35, 36, 37, 38, 39 or 40 base pairs of sequence upstream of the V gene recombination signal sequence (RSS), and in certain preferred embodiments greater than 40 base pairs of sequence upstream of the V gene recombination signal sequence (RSS), and at least 1, 2, 3, 4, 5, 6, 7, 8, 9, 10, 11, 12, 13, 14, 15, 16, 17, 18, 19 or 20 base pairs downstream of the J gene RSS, preferably at least about 22, 24, 26, 28 or 30 base pairs downstream of the J gene RSS, and in certain preferred embodiments greater than 30 base pairs downstream of the J gene RSS).

This feature stands in contrast to oligonucleotide primers described in the art for amplification of TCR-encoding or Ig-encoding gene sequences, which rely primarily on the amplification reaction merely for detection of presence or absence of products of appropriate sizes for V and J segments (*e.g.*, the presence in PCR reaction products of an amplicon of a particular size indicates presence of a V or J segment but fails to provide the sequence of the amplified PCR product and hence fails to confirm its identity, such as the common practice of spectratyping).

Oligonucleotides (*e.g.*, primers) can be prepared by any suitable method, including direct chemical synthesis by a method such as the phosphotriester method of Narang et al., 1979, Meth. Enzymol. 68:90-99; the phosphodiester method of Brown et al., 1979, Meth. Enzymol. 68:109-151; the diethylphosphoramidite method of Beaucage et al., 1981, Tetrahedron Lett. 22:1859-1862; and the solid support method of U.S. Pat. No. 4,458,066. A review of synthesis methods of conjugates of oligonucleotides and modified nucleotides is provided in Goodchild, 1990, Bioconjugate Chemistry 1(3): 165-187.

The term "primer," as used herein, refers to an oligonucleotide capable of acting as a point of initiation of DNA synthesis under suitable

conditions. Such conditions include those in which synthesis of a primer extension product complementary to a nucleic acid strand is induced in the presence of four different nucleoside triphosphates and an agent for extension (*e.g.*, a DNA polymerase or reverse transcriptase) in an appropriate buffer and at a suitable temperature.

A primer is preferably a single-stranded DNA. The appropriate length of a primer depends on the intended use of the primer but typically ranges from 6 to 50 nucleotides, or in certain embodiments, from 15-35 nucleotides. Short primer molecules generally require cooler temperatures to form sufficiently stable hybrid complexes with the template. A primer need not reflect the exact sequence of the template nucleic acid, but must be sufficiently complementary to hybridize with the template. The design of suitable primers for the amplification of a given target sequence is well known in the art and described in the literature cited herein.

As described herein, primers can incorporate additional features which allow for the detection or immobilization of the primer but do not alter the basic property of the primer, that of acting as a point of initiation of DNA synthesis. For example, primers may contain an additional nucleic acid sequence at the 5' end which does not hybridize to the target nucleic acid, but which facilitates cloning, detection, or sequencing of the amplified product. The region of the primer which is sufficiently complementary to the template to hybridize is referred to herein as the hybridizing region.

As used herein, a primer is "specific," for a target sequence if, when used in an amplification reaction under sufficiently stringent conditions, the primer hybridizes primarily to the target nucleic acid. Typically, a primer is specific for a target sequence if the primer-target duplex stability is greater than the stability of a duplex formed between the primer and any other sequence found in the sample. One of skill in the art will recognize that various factors, such as salt conditions as well as base composition of the primer and the location of the mismatches, will affect the specificity of the primer, and that routine experimental confirmation of the primer specificity will be needed in many cases. Hybridization conditions can be chosen under which the primer can form stable duplexes only with a target sequence. Thus, the use of target-specific primers under suitably stringent amplification conditions enables the selective amplification of those target sequences which contain the target primer binding sites.

In particular embodiments, primers for use in the methods described herein comprise or consist of a nucleic acid of at least about 15 nucleotides long that has the same sequence as, or is complementary to, a 15 nucleotide long contiguous sequence of the target V or J segment. Longer primers, *e.g.*, those of about 16, 17, 18, 19, 20, 21, 22, 23, 24, 25, 26, 27, 28, 29, 30, 31, 32, 33, 34, 35, 36, 37, 38, 39, 40, 45, or 50, nucleotides long that have the same sequence as, or sequence complementary to, a contiguous sequence of the target V or J segment, will also be of use in certain embodiments. All intermediate lengths of the aforementioned primers are contemplated for use herein. As would be recognized by the skilled person, the primers may have additional sequence added (*e.g.*, nucleotides that may not be the same as or complementary to the target V or J segment), such as restriction enzyme recognition sites, adaptor sequences for sequencing, barcode sequences, and the like (see *e.g.,* primer sequences provided herein and in the sequence listing). Therefore, the length of the primers may be longer, such as 55, 56, 57, 58, 59, 60, 65, 70, 75, nucleotides in length or more, depending on the specific use or need. For example, in one embodiment, the forward and reverse primers are both modified at the 5' end with the universal forward primer sequence compatible with a DNA sequencer.

Also contemplated for use in certain embodiments are adaptive immune receptor V-segment or J-segment oligonucleotide primer variants that may share a high degree of sequence identity to the oligonucleotide primers for which nucleotide sequences are presented herein, including those set forth in the Sequence Listing. Thus, in these and related embodiments, adaptive immune receptor V-segment or J-segment oligonucleotide primer variants may have substantial identity to the adaptive immune receptor V-segment or J-segment oligonucleotide primer sequences disclosed herein, for example, such oligonucleotide primer variants may comprise at least 70% sequence identity, preferably at least 75%, 80%, 85%, 90%, 91%, 92%, 93%, 94%, 95%, 96%, 97%, 98%, or 99% or higher sequence identity compared to a reference polynucleotide sequence such as the oligonucleotide primer sequences disclosed herein, using the methods described herein (*e.g.,* BLAST analysis using standard parameters). One skilled in this art will recognize that these values can be appropriately adjusted to determine corresponding ability of an oligonucleotide primer variant to anneal to an adaptive immune receptor segment-encoding polynucleotide by taking into account codon degeneracy, reading frame positioning and the like.

Typically, oligonucleotide primer variants will contain one or more substitutions, additions, deletions and/or insertions, preferably such that the annealing ability of the variant oligonucleotide is not substantially diminished relative to that of an adaptive immune receptor V-segment or J-segment oligonucleotide primer sequence that is specifically set forth herein. As also noted elsewhere herein, in preferred embodiments adaptive immune receptor V-segment and J-segment oligonucleotide primers are designed to be capable of amplifying a rearranged TCR or IGH sequence that includes the coding region for CDR3.

According to certain embodiments contemplated herein, the primers for use in the multiplex PCR methods of the present disclosure may be functionally blocked to prevent non-specific priming of non-T or B cell sequences. For example, the primers may be blocked with chemical modifications as described in U.S. patent application publication US2010/0167353. According to certain herein disclosed embodiments, the use of such blocked primers in the present multiplex PCR reactions involves primers that may have an inactive configuration wherein DNA replication (*i.e.,* primer extension) is blocked, and an activated configuration wherein DNA replication proceeds. The inactive configuration of the primer is present when the primer is either single-stranded, or when the primer is specifically hybridized to the target DNA sequence of interest but primer extension remains blocked by a chemical moiety that is linked at or near to the 3' end of the primer.

The activated configuration of the primer is present when the primer is hybridized to the target nucleic acid sequence of interest and is subsequently acted upon by RNase H or another cleaving agent to remove the 3' blocking group, thereby allowing an enzyme (*e.g.,* a DNA polymerase) to catalyze primer extension in an amplification reaction. Without wishing to be bound by theory, it is believed that the kinetics of the hybridization of such primers are akin to a second order reaction, and are therefore a function of the T cell or B cell gene sequence concentration in the mixture. Blocked primers minimize non-specific reactions by requiring hybridization to the target followed by cleavage before primer extension can proceed. If a primer hybridizes incorrectly to a sequence that is related to the desired target sequence but which differs by having one or more non-complementary nucleotides that result in base-pairing mismatches, cleavage of the primer is inhibited, especially when there is a mismatch that lies at or near the cleavage site. This strategy to improve the fidelity of amplification reduces the frequency of false priming at such locations, and thereby increases the specificity of the reaction. As would be recognized by the skilled person, reaction conditions, particularly the concentration of RNase H and the time allowed for hybridization and extension in each cycle, can be optimized to maximize the difference in cleavage efficiencies between highly efficient cleavage of the primer when it is correctly hybridized to its true target sequence, and poor cleavage of the primer when there is a mismatch between the primer and the template sequence to which it may be incompletely annealed.

As described in US2010/0167353, a number of blocking groups are known in the art that can be placed at or near the 3' end of the oligonucleotide (*e.g.,* a primer) to prevent extension. A primer or other oligonucleotide may be modified at the 3'-terminal nucleotide to prevent or inhibit initiation of DNA synthesis by, for example, the addition of a 3' deoxyribonucleotide residue (*e.g.,* cordycepin), a 2',3'-dideoxyribonucleotide residue, non-nucleotide linkages or alkane-diol modifications (U.S. Pat. No. 5,554,516). Alkane diol modifications which can be used to inhibit or block primer extension have also been described by Wilk et al., (1990 Nucleic Acids Res. 18 (8):2065), and by Arnold et al. (U.S. Pat. No. 6,031,091). Additional examples of suitable blocking groups include 3' hydroxyl substitutions (*e.g.,* 3'-phosphate, 3'-triphosphate or 3'-phosphate diesters with alcohols such as 3-hydroxypropyl), 2'3'-cyclic phosphate, 2' hydroxyl substitutions of a terminal RNA base (e.g., phosphate or sterically bulky groups such as triisopropyl silyl (TIPS) or tert-butyl dimethyl silyl (TBDMS)). 2'-alkyl silyl groups such as TIPS and TBDMS substituted at the 3'-end of an oligonucleotide are described by Laikhter et al., U.S. patent application Ser. No. US2007/0218490. Bulky substituents can also be incorporated on the base of the 3'-terminal residue of the oligonucleotide to block primer extension.

In certain embodiments, the oligonucleotide may comprise a cleavage domain that is located upstream (*e.g.,* 5' to) of the blocking group used to inhibit primer extension. As examples, the cleavage domain may be an RNase H cleavage domain, or the cleavage domain may be an RNase H2 cleavage domain comprising a single RNA residue, or the oligonucleotide may comprise replacement of the RNA base with one or more alternative nucleosides. Additional illustrative cleavage domains are described in US2010/0167353.

Thus, a multiplex PCR system may use 40, 45, 50, 55, 60, 65, 70, 75, 80, 85, or more forward primers, wherein each forward primer is complementary to a single functional TCR or Ig V segment or a small family of functional TCR or Ig V segments, *e.g.,* a TCR Vβ segment, (see *e.g.,* the TCRBV primers as shown in Table 2, SEQ ID NOS:1644-1695), and, for example, thirteen reverse primers, each specific to a TCR or Ig J segment, such as TCR Jβ segment (see *e.g.,*

TCRBJ primers in Table 2, SEQ ID NOS:1631-1643). In another embodiment, a multiplex PCR reaction may use four forward primers each specific to one or more functional TCRγ V segment and four reverse primers each specific for one or more TCRγ J segments. In another embodiment, a multiplex PCR reaction may use 84 forward primers each specific to one or more functional V segments and six reverse primers each specific for one or more J segments.

Thermal cycling conditions may follow methods of those skilled in the art. For example, using a PCR Express™ thermal cycler (Hybaid, Ashford, UK), the following cycling conditions may be used: 1 cycle at 95°C for 15 minutes, 25 to 40 cycles at 94°C for 30 seconds, 59°C for 30 seconds and 72°C for 1 minute, followed by one cycle at 72°C for 10 minutes. As will be recognized by the skilled person, thermal cycling conditions may be optimized, for example, by modifying annealing temperatures, annealing times, number of cycles and extension times. As would be recognized by the skilled person, the amount of primer and other PCR reagents used, as well as PCR parameters (*e.g.*, annealing temperature, extension times and cycle numbers), may be optimized to achieve desired PCR amplification efficiency.

Alternatively, in certain related embodiments also contemplated herein, "digital PCR" methods can be used to quantitate the number of target genomes in a sample, without the need for a standard curve. In digital PCR, the PCR reaction for a single sample is performed in a multitude of more than 100 microcells or droplets, such that each droplet either amplifies (*e.g.*, generation of an amplification product provides evidence of the presence of at least one template molecule in the microcell or droplet) or fails to amplify (evidence that the template was not present in a given microcell or droplet). By simply counting the number of positive microcells, it is possible directly to count the number of target genomes that are present in an input sample. Digital PCR methods typically use an endpoint readout, rather than a conventional quantitative PCR signal that is measured after each cycle in the thermal cycling reaction (see, *e.g.,* Pekin et al., 2011 Lab. Chip 11(13):2156; Zhong et al., 2011 Lab. Chip 11(13):2167; Tewhey et al., 2009 Nature Biotechnol. 27:1025; 2010 Nature Biotechnol. 28:178). Accordingly, any of the herein described compositions (*e.g.*, adaptive immune receptor gene-specific oligonucleotide primer sets) and methods may be adapted for use in such digital PCR methodology, for example, the ABI QuantStudio™ 12K Flex System (Life Technologies, Carlsbad, CA), the QuantaLife™ digital PCR system (BioRad, Hercules, CA) or the RainDance™ microdroplet digital PCR system (RainDance Technologies, Lexington, MA).

**Adaptors**

The herein described oligonucleotides may in certain embodiments comprise first (U1) and second (U2) (and optionally third (U3) and fourth (U4)) universal adaptor oligonucleotide sequences, or may lack either or both of U1 and U2 (or U3 or U4). A universal adaptor oligonucleotide U thus may comprise either nothing or an oligonucleotide having a sequence that is selected from (i) a first universal adaptor oligonucleotide sequence, and (ii) a first sequencing platform-specific oligonucleotide sequence that is linked to and positioned 5' to a first universal adaptor oligonucleotide sequence, and U2 may comprise either nothing or an oligonucleotide having a sequence that is selected from (i) a second universal adaptor oligonucleotide sequence, and (ii) a second sequencing platform-specific oligonucleotide sequence that is linked to and positioned 5' to a second universal adaptor oligonucleotide sequence. A similar relationship pertains for U3 and U4.

U1 and/or U2 may, for example, comprise universal adaptor oligonucleotide sequences and/or sequencing platform-specific oligonucleotide sequences that are specific to a single-molecule sequencing technology being employed, for example the HiSeq™ or GeneAnalyzer™-2 (GA-2) systems (Illumina, Inc., San Diego, CA) or another suitable sequencing suite of instrumentation, reagents and software. Inclusion of such platform-specific adaptor sequences permits direct quantitative sequencing of the presently described dsDNA amplification products into which U has been incorporated as described herein, using a nucleotide sequencing methodology such as the HiSeq™ or GA2 or equivalent. This feature therefore advantageously permits qualitative and quantitative characterization of the dsDNA composition.

For example, dsDNA amplification products may be generated that have universal adaptor sequences at both ends, so that the adaptor sequences can be used to further incorporate sequencing platform-specific oligonucleotides at each end of each template.

Without wishing to be bound by theory, platform-specific oligonucleotides may be added onto the ends of such dsDNA using 5' (5'-platform sequence-universal adaptor-1 sequence-3') and 3' (5'-platform sequence-universal adaptor-2 sequence-3') oligonucleotides in three cycles of denaturation, annealing and extension, so that the relative representation in the dsDNA composition of each of the component dsDNAs is not quantitatively altered. Unique identifier sequences (*e.g.*, barcode sequences B that are associated with and thus identify individual V and/or J regions, or sample-identifier barcodes as described herein) are placed adjacent to the adaptor sequences, thus permitting quantitative sequencing in short sequence reads, in order to characterize the DNA population by the criterion of the relative amount of each unique sequence that is present.

In addition to adaptor sequences described in the Examples and included in the exemplary template sequences in the Sequence Listing (*e.g.*, at the 5' and 3' ends of SEQ ID NOS:1-1630), other oligonucleotide sequences that may be used as universal adaptor sequences will be known to those familiar with the art in view of the present disclosure. Non-limiting examples of additional adaptor sequences are shown in Table 3 and set forth in SEQ ID NOS:1710-1731.

*Table 3. Exemplary Adaptor Sequences*

| Adaptor (primer) name | Sequence | SEQ ID NO: |
|---|---|---|
| T7 Promotor | AATACGACTCACTATAGG | 1710 |
| T7 Terminator | GCTAGTTATTGCTCAGCGG | 1711 |
| T3 | ATTAACCCTCACTAAAGG | 1712 |
| SP6 | GATTTAGGTGACACTATAG | 1713 |
| M13F(-21) | TGTAAAACGACGGCCAGT | 1714 |
| M13F(-40) | GTTTTCCCAGTCACGAC | 1715 |
| M13R Reverse | CAGGAAACAGCTATGACC | 1716 |
| AOX1 Forward | GACTGGTTCCAATTGACAAGC | 1717 |
| AOX1 Reverse | GCAAATGGCATTCTGACATCC | 1718 |
| pGEX Forward (GST 5, pGEX 5') | GGGCTGGCAAGCCACGTTTGGTG | 1719 |
| pGEX Reverse (GST 3, pGEX 3') | CCGGGAGCTGCATGTGTCAGAGG | 1720 |
| BGH Reverse | AACTAGAAGGCACAGTCGAGGC | 1721 |
| GFP (C' terminal, CFP, YFP or BFP) | CACTCTCGGCATGGACGAGC | 1722 |
| GFP Reverse | TGGTGCAGATGAACTTCAGG | 1723 |
| GAG | GTTCGACCCCGCCTCGATCC | 1724 |
| GAG Reverse | TGACACACATTCCACAGGGTC | 1725 |
| CYC1 Reverse | GCGTGAATGTAAGCGTGAC | 1726 |
| pFastBacF | 5'-d(GGATTATTCATACCGTCCCA)-3' | 1727 |
| pFastBacR | 5'-d(CAAATGTGGTATGGCTGATT)-3' | 1728 |
| pBAD Forward | 5'-d(ATGCCATAGCATTTTTATCC)-3' | 1729 |
| pBAD Reverse | 5'-d(GATTTAATCTGTATCAGG)-3' | 1730 |
| CMV-Forward | 5'-d(CGCAAATGGGCGGTAGGCGTG)-3' | 1731 |

### Barcodes

As described herein, certain embodiments contemplate designing oligonucleotide sequences to contain short signature sequences that permit unambiguous identification of the polynucleotide sequence into which they are incorporated, and hence of at least one primer responsible for amplifying that product, without having to sequence the entire amplification product. In the herein described oligonucleotides, such barcodes B (*e.g.,* B1, B2) are each either nothing or each comprise an oligonucleotide B that comprises an oligonucleotide barcode sequence of 3, 4, 5, 6, 7, 8, 9, 10, 11, 12, 13, 14, 15, 16, 17, 18, 19, 20, 25, 30, 35, 40, 45, 50 or more contiguous nucleotides (including all integer values therebetween), wherein in each of the plurality of oligonucleotide sequences B comprises a unique oligonucleotide sequence which uniquely identifies a particular V and/or J oligonucleotide primer sequence.

Exemplary barcodes may comprise a first barcode oligonucleotide of 5, 6, 7, 8, 9, 10, 11, 12, 13, 14, 15 or 16 nucleotides that uniquely identifies each oligonucleotide primer (*e.g.,* a V or a J primer) in the primer composition, and optionally in certain embodiments a second barcode oligonucleotide of 5, 6, 7, 8, 9, 10, 11, 12, 13, 14, 15 or 16 nucleotides that uniquely identifies each partner primer in a primer set (*e.g.,* a J or a V primer), to provide barcodes of, respectively, 5, 6, 7, 8, 9, 10, 11, 12, 13, 14, 15, 16, 17, 18, 19, 20, 21, 22, 23, 24, 25, 26, 27, 28, 29, 30, 31 or 32 nucleotides in length, but these and related embodiments are not intended to be so limited. Barcode oligonucleotides may comprise oligonucleotide sequences of any length, so long as a minimum barcode length is obtained that precludes occurrence of a given barcode sequence in two or more product polynucleotides having otherwise distinct sequences (*e.g.*, V and J sequences).

Thus, the minimum barcode length, to avoid such redundancy amongst the barcodes that are used to uniquely identify different V-J sequence pairings, is X nucleotides, where $4^x$ is greater than the number of distinct template species that are to be differentiated on the basis of having non-identical sequences. In practice, barcode oligonucleotide sequence read lengths may be limited only by the sequence read-length limits of the nucleotide sequencing instrument to be employed. For certain embodiments, different barcode oligonucleotides that will distinguish individual species of template oligonucleotides should have at least two nucleotide mismatches (*e.g.*, a minimum hamming distance of 2) when aligned to maximize the number of nucleotides that match at particular positions in the barcode oligonucleotide sequences.

The skilled artisan will be familiar with the design, synthesis, and incorporation into a larger oligonucleotide or polynucleotide construct, of oligonucleotide barcode sequences of, for instance, at least 3, 4, 5, 6, 7, 8, 9, 10, 11, 12, 13, 14, 15, 16, 17, 18, 19, 20, 21, 22, 23, 24, 25, 30, 35 or more contiguous nucleotides, including all integer values therebetween. For non-limiting examples of the design and implementation of oligonucleotide barcode sequence identification strategies, see, *e.g.,* de Career et al., 2011 Adv. Env. Microbiol. 77:6310; Parameswaran et al., 2007 Nucl. Ac. Res. 35(19):330; Roh et al., 2010 Trends Biotechnol. 28:291.

Typically, barcodes are placed in oligonucleotides at locations where they are not found naturally, **i.e.,** barcodes comprise nucleotide sequences that are distinct from any naturally occurring oligonucleotide sequences that may be found in the vicinity of the sequences adjacent to which the barcodes are situated (*e.g.*, V and/or J sequences). Such barcode sequences may be included, according to certain embodiments described herein, as elements B1 and/or B2 of the presently disclosed oligonucleotides. Accordingly, certain of the herein described oligonucleotide compositions may in certain embodiments comprise one, two or more barcodes, while in certain other embodiments some or all of these barcodes may be absent. In certain embodiments all barcode sequences will have identical or similar GC content (*e.g.*, differing in GC content by no more than 20%, or by no more than 19, 18, 17, 16, 15, 14, 13, 12, 11 or 10%).

**Sequencing**

Sequencing may be performed using any of a variety of available high throughput single molecule sequencing machines and systems. Illustrative sequence systems include sequence-by-synthesis systems such as the Illumina Genome Analyzer and associated instruments (Illumina, Inc., San Diego, CA), Helicos Genetic Analysis System (Helicos BioSciences Corp., Cambridge, MA), Pacific Biosciences PacBio *RS* (Pacific Biosciences, Menlo Park, CA), or other systems having similar capabilities. Sequencing is achieved using a set of sequencing oligonucleotides that hybridize to a defined region within the amplified DNA molecules. The sequencing oligonucleotides are designed such that the V- and J-encoding gene segments can be uniquely identified by the sequences that are generated, based on the present disclosure and in view of known adaptive immune receptor gene sequences that appear in publicly available databases. See, *e.g.,* US201070330571; WO2011106738; or WO2012027503. Exemplary TCRB J-region sequencing primers are set forth in Table 4:

*Table 4: TCRBJ Sequencing Primers*

| PRIMER | SEQUENCE | SEQ ID NO: |
|--------|----------|------------|
| >Jseq1-1 | ACAACTGTGAGTCTGGTGCCTTGTCCAAAGAAA | 1696 |
| >Jseq1-2 | ACAACGGTTAACCTGGTCCCCGAACCGAAGGTG | 1697 |
| >Jseq1-3 | ACAACAGTGAGCCAACTTCCCTCTCCAAAATAT | 1698 |
| >Jseq1-4 | AAGACAGAGAGCTGGGTTCCACTGCCAAAAAAC | 1699 |
| >Jseq1-5 | AGGATGGAGAGTCGAGTCCCATCACCAAAATGC | 1700 |
| >Jseq1-6 | GTCACAGTGAGCCTGGTCCCGTTCCCAAAGTGG | 1701 |
| >Jseq2-1 | AGCACGGTGAGCCGTGTCCCTGGCCCGAAGAAC | 1702 |
| >Jseq2-2 | AGTACGGTCAGCCTAGAGCCTTCTCCAAAAAAC | 1703 |
| >Jseq2-3 | AGCACTGTCAGCCGGGTGCCTGGGCCAAAATAC | 1704 |
| >Jseq2-4 | AGCACTGAGAGCCGGGTCCCGGCGCCGAAGTAC | 1705 |
| >Jseq2-5 | AGCACCAGGAGCCGCGTGCCTGGCCCGAAGTAC | 1706 |
| >Jseq2-6 | AGCACGGTCAGCCTGCTGCCGGCCCCGAAAGTC | 1707 |
| >Jseq2-7 | GTGACCGTGAGCCTGGTGCCCGGCCCGAAGTAC | 1708 |

The term "gene" means the segment of DNA involved in producing a polypeptide chain such as all or a portion of a TCR or Ig polypeptide *(e.g.,* a CDR3-containing polypeptide); it includes regions preceding and following the coding region "leader and trailer" as well as intervening sequences (introns) between individual coding segments (exons), and may also include regulatory elements (*e.g.*, promoters, enhancers, repressor binding sites and the like), and may also include recombination signal sequences (RSSs) as described herein.

The nucleic acids of the present embodiments, also referred to herein as polynucleotides, may be in the form of RNA or in the form of DNA, which DNA includes cDNA, genomic DNA, and synthetic DNA. The DNA may be double-stranded or single-stranded, and if single stranded may be the coding strand or non-coding (anti-sense) strand. A coding sequence which encodes a TCR or an immunoglobulin or a region thereof (*e.g.*, a V region, a D segment, a J region, a C region, etc.) for use according to the present embodiments may be identical to the coding sequence known in the art for any given TCR or immunoglobulin gene regions or polypeptide domains (*e.g.*, V-region domains, CDR3 domains, etc.), or may be a different coding sequence, which, as a result of the redundancy or degeneracy of the genetic code, encodes the same TCR or immunoglobulin region or polypeptide.

In certain embodiments, the amplified J-region encoding gene segments may each have a unique sequence-defined identifier tag of 2, 3, 4, 5, 6, 7, 8, 9, 10 or about 15, 20 or more nucleotides, situated at a defined position relative to a RSS site. For example, a four-base tag may be used, in the Jβ-region encoding segment of amplified TCRβ CDR3-encoding regions, at positions +11 through +14 downstream from the RSS site. However, these and related embodiments need not be so limited and also contemplate other relatively short nucleotide sequence-defined identifier tags that may be detected in J-region encoding gene segments and defined based on their positions relative to an RSS site. These may vary between different adaptive immune receptor encoding loci.

The recombination signal sequence (RSS) consists of two conserved sequences (heptamer, 5'-CACAGTG-3', and nonamer, 5'-ACAAAAACC-3'), separated by a spacer of either 12 +/- 1 bp ("12-signal") or 23 +/- 1 bp ("23-signal"). A number of nucleotide positions have been identified as important for recombination including the CA dinucleotide at position one and two of the heptamer, and a C at heptamer position three has also been shown to be strongly preferred as well as an A nucleotide at positions 5, 6, 7 of the nonamer. (Ramsden et. al 1994; Akamatsu et. al. 1994; Hesse et. al. 1989). Mutations of other nucleotides have minimal or inconsistent effects. The spacer, although more variable, also has an impact on recombination, and single-nucleotide replacements have been shown to significantly impact recombination efficiency (Fanning et. al. 1996, Larijani et. al 1999; Nadel et. al. 1998). Criteria have been described for identifying RSS polynucleotide sequences having significantly different recombination efficiencies (Ramsden et. al 1994; Akamatsu et. al. 1994; Hesse et. al. 1989 and Cowell et. al. 1994). Accordingly, the sequencing oligonucleotides may hybridize adjacent to a four base tag within the amplified J-encoding gene segments at positions +11 through +14 downstream of the RSS site. For example, sequencing oligonucleotides for TCRB may be designed to anneal to a consensus nucleotide motif observed just downstream of this "tag", so that the first four bases of a sequence read will uniquely identify the J-encoding gene segment (see, *e.g.*, WO/2012/027503).

The average length of the CDR3-encoding region, for the TCR, defined as the nucleotides encoding the TCR polypeptide between the second conserved cysteine of the V segment and the conserved phenylalanine of the J segment, is 35+/-3 nucleotides. Accordingly and in certain embodiments, PCR amplification using V-segment oligonucleotide primers with J-segment oligonucleotide primers that start from the J segment tag of a particular TCR or IgH J region (*e.g.*, TCR Jβ, TCR Jγ or IgH JH as described herein) will nearly always capture the complete V-D-J junction in a 50 base pair read. The average length of the IgH CDR3 region, defined as the nucleotides between the conserved cysteine in the V segment and the conserved phenylalanine in the J segment, is less constrained than at the TCRβ locus, but will typically be between about 10 and about 70 nucleotides. Accordingly and in certain embodiments, PCR amplification using V-segment oligonucleotide primers with J-segment oligonucleotide primers that start from the IgH J segment tag will capture the complete V-D-J junction in a 100 base pair read.

PCR primers that anneal to and support polynucleotide extension on mismatched template sequences are referred to as promiscuous primers. In certain embodiments, the TCR and Ig J-segment reverse PCR primers may be designed to minimize overlap with the sequencing oligonucleotides, in order to minimize promiscuous priming in the context of multiplex PCR. In one embodiment, the TCR and Ig J-segment reverse primers may be anchored at the 3' end by annealing to the consensus splice site motif, with minimal overlap of the sequencing primers. Generally, the TCR and Ig V and J-segment primers may be selected to operate in PCR at consistent annealing temperatures using known sequence/primer design and analysis programs under default parameters.

For the sequencing reaction, the exemplary IGHJ sequencing primers extend three nucleotides across the conserved CAG sequences as described in WO/2012/027503.

## Samples

The subject or biological source, from which a test biological sample may be obtained, may be a human or non-human animal, or a transgenic or cloned or tissue-engineered (including through the use of stem cells) organism. In certain preferred embodiments of the invention, the subject or biological source may be known to have, or may be suspected of having or being at risk for having, a circulating or solid tumor or other malignant condition, or an autoimmune disease, or an inflammatory condition, and in certain preferred embodiments of the invention the subject or biological source may be known to be free of a risk or presence of such disease.

Certain preferred embodiments contemplate a subject or biological source that is a human subject such as a patient that has been diagnosed as having or being at risk for developing or acquiring cancer according to art-accepted clinical diagnostic criteria, such as those of the U.S. National Cancer Institute (Bethesda, MD, USA) or as described in DeVita, Hellman, and Rosenberg's Cancer: Principles and Practice of Oncology (2008, Lippincott, Williams and Wilkins, Philadelphia/ Ovid, New York); Pizzo and Poplack, Principles and Practice of Pediatric Oncology (Fourth edition, 2001, Lippincott, Williams and Wilkins, Philadelphia/ Ovid, New York); and Vogelstein and Kinzler, The Genetic Basis of Human Cancer (Second edition, 2002, McGraw Hill Professional, New York); certain embodiments contemplate a human subject that is known to be free of a risk for having, developing or acquiring cancer by such criteria.

Certain other embodiments contemplate a non-human subject or biological source, for example a non-human primate such as a macaque, chimpanzee, gorilla, vervet, orangutan, baboon or other non-human primate, including such non-human subjects that may be known to the art as preclinical models, including preclinical models for solid tumors and/or other cancers. Certain other embodiments contemplate a non-human subject that is a mammal, for example, a mouse, rat, rabbit, pig, sheep, horse, bovine, goat, gerbil, hamster, guinea pig or other mammal; many such mammals may be subjects that are known to the art as preclinical models for certain diseases or disorders, including circulating or solid tumors and/or other cancers (*e.g.,* Talmadge et al., 2007 Am. J. Pathol. 170:793; Kerbel, 2003 Canc. Biol. Therap. 2(4 Suppl 1):S134; Man et al., 2007 Canc. Met. Rev. 26:737; Cespedes et al., 2006 Clin. Transl. Oncol. 8:318). The range of embodiments is not intended to be so limited, however, such that there are also contemplated other embodiments in which the subject or biological source may be a non-mammalian vertebrate, for example, another higher vertebrate, or an avian, amphibian or reptilian species, or another subject or biological source.

Biological samples may be provided by obtaining a blood sample, biopsy specimen, tissue explant, organ culture, biological fluid or any other tissue or cell preparation from a subject or a biological source. Preferably the sample comprises DNA from lymphoid cells of the subject or biological source, which, by way of illustration and not limitation, may contain rearranged DNA at one or more TCR or BCR loci. In certain embodiments a test biological sample may be obtained from a solid tissue *(e.g.,* a solid tumor), for example by surgical resection, needle biopsy or other means for obtaining a test biological sample that contains a mixture of cells.

According to certain embodiments it may be desirable to isolate lymphoid cells *(e.g.,* T cells and/or B cells) according to any of a large number of established methodologies, where isolated lymphoid cells are those that have been removed or separated from the tissue, environment or milieu in which they naturally occur. B cells and T cells can thus be obtained from a biological sample, such as from a variety of tissue and biological fluid samples including bone marrow, thymus, lymph glands, lymph nodes, peripheral tissues and blood, but peripheral blood is most easily accessed. Any peripheral tissue can be sampled for the presence of B and T cells and is therefore contemplated for use in the methods described herein. Tissues and biological fluids from which adaptive immune cells, may be obtained include, but are not limited to skin, epithelial tissues, colon, spleen, a mucosal secretion, oral mucosa, intestinal mucosa, vaginal mucosa or a vaginal secretion, cervical tissue, ganglia, saliva, cerebrospinal fluid (CSF), bone marrow, cord blood, serum, serosal fluid, plasma, lymph, urine, ascites fluid, pleural fluid, pericardial fluid, peritoneal fluid, abdominal fluid, culture medium, conditioned culture medium or lavage fluid. In certain embodiments, adaptive immune cells may be isolated from an apheresis sample. Peripheral blood samples may be obtained by phlebotomy from subjects. Peripheral blood mononuclear cells (PBMC) are isolated by techniques known to those of skill in the art, *e.g.,* by Ficoll-Hypaque® density gradient separation. In certain embodiments, whole PBMCs are used for analysis.

For nucleic acid extraction, total genomic DNA may be extracted from cells using methods known in the art and/or commercially available kits, *e.g.,* by using the QIAamp® DNA blood Mini Kit (QIAGEN®). The approximate mass of a single haploid genome is 3 pg. Preferably, at least 100,000 to 200,000 cells are used for analysis, *i.e.,* about 0.6 to 1.2 μg DNA from diploid T or B cells. Using PBMCs as a source, the number of T cells can be estimated to be about 30% of total cells. The number of B cells can also be estimated to be about 30% of total cells in a PBMC preparation.

The Ig and TCR gene loci contain many different variable (V), diversity (D), and joining (J) gene segments, which are subjected to rearrangement processes during early lymphoid differentiation. Ig and TCR V, D and J gene segment sequences are known in the art and are available in public databases such as GENBANK. The V-D-J rearrangements are mediated via a recombinase enzyme complex in which the RAG1 and RAG2 proteins play a key role by recognizing and cutting the DNA at the recombination signal sequences (RSS), which are located downstream of the V gene segments, at both sides of the D gene segments, and upstream of the J gene segments. Inappropriate RSS reduce or even completely prevent rearrangement. The recombination signal sequence (RSS) consists of two conserved sequences (heptamer, 5'-CACAGTG-3', and nonamer, 5'-ACAAAAACC-3'), separated by a spacer of either 12 +/- 1 bp ("12-signal") or 23 +/- 1 bp ("23-signal").

A number of nucleotide positions have been identified as important for recombination including the CA dinucleotide at position one and two of the heptamer, and a C at heptamer position three has also been shown to be strongly preferred as well as an A nucleotide at positions 5, 6, 7 of the nonamer. (Ramsden et al. 1994 Nucl. Ac. Res. 22:1785;

Akamatsu et. al. 1994 J. Immunol. 153:4520; Hesse et. al. 1989 Genes Dev. 3:1053). Mutations of other nucleotides have minimal or inconsistent effects. The spacer, although more variable, also has an impact on recombination, and single-nucleotide replacements have been shown to significantly impact recombination efficiency (Fanning et. al. 1996 Cell. Immunol. Immunopath. 79:1, Larijani et al. 1999 Nucl. Ac. Res. 27:2304; Nadel et al. 1998 J. Immunol. 161:6068; Nadel et al., 1998 J. Exp. Med. 187:1495). Criteria have been described for identifying RSS polynucleotide sequences having significantly different recombination efficiencies (Ramsden et al 1994 Nucl. Ac. Res. 22:1785; Akamatsu et. al. 1994 J. Immunol. 153:4520; Hesse et al. 1989 Genes Dev. 3:1053, and Lee et al., 2003 PLoS 1(1):E1).

The rearrangement process generally starts with a D to J rearrangement followed by a V to D-J rearrangement in the case of Ig heavy chain (IgH), TCR beta (TCRB), and TCR delta (TCRD) genes or concerns direct V to J rearrangements in case of Ig kappa (IgK), Ig lambda (IgL), TCR alpha (TCRA), and TCR gamma (TCRG) genes. The sequences between rearranging gene segments are generally deleted in the form of a circular excision product, also called TCR excision circle (TREC) or B cell receptor excision circle (BREC).

The many different combinations of V, D, and J gene segments represent the so-called combinatorial repertoire, which is estimated to be ~$2 \times 10^6$ for Ig molecules, ~$3 \times 10^6$ for TCR$\alpha\beta$ and ~$5 \times 10^3$ for TCR$\gamma\delta$ molecules. At the junction sites of the V, D, and J gene segments, deletion and random insertion of nucleotides occurs during the rearrangement process, resulting in highly diverse junctional regions, which significantly contribute to the total repertoire of Ig and TCR molecules, estimated to be > $10^{12}$.

Mature B-lymphocytes further extend their Ig repertoire upon antigen recognition in follicle centers via somatic hypermutation, a process, leading to affinity maturation of the Ig molecules. The somatic hypermutation process focuses on the V- (D-) J exon of IgH and Ig light chain genes and concerns single nucleotide mutations and sometimes also insertions or deletions of nucleotides. Somatically-mutated Ig genes are also found in mature B-cell malignancies of follicular or post-follicular origin.

In certain embodiments described herein, V-segment and J-segment primers may be employed in a PCR reaction to amplify rearranged TCR or BCR CDR3-encoding DNA regions in a test biological sample, wherein each functional TCR or Ig V-encoding gene segment comprises a V gene recombination signal sequence (RSS) and each functional TCR or Ig J-encoding gene segment comprises a J gene RSS. In these and related embodiments, each amplified rearranged DNA molecule may comprise (i) at least about 10, 20, 30, 40, 50, 60, 70, 80, 90, 100, 200, 300, 400, 500, 600, 700, 800, 900, 1000 (including all integer values therebetween) or more contiguous nucleotides of a sense strand of the TCR or Ig V-encoding gene segment, with the at least about 10, 20, 30, 40, 50, 60, 70, 80, 90, 100, 200, 300, 400, 500, 600, 700, 800, 900, 1000 or more contiguous nucleotides being situated 5' to the V gene RSS and/or each amplified rearranged DNA molecule may comprise (ii) at least about 10, 20, 30, 40, 50, 60, 70, 80, 90, 100, 200, 300, 400, 500 (including all integer values therebetween) or more contiguous nucleotides of a sense strand of the TCR or Ig J-encoding gene segment, with the at least about 10, 20, 30, 40, 50, 60, 70, 80, 90, 100, 200, 300, 400, 500 or more contiguous nucleotides being situated 3' to the J gene RSS.

The practice of certain embodiments of the present invention will employ, unless indicated specifically to the contrary, conventional methods in microbiology, molecular biology, biochemistry, molecular genetics, cell biology, virology and immunology techniques that are within the skill of the art, and reference to several of which is made below for the purpose of illustration. Such techniques are explained fully in the literature. See, *e.g.*, Sambrook, et al., Molecular Cloning: A Laboratory Manual (3rd Edition, 2001); Sambrook, et al., Molecular Cloning: A Laboratory Manual (2nd Edition, 1989); Maniatis et al., Molecular Cloning: A Laboratory Manual (1982); Ausubel et al., Current Protocols in Molecular Biology (John Wiley and Sons, updated July 2008); Short Protocols in Molecular Biology: A Compendium of Methods from Current Protocols in Molecular Biology, Greene Pub. Associates and Wiley-Interscience; Glover, DNA Cloning: A Practical Approach, vol. I & II (IRL Press, Oxford Univ. Press USA, 1985); Current Protocols in Immunology (Edited by: John E. Coligan, Ada M. Kruisbeek, David H. Margulies, Ethan M. Shevach, Warren Strober 2001 John Wiley & Sons, NY, NY); Real-Time PCR: Current Technology and Applications, Edited by Julie Logan, Kirstin Edwards and Nick Saunders, 2009, Caister Academic Press, Norfolk, UK; Anand, Techniques for the Analysis of Complex Genomes, (Academic Press, New York, 1992); Guthrie and Fink, Guide to Yeast Genetics and Molecular Biology (Academic Press, New York, 1991); Oligonucleotide Synthesis (N. Gait, Ed., 1984); Nucleic Acid Hybridization (B. Hames & S. Higgins, Eds., 1985); Transcription and Translation (B. Hames & S. Higgins, Eds., 1984); Animal Cell Culture (R. Freshney, Ed., 1986); Perbal, A Practical Guide to Molecular Cloning (1984); Next-Generation Genome Sequencing (Janitz, 2008 Wiley-VCH); PCR Protocols (Methods in Molecular Biology) (Park, Ed., 3rd Edition, 2010 Humana Press); Immobilized Cells And Enzymes (IRL Press, 1986); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Harlow and Lane, Antibodies, (Cold Spring Harbor Laboratory Press, Cold Spring Harbor, N.Y., 1998); Immunochemical Methods In Cell And Molecular Biology (Mayer and Walker, eds., Academic Press, London, 1987); Handbook Of Experimental Immunology, Volumes I-IV (D. M. Weir and CC Blackwell, eds., 1986); Riott, Essential Immunology, 6th Edition, (Blackwell Scientific Publications, Oxford, 1988); Embryonic Stem Cells: Methods and Protocols (Methods in Molecular Biology) (Kurstad Turksen, Ed., 2002); Embryonic Stem Cell Protocols: Volume I: Isolation and Characterization (Methods in Molecular Biology) (Kurstad Turksen, Ed., 2006); Embryonic Stem Cell Protocols: Volume II: Differentiation Models (Methods in Molecular Biology) (Kurstad Turksen, Ed., 2006); Human Embryonic Stem Cell Protocols (Methods in Molecular Biology) (Kursad Turksen Ed., 2006); Mesenchymal Stem Cells: Methods and Protocols (Methods in Molecular Biology) (Darwin J. Prockop, Donald G. Phinney, and Bruce A. Bunnell Eds., 2008); Hematopoietic Stem Cell Protocols (Methods in Molecular Medicine) (Christopher A. Klug, and Craig T. Jordan Eds., 2001); Hematopoietic Stem Cell Protocols (Methods in Molecular Biology) (Kevin D. Bunting Ed., 2008) Neural Stem Cells: Methods and Protocols (Methods in Molecular Biology) (Leslie P. Weiner Ed., 2008).

Unless specific definitions are provided, the nomenclature utilized in connection with, and the laboratory procedures and techniques of, molecular biology, analytical chemistry, synthetic organic chemistry, and medicinal and pharmaceutical chemistry described herein are those well known and commonly used in the art. Standard techniques may be used for recombinant technology, molecular biological, microbiological, chemical syntheses, chemical analyses, pharmaceutical preparation, formulation, and delivery, and treatment of patients.

The term "isolated" means that the material is removed from its original environment (e.g., the natural environment if it is naturally occurring). For example, a naturally occurring tissue, cell, nucleic acid or polypeptide present in its original milieu in a living animal is not isolated, but the same tissue, cell, nucleic acid or polypeptide, separated from some or all of the co-existing materials in the natural system, is isolated. Such nucleic acid could be part of a vector and/or such nucleic acid or polypeptide could be part of a composition (e.g., a cell lysate), and still be isolated in that such vector or composition is not part of the natural environment for the nucleic acid or polypeptide. The term "gene" means the segment of DNA involved in producing a polypeptide chain; it includes regions preceding and following the coding region "leader and trailer" as well as intervening sequences (introns) between individual coding segments (exons).

Unless the context requires otherwise, throughout the present specification and claims, the word "comprise" and variations thereof, such as, "comprises" and "comprising" are to be construed in an open, inclusive sense, that is, as "including, but not limited to". By "consisting of' is meant including, and typically limited to, whatever follows the phrase "consisting of." By "consisting essentially of' is meant including any elements listed after the phrase, and limited to other elements that do not interfere with or contribute to the activity or action specified in the disclosure for the listed elements. Thus, the phrase "consisting essentially of' indicates that the listed elements are required or mandatory, but that no other elements are required and may or may not be present depending upon whether or not they affect the activity or action of the listed elements.

In this specification and the appended claims, the singular forms "a," "an" and "the" include plural references unless the content clearly dictates otherwise. As used herein, in particular embodiments, the terms "about" or "approximately" when preceding a numerical value indicates the value plus or minus a range of 5%, 6%, 7%, 8% or 9%. In other embodiments, the terms "about" or "approximately" when preceding a numerical value indicates the value plus or minus a range of 10%, 11%, 12%, 13% or 14%. In yet other embodiments, the terms "about" or "approximately" when preceding a numerical value indicates the value plus or minus a range of 15%, 16%, 17%, 18%, 19% or 20%.

## EXAMPLES

### Example 1: Single Molecule Labeling.

The single molecule labeling process used a Polymerase Chain Reaction approach to tag adaptive immune receptor encoding sequences with a unique barcode and a universal primer. The PCR reaction to tag the individual barcodes used QIAGEN Multiplex PCR master mix (QIAGEN part number 206145, Qiagen, Valencia, CA), 10% Q-solution (QIAGEN), and 300 ng of template DNA. The pooled primers were added so the final reaction had an aggregate forward primer concentration of 2 uM and an aggregate reverse primer concentration of 2 uM. The forward primers were composed of nucleotide sequence portions that annealed to V genes (segments that annealed to the V genes are shown in Table 2) and at the 5' end a universal primer (pGEX f, Table 3). The aggregate primer is listed in Table 6. These primers may, for greater specificity, have a random nucleotide insertion between the 3' end of the V primer and the 5' end of the universal primer sequence. The reverse primers have a section of nucleotides that can anneal to the J gene region (Table 2), on the 5' end of the J primer an 8 bp barcode composed of random nucleotides, and on the 5' end of the 8 bp random barcode a universal primer (pGEXr, Table 3). An example of these primers is listed in Table 5. The 8 bp barcode made of random nucleotides may be shorter or longer, additional basepairs increase the number of unique barcodes.

The nucleotide tags were incorporated onto the molecules in a 7 cycle PCR reaction. The thermocycle conditions were: 95° C for 5 minutes, followed by 7 cycles of 95° for 30 sec, 68° for 90 sec, and 72° for 30 sec. Following cycling, the rxn is held for 10 minutes at 72°.

Once the antigen receptor molecules were tagged by the primers carrying a random 8 bp tag, any remaining primers were destroyed using ExoSAP-IT (Product # 78200, Affymetrix, Santa Clara, CA). ExoSAP-IT is a product from Affymetrix that uses Exonuclease I and Shrimp Alkaline Phosphatase activities; the Exonuclease I destroys single stranded DNA and SAP degrades dNTPs. For this example, 10 ul of PCR reagents and 4 ul of exoSAP-IT were used. The reaction was incubated for 15 minutes at 37°C and the ExoSAP-it was inactivated by a 15 minute incubation at 80°C. At this point, the molecules were uniquely tagged with a barcode and a universal primer. To amplify the tagged products, another PCR reaction was performed with the universal pGEX primers. This reaction used QIAGEN Multiplex PCR master mix (QIAGEN part number 206145, Qiagen, Valencia, CA), 10% Q- solution (QIAGEN), and 6 ul of cleaned PCR reaction as template. The forward universal (pGEXf) primer was added to the mix so the final concentration was 2 uM and the reverse universal primer (pgEXr) was added to the reaction so its final concentration was 2uM. To sequence these molecules, an Illumina adapter was incorporated using the pGEX primers. The reaction conditions were the same as above, except that the primers were replaced with the tailing primers (Table 7 below (SEQ ID NOs: 5686-5877). The Illumina adapters, which also included an 8 bp tag and a 6 bp random set of nucleotides, were incorporated onto the molecules in a 7 cycle PCR reaction. The thermocycle conditions were: 95° C for 5 minutes, followed by 7 cycles of 95° for 30 sec, 68° for 90 sec, and 72° for 30 sec. Following cycling, the reaction was held for 10 minutes at 72°.

Once the labeled molecules were "tailed" with Illumina adaptors, they were amenable to sequencing. For this example, sequencing was conducted through the 8 bp randomer into the adaptive immune receptor encoding sequence on an Illumina HISEQ™ sequencing platform. The sequenced molecules included an 8 bp random tag. Every sequenced molecule having identical CDR3 and 8 bp random tag sequences was amplified from the adaptive immune receptor encoding polynucleotide sequences of a single cell.

Table 5 shows the J primers for the single molecule sequencing (reverse primers) and Table 6 shows the V primers (forward primers). The PCR protocol is short: 1st PCR (5 cycles) with the above primers to uniquely tag each molecule, followed by a second PCR (35 cycles) with a universal primer (PGEX) to amplify the molecules. These reactions are followed by a PCR reaction to tail on the Illumina adapters.

***Table 5***

| J Primer Name | Bases | SEQ ID NO: | Sequence |
|---|---|---|---|
| pGEXr_TCRBJ1-1 _vD12 | 60 | 5613 | CCG GGA GCT GCA TGT GTC AGA GGN NNN NNN NGT CTT ACC TAC AAC TGT GAG TCT GGT GCC |
| pGEXr_TCRBJ1-2 _vD12 | 59 | 5614 | CCG GGA GCT GCA TGT GTC AGA GGN NNN NNN NCC TTA CCT ACA ACG GTT AAC CTG GTC CC |
| pGEXr_TCRBJ1-3 _vD12 | 62 | 5615 | CCG GGA GCT GCA TGT GTC AGA GGN NNN NNN NCT TAC TCA CCT ACA ACA GTG AGC CAA CTT CC |
| pGEXr_TCRBJ1-4 _vD12 | 57 | 5616 | CCG GGA GCT GCA TGT GTC AGA GGN NNN NNN NAT ACC CAA GAC AGA GAG CTG GGT TCC |

(continued)

| J Primer Name | Bases | SEQ ID NO: | Sequence |
|---|---|---|---|
| pGEXr_TCRBJ1-5 _vD12 | 60 | 5617 | CCG GGA GCT GCA TGT GTC AGA GGN NNN NNN NAA CTT ACC TAG GAT GGA GAG TCG AGT CCC |
| pGEXr_TCRBJ1-6 _vD12 | 53 | 5618 | CCG GGA GCT GCA TGT GTC AGA GGN NNN NNN NCT GTC ACA GTG AGC CTG GTC CC |
| pGEXr_TCRBJ2-1 _vD12 | 49 | 5619 | CCG GGA GCT GCA TGT GTC AGA GGN NNN NNN NCA CGG TGA GCC GTG TCC C |
| pGEXr_TCRBJ2-2 _vD12 | 53 | 5620 | CCG GGA GCT GCA TGT GTC AGA GGN NNN NNN NCC AGT ACG GTC AGC CTA GAG CC |
| pGEXr_TCRBJ2-3 _vD12 | 49 | 5621 | CCG GGA GCT GCA TGT GTC AGA GGN NNN NNN NCA CTG TCA GCC GGG TGC C |
| pGEXr_TCRBJ2-4 _vD12 | 49 | 5622 | CCG GGA GCT GCA TGT GTC AGA GGN NNN NNN NCA CTG AGA GCC GGG TCC C |
| pGEXr_TCRBJ2-5 _vD12 | 48 | 5623 | CCG GGA GCT GCA TGT GTC AGA GGN NNN NNN NAC CAG GAG CCG CGT GCC |
| pGEXr_TCRBJ2-6 _vD12 | 49 | 5624 | CCG GGA GCT GCA TGT GTC AGA GGN NNN NNN NCA CGG TCA GCC TGC TGC C |
| pGEXr_TCRBJ2-7 _vD12 | 49 | 5625 | CCG GGA GCT GCA TGT GTC AGA GGN NNN NNN NGA CCG TGA GCC TGG TGC C |

*Table 6*

| Primer Name | SEQ ID NO: | Sequence |
|---|---|---|
| pGEXf_TCRB V01_verD10 | 5626 | GGGCTGGCAAGCCACGTTTGGTGGAATGCCCTGAC AGCTCTCGCTTATA |
| pGEXf_TCRB V02_verD10 | 5627 | GGGCTGGCAAGCCACGTTTGGTGCTCAGAGAAGTC TGAAATATTCGATGATCAATTCTCAGTTG |
| pGEXf_TCRB V03-1_verD10 | 5628 | GGGCTGGCAAGCCACGTTTGGTGCCAAATCGMTTC TCACCTAAATCTCCAGACAAAG |
| pGEXf_TCRB V03-2_verD10 | 5629 | GGGCTGGCAAGCCACGTTTGGTGCACCTGACTCTCC AGACAAAGCTCAT |
| pGEXf_TCRB V04-1/2/3_verD10 | 5630 | GGGCTGGCAAGCCACGTTTGGTGCCTGAATGCCCCA ACAGCTCTC |
| pGEXf_TCRB V05-1_verD10 | 5631 | GGGCTGGCAAGCCACGTTTGGTGGATTCTCAGGGC GCCAGTTCTCTA |
| pGEXf_TCRB V05-2_verD10 | 5632 | GGGCTGGCAAGCCACGTTTGGTGCCTAATTGATTCT CAGCTCACCACGTCCATA |
| pGEXf_TCRB V05-3_verD10 | 5633 | GGGCTGGCAAGCCACGTTTGGTGTCAGGGCGCCAG TTCCATG |
| pGEXf_TCRB V05-4_verD10 | 5634 | GGGCTGGCAAGCCACGTTTGGTGTCCTAGATTCTCA GGTCTCCAGTTCCCTA |
| pGEXf_TCRB V05-5_verD10 | 5635 | GGGCTGGCAAGCCACGTTTGGTGGAGGAAACTTCC CTGATCGATTCTCAGC |
| pGEXf_TCRB V05-6_verD10 | 5636 | GGGCTGGCAAGCCACGTTTGGTGCAACTTCCCTGAT CGATTCTCAGGTCA |
| pGEXf_TCRB V05-7_verD10 | 5637 | GGGCTGGCAAGCCACGTTTGGTGAGGAAACTTCCCT GATCAATTCTCAGGTCA |
| pGEXf_TCRB V05-8_verD10 | 5638 | GGGCTGGCAAGCCACGTTTGGTGGGAAACTTCCCTC CTAGATTTTCAGGTCG |
| pGEXf_TCRB V06-1_verD10 | 5639 | GGGCTGGCAAGCCACGTTTGGTGCCCCAATGGCTAC AATGTCTCCAGATT |
| pGEXf_TCRB V06-2/3_verD10 | 5640 | GGGCTGGCAAGCCACGTTTGGTGGGAGAGGTCCCT GATGGCTACAA |

(continued)

| Primer Name | SEQ ID NO: | Sequence |
|---|---|---|
| pGEXf_TCRB V06-4_verD10 | 5641 | GGGCTGGCAAGCCACGTTTGGTGTCCCTGATGGTTATAGTGTCTCCAGAGC |
| pGEXf_TCRB V06-5_verD10 | 5642 | GGGCTGGCAAGCCACGTTTGGTGGGAGAAGTCCCCAATGGCTACAATGTC |
| pGEXf_TCRB V06-6_verD10 | 5643 | GGGCTGGCAAGCCACGTTTGGTGAAAGGAGAAGTCCCGAATGGCTACAA |
| pGEXf_TCRB V06-7_verD10 | 5644 | GGGCTGGCAAGCCACGTTTGGTGGTTCCCAATGGCTACAATGTCTCCAGATC |
| pGEXf_TCRB V06-8_verD10 | 5645 | GGGCTGGCAAGCCACGTTTGGTGGAAGTCCCCAATGGCTACAATGTCTCTAGATT |
| pGEXf_TCRB V06-9_verD10 | 5646 | GGGCTGGCAAGCCACGTTTGGTGGAGAAGTCCCCGATGGCTACAATGTA |
| pGEXf_TCRB V07-1_verD10 | 5647 | GGGCTGGCAAGCCACGTTTGGTGGTGATCGGTTCTCTGCACAGAGGT |
| pGEXf_TCRB V07-2_verD10 | 5648 | GGGCTGGCAAGCCACGTTTGGTGCGCTTCTCTGCAGAGAGGACTGG |
| pGEXf_TCRB V07-3_verD10 | 5649 | GGGCTGGCAAGCCACGTTTGGTGGGTTCTTTGCAGTCAGGCCTGA |
| pGEXf_TCRB V07-4_verD10 | 5650 | GGGCTGGCAAGCCACGTTTGGTGCAGTGGTCGGTTCTCTGCAGAG |
| pGEXf_TCRB V07-5_verD10 | 5651 | GGGCTGGCAAGCCACGTTTGGTGGCTCAGTGATCAATTCTCCACAGAGAGGT |
| pGEXf_TCRB V07-6/7_verD10 | 5652 | GGGCTGGCAAGCCACGTTTGGTGTTCTCTGCAGAGAGGCCTGAGG |
| pGEXf_TCRB V07-8_verD10 | 5653 | GGGCTGGCAAGCCACGTTTGGTGCCCAGTGATCGCTTCTTTGCAGAAA |
| pGEXf_TCRB V07-9_verD10 | 5654 | GGGCTGGCAAGCCACGTTTGGTGCTGCAGAGAGGCCTAAGGGATCT |
| pGEXf_TCRB V08-1_verD10 | 5655 | GGGCTGGCAAGCCACGTTTGGTGGAAGGGTACAATGTCTCTGGAAACAAACTCAAG |

(continued)

| Primer Name | SEQ ID NO: | Sequence |
|---|---|---|
| pGEXf_TCRB V08-2_verD10 | 5656 | GGGCTGGCAAGCCACGTTTGGTGGGGGTACTGTGTT TCTTGAAACAAGCTTGAG |
| pGEXf_TCRB V09_verD10 | 5657 | GGGCTGGCAAGCCACGTTTGGTGCAGTTCCCTGACT TGCACTCTGAACTAAAC |
| pGEXf_TCRB V10-1_verD10 | 5658 | GGGCTGGCAAGCCACGTTTGGTGACTAACAAAGGA GAAGTCTCAGATGGCTACAG |
| pGEXf_TCRB V10-2_verD10 | 5659 | GGGCTGGCAAGCCACGTTTGGTGAGATAAAGGAGA AGTCCCCGATGGCTA |
| pGEXf_TCRB V10-3_ _verD10 | 5660 | GGGCTGGCAAGCCACGTTTGGTGGATACTGACAAA GGAGAAGTCTCAGATGGCTATAG |
| pGEXf_TCRB V11-1/2/3_verD10 | 5661 | GGGCTGGCAAGCCACGTTTGGTGCTAAGGATCGATT TTCTGCAGAGAGGCTC |
| pGEXf_TCRB V12-1_verD10 | 5662 | GGGCTGGCAAGCCACGTTTGGTGTTGATTCTCAGCA CAGATGCCTGATGT |
| pGEXf_TCRB V12-2_verD10 | 5663 | GGGCTGGCAAGCCACGTTTGGTGATTCTCAGCTGAG AGGCCTGATGG |
| pGEXf_TCRB V12-3/4_verD10 | 5664 | GGGCTGGCAAGCCACGTTTGGTGGGATCGATTCTCA GCTAAGATGCCTAATGC |
| pGEXf_TCRB V12-5_verD10 | 5665 | GGGCTGGCAAGCCACGTTTGGTGCTCAGCAGAGAT GCCTGATGCAACTTTA |
| pGEXf_TCRB V13_verD10 | 5666 | GGGCTGGCAAGCCACGTTTGGTGCTGATCGATTCTC AGCTCAACAGTTCAGT |
| pGEXf_TCRB V14_verD10 | 5667 | GGGCTGGCAAGCCACGTTTGGTGTAGCTGAAAGGA CTGGAGGGACGTAT |
| pGEXf_TCRB V15_verD10 | 5668 | GGGCTGGCAAGCCACGTTTGGTGCCAGGAGGCCGA ACACTTCTTTCT |
| pGEXf_TCRB V16_ verD10 | 5669 | GGGCTGGCAAGCCACGTTTGGTGGCTAAGTGCCTCC CAAATTCACCCT |
| pGEXf_TCRB V17_verD10 | 5670 | GGGCTGGCAAGCCACGTTTGGTGCACAGCTGAAAG ACCTAACGGAACGT |

(continued)

| Primer Name | SEQ ID NO: | Sequence |
|---|---|---|
| pGEXf_TCRB V18_verD10 | 5671 | GGGCTGGCAAGCCACGTTTGGTGCTGCTGAATTTCCCAAAGAGGGCC |
| pGEXf_TCRB V19_verD10 | 5672 | GGGCTGGCAAGCCACGTTTGGTGAGGGTACAGCGTCTCTCGGG |
| pGEXf_TCRB V20_verD10 | 5673 | GGGCTGGCAAGCCACGTTTGGTGGCCTGACCTTGTCCACTCTGACA |
| pGEXf_TCRB V21_verD10 | 5674 | GGGCTGGCAAGCCACGTTTGGTGATGAGCGATTTTTAGCCCAATGCTCCA |
| pGEXf_TCRB V22_verD10 | 5675 | GGGCTGGCAAGCCACGTTTGGTGTGAAGGCTACGTGTCTGCCAAGAG |
| pGEXf_TCRB V23_verD10 | 5676 | GGGCTGGCAAGCCACGTTTGGTGCTCATCTCAATGCCCCAAGAACGC |
| pGEXf_TCRB V24_verD10 | 5677 | GGGCTGGCAAGCCACGTTTGGTGAGATCTCTGATGGATACAGTGTCTCTCGACA |
| pGEXf_TCRB V25_verD10 | 5678 | GGGCTGGCAAGCCACGTTTGGTGAGATCTTTCCTCTGAGTCAACAGTCTCCAGAATA |
| pGEXf_TCRB V26_verD10 | 5679 | GGGCTGGCAAGCCACGTTTGGTGCACTGAAAAAGGAGATATCTCTGAGGGGTATCATG |
| pGEXf_TCRB V27_verD10 | 5680 | GGGCTGGCAAGCCACGTTTGGTGGTTCCTGAAGGGTACAAAGTCTCTCGAAAAG |
| pGEXf_TCRB V28_verD10 | 5681 | GGGCTGGCAAGCCACGTTTGGTGCTGAGGGGTACAGTGTCTCTAGAGAGA |
| pGEXf_TCRB V29_verD10 | 5682 | GGGCTGGCAAGCCACGTTTGGTGAGCCGCCCAAACCTAACATTCTCAA |
| pGEXf_TCRB V30_verD10 | 5683 | GGGCTGGCAAGCCACGTTTGGTGCCCAGGACCGGCAGTTCA |
| pGEXf_TCRB VA_verD10 | 5684 | GGGCTGGCAAGCCACGTTTGGTGTTGATTAGAGACATATCCCTATTGAAAATATTTCCTGGCA |
| pGEXf_TCRB VB_verD10 | 5685 | GGGCTGGCAAGCCACGTTTGGTGAGATGCCCTGAGTCAGCATAGTCATTCTAAC |

**Table 7. Exemplary Tailing Primers**

| Sequence | SEQ ID NO: |
|---|---|
| AAT GAT ACG GCG ACC ACC GAG ATC TAC ACC GGT CTC GGC ATT CCT GCT GAA CCG CTC TTC CGA TCT NNN NNN CAA GGT CAC CGG GAG CTG CAT GTG TCA GAG G | 5686 |
| AAT GAT ACG GCG ACC ACC GAG ATC TAC ACC GGT CTC GGC ATT CCT GCT GAA CCG CTC TTC CGA TCT NNN NNN GCA TAA CTC CGG GAG CTG CAT GTG TCA GAG G | 5687 |
| AAT GAT ACG GCG ACC ACC GAG ATC TAC ACC GGT CTC GGC ATT CCT GCT GAA CCG CTC TTC CGA TCT NNN NNN CTC TGA TTC CGG GAG CTG CAT GTG TCA GAG G | 5688 |
| AAT GAT ACG GCG ACC ACC GAG ATC TAC ACC GGT CTC GGC ATT CCT GCT GAA CCG CTC TTC CGA TCT NNN NNN TAC GTA CGC CGG GAG CTG CAT GTG TCA GAG G | 5689 |
| AAT GAT ACG GCG ACC ACC GAG ATC TAC ACC GGT CTC GGC ATT CCT GCT GAA CCG CTC TTC CGA TCT NNN NNN TAC GCG TTC CGG GAG CTG CAT GTG TCA GAG G | 5690 |
| AAT GAT ACG GCG ACC ACC GAG ATC TAC ACC GGT CTC GGC ATT CCT GCT GAA CCG CTC TTC CGA TCT NNN NNN CTC AGT GAC CGG GAG CTG CAT GTG TCA GAG G | 5691 |
| AAT GAT ACG GCG ACC ACC GAG ATC TAC ACC GGT CTC GGC ATT CCT GCT GAA CCG CTC TTC CGA TCT NNN NNN TCT GAT ATC CGG GAG CTG CAT GTG TCA GAG G | 5692 |

(continued)

| Sequence | SEQ ID NO: |
| --- | --- |
| AAT GAT ACG GCG ACC ACC GAG ATC TAC ACC GGT CTC GGC ATT CCT GCT GAA CCG CTC TTC CGA TCT NNN NNN CAT ATG CTC CGG GAG CTG CAT GTG TCA GAG G | 5693 |
| AAT GAT ACG GCG ACC ACC GAG ATC TAC ACC GGT CTC GGC ATT CCT GCT GAA CCG CTC TTC CGA TCT NNN NNN CGT AAT TAC CGG GAG CTG CAT GTG TCA GAG G | 5694 |
| AAT GAT ACG GCG ACC ACC GAG ATC TAC ACC GGT CTC GGC ATT CCT GCT GAA CCG CTC TTC CGA TCT NNN NNN ACG TAC TCC CGG GAG CTG CAT GTG TCA GAG G | 5695 |
| AAT GAT ACG GCG ACC ACC GAG ATC TAC ACC GGT CTC GGC ATT CCT GCT GAA CCG CTC TTC CGA TCT NNN NNN CTT CTA AGC CGG GAG CTG CAT GTG TCA GAG G | 5696 |
| AAT GAT ACG GCG ACC ACC GAG ATC TAC ACC GGT CTC GGC ATT CCT GCT GAA CCG CTC TTC CGA TCT NNN NNN ACT ATG ACC CGG GAG CTG CAT GTG TCA GAG G | 5697 |
| AAT GAT ACG GCG ACC ACC GAG ATC TAC ACC GGT CTC GGC ATT CCT GCT GAA CCG CTC TTC CGA TCT NNN NNN GAC GTT AAC CGG GAG CTG CAT GTG TCA GAG G | 5698 |
| AAT GAT ACG GCG ACC ACC GAG ATC TAC ACC GGT CTC GGC ATT CCT GCT GAA CCG CTC TTC CGA TCT NNN NNN ACA AGA TAC CGG GAG CTG CAT GTG TCA GAG G | 5699 |

| Sequence | SEQ ID NO: |
|---|---|
| AAT GAT ACG GCG ACC ACC GAG ATC TAC ACC GGT CTC GGC ATT CCT GCT GAA CCG CTC TTC CGA TCT NNN NNN GAC TAA GAC CGG GAG CTG CAT GTG TCA GAG G | 5700 |
| AAT GAT ACG GCG ACC ACC GAG ATC TAC ACC GGT CTC GGC ATT CCT GCT GAA CCG CTC TTC CGA TCT NNN NNN GTG TCT ACC CGG GAG CTG CAT GTG TCA GAG G | 5701 |
| AAT GAT ACG GCG ACC ACC GAG ATC TAC ACC GGT CTC GGC ATT CCT GCT GAA CCG CTC TTC CGA TCT NNN NNN TTC ACT AGC CGG GAG CTG CAT GTG TCA GAG G | 5702 |
| AAT GAT ACG GCG ACC ACC GAG ATC TAC ACC GGT CTC GGC ATT CCT GCT GAA CCG CTC TTC CGA TCT NNN NNN AAT CGG ATC CGG GAG CTG CAT GTG TCA GAG G | 5703 |
| AAT GAT ACG GCG ACC ACC GAG ATC TAC ACC GGT CTC GGC ATT CCT GCT GAA CCG CTC TTC CGA TCT NNN NNN AGT ACC GAC CGG GAG CTG CAT GTG TCA GAG G | 5704 |
| AAT GAT ACG GCG ACC ACC GAG ATC TAC ACC GGT CTC GGC ATT CCT GCT GAA CCG CTC TTC CGA TCT NNN NNN TTG CCT CAC CGG GAG CTG CAT GTG TCA GAG G | 5705 |
| AAT GAT ACG GCG ACC ACC GAG ATC TAC ACC GGT CTC GGC ATT CCT GCT GAA CCG CTC TTC CGA TCT NNN NNN TCG TTA GCC CGG GAG CTG CAT GTG TCA GAG G | 5706 |

EP 2 971 105 B1

50

| Sequence | SEQ ID NO: |
|---|---|
| AAT GAT ACG GCG ACC ACC GAG ATC TAC ACC GGT CTC GGC ATT CCT GCT GAA CCG CTC TTC CGA TCT NNN NNN TAT AGT TCC CGG GAG CTG CAT GTG TCA GAG G | 5707 |
| AAT GAT ACG GCG ACC ACC GAG ATC TAC ACC GGT CTC GGC ATT CCT GCT GAA CCG CTC TTC CGA TCT NNN NNN TGG CGT ATC CGG GAG CTG CAT GTG TCA GAG G | 5708 |
| AAT GAT ACG GCG ACC ACC GAG ATC TAC ACC GGT CTC GGC ATT CCT GCT GAA CCG CTC TTC CGA TCT NNN NNN TGG ACA TGC CGG GAG CTG CAT GTG TCA GAG G | 5709 |
| AAT GAT ACG GCG ACC ACC GAG ATC TAC ACC GGT CTC GGC ATT CCT GCT GAA CCG CTC TTC CGA TCT NNN NNN AGG TTG CTC CGG GAG CTG CAT GTG TCA GAG G | 5710 |
| AAT GAT ACG GCG ACC ACC GAG ATC TAC ACC GGT CTC GGC ATT CCT GCT GAA CCG CTC TTC CGA TCT NNN NNN ATA TGC TGC CGG GAG CTG CAT GTG TCA GAG G | 5711 |
| AAT GAT ACG GCG ACC ACC GAG ATC TAC ACC GGT CTC GGC ATT CCT GCT GAA CCG CTC TTC CGA TCT NNN NNN GTA CAG TGC CGG GAG CTG CAT GTG TCA GAG G | 5712 |
| AAT GAT ACG GCG ACC ACC GAG ATC TAC ACC GGT CTC GGC ATT CCT GCT GAA CCG CTC TTC CGA TCT NNN NNN ATC CAT GGC CGG GAG CTG CAT GTG TCA GAG G | 5713 |

| Sequence | SEQ ID NO: |
|---|---|
| AAT GAT ACG GCG ACC ACC GAG ATC TAC ACC GGT CTC GGC ATT CCT GCT GAA CCG CTC TTC CGA TCT NNN NNN TGA TGC GAC CGG GAG CTG CAT GTG TCA GAG G | 5714 |
| AAT GAT ACG GCG ACC ACC GAG ATC TAC ACC GGT CTC GGC ATT CCT GCT GAA CCG CTC TTC CGA TCT NNN NNN GTA GCA GTC CGG GAG CTG CAT GTG TCA GAG G | 5715 |
| AAT GAT ACG GCG ACC ACC GAG ATC TAC ACC GGT CTC GGC ATT CCT GCT GAA CCG CTC TTC CGA TCT NNN NNN GGA TCA TCC CGG GAG CTG CAT GTG TCA GAG G | 5716 |
| AAT GAT ACG GCG ACC ACC GAG ATC TAC ACC GGT CTC GGC ATT CCT GCT GAA CCG CTC TTC CGA TCT NNN NNN GTG AAC GTC CGG GAG CTG CAT GTG TCA GAG G | 5717 |
| AAT GAT ACG GCG ACC ACC GAG ATC TAC ACC GGT CTC GGC ATT CCT GCT GAA CCG CTC TTC CGA TCT NNN NNN ATT AAG CGC CGG GAG CTG CAT GTG TCA GAG G | 5718 |
| AAT GAT ACG GCG ACC ACC GAG ATC TAC ACC GGT CTC GGC ATT CCT GCT GAA CCG CTC TTC CGA TCT NNN NNN TAT TGG CGC CGG GAG CTG CAT GTG TCA GAG G | 5719 |
| AAT GAT ACG GCG ACC ACC GAG ATC TAC ACC GGT CTC GGC ATT CCT GCT GAA CCG CTC TTC CGA TCT NNN NNN CGA TTA CAC CGG GAG CTG CAT GTG TCA GAG G | 5720 |

(continued)

| Sequence | SEQ ID NO: |
|---|---|
| AAT GAT ACG GCG ACC ACC GAG ATC TAC ACC GGT CTC GGC ATT CCT GCT GAA CCG CTC TTC CGA TCT NNN NNN TGT CAT CGC CGG GAG CTG CAT GTG TCA GAG G | 5721 |
| AAT GAT ACG GCG ACC ACC GAG ATC TAC ACC GGT CTC GGC ATT CCT GCT GAA CCG CTC TTC CGA TCT NNN NNN TAT CAA GTC CGG GAG CTG CAT GTG TCA GAG G | 5722 |
| AAT GAT ACG GCG ACC ACC GAG ATC TAC ACC GGT CTC GGC ATT CCT GCT GAA CCG CTC TTC CGA TCT NNN NNN AGG CTT GAC CGG GAG CTG CAT GTG TCA GAG G | 5723 |
| AAT GAT ACG GCG ACC ACC GAG ATC TAC ACC GGT CTC GGC ATT CCT GCT GAA CCG CTC TTC CGA TCT NNN NNN GAT AAC CAC CGG GAG CTG CAT GTG TCA GAG G | 5724 |
| AAT GAT ACG GCG ACC ACC GAG ATC TAC ACC GGT CTC GGC ATT CCT GCT GAA CCG CTC TTC CGA TCT NNN NNN AAT CCT GCC CGG GAG CTG CAT GTG TCA GAG G | 5725 |
| AAT GAT ACG GCG ACC ACC GAG ATC TAC ACC GGT CTC GGC ATT CCT GCT GAA CCG CTC TTC CGA TCT NNN NNN GTT ATA TCC CGG GAG CTG CAT GTG TCA GAG G | 5726 |
| AAT GAT ACG GCG ACC ACC GAG ATC TAC ACC GGT CTC GGC ATT CCT GCT GAA CCG CTC TTC CGA TCT NNN NNN ACA CAC GTC CGG GAG CTG CAT GTG TCA GAG G | 5727 |

| Sequence | SEQ ID NO: |
|---|---|
| AAT GAT ACG GCG ACC ACC GAG ATC TAC ACC GGT CTC GGC ATT CCT GCT GAA CCG CTC TTC CGA TCT NNN NNN ATA CGA CTC CGG GAG CTG CAT GTG TCA GAG G | 5728 |
| AAT GAT ACG GCG ACC ACC GAG ATC TAC ACC GGT CTC GGC ATT CCT GCT GAA CCG CTC TTC CGA TCT NNN NNN ATC TTC GTC CGG GAG CTG CAT GTG TCA GAG G | 5729 |
| AAT GAT ACG GCG ACC ACC GAG ATC TAC ACC GGT CTC GGC ATT CCT GCT GAA CCG CTC TTC CGA TCT NNN NNN ACA TGT ATC CGG GAG CTG CAT GTG TCA GAG G | 5730 |
| AAT GAT ACG GCG ACC ACC GAG ATC TAC ACC GGT CTC GGC ATT CCT GCT GAA CCG CTC TTC CGA TCT NNN NNN TCC ACA GTC CGG GAG CTG CAT GTG TCA GAG G | 5731 |
| AAT GAT ACG GCG ACC ACC GAG ATC TAC ACC GGT CTC GGC ATT CCT GCT GAA CCG CTC TTC CGA TCT NNN NNN CAG TCT GTC CGG GAG CTG CAT GTG TCA GAG G | 5732 |
| AAT GAT ACG GCG ACC ACC GAG ATC TAC ACC GGT CTC GGC ATT CCT GCT GAA CCG CTC TTC CGA TCT NNN NNN TCC ATG TGC CGG GAG CTG CAT GTG TCA GAG G | 5733 |
| AAT GAT ACG GCG ACC ACC GAG ATC TAC ACC GGT CTC GGC ATT CCT GCT GAA CCG CTC TTC CGA TCT NNN NNN TCA CTG CAC CGG GAG CTG CAT GTG TCA GAG G | 5734 |

(continued)

| Sequence | SEQ ID NO: |
|---|---|
| AAT GAT ACG GCG ACC ACC GAG ATC TAC ACC GGT CTC GGC ATT CCT GCT GAA CCG CTC TTC CGA TCT NNN NNN ATG GTC AAC CGG GAG CTG CAT GTG TCA GAG G | 5735 |
| AAT GAT ACG GCG ACC ACC GAG ATC TAC ACC GGT CTC GGC ATT CCT GCT GAA CCG CTC TTC CGA TCT NNN NNN CAA GTC ACC CGG GAG CTG CAT GTG TCA GAG G | 5736 |
| AAT GAT ACG GCG ACC ACC GAG ATC TAC ACC GGT CTC GGC ATT CCT GCT GAA CCG CTC TTC CGA TCT NNN NNN TAG ACG GAC CGG GAG CTG CAT GTG TCA GAG G | 5737 |
| AAT GAT ACG GCG ACC ACC GAG ATC TAC ACC GGT CTC GGC ATT CCT GCT GAA CCG CTC TTC CGA TCT NNN NNN CAG CTC TTC CGG GAG CTG CAT GTG TCA GAG G | 5738 |
| AAT GAT ACG GCG ACC ACC GAG ATC TAC ACC GGT CTC GGC ATT CCT GCT GAA CCG CTC TTC CGA TCT NNN NNN GAG CGA TAC CGG GAG CTG CAT GTG TCA GAG G | 5739 |
| AAT GAT ACG GCG ACC ACC GAG ATC TAC ACC GGT CTC GGC ATT CCT GCT GAA CCG CTC TTC CGA TCT NNN NNN CTC GAG AAC CGG GAG CTG CAT GTG TCA GAG G | 5740 |
| AAT GAT ACG GCG ACC ACC GAG ATC TAC ACC GGT CTC GGC ATT CCT GCT GAA CCG CTC TTC CGA TCT NNN NNN ATG ACA CCC CGG GAG CTG CAT GTG TCA GAG G | 5741 |

(continued)

| Sequence | SEQ ID NO: |
|---|---|
| AAT GAT ACG GCG ACC ACC GAG ATC TAC ACC GGT CTC GGC ATT CCT GCT GAA CCG CTC TTC CGA TCT NNN NNN CTT CAC GAC CGG GAG CTG CAT GTG TCA GAG G | 5742 |
| AAT GAT ACG GCG ACC ACC GAG ATC TAC ACC GGT CTC GGC ATT CCT GCT GAA CCG CTC TTC CGA TCT NNN NNN CTA TAA GGC CGG GAG CTG CAT GTG TCA GAG G | 5743 |
| AAT GAT ACG GCG ACC ACC GAG ATC TAC ACC GGT CTC GGC ATT CCT GCT GAA CCG CTC TTC CGA TCT NNN NNN CGT AGA GTC CGG GAG CTG CAT GTG TCA GAG G | 5744 |
| AAT GAT ACG GCG ACC ACC GAG ATC TAC ACC GGT CTC GGC ATT CCT GCT GAA CCG CTC TTC CGA TCT NNN NNN ATA GAT ACC CGG GAG CTG CAT GTG TCA GAG G | 5745 |
| AAT GAT ACG GCG ACC ACC GAG ATC TAC ACC GGT CTC GGC ATT CCT GCT GAA CCG CTC TTC CGA TCT NNN NNN TCG TCG ATC CGG GAG CTG CAT GTG TCA GAG G | 5746 |
| AAT GAT ACG GCG ACC ACC GAG ATC TAC ACC GGT CTC GGC ATT CCT GCT GAA CCG CTC TTC CGA TCT NNN NNN TAA GAA TCC CGG GAG CTG CAT GTG TCA GAG G | 5747 |
| AAT GAT ACG GCG ACC ACC GAG ATC TAC ACC GGT CTC GGC ATT CCT GCT GAA CCG CTC TTC CGA TCT NNN NNN AAT GAC AGC CGG GAG CTG CAT GTG TCA GAG G | 5748 |

56

(continued)

| Sequence | SEQ ID NO: |
|---|---|
| AAT GAT ACG GCG ACC ACC GAG ATC TAC ACC GGT CTC GGC ATT CCT GCT GAA CCG CTC TTC CGA TCT NNN NNN AGC TAG TGC CGG GAG CTG CAT GTG TCA GAG G | 5749 |
| AAT GAT ACG GCG ACC ACC GAG ATC TAC ACC GGT CTC GGC ATT CCT GCT GAA CCG CTC TTC CGA TCT NNN NNN TGA GAC CTC CGG GAG CTG CAT GTG TCA GAG G | 5750 |
| AAT GAT ACG GCG ACC ACC GAG ATC TAC ACC GGT CTC GGC ATT CCT GCT GAA CCG CTC TTC CGA TCT NNN NNN AGC GTA ATC CGG GAG CTG CAT GTG TCA GAG G | 5751 |
| AAT GAT ACG GCG ACC ACC GAG ATC TAC ACC GGT CTC GGC ATT CCT GCT GAA CCG CTC TTC CGA TCT NNN NNN TAA CCA AGC CGG GAG CTG CAT GTG TCA GAG G | 5752 |
| AAT GAT ACG GCG ACC ACC GAG ATC TAC ACC GGT CTC GGC ATT CCT GCT GAA CCG CTC TTC CGA TCT NNN NNN GAT GGC TTC CGG GAG CTG CAT GTG TCA GAG G | 5753 |
| AAT GAT ACG GCG ACC ACC GAG ATC TAC ACC GGT CTC GGC ATT CCT GCT GAA CCG CTC TTC CGA TCT NNN NNN GCA TCT GAC CGG GAG CTG CAT GTG TCA GAG G | 5754 |
| AAT GAT ACG GCG ACC ACC GAG ATC TAC ACC GGT CTC GGC ATT CCT GCT GAA CCG CTC TTC CGA TCT NNN NNN TTC CGG TAC CGG GAG CTG CAT GTG TCA GAG G | 5755 |

| Sequence | SEQ ID NO: |
|---|---|
| AAT GAT ACG GCG ACC ACC GAG ATC TAC ACC GGT CTC GGC ATT CCT GCT GAA CCG CTC TTC CGA TCT NNN NNN GAC ACT CTC CGG GAG CTG CAT GTG TCA GAG G | 5756 |
| AAT GAT ACG GCG ACC ACC GAG ATC TAC ACC GGT CTC GGC ATT CCT GCT GAA CCG CTC TTC CGA TCT NNN NNN TTA AGC ATC CGG GAG CTG CAT GTG TCA GAG G | 5757 |
| AAT GAT ACG GCG ACC ACC GAG ATC TAC ACC GGT CTC GGC ATT CCT GCT GAA CCG CTC TTC CGA TCT NNN NNN TGC TAC ACC CGG GAG CTG CAT GTG TCA GAG G | 5758 |
| AAT GAT ACG GCG ACC ACC GAG ATC TAC ACC GGT CTC GGC ATT CCT GCT GAA CCG CTC TTC CGA TCT NNN NNN TCA GCT TGC CGG GAG CTG CAT GTG TCA GAG G | 5759 |
| AAT GAT ACG GCG ACC ACC GAG ATC TAC ACC GGT CTC GGC ATT CCT GCT GAA CCG CTC TTC CGA TCT NNN NNN CAT GTA GAC CGG GAG CTG CAT GTG TCA GAG G | 5760 |
| AAT GAT ACG GCG ACC ACC GAG ATC TAC ACC GGT CTC GGC ATT CCT GCT GAA CCG CTC TTC CGA TCT NNN NNN TTC GGA ACC CGG GAG CTG CAT GTG TCA GAG G | 5761 |
| AAT GAT ACG GCG ACC ACC GAG ATC TAC ACC GGT CTC GGC ATT CCT GCT GAA CCG CTC TTC CGA TCT NNN NNN GCA ATT CGC CGG GAG CTG CAT GTG TCA GAG G | 5762 |

58

**EP 2 971 105 B1**

(continued)

| Sequence | SEQ ID NO: |
|---|---|
| AAT GAT ACG GCG ACC ACC GAG ATC TAC ACC GGT CTC GGC ATT CCT GCT GAA CCG CTC TTC CGA TCT NNN NNN CAA GAG GTC CGG GAG CTG CAT GTG TCA GAG G | 5763 |
| AAT GAT ACG GCG ACC ACC GAG ATC TAC ACC GGT CTC GGC ATT CCT GCT GAA CCG CTC TTC CGA TCT NNN NNN TCG ATT AAC CGG GAG CTG CAT GTG TCA GAG G | 5764 |
| AAT GAT ACG GCG ACC ACC GAG ATC TAC ACC GGT CTC GGC ATT CCT GCT GAA CCG CTC TTC CGA TCT NNN NNN GAA TGG ACC CGG GAG CTG CAT GTG TCA GAG G | 5765 |
| AAT GAT ACG GCG ACC ACC GAG ATC TAC ACC GGT CTC GGC ATT CCT GCT GAA CCG CTC TTC CGA TCT NNN NNN AGA ATC AGC CGG GAG CTG CAT GTG TCA GAG G | 5766 |
| AAT GAT ACG GCG ACC ACC GAG ATC TAC ACC GGT CTC GGC ATT CCT GCT GAA CCG CTC TTC CGA TCT NNN NNN AAC TGC CAC CGG GAG CTG CAT GTG TCA GAG G | 5767 |
| AAT GAT ACG GCG ACC ACC GAG ATC TAC ACC GGT CTC GGC ATT CCT GCT GAA CCG CTC TTC CGA TCT NNN NNN AAG TAA CGC CGG GAG CTG CAT GTG TCA GAG G | 5768 |
| AAT GAT ACG GCG ACC ACC GAG ATC TAC ACC GGT CTC GGC ATT CCT GCT GAA CCG CTC TTC CGA TCT NNN NNN ACT CAA TGC CGG GAG CTG CAT GTG TCA GAG G | 5769 |

| Sequence | SEQ ID NO: |
|---|---|
| AAT GAT ACG GCG ACC ACC GAG ATC TAC ACC GGT CTC GGC ATT CCT GCT GAA CCG CTC TTC CGA TCT NNN NNN CCT AGT AGC CGG GAG CTG CAT GTG TCA GAG G | 5770 |
| AAT GAT ACG GCG ACC ACC GAG ATC TAC ACC GGT CTC GGC ATT CCT GCT GAA CCG CTC TTC CGA TCT NNN NNN CTG ACG TTC CGG GAG CTG CAT GTG TCA GAG G | 5771 |
| AAT GAT ACG GCG ACC ACC GAG ATC TAC ACC GGT CTC GGC ATT CCT GCT GAA CCG CTC TTC CGA TCT NNN NNN TGC AGA CAC CGG GAG CTG CAT GTG TCA GAG G | 5772 |
| AAT GAT ACG GCG ACC ACC GAG ATC TAC ACC GGT CTC GGC ATT CCT GCT GAA CCG CTC TTC CGA TCT NNN NNN AGT TGA CCC CGG GAG CTG CAT GTG TCA GAG G | 5773 |
| AAT GAT ACG GCG ACC ACC GAG ATC TAC ACC GGT CTC GGC ATT CCT GCT GAA CCG CTC TTC CGA TCT NNN NNN GTC TCC TAC CGG GAG CTG CAT GTG TCA GAG G | 5774 |
| AAT GAT ACG GCG ACC ACC GAG ATC TAC ACC GGT CTC GGC ATT CCT GCT GAA CCG CTC TTC CGA TCT NNN NNN CTG CAA TCC CGG GAG CTG CAT GTG TCA GAG G | 5775 |
| AAT GAT ACG GCG ACC ACC GAG ATC TAC ACC GGT CTC GGC ATT CCT GCT GAA CCG CTC TTC CGA TCT NNN NNN TGA GCG AAC CGG GAG CTG CAT GTG TCA GAG G | 5776 |

(continued)

| Sequence | SEQ ID NO: |
|---|---|
| AAT GAT ACG GCG ACC ACC GAG ATC TAC ACC GGT CTC GGC ATT CCT GCT GAA CCG CTC TTC CGA TCT NNN NNN TTG GAC TGC CGG GAG CTG CAT GTG TCA GAG G | 5777 |
| AAT GAT ACG GCG ACC ACC GAG ATC TAC ACC GGT CTC GGC ATT CCT GCT GAA CCG CTC TTC CGA TCT NNN NNN AGC AAT CCC CGG GAG CTG CAT GTG TCA GAG G | 5778 |
| AAT GAT ACG GCG ACC ACC GAG ATC TAC ACC GGT CTC GGC ATT CCT GCT GAA CCG CTC TTC CGA TCT NNN NNN CGA ACT ACC CGG GAG CTG CAT GTG TCA GAG G | 5779 |
| AAT GAT ACG GCG ACC ACC GAG ATC TAC ACC GGT CTC GGC ATT CCT GCT GAA CCG CTC TTC CGA TCT NNN NNN TTA ATG GCC CGG GAG CTG CAT GTG TCA GAG G | 5780 |
| AAT GAT ACG GCG ACC ACC GAG ATC TAC ACC GGT CTC GGC ATT CCT GCT GAA CCG CTC TTC CGA TCT NNN NNN GCT TAG TAC CGG GAG CTG CAT GTG TCA GAG G | 5781 |
| CAA GCA GAA GAC GGC ATA CGA GAT ACA CTC TTT CCC TAC ACG ACG CTC TTC CGA TCT CAA GGT CAN NNN NNG GGC TGG CAA GCC ACG TTT GGT G | 5782 |
| CAA GCA GAA GAC GGC ATA CGA GAT ACA CTC TTT CCC TAC ACG ACG CTC TTC CGA TCT GCA TAA CTN NNN NNG GGC TGG CAA GCC ACG TTT GGT G | 5783 |

| Sequence | SEQ ID NO: |
|---|---|
| CAA GCA GAA GAC GGC ATA CGA GAT ACA CTC TTT CCC TAC ACG ACG CTC TTC CGA TCT CTC TGA TTN NNN NNG GGC TGG CAA GCC ACG TTT GGT G | 5784 |
| CAA GCA GAA GAC GGC ATA CGA GAT ACA CTC TTT CCC TAC ACG ACG CTC TTC CGA TCT TAC GTA CGN NNN NNG GGC TGG CAA GCC ACG TTT GGT G | 5785 |
| CAA GCA GAA GAC GGC ATA CGA GAT ACA CTC TTT CCC TAC ACG ACG CTC TTC CGA TCT TAC GCG TTN NNN NNG GGC TGG CAA GCC ACG TTT GGT G | 5786 |
| CAA GCA GAA GAC GGC ATA CGA GAT ACA CTC TTT CCC TAC ACG ACG CTC TTC CGA TCT CTC AGT GAN NNN NNG GGC TGG CAA GCC ACG TTT GGT G | 5787 |
| CAA GCA GAA GAC GGC ATA CGA GAT ACA CTC TTT CCC TAC ACG ACG CTC TTC CGA TCT TCT GAT ATN NNN NNG GGC TGG CAA GCC ACG TTT GGT G | 5788 |
| CAA GCA GAA GAC GGC ATA CGA GAT ACA CTC TTT CCC TAC ACG ACG CTC TTC CGA TCT CAT ATG CTN NNN NNG GGC TGG CAA GCC ACG TTT GGT G | 5789 |
| CAA GCA GAA GAC GGC ATA CGA GAT ACA CTC TTT CCC TAC ACG ACG CTC TTC CGA TCT CGT AAT TAN NNN NNG GGC TGG CAA GCC ACG TTT GGT G | 5790 |

(continued)

| Sequence | SEQ ID NO: |
|---|---|
| CAA GCA GAA GAC GGC ATA CGA GAT ACA CTC TTT CCC TAC ACG ACG CTC TTC CGA TCT ACG TAC TCN NNN NNG GGC TGG CAA GCC ACG TTT GGT G | 5791 |
| CAA GCA GAA GAC GGC ATA CGA GAT ACA CTC TTT CCC TAC ACG ACG CTC TTC CGA TCT CTT CTA AGN NNN NNG GGC TGG CAA GCC ACG TTT GGT G | 5792 |
| CAA GCA GAA GAC GGC ATA CGA GAT ACA CTC TTT CCC TAC ACG ACG CTC TTC CGA TCT ACT ATG ACN NNN NNG GGC TGG CAA GCC ACG TTT GGT G | 5793 |
| CAA GCA GAA GAC GGC ATA CGA GAT ACA CTC TTT CCC TAC ACG ACG CTC TTC CGA TCT GAC GTT AAN NNN NNG GGC TGG CAA GCC ACG TTT GGT G | 5794 |
| CAA GCA GAA GAC GGC ATA CGA GAT ACA CTC TTT CCC TAC ACG ACG CTC TTC CGA TCT ACA AGA TAN NNN NNG GGC TGG CAA GCC ACG TTT GGT G | 5795 |
| CAA GCA GAA GAC GGC ATA CGA GAT ACA CTC TTT CCC TAC ACG ACG CTC TTC CGA TCT GAC TAA GAN NNN NNG GGC TGG CAA GCC ACG TTT GGT G | 5796 |
| CAA GCA GAA GAC GGC ATA CGA GAT ACA CTC TTT CCC TAC ACG ACG CTC TTC CGA TCT GTG TCT ACN NNN NNG GGC TGG CAA GCC ACG TTT GGT G | 5797 |

(continued)

| Sequence | SEQ ID NO: |
|---|---|
| CAA GCA GAA GAC GGC ATA CGA GAT ACA CTC TTT CCC TAC ACG ACG CTC TTC CGA TCT TTC ACT AGN NNN NNG GGC TGG CAA GCC ACG TTT GGT G | 5798 |
| CAA GCA GAA GAC GGC ATA CGA GAT ACA CTC TTT CCC TAC ACG ACG CTC TTC CGA TCT AAT CGG ATN NNN NNG GGC TGG CAA GCC ACG TTT GGT G | 5799 |
| CAA GCA GAA GAC GGC ATA CGA GAT ACA CTC TTT CCC TAC ACG ACG CTC TTC CGA TCT AGT ACC GAN NNN NNG GGC TGG CAA GCC ACG TTT GGT G | 5800 |
| CAA GCA GAA GAC GGC ATA CGA GAT ACA CTC TTT CCC TAC ACG ACG CTC TTC CGA TCT TTG CCT CAN NNN NNG GGC TGG CAA GCC ACG TTT GGT G | 5801 |
| CAA GCA GAA GAC GGC ATA CGA GAT ACA CTC TTT CCC TAC ACG ACG CTC TTC CGA TCT TCG TTA GCN NNN NNG GGC TGG CAA GCC ACG TTT GGT G | 5802 |
| CAA GCA GAA GAC GGC ATA CGA GAT ACA CTC TTT CCC TAC ACG ACG CTC TTC CGA TCT TAT AGT TCN NNN NNG GGC TGG CAA GCC ACG TTT GGT G | 5803 |
| CAA GCA GAA GAC GGC ATA CGA GAT ACA CTC TTT CCC TAC ACG ACG CTC TTC CGA TCT TGG CGT ATN NNN NNG GGC TGG CAA GCC ACG TTT GGT G | 5804 |

(continued)

| Sequence | SEQ ID NO: |
|---|---|
| CAA GCA GAA GAC GGC ATA CGA GAT ACA CTC TTT CCC TAC ACG ACG CTC TTC CGA TCT TGG ACA TGN NNN NNG GGC TGG CAA GCC ACG TTT GGT G | 5805 |
| CAA GCA GAA GAC GGC ATA CGA GAT ACA CTC TTT CCC TAC ACG ACG CTC TTC CGA TCT AGG TTG CTN NNN NNG GGC TGG CAA GCC ACG TTT GGT G | 5806 |
| CAA GCA GAA GAC GGC ATA CGA GAT ACA CTC TTT CCC TAC ACG ACG CTC TTC CGA TCT ATA TGC TGN NNN NNG GGC TGG CAA GCC ACG TTT GGT G | 5807 |
| CAA GCA GAA GAC GGC ATA CGA GAT ACA CTC TTT CCC TAC ACG ACG CTC TTC CGA TCT GTA CAG TGN NNN NNG GGC TGG CAA GCC ACG TTT GGT G | 5808 |
| CAA GCA GAA GAC GGC ATA CGA GAT ACA CTC TTT CCC TAC ACG ACG CTC TTC CGA TCT ATC CAT GGN NNN NNG GGC TGG CAA GCC ACG TTT GGT G | 5809 |
| CAA GCA GAA GAC GGC ATA CGA GAT ACA CTC TTT CCC TAC ACG ACG CTC TTC CGA TCT TGA TGC GAN NNN NNG GGC TGG CAA GCC ACG TTT GGT G | 5810 |
| CAA GCA GAA GAC GGC ATA CGA GAT ACA CTC TTT CCC TAC ACG ACG CTC TTC CGA TCT GTA GCA GTN NNN NNG GGC TGG CAA GCC ACG TTT GGT G | 5811 |

65

| Sequence | SEQ ID NO: |
|---|---|
| CAA GCA GAA GAC GGC ATA CGA GAT ACA CTC TTT CCC TAC ACG ACG CTC TTC CGA TCT GGA TCA TCN NNN NNG GGC TGG CAA GCC ACG TTT GGT G | 5812 |
| CAA GCA GAA GAC GGC ATA CGA GAT ACA CTC TTT CCC TAC ACG ACG CTC TTC CGA TCT GTG AAC GTN NNN NNG GGC TGG CAA GCC ACG TTT GGT G | 5813 |
| CAA GCA GAA GAC GGC ATA CGA GAT ACA CTC TTT CCC TAC ACG ACG CTC TTC CGA TCT ATT AAG CGN NNN NNG GGC TGG CAA GCC ACG TTT GGT G | 5814 |
| CAA GCA GAA GAC GGC ATA CGA GAT ACA CTC TTT CCC TAC ACG ACG CTC TTC CGA TCT TAT TGG CGN NNN NNG GGC TGG CAA GCC ACG TTT GGT G | 5815 |
| CAA GCA GAA GAC GGC ATA CGA GAT ACA CTC TTT CCC TAC ACG ACG CTC TTC CGA TCT CGA TTA CAN NNN NNG GGC TGG CAA GCC ACG TTT GGT G | 5816 |
| CAA GCA GAA GAC GGC ATA CGA GAT ACA CTC TTT CCC TAC ACG ACG CTC TTC CGA TCT TGT CAT CGN NNN NNG GGC TGG CAA GCC ACG TTT GGT G | 5817 |
| CAA GCA GAA GAC GGC ATA CGA GAT ACA CTC TTT CCC TAC ACG ACG CTC TTC CGA TCT TAT CAA GTN NNN NNG GGC TGG CAA GCC ACG TTT GGT G | 5818 |

66

(continued)

| Sequence | SEQ ID NO: |
|---|---|
| CAA GCA GAA GAC GGC ATA CGA GAT ACA CTC TTT CCC TAC ACG ACG CTC TTC CGA TCT AGG CTT GAN NNN NNG GGC TGG CAA GCC ACG TTT GGT G | 5819 |
| CAA GCA GAA GAC GGC ATA CGA GAT ACA CTC TTT CCC TAC ACG ACG CTC TTC CGA TCT GAT AAC CAN NNN NNG GGC TGG CAA GCC ACG TTT GGT G | 5820 |
| CAA GCA GAA GAC GGC ATA CGA GAT ACA CTC TTT CCC TAC ACG ACG CTC TTC CGA TCT AAT CCT GCN NNN NNG GGC TGG CAA GCC ACG TTT GGT G | 5821 |
| CAA GCA GAA GAC GGC ATA CGA GAT ACA CTC TTT CCC TAC ACG ACG CTC TTC CGA TCT GTT ATA TCN NNN NNG GGC TGG CAA GCC ACG TTT GGT G | 5822 |
| CAA GCA GAA GAC GGC ATA CGA GAT ACA CTC TTT CCC TAC ACG ACG CTC TTC CGA TCT ACA CAC GTN NNN NNG GGC TGG CAA GCC ACG TTT GGT G | 5823 |
| CAA GCA GAA GAC GGC ATA CGA GAT ACA CTC TTT CCC TAC ACG ACG CTC TTC CGA TCT ATA CGA CTN NNN NNG GGC TGG CAA GCC ACG TTT GGT G | 5824 |
| CAA GCA GAA GAC GGC ATA CGA GAT ACA CTC TTT CCC TAC ACG ACG CTC TTC CGA TCT ATC TTC GTN NNN NNG GGC TGG CAA GCC ACG TTT GGT G | 5825 |

(continued)

| Sequence | SEQ ID NO: |
|---|---|
| CAA GCA GAA GAC GGC ATA CGA GAT ACA CTC TTT CCC TAC ACG ACG CTC TTC CGA TCT ACA TGT ATN NNN NNG GGC TGG CAA GCC ACG TTT GGT G | 5826 |
| CAA GCA GAA GAC GGC ATA CGA GAT ACA CTC TTT CCC TAC ACG ACG CTC TTC CGA TCT TCC ACA GTN NNN NNG GGC TGG CAA GCC ACG TTT GGT G | 5827 |
| CAA GCA GAA GAC GGC ATA CGA GAT ACA CTC TTT CCC TAC ACG ACG CTC TTC CGA TCT CAG TCT GTN NNN NNG GGC TGG CAA GCC ACG TTT GGT G | 5828 |
| CAA GCA GAA GAC GGC ATA CGA GAT ACA CTC TTT CCC TAC ACG ACG CTC TTC CGA TCT TCC ATG TGN NNN NNG GGC TGG CAA GCC ACG TTT GGT G | 5829 |
| CAA GCA GAA GAC GGC ATA CGA GAT ACA CTC TTT CCC TAC ACG ACG CTC TTC CGA TCT TCA CTG CAN NNN NNG GGC TGG CAA GCC ACG TTT GGT G | 5830 |
| CAA GCA GAA GAC GGC ATA CGA GAT ACA CTC TTT CCC TAC ACG ACG CTC TTC CGA TCT ATG GTC AAN NNN NNG GGC TGG CAA GCC ACG TTT GGT G | 5831 |
| CAA GCA GAA GAC GGC ATA CGA GAT ACA CTC TTT CCC TAC ACG ACG CTC TTC CGA TCT CAA GTC ACN NNN NNG GGC TGG CAA GCC ACG TTT GGT G | 5832 |

| Sequence | SEQ ID NO: |
|---|---|
| CAA GCA GAA GAC GGC ATA CGA GAT ACA CTC TTT CCC TAC ACG ACG CTC TTC CGA TCT TAG ACG GAN NNN NNG GGC TGG CAA GCC ACG TTT GGT G | 5833 |
| CAA GCA GAA GAC GGC ATA CGA GAT ACA CTC TTT CCC TAC ACG ACG CTC TTC CGA TCT CAG CTC TTN NNN NNG GGC TGG CAA GCC ACG TTT GGT G | 5834 |
| CAA GCA GAA GAC GGC ATA CGA GAT ACA CTC TTT CCC TAC ACG ACG CTC TTC CGA TCT GAG CGA TAN NNN NNG GGC TGG CAA GCC ACG TTT GGT G | 5835 |
| CAA GCA GAA GAC GGC ATA CGA GAT ACA CTC TTT CCC TAC ACG ACG CTC TTC CGA TCT CTC GAG AAN NNN NNG GGC TGG CAA GCC ACG TTT GGT G | 5836 |
| CAA GCA GAA GAC GGC ATA CGA GAT ACA CTC TTT CCC TAC ACG ACG CTC TTC CGA TCT ATG ACA CCN NNN NNG GGC TGG CAA GCC ACG TTT GGT G | 5837 |
| CAA GCA GAA GAC GGC ATA CGA GAT ACA CTC TTT CCC TAC ACG ACG CTC TTC CGA TCT CTT CAC GAN NNN NNG GGC TGG CAA GCC ACG TTT GGT G | 5838 |
| CAA GCA GAA GAC GGC ATA CGA GAT ACA CTC TTT CCC TAC ACG ACG CTC TTC CGA TCT CTA TAA GGN NNN NNG GGC TGG CAA GCC ACG TTT GGT G | 5839 |

(continued)

| SEQ ID NO: | Sequence |
|---|---|
| 5840 | CAA GCA GAA GAC GGC ATA CGA GAT ACA CTC TTT CCC TAC ACG ACG CTC TTC CGA TCT CGT AGA GTN NNN NNG GGC TGG CAA GCC ACG TTT GGT G |
| 5841 | CAA GCA GAA GAC GGC ATA CGA GAT ACA CTC TTT CCC TAC ACG ACG CTC TTC CGA TCT ATA GAT ACN NNN NNG GGC TGG CAA GCC ACG TTT GGT G |
| 5842 | CAA GCA GAA GAC GGC ATA CGA GAT ACA CTC TTT CCC TAC ACG ACG CTC TTC CGA TCT TCG TCG ATN NNN NNG GGC TGG CAA GCC ACG TTT GGT G |
| 5843 | CAA GCA GAA GAC GGC ATA CGA GAT ACA CTC TTT CCC TAC ACG ACG CTC TTC CGA TCT TAA GAA TCN NNN NNG GGC TGG CAA GCC ACG TTT GGT G |
| 5844 | CAA GCA GAA GAC GGC ATA CGA GAT ACA CTC TTT CCC TAC ACG ACG CTC TTC CGA TCT AAT GAC AGN NNN NNG GGC TGG CAA GCC ACG TTT GGT G |
| 5845 | CAA GCA GAA GAC GGC ATA CGA GAT ACA CTC TTT CCC TAC ACG ACG CTC TTC CGA TCT AGC TAG TGN NNN NNG GGC TGG CAA GCC ACG TTT GGT G |
| 5846 | CAA GCA GAA GAC GGC ATA CGA GAT ACA CTC TTT CCC TAC ACG ACG CTC TTC CGA TCT TGA GAC CTN NNN NNG GGC TGG CAA GCC ACG TTT GGT G |

| Sequence | SEQ ID NO: |
|---|---|
| CAA GCA GAA GAC GGC ATA CGA GAT ACA CTC TTT CCC TAC ACG ACG CTC TTC CGA TCT AGC GTA ATN NNN NNG GGC TGG CAA GCC ACG TTT GGT G | 5847 |
| CAA GCA GAA GAC GGC ATA CGA GAT ACA CTC TTT CCC TAC ACG ACG CTC TTC CGA TCT TAA CCA AGN NNN NNG GGC TGG CAA GCC ACG TTT GGT G | 5848 |
| CAA GCA GAA GAC GGC ATA CGA GAT ACA CTC TTT CCC TAC ACG ACG CTC TTC CGA TCT GAT GGC TTN NNN NNG GGC TGG CAA GCC ACG TTT GGT G | 5849 |
| CAA GCA GAA GAC GGC ATA CGA GAT ACA CTC TTT CCC TAC ACG ACG CTC TTC CGA TCT GCA TCT GAN NNN NNG GGC TGG CAA GCC ACG TTT GGT G | 5850 |
| CAA GCA GAA GAC GGC ATA CGA GAT ACA CTC TTT CCC TAC ACG ACG CTC TTC CGA TCT TTC CGG TAN NNN NNG GGC TGG CAA GCC ACG TTT GGT G | 5851 |
| CAA GCA GAA GAC GGC ATA CGA GAT ACA CTC TTT CCC TAC ACG ACG CTC TTC CGA TCT GAC ACT CTN NNN NNG GGC TGG CAA GCC ACG TTT GGT G | 5852 |
| CAA GCA GAA GAC GGC ATA CGA GAT ACA CTC TTT CCC TAC ACG ACG CTC TTC CGA TCT TTA AGC ATN NNN NNG GGC TGG CAA GCC ACG TTT GGT G | 5853 |

| Sequence | SEQ ID NO: |
|---|---|
| CAA GCA GAA GAC GGC ATA CGA GAT ACA CTC TTT CCC TAC ACG ACG CTC TTC CGA TCT TGC TAC ACN NNN NNG GGC TGG CAA GCC ACG TTT GGT G | 5854 |
| CAA GCA GAA GAC GGC ATA CGA GAT ACA CTC TTT CCC TAC ACG ACG CTC TTC CGA TCT TCA GCT TGN NNN NNG GGC TGG CAA GCC ACG TTT GGT G | 5855 |
| CAA GCA GAA GAC GGC ATA CGA GAT ACA CTC TTT CCC TAC ACG ACG CTC TTC CGA TCT CAT GTA GAN NNN NNG GGC TGG CAA GCC ACG TTT GGT G | 5856 |
| CAA GCA GAA GAC GGC ATA CGA GAT ACA CTC TTT CCC TAC ACG ACG CTC TTC CGA TCT TTC GGA ACN NNN NNG GGC TGG CAA GCC ACG TTT GGT G | 5857 |
| CAA GCA GAA GAC GGC ATA CGA GAT ACA CTC TTT CCC TAC ACG ACG CTC TTC CGA TCT GCA ATT CGN NNN NNG GGC TGG CAA GCC ACG TTT GGT G | 5858 |
| CAA GCA GAA GAC GGC ATA CGA GAT ACA CTC TTT CCC TAC ACG ACG CTC TTC CGA TCT CAA GAG GTN NNN NNG GGC TGG CAA GCC ACG TTT GGT G | 5859 |
| CAA GCA GAA GAC GGC ATA CGA GAT ACA CTC TTT CCC TAC ACG ACG CTC TTC CGA TCT TCG ATT AAN NNN NNG GGC TGG CAA GCC ACG TTT GGT G | 5860 |

**EP 2 971 105 B1**

EP 2 971 105 B1

| Sequence | SEQ ID NO: |
|---|---|
| CAA GCA GAA GAC GGC ATA CGA GAT ACA CTC TTT CCC TAC ACG ACG CTC TTC CGA TCT GAA TGG ACN NNN NNG GGC TGG CAA GCC ACG TTT GGT G | 5861 |
| CAA GCA GAA GAC GGC ATA CGA GAT ACA CTC TTT CCC TAC ACG ACG CTC TTC CGA TCT AGA ATC AGN NNN NNG GGC TGG CAA GCC ACG TTT GGT G | 5862 |
| CAA GCA GAA GAC GGC ATA CGA GAT ACA CTC TTT CCC TAC ACG ACG CTC TTC CGA TCT AAC TGC CAN NNN NNG GGC TGG CAA GCC ACG TTT GGT G | 5863 |
| CAA GCA GAA GAC GGC ATA CGA GAT ACA CTC TTT CCC TAC ACG ACG CTC TTC CGA TCT AAG TAA CGN NNN NNG GGC TGG CAA GCC ACG TTT GGT G | 5864 |
| CAA GCA GAA GAC GGC ATA CGA GAT ACA CTC TTT CCC TAC ACG ACG CTC TTC CGA TCT ACT CAA TGN NNN NNG GGC TGG CAA GCC ACG TTT GGT G | 5865 |
| CAA GCA GAA GAC GGC ATA CGA GAT ACA CTC TTT CCC TAC ACG ACG CTC TTC CGA TCT CCT AGT AGN NNN NNG GGC TGG CAA GCC ACG TTT GGT G | 5866 |
| CAA GCA GAA GAC GGC ATA CGA GAT ACA CTC TTT CCC TAC ACG ACG CTC TTC CGA TCT CTG ACG TTN NNN NNG GGC TGG CAA GCC ACG TTT GGT G | 5867 |

(continued)

| Sequence | SEQ ID NO: |
|---|---|
| CAA GCA GAA GAC GGC ATA CGA GAT ACA CTC TTT CCC TAC ACG ACG CTC TTC CGA TCT TGC AGA CAN NNN NNG GGC TGG CAA GCC ACG TTT GGT G | 5868 |
| CAA GCA GAA GAC GGC ATA CGA GAT ACA CTC TTT CCC TAC ACG ACG CTC TTC CGA TCT AGT TGA CCN NNN NNG GGC TGG CAA GCC ACG TTT GGT G | 5869 |
| CAA GCA GAA GAC GGC ATA CGA GAT ACA CTC TTT CCC TAC ACG ACG CTC TTC CGA TCT GTC TCC TAN NNN NNG GGC TGG CAA GCC ACG TTT GGT G | 5870 |
| CAA GCA GAA GAC GGC ATA CGA GAT ACA CTC TTT CCC TAC ACG ACG CTC TTC CGA TCT CTG CAA TCN NNN NNG GGC TGG CAA GCC ACG TTT GGT G | 5871 |
| CAA GCA GAA GAC GGC ATA CGA GAT ACA CTC TTT CCC TAC ACG ACG CTC TTC CGA TCT TGA GCG AAN NNN NNG GGC TGG CAA GCC ACG TTT GGT G | 5872 |
| CAA GCA GAA GAC GGC ATA CGA GAT ACA CTC TTT CCC TAC ACG ACG CTC TTC CGA TCT TTG GAC TGN NNN NNG GGC TGG CAA GCC ACG TTT GGT G | 5873 |
| CAA GCA GAA GAC GGC ATA CGA GAT ACA CTC TTT CCC TAC ACG ACG CTC TTC CGA TCT AGC AAT CCN NNN NNG GGC TGG CAA GCC ACG TTT GGT G | 5874 |

74

(continued)

| Sequence | SEQ ID NO: |
|---|---|
| CAA GCA GAA GAC GGC ATA CGA GAT ACA CTC TTT CCC TAC ACG ACG CTC TTC CGA TCT CGA ACT ACN NNN NNG GGC TGG CAA GCC ACG TTT GGT G | 5875 |
| CAA GCA GAA GAC GGC ATA CGA GAT ACA CTC TTT CCC TAC ACG ACG CTC TTC CGA TCT TTA ATG GCN NNN NNG GGC TGG CAA GCC ACG TTT GGT G | 5876 |
| CAA GCA GAA GAC GGC ATA CGA GAT ACA CTC TTT CCC TAC ACG ACG CTC TTC CGA TCT GCT TAG TAN NNN NNG GGC TGG CAA GCC ACG TTT GGT G | 5877 |

**Example 2: Single Cell Labeling of Adaptive Immune Receptor Encoding Sequences**

This example describes single cell labeling of immunoglobulin and T cell receptor heavy and light chain encoding sequences by RT-PCR. Freshly drawn blood from healthy human volunteers is used as a source of leukocytes. The amount of whole blood required to obtain 100,000 - 300,000 leukocytes is less than 1mL; 1-3 mL of blood are used for isolation of blood cells. Peripheral blood mononuclear cells (PBMC) are isolated from blood by density gradient centrifugation on Histopaque®-1077 (Sigma, St. Louis, MO) according to the supplier's instructions. CD45⁺ hematopoietic cells are isolated by binding to anti-CD45 coated magnetic beads using Whole Blood CD45 Microbeads (Miltenyi Biotec, Auburn, CA) as instructed by the manufacturer and essentially as described in Koehl et al. (2003 Leukemia 17:232). Leukocyte cell suspensions are washed in phosphate-buffered saline solution (PBS) and adjusted to a concentration of $1 \times 10^6$ cells/ mL. Aliquots of 1-3 $\mu$L (1-3 x $10^3$ cells) are distributed into wells of 96-well PCR multiwell plates held on ice in pre-chilled plate racks. Immediately after all plate wells are filled, the plates are sealed and placed on dry ice to freeze and lyse the cells. Plates are held on dry ice during the reverse transcription preparation steps below.

Reverse transcription is performed using the SMARTer™ Ultra Low RNA kit for Illumina sequencing (Clontech, Mountain View, CA) essentially according to the supplier's instructions. Stock Reaction Buffer is prepared by mixing 380 $\mu$l of Dilution Buffer with 20 $\mu$l of RNase inhibitor (40U/$\mu$l). 250 $\mu$l of Reaction Buffer is then mixed with 100 $\mu$l of a 12 $\mu$M solution of the 3' Smarter™ CDS II oligonucleotide (5'-Bio-AAGCAGTGGTATCAACGCAGAGTACT$_{(30)}$NN-3' [SEQ ID NO: 5878], where Bio is a biotin moiety; AAGCAGTGGTATCAACGCAGAGTAC [SEQ ID NO: 5879] is a universal adapter sequence, $T_{(30)}$ (SEQ ID NO: 5880) is a 30-mer of thymine residues, and N is any nucleotide (A, C, G or T).

The first-step annealing reactions for reverse transcription are set up by adding 3.5 $\mu$l of the Reaction Buffer containing the 3' Smarter™ CDS II oligonucleotide primer to each well of the 96-well plate containing the lysed cells, sealing the plate and incubating it for 3 minutes at 72°C, after which it is returned to a chilled rack on ice.

Reverse Transcription Master Mix (450 $\mu$l for 100 rxns) is prepared by combining 200 $\mu$l of 5x First Strand Buffer, 25 $\mu$l of 100 mM dithithreitol (DTT), 100 $\mu$l of dNTPs (10mM), 25 $\mu$l of RNase inhibitor (40U/$\mu$l), and 100 $\mu$l of reverse transcriptase. A 96-well working plate is prepared containing 1.0 $\mu$l of a barcoded 3'-Smart™ CDSII oligonucleotide per well. The 3'-Smart CDSII oligo sequence is: 5'-AAGCAGTGGTATCAACGCAGAGTACBBBBBBBBrGrGrG-P-3' [SEQ ID NO: 5881] where AAGCAGTGGTATCAACGCAGAGTAC [SEQ ID NO: 5879] is a universal adapter sequence; BBBBBBBB is an 8-nucleotide barcode (see list below for examples of barcodes); rG is riboguanine; and P is a 3' phosphate blocking moiety.

*Table 8. Barcode list (96 JS barcodes):*

| Name | Sequence |
|------|----------|
| JS01 | CAAGGTCA |
| JS02 | GCATAACT |
| JS03 | CTCTGATT |
| JS04 | TACGTACG |
| JS05 | TACGCGTT |
| JS06 | CTCAGTGA |
| JS07 | TCTGATAT |
| JS08 | CATATGCT |
| JS09 | CGTAATTA |
| JS10 | ACGTACTC |
| JS11 | CTTCTAAG |
| JS12 | ACTATGAC |
| JS13 | GACGTTAA |
| JS14 | ACAAGATA |
| JS15 | GACTAAGA |
| JS16 | GTGTCTAC |
| JS17 | TTCACTAG |

(continued)

| Name | Sequence |
|------|----------|
| JS18 | AATCGGAT |
| JS19 | AGTACCGA |
| JS20 | TTGCCTCA |
| JS21 | TCGTTAGC |
| JS22 | TATAGTTC |
| JS23 | TGGCGTAT |
| JS24 | TGGACATG |
| JS25 | AGGTTGCT |
| JS26 | ATATGCTG |
| JS27 | GTACAGTG |
| JS28 | ATCCATGG |
| JS29 | TGATGCGA |
| JS30 | GTAGCAGT |
| JS31 | GGATCATC |
| JS32 | GTGAACGT |
| JS33 | ATTAAGCG |
| JS34 | TATTGGCG |
| JS35 | CGATTACA |
| JS36 | TGTCATCG |
| JS37 | TATCAAGT |
| JS38 | AGGCTTGA |
| JS39 | GATAACCA |
| JS40 | AATCCTGC |
| JS41 | GTTATATC |
| JS42 | ACACACGT |
| JS43 | ATACGACT |
| JS44 | ATCTTCGT |
| JS45 | ACATGTAT |
| JS46 | TCCACAGT |
| JS47 | CAGTCTGT |
| JS48 | TCCATGTG |
| JS49 | TCACTGCA |
| JS50 | ATGGTCAA |
| JS51 | CAAGTCAC |
| JS52 | TAGACGGA |
| JS53 | CAGCTCTT |
| JS54 | GAGCGATA |
| JS55 | CTCGAGAA |

(continued)

| Name | Sequence |
|------|----------|
| JS56 | ATGACACC |
| JS57 | CTTCACGA |
| JS58 | CTATAAGG |
| JS59 | CGTAGAGT |
| JS60 | ATAGATAC |
| JS61 | TCGTCGAT |
| JS62 | TAAGAATC |
| JS63 | AATGACAG |
| JS64 | AGCTAGTG |
| JS65 | TGAGACCT |
| JS66 | AGCGTAAT |
| JS67 | TAACCAAG |
| JS68 | GATGGCTT |
| JS69 | GCATCTGA |
| JS70 | TTCCGGTA |
| JS71 | GACACTCT |
| JS72 | TTAAGCAT |
| JS73 | TGCTACAC |
| JS74 | TCAGCTTG |
| JS75 | CATGTAGA |
| JS76 | TTCGGAAC |
| JS77 | GCAATTCG |
| JS78 | CAAGAGGT |
| JS79 | TCGATTAA |
| JS80 | GAATGGAC |
| JS81 | AGAATCAG |
| JS82 | AACTGCCA |
| JS83 | AAGTAACG |
| JS84 | ACTCAATG |
| JS85 | CCTAGTAG |
| JS86 | CTGACGTT |
| JS87 | TGCAGACA |
| JS88 | AGTTGACC |
| JS89 | GTCTCCTA |
| JS90 | CTGCAATC |
| JS91 | TGAGCGAA |
| JS92 | TTGGACTG |
| JS93 | AGCAATCC |

(continued)

| Name | Sequence |
|------|----------|
| JS94 | CGAACTAC |
| JS95 | TTAATGGC |
| JS96 | GCTTAGTA |

To each well of the 96-well working plate containing 1.0 µl of a barcoded 3'-Smart™ CDSII oligonucleotide is added 4.5 µl of the Master Mix, and following completion of the annealing reaction, 5.5µl of the Master Mix containing barcoded 3'-Smart™ CDSII oligonucleotide is transferred from each well of the 96-well working plate to the correspondingly positioned (respective) wells of the reverse transcription annealing plate. The reverse transcription annealing plate is placed onto a thermocycler and a program is run with the steps 42°C for 90 minutes followed by 70°C for 10 minutes. This temperature profile performs first cDNA strand synthesis on all poly-A mRNA transcript molecules released from leukocytes in each well. According to non-limiting theory, after the first cDNA strand synthesis, each cDNA molecule in a well contains universal adaptor sequences at both the 5' and 3' ends, and is uniquely tagged with an 8-nt barcode at the 5' end.

Optionally, the barcoded cDNA molecules from all 96 reactions can be pooled at this step, and re-aliquoted onto a PCR plate where PCR amplification of immunoglobulin or T cell receptor cDNA takes place. The combining and splitting step permit substantially all barcoded cDNA molecules to be substantially evenly represented in subsequent PCR amplification reactions with adaptive immune receptor encoding (*e.g.,* IG or TCR) C-segment gene specific primers.

The products of reverse transcription/ cDNA first strand synthesis are next isolated by Solid Phase Reversible Immobilization Purification (SPRI) by mixing the contents of each well from the reverse transcription reaction plate with 25 µl of a suspension of Ampure™ XP SPRI magnetic beads (Beckman-Coulter Inc., Brea, CA) and incubating for 8 minutes at room temperature, followed by bead separation using a MagnaBot™ magnetic separator (Promega, Madison, WI) at room temperature according to the suppliers' instructions.

SPRI bead-immobilized cDNA first strands are immediately added to 5'RACE (rapid amplification of cDNA ends) PCR amplification reactions using Advantage 2™ PCR reagents (Clontech) according to the manufacturer's instructions. For each reaction, 50 µl of PCR Master Mix is added containing dNTPs, UPM primer mix, IG/TCR primer mix as described elsewhere herein, and Advantage 2™ polymerase and PCR buffer. The thermocycling conditions are: 95°C for 1 minute; 30 cycles of 95°C for 30 seconds, 63°C for 30 seconds, and 72°C for 3 minutes; 72°C for 7 minutes; and then reactions are held at 10°C prior to preparation for Illumina sequencing. PCR primer sequences are:

| 5'RACE UPM long | 5'-CTAATACGACTCACTATAGGGCAAGCAGTGGTATCAACGCAGAGT-3'(SEQ ID NO: 5611) |
|---|---|
| 5'RACE UPM short | 5'-CTAATACGACTCACTATAGGGC-3' (SEQ ID NO: 5612) |
| IgM_RACE | 5'-GATGGAGTCGGGAAGGAAGTCCTGTGCGAG-3' (SEQ ID NO: 5601) |
| IgG_RACE | 5'-GGGAAGACSGATGGGCCCTTGGTGG-3' (SEQ ID NO: 5602) |
| IgA_RACE | 5'-CAGGCAKGCGAYGACCACGTTCCCATC-3' (SEQ ID NO: 5603) |
| Igκ_RACE | 5'-CATCAGATGGCGGGAAGATGAAGACAGATGGTGC-3' (SEQ ID NO: 5604) |
| Igλ_RACE | 5'-CCTCAGAGGAGGGTGGGAACAGAGTGAC-3' (SEQ ID NO: 5605) |
| TCRB_RACE | 5'-GCTCAAACACAGCGACCTCGGGTGGGAACAC-3' (SEQ ID NO: 5606) |
| TCRA_RACE_JB2 | 5'-AGTCTCTCAGCTGGTACACGGCAGGGTC-3' (SEQ ID NO: 5591) |
| TCRA_50 | 5'- ACA GAC TTG TCA CTG GAT TTA GAG TCT CTC AGC TGG TAC ACG GCA GGG TC -3' (SEQ ID NO: 5592) |

(continued)

| | |
|---|---|
| TCRB_50 | 5'- GAG ATC TCT GCT TCT GAT GGC TCA AAC ACA GCG ACC TCG GGT GGG AAC AC -3' (SEQ ID NO: 5593) |
| S | G or C |
| K | G or T |
| Y | C or T |

**Illumina Sequencing Library Preparation**

PCR products are pooled by inverted centrifugation of the 96-well plates and the pooled products are purified to remove DNA fragments shorter than 200-300bp using Beckman Coulter Ampure™ XP beads according to the supplier's instructions. DNA purity is assessed by capillary electrophoresis using a Caliper Bioanalyzer (Perkin Elmer, Norwalk, CT) to confirm that most of the dsDNA is within a size range of 600-700 bp. dsDNA products are quantified fluorometrically or by A260 UV absorbance.

Sequencing library construction is conducted using 1 μg of purified DNA as an input for the Illumina TruSeq® sample preparation protocol (Illumina Inc., San Diego, CA) according to the Illumina TruSeq® DNA Sample Preparation Guide (Part number 15026486 Rev. C, July 2012, Illumina, Inc., San Diego, CA). This protocol generates a sequencing library that can be sequenced using the paired-end flow cell on the Illumina MiSeq®, HiSeq®2000, and HiSeq®2500 sequencers.

Illumina sequencing is conducted according to a sequencing protocol on the Illumina MiSeq® sequencer that utilizes the MiSeq® reagents kit v2, for 500 cycles. This chemistry provides kitted reagents for up to 525 cycles of sequencing on the MiSeq® instrument and provides sufficient reagents for a 251-cycle paired-end run, plus two eight-cycle indexed reads. The Illumina sequencing protocol is described in MiSeq® ReagentKit v2 ReagentPrepGuide, Part number 15034097 Rev. B, October 2012 (Illumina Inc., San Diego, CA). A schematic representation of the structure of DNA targets to be sequenced is shown in FIG. 6 (in which Ig heavy chain is used as an example).

**Example 3: High-Throughput Pairing of T-Cell Receptor Alpha and Beta Sequences**

An example is provided for applying the high-throughput pairing methods of the invention to pairing of α and β polypeptide chains of the T cell receptor (TCR) protein. The αβ T cell receptor (TCR) protein, which determines the antigenic specificity of an αβ T cell, is a heterodimer composed of two peptides: a longer β chain (TCRB) and a shorter α chain (TCRA) (*1-6*). Recently, high-throughput sequencing assays have been developed to profile TCR α and β chains and immunoglobulin heavy and light chains, with multiple diagnostic applications (*7-15*), including clinical diagnostics for the detection of cancer clones and the measurement of minimal residual disease in lymphoid malignancies (*13, 16-20*). However, high-throughput methods currently can sequence only one chain of the TCR at a time. In order to reconstitute T cell receptors for functional analysis, therapeutic use, or modeling of receptor-antigen binding, the α and β chains from a complete TCR must be identified as a pair.

There have been multiple attempts to pair α and β chains using single-cell technology. One approach has been to isolate individual B or T cells and physically link the heavy and light chains by bridge PCR prior to sequencing (See References *21-26* below). Alternatively, the heavy and light chains can be barcoded at the single cell level (See References 25, 27-31). Although single-cell methods have improved significantly, they are still technically challenging and limited in throughput. They also require intact single cells, which makes it difficult to assess infiltrating T cells in tissue or solid tumors. The invention provides a method for pairing α and β chain sequences at high throughput without the need for single-cell methods using a combinatorics approach, rather than physical isolation, to match the pairs. As a first demonstration, Applicants have applied the technology to peripheral blood from a healthy donor to identify ~35,000 paired α and β TCR gene sequences in a single experiment. The method can also be applied to tissue samples or both blood and tissue samples from a subject.

The method of the invention relies on the observation that rearranged TCRA and TCRB nucleotide sequences are nearly unique for each clonal population of T cells. Distinctive TCR sequences arise through recombination of gene segments and template-independent deletion or insertion of nucleotides at the V-J, V-D, and D-J junctions in somatic cells during lymphocyte development (*32*). This extraordinary diversity means that mRNAs encoding the TCRA and TCRB chains of a specific T cell clone will usually be present only in sets of cells that include that clone. Applicants leverage this extreme diversity by splitting a sample of T cells into multiple subsets and then sequencing the TCRA and TCRB mRNA molecules to determine the presence or absence of each TCR chain in each subset. The TCRA and TCRB sequences from a clone should be seen in the same subsets of T cells, and only those subsets.

In some embodiments, the method can involve extracting genomic DNA, rather than mRNA from cells in a sample, to amplify up the polypeptide chains of a specific adaptive immune receptor heterodimer.

Pairing the TCRA and TCRB chains then becomes a statistical problem: to declare a unique pairing, one must show that it is highly improbable for a given clone to occupy the same collection of T cell subsets as another clone. The probability that a given clone occupies the same collection of T cell subsets as another clone is close to zero for thousands of clones in an experiment using the methods of the invention.

FIG. 7 shows a graph of the number of occupied T cell subsets vs. the probability of shared subsets. For a simulated experiment using the methods of the invention, in which T cells are divided into 96 subsets containing 70,000 T cells each, this plot gives the probability (y-axis) that *any* clones present in a given number of subsets (x-axis) will occur in exactly the same subsets.

The methods of the invention provide a way to pair a clone's TCRA and TCRB sequences as a function of the frequency of that clone in the original sample, the total number of T cells in each subset, and the number of subsets created. The last two parameters are part of the experimental design, and they can be tuned to choose which range of T cell clonal frequencies will be accurately paired.

FIG. 8 shows a schematic of the methods of the invention, according to an embodiment of the invention. In FIG. 8A, a fixed number of T cells (e.g., 70,000 peripheral blood mononuclear cells (PBMCs)) are randomly allocated to each well on a 96-well plate to create 96 distinct subsets. The total cellular RNA is extracted, and cDNA is reverse transcribed form the RNA in the wells. The TCRA and TCRB sequences are PCR-amplified within each well and well-specific oligonucleotide DNA bar codes are attached to the amplified receptor molecules. In FIG. 8B, the barcoded TCRA and TCRB amplicons are pooled together. In FIG. 8C, pairs of TCRA/TCRB sequences consistently observed in the same libraries represent sequences carried by clones of the same cell.

Next, the amplified molecules are pooled and high-throughput DNA sequencing is performed, reading both the receptor sequence and the bar code for each strand. Sequenced bar codes are used to assign the receptor sequences to wells of origin, and we identify putative pairs by finding TCRA sequences that share many more wells with specific TCRB sequences than expected by chance. This involves computational de-multiplexing to map each TCR sequence back to the wells in which it originated. The immune repertoire is highly diverse, and the probability that two clones will share a well pattern is miniscule, so any TCRA/TCRB pair that shares a well pattern can be inferred to have come from the same clone.

To demonstrate the unprecedented throughput of Applicant's methods, we sampled 9.6 million human PBMCs and subdivided them equally among the wells of a 96-well microtiter plate. Each well thus contained 100,000 PBMCs, of which ~70,000 were expected to be T cells. After RNA extraction and cDNA synthesis, our sequencing runs produced 298 million reads, for an average coverage of 15 reads per T cell after filtering. (See Supplementary Materials for details).

We identified putative cognate pairs by comparing the well occupancy pattern of every TCRA sequence against that of every TCRB sequence. The well occupancy pattern can also be referred to as a container occupancy pattern. In some embodiments, the well occupancy pattern of every TCRB sequence is compared against that of every TCRA sequence. The TCRB that produced the smallest p-value with a given TCRA was marked as a possible pairing partner.

We then estimated the false discovery rate (FDR) for sets of putative pairs by computationally permuting the observed well patterns. This procedure quantifies the certainty of pairing while allowing for experimental inefficiencies, which is described in more detail below. Description about calculating the FDR are provided below.

The results of our experiment are shown in FIGs. 9A and 9B. At an FDR of 1% (shown in FIG. 9A, dotted line), we discovered 34,763 pairs of TCRA and TCRB sequences from 25,000 distinct clones. FIG. 9A shows the false discovery rate curve for an experiment with 96 wells and 70,000 T cells per well. The FDR curve is L-shaped, which implies that most of the TCRA/TCRB pairs stood out clearly from the background noise. Most of these pairs were called with extremely high confidence.

FIG. 9B illustrates the completeness of this pairing experiment. The grey (light shaded) bars show the total number of observed TCRB sequences as a function of well occupancy, and the dark shaded bars show how many of these were paired at FDR<1%. We successfully paired more than 85% of the TCRB sequences that occupied 15-70 wells. The remaining sequences could not be paired with high confidence, possibly because low TCRA and TCRB mRNA levels can lead to sequence drop-outs.

FIG. 10 shows an example workflow that was used for this experiment. In the example workflow, one starts with anticoagulated blood (1), separates PBMCs on a Ficoll gradient (2), aliquots even numbers of cells into the wells of a 96-well plate (3), isolates RNA in a 96-well plate (4), synthesizes cDNA (5), amplifies TCRA sequences with gene-specific PCR primers(6), amplifies TCRB sequences with gene-specific PCR primers (7), runs a nested PCR to add sequencing adaptors and well-specific barcodes to TCRA sequences (8), rusn a nested PCR to add sequencing adaptors and well-specific barcodes to TCRA sequences (9), and combines amplicons to create a sequencing library (10).

FIG. 11 illustrates the well occupancy vs. fraction of paired TCRB sequences. FIG. 11 shows the pairing yield (fraction of paired sequences) for TCRB clones from an experiment with 96 wells and 70,000 T cells per well. We grouped TCRB clones by the number of wells in which they were observed (x-axis) and computed the fraction of these sequences that were paired at FDR<1% (y-axis).

To validate the experimental design and statistical framework, we directly measured our FDR by performing a sample-mixing experiment outlined in FIG. 12. FIG. 12 shows the schematic of the two-subject validation experiment. Peripheral blood was collected from two healthy adult subjects, labeled 'X' and 'Y'. Deep immunosequencing was used to characterize the TCRA and TCRB repertoires (*14, 15, 33*) of each subject. PBMCs from the two subjects were then mixed, and the resulting mix was used to perform the methods of the invention. True-positive pairs must include a TCRA and a TCRB from the same subject, while approximately half of false-positive results will be cross-subject TCRA/TCRB pairs.

From these data, we identified 652,027 TCRA and 703,909 TCRB sequences that were unique to Subject X, and 395,476 TCRA and 433,086 TCRB sequences that were unique to Subject Y. Next, we combined 1.2 million PBMCs from each of the two subjects and performed the methods of the invention on the mixed sample, which had been distributed into 96 wells at 25,000 PBMC (~17,500 T cells) per well. True pairings must include a TCRA and a TCRB from the unique repertoire of a single subject, and roughly half of false pairings should include a TCRA sequence from one subject and a TCRB sequence from the other, so this experiment directly assesses the credibility of the identified pairs and the method's ability to control the rate of false pairings.

An FDR analysis of our sample-mixing experiment is shown in FIG. 13, where we divided the pairs into three categories: (i) pairs in which both TCR sequences were exclusive to Subject X ("X/X"); (ii) pairs in which both TCR sequences were exclusive to Subject Y ("Y/Y"); and (iii) pairs in which one sequence was exclusive to Subject X and the other to Subject Y ("X/Y"). The vertical dotted line in FIG. 13A shows the cutoff for an estimated FDR<1%. At a predicted FDR of 1%, we observed 1,115 X/X pairs, 706 Y/Y pairs, and 7 X/Y pairs.

In this experiment, fewer TCRA and TCRB pairs were detected in than in the first one because fewer T cells were allocated to each well, which shifts the sensitivity range toward more common clones.

If cross-donor pairs represented half of the false pairings, as expected, the FDR at this pairing threshold would be 1.06%, which shows excellent agreement with the prediction from the method of the invention. FIG. 13B extends this false pairing analysis to predicted FDR values ranging from 0.1% to 20%. The predicted values closely track the empirical values across this range, which shows that our analysis framework provides valid FDR estimates that can be tuned to balance specificity and sensitivity for different applications.

In FIGs. 14A-D, the well dropout rates in mRNA subsamples and within pairs are shown. FIG. 14A shows the well dropout rates in the first subsample of mRNA from the experiment using the methods described herein with 70,000 T cells per well. Median dropout rates (dotted vertical lines) are 20% for TCRA and 14% for TCRB. FIG. 14B shows the well dropout rates in the second subsample of mRNA from a experiment using the methods described herein. Median dropout rates are 24% for TCRA and 21% for TCRB. FIG. 14C shows the well dropout rates estimated from confidently paired sequences (FDR<1%) in the first subsample of mRNA (same data as in FIG. 14A). Median dropout rates are 14% for TCRA and 10% for TCRB, which are biased downward from the estimates in panel A. FIG. 14D shows the dropout rates estimated from confidently paired sequences (FDR<1%) in the full combined sample of mRNA, i.e., the data used in our discovery of ~35,000 pairs. Median dropout rates are 7% for TCRA and 3% for TCRB. After accounting for bias, we expect that the true median dropout rates are 10% and 5%, respectively

FIG. 15 shows the simulated well occupancy as a function of clone frequency and number of input T cells. A key design parameter in an experiment using the methods of the invention is the number of T cells allocated to each well. FIG. 15 shows the outcomes of simulated experiments for a 96-well plate and T cell inputs ranging from 100-100,000 cells per well (x-axis). Different colors depict clones with different repertoire frequencies, and the plot shows the number of wells they occupied in our simulations. To capture clones from different frequency bands, one can simply change the number of input T cells in an experiment using the methods of the invention. It is also possible to capture multiple frequency bands in a single experiment by varying the number of input T cells across the wells on a plate.

Finally, we confirmed our method's ability to correctly pair TCR chains from a known clonal sample by performing a spike-in experiment with a Jurkat E6-1 T-ALL cell line, for which the TCRB V, D, J and CDR3 sequence is known (34). We spiked Jurkat cells into a bulk PBMC sample at a target frequency < 1 in 70,000 PBMC (measured at ~1/150,000), allocated the cells to a 96-well microtiter plate at ~50,000 PBMCs per well, and performed a blind pairing analysis of the PBMC and spike-in sequences. The known Jurkat sequences were observed in 21 wells, of which 14 contained both the Jurkat TCRA and TCRB. This level of overlap produced a p-value of $2.6 \times 10^{-11}$, which is well below our FDR threshold and far stronger evidence of pairing than either sequence showed with any non-Jurkat sequence (the next-smallest p-value was $5.2 \times 10^{-5}$).

## *Subjects and Sample Preparation*

In this example, study donors were two healthy males, aged 44 and 51 years. Upon obtaining informed consent, venous blood was drawn from each donor into 10ml EDTA blood tubes (Beckton Dickinson, Franklin Lakes, NJ). Fresh anticoagulated blood was separated on Ficoll Histopaque gradients (Sigma, St. Louis, MO) and the peripheral blood mononuclear cell (PBMC) layer was aspirated. Red blood cells were lysed using 1x RBC Lysis solution (Miltenyi Biotec, Auburn, CA). Cells were washed in 1xPBS pH 7.2 (Ambion - Life Technologies, Foster City, CA) and resuspended in RNAlater (Qiagen, Valencia, CA). Cells were counted using a TC-20 cell counter (Bio-Rad, Hercules, CA) and concentration of cell suspensions was adjusted using RNAlater. Cell suspensions were stored at 4°C until use. Prior to cell aliquoting, cell suspensions were adjusted to a concentration of 500 or 2,000 cells/$\mu$l in RNAlater and 50$\mu$l aliquots were distributed into wells of 0.8ml deep well plates (Axygen, Union City, CA) by the Biomek FX liquid handler (Beckman Coulter, Danvers, MA).

The robotic method for distributing an equal volume of cell suspensions onto 96-well plates from a common, homogeneous cell suspension reservoir ensured that similar numbers of cells were deposited into each plate well. The final number of mononuclear cells deposited in each well was 100,000 for our initial pairing experiment (results in FIG. 9), 25,000 for our sample-mixing experiment (schematic in FIG. 12 and results in FIG. 13), and 50,000 for our Jurkat T-ALL spike-in experiment.

### RNA-Isolation and Reverse Transcription

Cells were lysed by addition of 400$\mu$l of lysis buffer RLT Plus (Qiagen), mixing and incubation at room temperature for 30 min. Total cellular RNA was isolated using an automated protocol on the QIAsymphony laboratory robot (Qiagen). RNA was eluted into a 96-deep well plate in 50$\mu$l of RNase-free water and immediately stored at -80oC. The quality and quantity of each RNA sample was assessed using the Caliper RNA Pico Sensitivity assay (PerkinElmer, Waltham, MA). Fifteen microliters (equivalent to 4-5ng of RNA) of RNA were reverse transcribed into single stranded cDNA using the SuperScript VILO cDNA synthesis kit (Invitrogen, Carlsbad, CA) according to the manufacturer's protocol. During RNA isolation and reverse transcription reaction, well identity was maintained relative to the original source plate with 96 cell suspension aliquots.

### Multiplex PCR and Barcodes

Multiplex PCR using either TCRA or TCRB gene-specific primers was performed on two 96-well plate replicates of the cDNA plate, using multiple V gene-specific primers for TCRA and TCRB and a single C segment reverse primer for each isotype (for lists of PCR primers, see Table S1 and Table S2). The Multiplex PCR Plus reagents (Qiagen) were used in 50$\mu$l reactions and 35 cycles of PCR amplification under the following conditions: 95°C denaturation for 300 s, followed by 35 cycles of denaturation at 90°C for 30 s, annealing at 60°C for 90 s and extension at 72°C for 90 s.

PCR products were purified using the SPRI Select magnetic beads (Beckman Coulter) and a 5$\mu$l aliquot of each PCR reaction was used in a nested PCR. The eight cycles of the second nested PCR incorporated Illumina paired-end adaptor sequences at each end of the amplicon, allowing the samples to be sequenced on Illumina MiSeq or HiSeq sequencers (Illumina, San Diego, CA). In addition, well-specific eight-base DNA barcodes were incorporated into each PCR product as described previously (33). For a complete list of barcodes, see Table S3.

During the TCRA and TCRB specific PCR and subsequent purification, as well as during the setup of nested PCR reactions, plate orientation was preserved relative to the original source plate. The nested second PCR barcoded DNA in each well on each plate with a specific barcode. Barcodes 1-96 were used for TCRB amplicon wells, and barcodes 97-192 were used for TCRA amplicon wells. After PCR amplification, 5$\mu$l aliquots were pooled for all 96 TCRA and TCRB wells, respectively, resulting in two DNA sample pools. The DNA concentration of each pool was determined by fluorometry and the DNA products were separated using the Caliper DNA 1K assay (PerkinElmer) to verify correct amplicon sizes and the absence of excess primer and primer-dimer. The two pools (TCRA and TCRB) were combined in equimolar ratio to a final sequencing library sample, now containing 192 individually barcoded DNA species.

### High - Throughput Sequencing

One sequencing library was prepared per one starting 96-well plate with cells, according to the method described above. Sequencing libraries were diluted, denatured and loaded onto the Illumina HiSeq 2500 sequencer flow cell as per Illumina protocols, for on-board cluster generation and sequencing by synthesis in a Rapid Run format. This run format analyzed DNA from one donor per sequencing run, using a single read sequencing (15 cycles of an index read followed by 150 cycles of sequencing through the V(D)JC receptor domain). The TCR sequencing reads were primed from the TCRA and TCRB constant regions with two sequencing primers specific to TCRA and TCRB, respectively. These primers annealed 14 bases from the J-C splice site, thus allowing us to confirm whether a sequence was either TCRA or TCRB, in addition to isotype and well-specific DNA barcodes. Methods for high throughput sequencing are described in detail above and in U.S.A.N. 13/217,126, U.S.A.N. 12/794,507, PCT/US2011/026373, or PCT/US2011/049012.

*T-all Clone Spike-In*

As a positive control of pairing, we purchased a human T cell leukemia (T-ALL) cell line, Jurkat Clone E6-1(ATCC® TIB-152™), known to be positive for $\alpha\beta$ TCR (35). The sequences of Jurkat TCRA and TCRB are known and were also confirmed by deep sequencing (34). Jurkat cells were propagated in HyClone RPMI 1640 media (Thermo Scientific, San Jose, CA) supplemented with 10% fetal bovine serum (Fischer Scientific, Waltham, MA) in T25 flasks at 37oC and 5% CO2. Harvested cells were diluted and approximately 450 Jurkat cells were added to a sample of 30 million PBMC, resulting in the Jurkat clone being present in the PBMC sample at a frequency of 1.5x10-5, or 0.75 Jurkat cells per well of a 96-well plate. The cell suspension concentration was adjusted to 1000 cells/$\mu$l and 50$\mu$l per well were robotically distributed onto a 96 well plate. Each well on the plate contained 50,000 PBMCs after distributing the sample cell suspension. The sample plate was then processed through the standard laboratory workflow of the invention, as described in FIG 7.

Based on the Poisson statistic, it was expected that at this level of spiked-in Jurkat clone, 51 wells would contain the known TCR $\alpha\beta$ pair sequences. The abundance of Jurkat TCR$\alpha\beta$ sequences in a well should be proportional to the level of TCR receptor mRNA expression. At least one literature report compared the number of TCR transcripts between peripheral blood CD4+ T-cells and Jurkat E6-1 cells. Using semi-quantitative PCR, the authors showed that TCR transcripts in Jurkat cells were not more abundant than TCR transcripts from CD4+ cells obtained from a healthy blood donor (36). Transcription levels of TCRB chain were compared across T-cell subsets using next-generation sequencing of TCRB cDNA and determined to be similar across naive, activated and memory CD8 T-cell subsets, ranging from 3-12 TCRB transcripts per cell (37). Based on the literature data, we expected that Jurkat clone sequences would be present in wells at levels below those of TCR repertoire sequences originating from PBMC. Relative expression of TCRA and TCB mRNA by the Jurkat clone is another biological characteristic that would have an impact on our ability to recover the expected clone TCR $\alpha\beta$ pair. It has been reported that transcriptional activity of alpha and beta chains is regulated during intra-thymic ontogeny of T cells and differs markedly depending on stage of thymic differentiation (38). Tumor cell lines derived from various stages of thymic development were shown to confirm the relative expression differences. The Jurkat cell line, being a "stage II T-cell" is thus expected to have lower expression of TCRA compared to expression of TCRB (39).

*Two Donors Experiment*

To directly measure our false positive rate for pairing, we isolated PBMCs from the venous blood of two healthy adults using standard techniques, as described above. Cellular RNA in freshly isolated PBMC was protected by storing the cell suspensions in RNAlater (a high salt, ammonium based RNA preservative that is known to protect RNA from degradation and to preserve mRNA expression profiles in stored tissue or cell samples (40)). Samples of the cell suspensions were distributed across 96 wells of a microtiter plate using a robotic liquid handler. We varied the number of cells placed in each well between 25,000 and 100,000, depending on experimental setup. The well-to-well variability in cell counts across the plate was thus dependent on pipetting precision of the liquid handler and was estimated to be below 25% CV. Starting with PBMC samples from two healthy adults, we divided the cells evenly into the wells of three 96 well plates. The first plate contained cells from donor X, the second plate contained cells from donor Y, and the third plate contained cells prepared by mixing samples from both donors in a 1:1 ratio. Each 96-well plate of donor cells sample analyzed was processed as a unit (all 96 wells in parallel) through the first two workflow steps: total cellular RNA isolation and cDNA synthesis. All 96 cDNA pools were then replicated for specific PCR amplification of TCRA and TCRB cDNA on two separate 96-well plates while preserving the well coordinates. We have designed the forward V-gene and reverse C-segment PCR primers to contain universal adapter complementary sequences, which allowed us to attach uniquely barcoded nested oligonucleotide adapters to each DNA template in a subsequent limited cycle PCR reaction. In that second PCR, all well-specific TCRA and TCRB amplicons in each 96 well plate were tagged with one of 192 eight-nucleotide barcodes. In addition, the second PCR also incorporated Illumina specific adapters into the amplicons, thus allowing pooling of all 192 wells into one sequencing library for sequencing on the HiSeq 2500. The design of TCRA and TCRB PCR products allowed us to sequence the amplified cDNA library as described previously (33). Briefly, a short, 15 cycle read determined the unique well barcode first, followed by a 150 cycle sequencing read which spanned the V(D)JC domains. Sequencing primers for the second read were positioned in the TCRA and TCRB C-segments, 14 nucleotides from the J-C splice junction. The read length of 150 bases thus allowed us to resolve all V, D (in case of TCRB), and J genes. The residual 14 bases of the C segment gene were specific to either TCRA or TCRB isotype, confirming a read belonging to either isotype independently of the well barcodes.

*Statistics for Adaptive Immune Receptor Pairing*

For every adaptive immune receptor pair considered, we compute a p-value for the number of wells that the receptor sequences share. For example, for a TCRA/TCRB pairing, we consider a TCRA sequence that occupies $N\alpha$ wells and a TCRB sequence that occupies $N\beta$ wells. If these sequences share $N\alpha\beta$ wells, then $N^*\alpha = N\alpha - N\alpha\beta$ wells are occupied only by the TCRA sequence and $N^*\beta = N\beta - N\alpha\beta$ wells are occupied only by the TCRB sequence. If each well contains the same number of T cells, the probability of seeing this amount of well-sharing by chance is

$$P(N_{\alpha\beta}|N_\alpha, N_\beta, N_{tot}) = \frac{\binom{N_{tot}}{N_{\alpha\beta}} \binom{N_{tot}-N_{\alpha\beta}}{N_\alpha^*} \binom{N_{tot}-N_{\alpha\beta}-N_\alpha^*}{N_\beta^*}}{\binom{N_{tot}}{N_\alpha} \binom{N_{tot}}{N_\beta}},$$

conditional on the total number of wells $N_{tot}$ and the marginal well counts $N\alpha$ and $N\beta$. We obtain a p-value for a putative TCR pair by summing the probabilities for all well configurations that have the same marginal counts and an equal or greater overlap of occupied wells.

Computing p-values in this way serves two purposes: it accounts for the fact that TCR sequences in different numbers of wells have different probabilities of overlapping by chance, and it captures departures from chance pairing without requiring perfect overlap between occupied wells. Imperfect well overlap is common among the members of a TCR pair because low numbers of mRNA transcripts per cell can lead to well dropouts (see section on "Well dropout rates in mRNA subsamples and within pairs" below), so it is essential to use a statistic that accounts for this feature of the data.

This analysis can be performed for other TCR or IG pairings, such as TCRG/TCRD and IGH with IGK or IGL.

### Simulating Null p-value Distributions

Unlike many applications in biological data analysis, the null distribution of p-values in an analysis using the methods of the invention is neither continuous nor uniform. Discrete well occupancy patterns lead to discrete p-values, and the p-value for each TCR sequence is chosen as the smallest seen in many comparisons with possible pairing partners, which skews the null distribution toward smaller p-values. TCR clones with different well occupancies also have different null distributions: more extreme p-values can be observed in clones that occupy a moderate number of wells, but such wells also tend to be involved in relatively few comparisons that could generate extreme p-values.

To account for these peculiarities, a permutation algorithm of the disclosure was used to fully model the structure of an experiment using the methods of the invention. We designated one locus (typically TCRA) as the "query" locus and the other as the "target" locus. We started by counting the number of target sequences Ti that occupy i wells for i = 1,...,96. One permutation involved the following steps:

For each occupancy level $i$, sample $T_i$ random numbers in [0,1]. Denote the largest sampled number $\gamma_i$.

For each occupancy level $j$, use $\gamma_i$ to determine the number of shared wells $N_{ij}$ in a cumulative distribution function for sequences that occupy $i$ and $j$ wells. This represents the largest number of wells that a sequence in $j$ wells would share with the best sequence in $i$ wells after sampling $T_i$ random configurations.

Use $N_{ij}$ to compute a p-value $\delta_j$ for occupancy level $j$. If $\delta_j$ is smaller than the smallest p-value seen so far at level $j$, store it.

Once we completed these steps for every number of occupied target wells $i$, we will have a minimum p-value $\delta_j$ for each possible number of query wells $j$. These values are stored as the outcome of one permutation, and they are directly analogous to the smallest p-values seen when comparing a given query sequence against a collection of target sequences like the one seen in an experiment of the invention. The results obtained by Applicants were based on running 10,000 permutations per experiment.

### False Discovery Rate Estimation

In one example, the simulated null distributions described above were used to estimate false discovery rates in an exemplary experiment. Clones at each well occupancy level have different null p-value distributions, so they also require separate FDR estimates. The approach follows that of Bancroft et al. (41) who developed a method for estimating false discovery rates with sequential permutation p-values. Like the p-values in the high-throughput pairing experiment, sequential permutation p-values are discrete and non-uniform, so their method is directly applicable to our scenario.

In one embodiment, the steps of the method were as follows:

For each well occupancy level $i$=1,...,96, construct as many bins as possible that have probability at least 0.05 under the null. This accounts for discontinuities in discrete distributions that can lead to over-conservative estimates of the number of truly null hypotheses.

For each well occupancy level $i$, estimate the number of null hypotheses $m_{0i}$ among the observed p-values via Theorem 2 of Bancroft et al., which is related to histogram-based estimators of $m_0$.

Compute the FDR at any rejection threshold $\alpha$ in the normal way for p-values at occupancy level $i$. To get experiment-wide FDR estimates like the ones shown in FIGs. 9 and 13, sum the numbers of expected type I errors and total pairing calls across occupancy levels and divide the former sum by the latter.

Estimates of $m_0$ can be unstable when the number of observed p-values is small, as can happen for occupancy levels with few TCR sequences. To get better estimates, occupancy levels that have small sequence counts and similar null distributions were combined.

Other methods for determining FDR can be used. In one embodiment, determining a false discovery rate estimation for a possible false pairing of a unique first adaptor immune receptor amplicon sequence and a unique second adaptor immune receptor amplicon sequence comprises calculating p-values for each of the plurality of putative cognate pairs identified in the sample; comparing the p-values for all of the plurality of putative cognate pairs with an expected p-value distribution, where the expected p-value distribution calculated to represent an experiment where no true cognate pairs are present; and determining for each putative cognate pair, an expected proportion of false positive results such that all p-values at or below the p-value of the putative cognate pair are determined to represent a true cognate pairing.

### Well Dropout Rates in mRNA Subsamples and Within Pairs

The pairing approach of the invention depends on reliably detecting the TCRA and TCRB sequences from a T cell clone in each well it occupies. A given T cell will typically carry just a few mRNA copies (possibly just 5-10) of each TCR rearrangement, and experimental inefficiencies can cause either locus to go unobserved in a subset of wells. To characterize this well dropout rate, the experiment was structured with 70,000 T cells per well to include two separate draws from the same pools of RNA on the plate. By processing each RNA draw separately, the effects of imperfect mRNA sampling by design were characterized, which is a proxy for imperfect mRNA sampling due to experimental inefficiencies.

In the section below, the well dropout rates in these mRNA subsamples are described. Well overlaps between paired sequences are used to estimate dropout rates in the complete set of sequenced mRNA, which was used for our main analysis.

FIG. 13 shows estimated rates of well dropouts for each of the two mRNA subsamples in this experiment. In these panels, the well dropout rate for each TCR sequence was determined by comparing the number of wells it occupied in one RNA draw against the combined data from both draws. The median dropout rates in this split experiment ranged from 20-24% for TCRA (Y/Y) and from 14-21% for TCRB (X/X). The high rates of well dropouts in these subsamples underscore the importance of thoroughly sampling the mRNA pool from the cells in an experiment. The dropout rates tended to be lower for TCRB than TCRA, which could reflect higher numbers of TCRB mRNA molecules per cell.

FIGs. 14A-D show the results of using a different estimator of well dropout rates. For a given experiment, we computed a dropout rate by comparing the well overlap within confidently paired (FDR<1%) sequences. Wells that are seen in one member of a TCR pair but not the other are considered missing. FIG. 14C shows the results of applying this estimator to the mRNA subsample that was used for FIG. 14A. As expected, the pairing-based estimates are biased downward since the power to detect pairs declines as wells drop out. For both TCRA and TCRB, the apparent dropout rate from paired sequences (FIG. 14C) was 30% lower than the rate seen by comparison to the complete mRNA sample (FIG. 14A).

Having determined the bias in a pairing-based estimator, we used this approach to evaluate well dropout rates in the full sample of mRNA from the high-throughput pairing experiment. The results are shown in FIG. 14D. The median dropout rates were 30-50% lower here than in the mRNA subset used for FIG. 14C, which further demonstrates the value of exhaustively sampling the mRNA pool. If we assume that the rates in FIG. 14D are 30% lower than the true rates, our final median dropout rates would be 10% for TCRA and 5% for TCRB. There are still substantial dropout rates for some TCR sequences, but we were nonetheless able to confidently assign ~35,000 TCR pairs in this experiment, which demonstrates the robustness of the methods of the invention.

### Example 4: Applications of High-Throughput Pairing to Adaptive Immune Receptor Heterodimer Polypeptide Sequences

In the example above, the application of the methods of the invention was focused on the ability of this approach to accurately pair TCR sequences at high throughput, but the methods of the invention can be used as a flexible tool that can broaden the scope of immune repertoire analysis in a variety of ways. For example, the method can be tuned to pair cognate TCR chains in any desired frequency range simply by changing the number of input T cells per well. One can also assay cognate pairs from multiple frequency bands in a single experiment by stratifying the number of input T cells across subsets of wells on a plate.

As described above, the method can be used to accurately pair TCR or IG sequences at high throughput. For example, the methods of the invention can be used to pair a first polypeptide chain of an adaptive immune receptor heterodimer comprising a TCR alpha (TCRA) chain and a second polypeptide of the adaptive immune receptor heterodimer comprising a TCR beta (TCRB) chain. In addition, the methods of the invention can be used to pair a first polypeptide of the adaptive immune receptor heterodimer comprising a TCR gamma (TCRG) chain and a second polypeptide of the adaptive immune receptor heterodimer comprising a TCR delta (TCRD) chain. In another example, the methods of the invention can be used to pair a first polypeptide of an adaptive immune receptor heterodimer comprising an immunoglobulin heavy (IGH) chain and a second polypeptide of the adaptive immune receptor heterodimer that is selected from an immunoglobulin light IGL or an IGK chain.

The method provides steps for identifying a plurality of cognate pairs comprising a first polypeptide and a second polypeptide that form an adaptive immune receptor heterodimer, said adaptive immune receptor heterodimer comprising a T cell receptor (TCR) or Immunoglobulin (IG) from a single clone in a sample, the sample comprising a plurality of lymphoid cells from a mammalian subject. As described above, the method includes steps for distributing a plurality of lymphoid cells among a plurality of containers, each container comprising a plurality of lymphoid cells; generating a library of amplicons in the plurality of containers by performing multiplex PCR of cDNA molecules that have been reverse-transcribed from mRNA molecules obtained from the plurality of lymphoid cells. The library of amplicons include: i) a plurality of first adaptive immune receptor amplicons encoding the first polypeptide, each comprising a unique variable (V) region encoding sequence, a unique J region encoding sequence or both a unique J region encoding sequence and a unique C region encoding sequence, at least one barcode sequence, at least one universal adaptor sequence, and a sequencing platform tag sequence, and ii) a plurality of second adaptive immune receptor amplicons encoding the second polypeptide, each comprising a unique V region encoding sequence, a unique J region encoding sequence or both a unique J region encoding sequence and a unique C region encoding sequence, at least one barcode sequence, at least one universal adaptor sequence, and a sequencing platform tag sequence. The method also includes steps for performing high throughput sequencing of the library of amplicons to obtain a data set of a plurality of first and second adaptive immune receptor amplicon sequences.

In addition, the method includes determining a container occupancy pattern for each unique first adaptor immune receptor amplicon sequence by assigning each unique first adaptor immune receptor amplicon sequence to one or more containers, and a container occupancy pattern for each unique second adaptor immune receptor amplicon sequence by assigning each unique second adaptor immune receptor amplicon sequence to one or more containers, wherein each barcode sequence in the unique first or second adaptor immune receptor amplicon sequences is associated with a particular container.

For each possible pairing of a unique first and second adaptive immune receptor amplicon sequence to form a putative cognate pair, the method involves calculating a statistical probability of observing the container occupancy patterns, or observing any larger proportion of shared containers than expected by chance, given that the first and second adaptor immune receptor amplicon sequences do not originate from the same clonal population of lymphoid cells, and identifying a plurality of a putative cognate pairs based on the statistical probability having a score lower than a predetermined likelihood cutoff.

Then, for each identified putative cognate pair, a false discovery rate estimation is determined for a possible false pairing of the unique first adaptor immune receptor amplicon sequence and the unique second adaptor immune receptor amplicon sequence. The method includes steps for identifying a plurality of cognate pairs of unique first and second adaptive immune receptor sequences as true cognate pairs that encode said adaptive immune receptors in said sample based on said statistical probability and said false discovery rate estimation.

In some embodiments, the statistical score can be a p-value calculated for pairing each putative cognate pair of unique first and second adaptive immune receptor amplicon sequences. In one embodiment, calculating the statistical score comprises calculating a probability that the unique first and second adaptive immune receptor amplicon sequences should jointly occupy as many or more containers than they are observed to jointly occupy, assuming no true cognate pairing and given the number of containers occupied by said unique first adaptive immune receptor amplicon sequence and the number of containers occupied by the unique second adaptive immune receptor amplicon sequence.

Essentially, given any two adaptive immune receptor sequences, the method analyzes whether the two sequences co-occur in more containers than would be expected by chance. Given a total of N containers, a first adaptive immune receptor sequence (A) observed in a total of X containers, a second adaptive immune receptor sequence (B) observed in a total of Y containers, and Z containers in which both adaptive immune receptor sequences (A) and (B) are observed, the method provides that given sequence (A) is found in X out of N containers (X / N) and sequence (B) is found in Y out of N (Y / N) containers, a calculation of the probability that both sequences are found in Z or more containers.

In some embodiments, the lower the probability that the observed number of overlapping containers between A and B sequences could occur by chance, the more highly likely that their co-occurrence is not by chance, but is instead due to true cognate pairing.

Next, identifying a plurality of a putative cognate pairs that have a high likelihood of pairing based on the statistical probability can comprise for each unique first adaptor immune receptor amplicon sequence identifying the unique second adaptor immune receptor amplicon sequence that has the lowest p-value score of matching, or for each unique second adaptor immune receptor amplicon sequence finding the unique first adaptor immune receptor amplicon sequence that has the lowest p-value score of matching.

In other embodiments, determining a false discovery rate estimation comprises: calculating p-values for each of the plurality of putative cognate pairs identified in the sample; comparing the p-values for all of the plurality of putative cognate pairs with an expected p-value distribution, said expected p-value distribution calculated to represent an experiment where no true cognate pairs are present; and determining for each putative cognate pair, an expected proportion of false positive results such that all p-values at or below the p-value of the putative cognate pair are determined to represent a true cognate pairing.

In certain embodiments, calculating the expected p-value distribution comprises: permuting the containers in which each first and second adaptive immune receptor sequence has been observed in an otherwise-identical experiment with no true cognate pairs, and calculating the distribution of p-values associated with each putative cognate pair.

The method includes identifying a plurality of cognate pairs of unique first and second adaptive immune receptor sequences as true cognate pairs by selecting a plurality of putative cognate pairs that have p-values below a threshold calculated based on the false discovery rate estimation.

In one embodiment, the identified cognate pair of unique first and second adaptive immune receptor amplicon sequences has a false discovery rate estimation of less than 1%. In other embodiments, the identified cognate pair of unique first and second adaptive immune receptor amplicon sequences has a false discovery rate estimation of less than 2%, 3%, 4%, 5%, 6%, 7%, 8%, 9%, or 10%.

The method can also include contacting each of said plurality of containers, under conditions and for a time sufficient to promote reverse transcription of mRNA molecules obtained from said plurality of lymphoid cells, with a first reverse transcription primer set. In certain embodiments, the (A) first oligonucleotide reverse transcription primer set comprises primers capable of reverse transcribing a plurality of mRNA sequences encoding the plurality of first and second adaptive immune receptor polypeptides for generating a plurality of first and second reverse-transcribed adaptive immune receptor cDNA amplicons, wherein the plurality of first reverse-transcribed adaptive immune receptor cDNA amplicons encoding the first adaptive immune receptor polypeptide comprise 1) a unique V region encoding gene sequence, and 2) a unique J region encoding gene sequence or both a unique J region encoding gene sequence and a unique C region encoding gene sequence, and wherein the plurality of second reverse-transcribed adaptive immune receptor cDNA amplicons encoding the second adaptive immune receptor polypeptide comprise 1) a unique V region encoding gene sequence, and 2) a unique J region encoding gene sequence or both a unique J region encoding gene sequence and a unique C region encoding gene sequence.

The first and second reverse-transcribed adaptive immune receptor cDNA amplicons are then amplified in a second reaction. The reaction begins by contacting each of said plurality of containers, under conditions and for a time sufficient to promote a multiplex PCR amplification of the first and second reverse-transcribed adaptive immune receptor cDNA amplicons with a second (B) and third (C) oligonucleotide primer sets. In some aspects, the (B) second oligonucleotide primer set comprises forward and reverse primers capable of amplifying the plurality of first reverse-transcribed adaptor immune receptor cDNA amplicons, wherein said forward and reverse primers each are capable of hybridizing to the first reverse-transcribed adaptive immune receptor cDNA amplicons.

Each pair of forward and reverse primers in the second oligonucleotide primer set is capable of amplifying the first reverse-transcribed adaptive immune receptor cDNA amplicons. The forward primers in the second oligonucleotide primer set comprise a first universal adaptor sequence and a region complementary to the V region encoding gene sequence. The reverse primers in the second oligonucleotide primer set comprise a second universal adaptor sequence and a region complementary to the J region encoding gene sequence or the C region encoding gene sequence.

The (C) third oligonucleotide primer set comprises forward and reverse primers capable of amplifying the plurality of reverse-transcribed second adaptive immune receptor cDNA amplicons. Each pair of forward and reverse primers in the third oligonucleotide primer set is capable of amplifying the second reverse-transcribed adaptive immune receptor cDNA amplicons. In one aspect, the forward primers in the third oligonucleotide primer set comprise a first universal adaptor sequence and a region complementary to the V region encoding gene sequence. The reverse primers in the third oligonucleotide primer set comprise a second universal adaptor sequence and a region complementary to the J region encoding gene sequence or complementary to the C region encoding gene sequence.

The method also includes generating i) a plurality of third adaptive immune receptor amplicons each comprising a unique V region encoding gene sequence, or complement thereof, a unique J region encoding gene sequence or both a unique J region encoding gene sequence and a unique C region encoding gene sequence, or complement thereof, and the first and second universal adaptor sequences, and ii) a plurality of fourth adaptive immune receptor amplicons each comprising a unique V region encoding gene sequence, or complement thereof, a unique J region encoding gene sequence or both a unique J region encoding gene sequence and a unique C region encoding gene sequence, or complement thereof, and the first and second universal adaptor sequences.

The plurality of third adaptive immune receptor amplicons and the plurality of fourth adaptive immune receptor amplicons are then amplified with additional primers. The method includes contacting each of the plurality of containers, under conditions and for a time sufficient to promote a second multiplex PCR amplification of the plurality of third and fourth adaptive immune receptor amplicons with a fourth (D) oligonucleotide primer set and fifth (E) oligonucleotide primer set.

In one embodiment, the (D) fourth oligonucleotide primer set comprises forward and reverse primers capable of amplifying the plurality of third adaptor immune receptor amplicons, wherein the forward and reverse primers each are capable of hybridizing to the third adaptive immune receptor amplicons. Each pair of forward and reverse primers in the fourth oligonucleotide primer set is capable of amplifying said third adaptor immune receptor amplicons.

The forward primer in the fourth oligonucleotide primer set comprises a sequencing platform tag sequence and a region complementary to the first universal adaptor sequence in the plurality of third adaptive immune receptor amplicon and the reverse primer comprises a sequencing platform tag sequence and a region complementary to the second universal adaptor sequence in the plurality of third adaptive immune receptor amplicons. In another embodiment, either one or both of the forward and reverse primers in the fourth oligonucleotide primer set comprises a unique barcode sequence associated with the container in which the fourth oligonucleotide primer set is introduced.

The (E) fifth oligonucleotide primer set comprises forward and reverse primers capable of amplifying the plurality of fourth adaptor immune receptor amplicons, wherein the forward and reverse primers each are capable of hybridizing to the fourth adaptive immune receptor amplicons. Each pair of forward and reverse primers in said fourth oligonucleotide primer set is capable of amplifying said plurality of fourth adaptor immune receptor amplicons. The forward primer in the fifth oligonucleotide primer set comprises a sequencing platform tag sequence and a region complementary to the first universal adaptor sequence in the plurality of fourth adaptive immune receptor amplicons, and the reverse primer in the fifth oligonucleotide primer set comprises a sequencing platform tag sequence and a region complementary to the second universal adaptor sequence in the plurality of fourth adaptive immune receptor amplicons.

Either one or both of the forward and reverse primers of the fourth oligonucleotide primer set comprises a unique barcode sequence associated with the container in which the fourth oligonucleotide primer set is introduced, thereby generating the library of amplicons comprising the plurality of first adaptive immune receptor amplicons and the plurality of second adaptive immune receptor amplicons.

Next, the method includes combining the library of amplicons from the plurality of containers into a mixture for sequencing. Methods for high throughput sequencing are described in detail above and in US201070330571, WO2011106738, or WO2012027503.

In one aspect, the plurality of first adaptive immune receptor amplicons comprise a C region encoding sequence. In some aspects, the plurality of second adaptive immune receptor amplicons comprise a C region encoding sequence.

In some cases, the sample comprises a blood sample. In another embodiment, the sample comprises a tissue sample. In certain embodiments, the sample comprises a sample purified or cultured human lymphoid cells. In other embodiments, the container comprises at least $10^4$ lymphoid cells. In another embodiment, the sample comprises at least $10^4$ cells.

The method is applicable to various adaptive immune receptor loci, as described above, such as pairing of a TCR alpha (TCRA) chain and a TCR beta (TCRB) chain, a TCR gamma (TCRG) chain and a TCR delta (TCRD) chain, or an immunoglobulin heavy (IGH) chain and an immunoglobulin light IGL or an IGK chain.

Where the first polypeptide of the adaptive immune receptor heterodimer is an IGH chain and the second polypeptide of the adaptive immune receptor heterodimer is both IGL and IGK, then three different amplification primer sets are used comprising: a first oligonucleotide amplification primer set for IGH, a second oligonucleotide amplification primer set for IGK, and a third oligonucleotide amplification primer set for IGL.

Thus, the methods of the invention can be found useful in many applications in immunology, medicine, and therapeutic development. The methods of the invention offer opportunities for investigating connections between the primary sequences of a collection of selected immune receptors and the target(s) (and epitopes) that caused their selection. With attention to experimental design and control of variables (e.g., HLA type), the methods of the invention can be a useful approach for identifying critical TCRs from tumor-infiltrating lymphocytes, for establishing new criteria for responsiveness to routine or experimental vaccination, and for epidemiological analysis of public exposures and shared responses. The methods of the invention also provide information on the relative contribution of each independent chain to a given response. In addition, our approach provides data on whether there might be physical TCR chain attributes that govern a particular immune response. For example, constraints on the length or biophysical parameters of one or both chains for a given type of response to a given type of antigenic challenge. The methods of the invention can be run with standard laboratory supplies and equipment, without the need for specialized expertise, and the starting sample type has a broad potential range (tumor samples, sorted cells, cells in suspension, etc.). This technology is designed to be scalable and accessible to a variety of laboratories.

It is important to recognize that the methods of the invention can be applied to and will work equally well for TCRγ/δ, and for linking the immunoglobulin heavy and light chains (IGH with IGK or IGL). Given the practical interest in monoclonal antibody development, as well as the general importance of the humoral immune response, the methods of the invention have the potential to become an important technology for biomedical discovery.

**TABLES**

**Table S1.**

| Sequences of TCRAD and TCRB V-gene forward PCR primers | | | |
|---|---|---|---|
| **TCRAD V-gene primers** | | **TCRB V-gene primers** | |
| Sequence Name | Sequence | Sequence Name | Sequence |
| TRAV01-1 | TCATTCCTTAGTCGCTCTGATAGTTATGGTTA | TCRBV01 | GAATGCCCTGACAGCTCTCGCTTATA |
| TRAV01-2 | CATTCCTTAGTCGGTCTAAAGGGTACAGTTA | TCRBV02 | CTCAGAGAAGTCTGAAATATTCGATGATCAATTCTCAGTTG |
| TRAV02 | ACAACATGACCTATGAACGGTTCTCTTCATC | TCRBV03-1 | CCAAATCGMTTCTCACCTAAATCTCCAGACAAAG |
| TRAV03 | CTGAATTTAACAAGAGCCAAACCTCCTTCCA | TCRBV03-2 | CACCTGACTCTCCAGACAAAGCTCAT |
| TRAV048 | CCGACAGAAAGTCCAGCACTCTGAG | TCRBV04-1/2/3 | CCTGAATGCCCCAACAGCTCTC |
| TRAV05 | CACTGTTCTATTGAATAAAAAGGATAAACATCTGTC | TCRBV05-1 | GATTCTCAGGGCGCCAGTTCTCTA |
| TRAV06 | GTCACCTTTGATACCACCCTTAAACAGAGTTT | TCRBV05-2 | CCTAATTGATTCTCAGCTCACCACGTCCATA |
| TRAV07 | AGACTAAATGCTACATTACTGAAGAATGGAAGCAG | TCRBV05-3 | TCAGGGCGCCAGTTCCATG |
| TRAV08-1 | TGAGGCTGAATTTATAAAGAGTAAATTCTCCTTTAA | TCRBV05-4 | TCCTAGATTCTCAGGTCTCCAGTTCCCTA |
| TRAV08-2 | GCTGAATTTAAGAAGAGTGAAACCTCCTTCCA | TCRBV05-5 | GAGGAAACTTCCCTGATCGATTCTCAGC |
| TRAV08-3 | GGCTGAATTTAAGAGGAGTCAATCTTCCTTCAA | TCRBV05-6 | CAACTTCCCTGATCGATTCTCAGGTCA |
| TRAV08-5P | GACACTTATCACTTCCCCAATCAATACCCC | TCRBV05-7 | AGGAAACTTCCCTGATCAATTCTCAGGTCA |
| TRAV08-6 | GGCTGAATTTAACAAGAGTCAAACTTCCTTCCA | TCRBV05-8 | GGAAACTTCCCTCCTAGATTTTCAGGTCG |
| TRAV08-7ORF | GCTGAATTTAAGAAGAGCGAAACCTCCTTCTA | TCRBV06-1 | CCCCAATGGCTACAATGTCTCCAGATT |
| TRAV09-1 | CCATGTACCGTAAAGAAACCACTTCTTTCCA | TCRBV06-2/3 | GGAGAGGTCCCTGATGGCTACAA |
| TRAV09-2 | CCACATACCGTAAAGAAACCACTTCTTTCCA | TCRBV06-4 | TCCCTGATGGTTATAGTGTCTCCAGAGC |
| TRAV10 | TGGATGCAGACACAAAGCAAAGCTC | TCRBV06-5 | GGAGAAGTCCCCAATGGCTACAATGTC |
| TRAV11P | TAAAGAACTGCTTGGAAAAGAAAAATTTTATAGTGT | TCRBV06-6 | AAAGGAGAAGTCCCGAATGGCTACAA |
| TRAV12-1 | ACAGCTCAATAGAGCCAGCCAGTATATTTC | TCRBV06-7 | GTTCCCAATGGCTACAATGTCTCCAGATC |
| TRAV12-2 | CAGCTCAATAAAGCCAGCCAGTATGTTTC | TCRBV06-8 | GAAGTCCCCAATGGCTACAATGTCTCTAGATT |
| TRAV12-3 | GCACAGGTCGATAAATCCAGCAAGTATATCTC | TCRBV06-9 | GAGAAGTCCCCGATGGCTACAATGTA |

(continued)

| Sequences of TCRAD and TCRB V-gene forward PCR primers | | | |
|---|---|---|---|
| **TCRAD V-gene primers** | | **TCRB V-gene primers** | |
| **Sequence Name** | **Sequence** | **Sequence Name** | **Sequence** |
| TRAV13-1 | CTGTTACATTGAACAAGACAGCCAAACATTTCTC | TCRBV07-1 | GTGATCGGTTCTCTGCACAGAGGT |
| TRAV13-2 | CACCGTTTTATTGAATAAGACAGTGAAACATCTCTC | TCRBV07-2 | CGCTTCTCTGCAGAGAGGACTGG |
| TRAV14/DV4 | CCAGAAGGCAAGAAAATCCGCCAA | TCRBV07-3 | GGTTCTTTGCAGTCAGGCCTGA |
| TRAV15P | AGAAGCGCTTGGAAAAGAGAAGTTTTATAGTGT | TCRBV07-4 | CAGTGGTCGGTTCTCTGCAGAG |
| TRAV16 | TGACCTTAACAAAGGCGAGACATCTTTCCA | TCRBV07-5 | GCTCAGTGATCAATTCTCCACAGAGAGGT |
| TRAV17 | CGCTTGACACTTCCAAGAAAAGCAGTTC | TCRBV07-6/7 | TTCTCTGCAGAGAGGCCTGAGG |
| TRAV18 | CAGTCCTATCAAGAGTGACAGTTCCTTCCA | TCRBV07-8 | CCCAGTGATCGCTTCTTTGCAGAAA |
| TRAV19 | GAACTTCCAGAAATCCACCAGTTCCTTCAA | TCRBV07-9 | CTGCAGAGAGGCCTAAGGGATCT |
| TRAV20 | GCTAAAAGCCACATTAACAAAGAAGGAAAGCTT | TCRBV08-1 | GAAGGGTACAATGTCTCTGGAAACAAACTCAAG |
| TRAV21 | CTCGCTGGATAAATCATCAGGACGTAGTAC | TCRBV08-2 | GGGGTACTGTGTTTCTTGAAACAAGCTTGAG |
| TRAV22 | TCGCTACGGAACGCTACAGCTT | TCRBV09 | CAGTTCCCTGACTTGCACTCTGAACTAAAC |
| TRAV23/DV6 | CTCCTTCAATAAAAGTGCCAAGCAGTTCTC | TCRBV10-1 | ACTAACAAAGGAGAAGTCTCAGATGGCTACAG |
| TRAV24 | CACTCTTAATACCAAGGAGGGTTACAGCTA | TCRBV10-2 | AGATAAAGGAGAAGTCCCCGATGGCTA |
| TRAV25 | TCAGTTTGGAGAAGCAAAAAAGAACAGCTC | TCRBV10-3 | GATACTGACAAAGGAGAAGTCTCAGATGGCTATAG |
| TRAV26-1 | GATCATCACAGAAGACAGAAAGTCCAGCAC | TCRBV11-1/2/3 | CTAAGGATCGATTTTCTGCAGAGAGGCTC |
| TRAV26-2 | GCAATCGCTGAAGACAGAAAGTCCAGTAC | TCRBV12-1 | TTGATTCTCAGCACAGATGCCTGATGT |
| TRAV27 | CAGTTTGGTGATGCAAGAAAGGACAGTTC | TCRBV12-2 | ATTCTCAGCTGAGAGGCCTGATGG |
| TRAV28P | CAGTCAAAGCTGAGGAACTTTATGGCCA | TCRBV12-3/4 | GGATCGATTCTCAGCTAAGATGCCTAATGC |
| TRAV29DV05 | CTTCTTAAACAAAGTGCCAAGCACCTCTC | TCRBV12-5 | CTCAGCAGAGATGCCTGATGCAACTTTA |
| TRAV30 | CTGCTTCATTTAATGAAAAAAAGCAGCAAAGCTC | TCRBV13 | CTGATCGATTCTCAGCTCAACAGTTCAGT |
| TRAV31P | TTCTGTGAGCTTCCAGAAAACAACTAAAACTATTCA | TCRBV14 | TAGCTGAAAGGACTGGAGGGACGTAT |
| TRAV32P | CACTGTACTGTTGAATAAAAATGCTAAACATGTCTC | TCRBV15 | CCAGGAGGCCGAACACTTCTTTCT |

| Sequences of TCRAD and TCRB V-gene forward PCR primers | | | |
|---|---|---|---|
| **TCRAD V-gene primers** | | **TCRB V-gene primers** | |
| **Sequence Name** | **Sequence** | **Sequence Name** | **Sequence** |
| TRAV33P | GCCTGTGAACTTTGAAAAAAAGAAAAAGTTCATCAA | TCRBV16 | GCTAAGTGCCTCCCAAATTCACCCT |
| TRAV34 | CCAAGTTGGATGAGAAAAAGCAGCAAAGTTC | TCRBV17 | CACAGCTGAAAGACCTAACGGAACGT |
| TRAV35 | TCAGTTTGGTATAACCAGAAAGGACAGCTT | TCRBV18 | CTGCTGAATTTCCCAAAGAGGGCC |
| TRAV36/TRDV7 | AAGTAGCATATTAGATAAGAAAGAACTTTCCAGCAT | TCRBV19 | AGGGTACAGCGTCTCTCGGG |
| TRAV37P | CAGGCTTAAAAAAGGAGACCAGCACATTTC | TCRBV20 | GCCTGACCTTGTCCACTCTGACA |
| TRAV38-1 | CTTCCAGAAAGCAGCCAAATCCTTCAG | TCRBV21 | ATGAGCGATTTTTAGCCCAATGCTCCA |
| TRAV39 | TGATACCAAAGCCCGTCTCAGCAC | TCRBV22 | TGAAGGCTACGTGTCTGCCAAGAG |
| TRAV40 | GAGGCGGAAATATTAAAGACAAAAACTCCCC | TCRBV23 | CTCATCTCAATGCCCCAAGAACGC |
| TRAV41 | CCACAATAAACATACAGGAAAAGCACAGCTC | TCRBV24 | AGATCTCTGATGGATACAGTGTCTCTCGACA |
| TRDV01 | AGAAAGCAGCGAAATCCGTCGC | TCRBV25 | AGATCTTTCCTCTGAGTCAACAGTCTCCAGAATA |
| TRDV02 | TGACATTGATATTGCAAAGAACCTGGCTGT | TCRBV26 | CACTGAAAAAGGAGATATCTCTGAGGGGTATCATG |
| TRDV035 | GAAACACATTCTGACCCAGAAAGCCTTTCA | TCRBV27 | GTTCCTGAAGGGTACAAAGTCTCTCGAAAAG |
| | | TCRBV28 | CTGAGGGGTACAGTGTCTCTAGAGAGA |
| | | TCRBV29 | AGCCGCCCAAACCTAACATTCTCAA |
| | | TCRBV30 | CCCAGGACCGGCAGTTCA |
| | | TCRBVA | TTGATTAGAGACATATCCCTATTGAAAATATTTCCTGGCA |
| | | TCRBVB | AGATGCCCTGAGTCAGCATAGTCATTCTAAC |

EP 2 971 105 B1

**Table S2.**

| Sequences of TCRA and TCRB C-gene reverse PCR primers | |
| --- | --- |
| **TCRA and TCRB C-segment primers** | |
| **Sequence Name** | **Sequence** |
| TCRA_C50 | ACAGACTTGTCACTGGATTTAGAGTCTCTCAGCTGGTACACGGCAGGGTC |
| TCRB_C50 | GAGATCTCTGCTTCTGATGGCTCAAACACAGCGACCTCGGGTGGGAACAC |

**Table S3.**

| Sequences of DNA barcodes | | | | | | | |
| --- | --- | --- | --- | --- | --- | --- | --- |
| **BarCode** | **Sequence** | **BarCode** | **Sequence** | **BarCode** | **Sequence** | **BarCode** | **Sequence** |
| JS01 | CAAGGTCA | JS49 | TCACTGCA | JS97 | TGACCTTG | JS145 | TGCAGTGA |
| JS02 | GCATAACT | JS50 | ATGGTCAA | JS98 | AGTTATGC | JS146 | TTGACCAT |
| JS03 | CTCTGATT | JS51 | CAAGTCAC | JS99 | AATCAGAG | JS147 | GTGACTTG |
| JS04 | TACGTACG | JS52 | TAGACGGA | JS100 | CGTACGTA | JS148 | TCCGTCTA |
| JS05 | TACGCGTT | JS53 | CAGCTCTT | JS101 | AACGCGTA | JS149 | AAGAGCTG |
| JS06 | CTCAGTGA | JS54 | GAGCGATA | JS102 | TCACTGAG | JS150 | TATCGCTC |
| JS07 | TCTGATAT | JS55 | CTCGAGAA | JS103 | ATATCAGA | JS151 | TTCTCGAG |
| JS08 | CATATGCT | JS56 | ATGACACC | JS104 | AGCATATG | JS152 | GGTGTCAT |
| JS09 | CGTAATTA | JS57 | CTTCACGA | JS105 | TAATTACG | JS153 | TCGTGAAG |
| JS10 | ACGTACTC | JS58 | CTATAAGG | JS106 | GAGTACGT | JS154 | CCTTATAG |
| JS11 | CTTCTAAG | JS59 | CGTAGAGT | JS107 | CTTAGAAG | JS155 | ACTCTACG |
| JS12 | ACTATGAC | JS60 | ATAGATAC | JS108 | GTCATAGT | JS156 | GTATCTAT |
| JS13 | GACGTTAA | JS61 | TCGTCGAT | JS109 | TTAACGTC | JS157 | ATCGACGA |
| JS14 | ACAAGATA | JS62 | TAAGAATC | JS110 | TATCTTGT | JS158 | GATTCTTA |
| JS15 | GACTAAGA | JS63 | AATGACAG | JS111 | TCTTAGTC | JS159 | CTGTCATT |
| JS16 | GTGTCTAC | JS64 | AGCTAGTG | JS112 | GTAGACAC | JS160 | CACTAGCT |
| JS17 | TTCACTAG | JS65 | TGAGACCT | JS113 | CTAGTGAA | JS161 | AGGTCTCA |
| JS18 | AATCGGAT | JS66 | AGCGTAAT | JS114 | ATCCGATT | JS162 | ATTACGCT |
| JS19 | AGTACCGA | JS67 | TAACCAAG | JS115 | TCGGTACT | JS163 | CTTGGTTA |
| JS20 | TTGCCTCA | JS68 | GATGGCTT | JS116 | TGAGGCAA | JS164 | AAGCCATC |
| JS21 | TCGTTAGC | JS69 | GCATCTGA | JS117 | GCTAACGA | JS165 | TCAGATGC |
| JS22 | TATAGTTC | JS70 | TTCCGGTA | JS118 | GAACTATA | JS166 | TACCGGAA |
| JS23 | TGGCGTAT | JS71 | GACACTCT | JS119 | ATACGCCA | JS167 | AGAGTGTC |
| JS24 | TGGACATG | JS72 | TTAAGCAT | JS120 | CATGTCCA | JS168 | ATGCTTAA |
| JS25 | AGGTTGCT | JS73 | TGCTACAC | JS121 | AGCAACCT | JS169 | GTGTAGCA |
| JS26 | ATATGCTG | JS74 | TCAGCTTG | JS122 | CAGCATAT | JS170 | CAAGCTGA |
| JS27 | GTACAGTG | JS75 | CATGTAGA | JS123 | CACTGTAC | JS171 | TCTACATG |
| JS28 | ATCCATGG | JS76 | TTCGGAAC | JS124 | CCATGGAT | JS172 | GTTCCGAA |
| JS29 | TGATGCGA | JS77 | GCAATTCG | JS125 | TCGCATCA | JS173 | CGAATTGC |

(continued)

| Sequences of DNA barcodes | | | | | | | |
|---|---|---|---|---|---|---|---|
| BarCode | Sequence | BarCode | Sequence | BarCode | Sequence | BarCode | Sequence |
| JS30 | GTAGCAGT | JS78 | CAAGAGGT | JS126 | ACTGCTAC | JS174 | ACCTCTTG |
| JS31 | GGATCATC | JS79 | TCGATTAA | JS127 | GATGATCC | JS175 | TTAATCGA |
| JS32 | GTGAACGT | JS80 | GAATGGAC | JS128 | ACGTTCAC | JS176 | GTCCATTC |
| JS33 | ATTAAGCG | JS81 | AGAATCAG | JS129 | CGCTTAAT | JS177 | CTGATTCT |
| JS34 | TATTGGCG | JS82 | AACTGCCA | JS130 | CGCCAATA | JS178 | TGGCAGTT |
| JS35 | CGATTACA | JS83 | AAGTAACG | JS131 | TGTAATCG | JS179 | CGTTACTT |
| JS36 | TGTCATCG | JS84 | ACTCAATG | JS132 | CGATGACA | JS180 | CATTGAGT |
| JS37 | TATCAAGT | JS85 | CCTAGTAG | JS133 | ACTTGATA | JS181 | CTACTAGG |
| JS38 | AGGCTTGA | JS86 | CTGACGTT | JS134 | TCAAGCCT | JS182 | AACGTCAG |
| JS39 | GATAACCA | JS87 | TGCAGACA | JS135 | TGGTTATC | JS183 | TGTCTGCA |
| JS40 | AATCCTGC | JS88 | AGTTGACC | JS136 | GCAGGATT | JS184 | GGTCAACT |
| JS41 | GTTATATC | JS89 | GTCTCCTA | JS137 | GATATAAC | JS185 | TAGGAGAC |
| JS42 | ACACACGT | JS90 | CTGCAATC | JS138 | ACGTGTGT | JS186 | GATTGCAG |
| JS43 | ATACGACT | JS91 | TGAGCGAA | JS139 | AGTCGTAT | JS187 | TTCGCTCA |
| JS44 | ATCTTCGT | JS92 | TTGGACTG | JS140 | ACGAAGAT | JS188 | CAGTCCAA |
| JS45 | ACATGTAT | JS93 | AGCAATCC | JS141 | ATACATGT | JS189 | GGATTGCT |
| JS46 | TCCACAGT | JS94 | CGAACTAC | JS142 | ACTGTGGA | JS190 | GTAGTTCG |
| JS47 | CAGTCTGT | JS95 | TTAATGGC | JS143 | ACAGACTG | JS191 | GCCATTAA |
| JS48 | TCCATGTG | JS96 | GCTTAGTA | JS144 | CACATGGA | JS192 | TACTAAGC |

## REFERENCES

1. N. R. Gascoigne, Y.-h. Chien, D. M. Becker, J. Kavaler, M. M. Davis, Genomic organization and sequence of T-cell receptor β-chain constant-and joining-region genes. Nature 310, 387-391 (1984).

2. G. Siu, S. P. Clark, Y. Yoshikai, M. Malissen, Y. Yanagi, E. Strauss, T. W. Mak, L. Hood, The human T cell antigen receptor is encoded by variable, diversity, and joining gene segments that rearrange to generate a complete V gene. Cell 37, 393-401 (1984).

3. Y. Yoshikai, S. P. Clark, S. Taylor, U. Sohn, B. I. Wilson, M. D. Minden, T. W. Mak, Organization and sequences of the variable, joining and constant region genes of the human T-cell receptor α-chain. (1985).

4. B. Toyonaga, Y. Yoshikai, V. Vadasz, B. Chin, T. W. Mak, Organization and sequences of the diversity, joining, and constant region genes of the human T-cell receptor beta chain. Proceedings of the National Academy of Sciences 82, 8624-8628 (1985); published online EpubDecember 1, 1985 (

5. M. M. Davis, P. J. Bjorkman, T-cell antigen receptor genes and T-cell recognition. Nature 334, (1988).

6. J. Nikolich-Žugich, M. K. Slifka, I. Messaoudi, The many important facets of T-cell repertoire diversity. Nature Reviews Immunology 4, 123-132 (2004).

7. H. S. Robins, P. V. Campregher, S. K. Srivastava, A. Wacher, C. J. Turtle, O. Kahsai, S. R. Riddell, E. H. Warren, C. S. Carlson, Comprehensive assessment of T-cell receptor beta-chain diversity in alphabeta T cells. Blood 114, 4099-4107 (2009); published online EpubNov 5 (10.1182/blood-2009-04-217604).

8. R. L. Warren, B. H. Nelson, R. A. Holt, Profiling model T-cell metagenomes with short reads. Bioinformatics 25, 458-464 (2009).

9. J. Glanville, W. Zhai, J. Berka, D. Telman, G. Huerta, G. R. Mehta, I. Ni, L. Mei, P. D. Sundar, G. M. Day, D. Cox, A. Rajpal, J. Pons, Precise determination of the diversity of a combinatorial antibody library gives insight into the human immunoglobulin repertoire. Proceedings of the National Academy of Sciences of the United States of America 106, 20216-20221 (2009); published online EpubDec 1 (10.1073/pnas.0909775106).

10. S. D. Boyd, E. L. Marshall, J. D. Merker, J. M. Maniar, L. N. Zhang, B. Sahaf, C. D. Jones, B. B. Simen, B. Hanczaruk, K. D. Nguyen, Measurement and clinical monitoring of human lymphocyte clonality by massively parallel VDJ pyrosequencing. Science translational medicine 1, 12ra23-12ra23 (2009).

11. H. S. Robins, S. K. Srivastava, P. V. Campregher, C. J. Turtle, J. Andriesen, S. R. Riddell, C. S. Carlson, E. H. Warren, Overlap and effective size of the human CD8+ T cell receptor repertoire. Science translational medicine 2, 47ra64 (2010).

12. R. L. Warren, J. D. Freeman, T. Zeng, G. Choe, S. Munro, R. Moore, J. R. Webb, R. A. Holt, Exhaustive T-cell repertoire sequencing of human peripheral blood samples reveals signatures of antigen selection and a directly measured repertoire size of at least 1 million clonotypes. Genome research 21, 790-797 (2011).

13. H. Robins, C. Desmarais, J. Matthis, R. Livingston, J. Andriesen, H. Reijonen, C. Carlson, G. Nepom, C. Yee, K. Cerosaletti, Ultra-sensitive detection of rare T cell clones. Journal of Immunological Methods, (2011)10.1016/j.jim.2011.09.001).

14. A. M. Sherwood, C. Desmarais, R. J. Livingston, J. Andriesen, M. Haussler, C. S. Carlson, H. Robins, Deep sequencing of the human TCRgamma and TCRbeta repertoires suggests that TCRbeta rearranges after alphabeta and gammadelta T cell commitment. Science translational medicine 3, 90ra61 (2011); published online EpubJul 6 (10.1126/scitranslmed.3002536).

15. K. Larimore, M. W. McCormick, H. S. Robins, P. D. Greenberg, Shaping of human germline IgH repertoires revealed by deep sequencing. Journal of immunology 189, 3221-3230 (2012); published online EpubSep 15 (10.4049/jimmunol.1201303).

16. M. Faham, J. Zheng, M. Moorhead, V. E. Carlton, P. Stow, E. Coustan-Smith, C. H. Pui, D. Campana, Deep-sequencing approach for minimal residual disease detection in acute lymphoblastic leukemia. Blood 120, 5173-5180 (2012); published online EpubDec 20 (10.1182/blood-2012-07-444042).

17. D. Wu, A. Sherwood, J. R. Fromm, S. S. Winter, K. P. Dunsmore, M. L. Loh, H. A. Greisman, D. E. Sabath, B. L. Wood, H. Robins, High-throughput sequencing detects minimal residual disease in acute T lymphoblastic leukemia. Science translational medicine 4, 134ra163-134ra163 (2012).

18. S. A. Grupp, M. Kalos, D. Barrett, R. Aplenc, D. L. Porter, S. R. Rheingold, D. T. Teachey, A. Chew, B. Hauck, J. F. Wright, Chimeric antigen receptor-Modified T cells for acute lymphoid leukemia. New England Journal of Medicine 368, 1509-1518 (2013).

19. W.-K. Weng, R. Armstrong, S. Arai, C. Desmarais, R. Hoppe, Y. H. Kim, Minimal Residual Disease Monitoring with High-Throughput Sequencing of T Cell Receptors in Cutaneous T Cell Lymphoma. Science translational medicine 5, 214ra171-214ra171 (2013).

20. M. L. Davila, I. Riviere, X. Wang, S. Bartido, J. Park, K. Curran, S. S. Chung, J. Stefanski, O. Borquez-Ojeda, M. Olszewska, Efficacy and Toxicity Management of 19-28z CAR T Cell Therapy in B Cell Acute Lymphoblastic Leukemia. Science translational medicine 6, 224ra225-224ra225 (2014).

21. M. Embleton, G. Gorochov, P. T. Jones, G. Winter, In-cell PCR from mRNA: amplifying and linking the rearranged immunoglobulin heavy and light chain V-genes within single cells. Nucleic acids research 20, 3831-3837 (1992).

22. P.-J. Meijer, P. S. Andersen, M. Haahr Hansen, L. Steinaa, A. Jensen, J. Lantto, M. B. Oleksiewicz, K. Tengbjerg, T. R. Poulsen, V. W. Coljee, Isolation of human antibody repertoires with preservation of the natural heavy and light chain pairing. Journal of molecular biology 358, 764-772 (2006).

23. N. Chapal, M. Bouanani, M. Embleton, I. Navarro-Teulon, M. Biard-Piechaczyk, B. Pau, S. Peraldi-Roux, In-cell assembly of scFv from human thyroid-infiltrating B cells. Biotechniques 23, 518-524 (1997).

24. S. M. Kim, L. Bhonsle, P. Besgen, J. Nickel, A. Backes, K. Held, S. Vollmer, K. Dornmair, J. C. Prinz, Analysis of the paired TCR alpha- and beta-chains of single human T cells. PloS one 7, e37338 (2012)10.1371/journal.pone.0037338).

25. B. J. DeKosky, G. C. Ippolito, R. P. Deschner, J. J. Lavinder, Y. Wine, B. M. Rawlings, N. Varadarajan, C. Giesecke, T. Dorner, S. F. Andrews, P. C. Wilson, S. P. Hunicke-Smith, C. G. Willson, A. D. Ellington, G. Georgiou, High-throughput sequencing of the paired human immunoglobulin heavy and light chain repertoire. Nature biotechnology 31, 166-169 (2013); published online EpubFeb (10.1038/nbt.2492).

26. M. A. Turchaninova, O. V. Britanova, D. A. Bolotin, M. Shugay, E. V. Putintseva, D. B. Staroverov, G. Sharonov, D. Shcherbo, I. V. Zvyagin, I. Z. Mamedov, C. Linnemann, T. N. Schumacher, D. M. Chudakov, Pairing of T-cell receptor chains via emulsion PCR. European journal of immunology 43, 2507-2515 (2013)10.1002/eji.201343453).

27. X. Sun, M. Saito, Y. Sato, T. Chikata, T. Naruto, T. Ozawa, E. Kobayashi, H. Kishi, A. Muraguchi, M. Takiguchi, Unbiased Analysis of TCR$\alpha/\beta$ Chains at the Single-Cell Level in Human CD8+ T-Cell Subsets. PloS one 7, e40386 (2012).

28. S.-M. Kim, L. Bhonsle, P. Besgen, J. Nickel, A. Backes, K. Held, S. Vollmer, K. Dornmair, J. C. Prinz, Analysis of the paired TCR $\alpha$-and $\beta$-chains of single human T cells. PloS one 7, e37338 (2012).

29. P. Dash, J. L. McClaren, T. H. Oguin III, W. Rothwell, B. Todd, M. Y. Morris, J. Becksfort, C. Reynolds, S. A. Brown, P. C. Doherty, Paired analysis of TCRα and TCRβ chains at the single-cell level in mice. The Journal of clinical investigation 121, 288 (2011).

30. C. E. Busse, I. Czogiel, P. Braun, P. F. Arndt, H. Wardemann, Single-cell based high-throughput sequencing of full-length immunoglobulin heavy and light chain genes. European journal of immunology 44, 597-603 (2014); published online EpubFeb (10.1002/eji.201343917).

31. Y.-C. Tan, L. K. Scalfone, S. Kongpachith, C.-H. Ju, X. Cai, T. M. Lindstrom, J. Sokolove, W. H. Robinson, Sequencing Antibody Repertoires Provides Evidence for Original Antigenic Sin Shaping the Antibody Response to Influenza Vaccination. Clinical Immunology, (2014).

32. J. D. Ashwell, A. Weissman, in Clinical Immunology, Principles and Practice, R. R. Rich, Ed. (Mosby International Limited, London, 2001), chap. 5, pp. 5.1-5.19.

33. C. S. Carlson, R. O. Emerson, A. M. Sherwood, C. Desmarais, M. W. Chung, J. M. Parsons, M. S. Steen, M. A. LaMadrid-Herrmannsfeldt, D. W. Williamson, R. J. Livingston, D. Wu, B. L. Wood, M. J. Rieder, H. Robins, Using synthetic templates to design an unbiased multiplex PCR assay. Nature communications 4, 2680 (2013)10.1038/ncomms3680).

34. http://www.imgt.org/IMGTrepertoire/Probes/Rearrangements%20and%20junc tions/human/HuTRrea.html.

35. Y. Sandberg, B. Verhaaf, E. J. van Gastel-Mol, I. L. Wolvers-Tettero, J. de Vos, R. A. Macleod, J. G. Noordzij, W. A. Dik, J. J. van Dongen, A. W. Langerak, Human T-cell lines with well-defined T-cell receptor gene rearrangements as controls for the BIOMED-2 multiplex polymerase chain reaction tubes. Leukemia 21, 230-237 (2007); published online EpubFeb (10.1038/sj.leu.2404486).

36. H. P. Bonarius, F. Baas, E. B. Remmerswaal, R. A. van Lier, I. J. ten Berge, P. P. Tak, N. de Vries, Monitoring the T-cell receptor repertoire at single-clone resolution. PloS one 1, e55 (2006)10.1371/journal.pone.0000055).

37. M. Klinger, K. Kong, M. Moorhead, L. Weng, J. Zheng, M. Faham, Combining next-generation sequencing and immune assays: a novel method for identification of antigen-specific T cells. PloS one 8, e74231 (2013)10.1371/journal.pone.0074231).

38. H. D. Royer, D. Ramarli, O. Acuto, T. J. Campen, E. L. Reinherz, Genes encoding the T-cell receptor alpha and beta subunits are transcribed in an ordered manner during intrathymic ontogeny. Proceedings of the National Academy of Sciences 82, 5510-5514 (1985).

39. R. N. Sangster, J. Minowada, N. Suciu-Foca, M. Minden, T. W. Mak, Rearrangement and expression of the alpha, beta, and gamma chain T cell receptor genes in human thymic leukemia cells and functional T cells. The Journal of experimental medicine 163, 1491-1508 (1986); published online EpubJune 1, 1986 (10.1084/jem.163.6.1491).

40. D. Chowdary, J. Lathrop, J. Skelton, K. Curtin, T. Briggs, Y. Zhang, J. Yu, Y. Wang, A. Mazumder, Prognostic gene expression signatures can be measured in tissues collected in RNAlater preservative. The journal of molecular diagnostics 8, 31-39 (2006).

41. T. Bancroft, C. Du, D. Nettleton, Estimation of False Discovery Rate Using Sequential Permutation p-Values. Biometrics 69, 1-7 (2013).

**Claims**

1. A method of identifying a plurality of adaptive immune receptor (AIR) cognate pairs comprising a first polypeptide and a second polypeptide that form an AIR heterodimer from a single clone in a sample, said sample comprising a plurality of lymphoid cells from a mammalian subject, said method comprising:

    A) distributing said plurality of lymphoid cells among a plurality of containers such that each container comprises a plurality of lymphoid cells;
    B) generating a library of amplicons in said plurality of containers by performing multiplex PCR of cDNA molecules, wherein the cDNA molecules have been reverse-transcribed from mRNA molecules obtained from the plurality of lymphoid cells, and wherein said library of amplicons comprises:

    i) a plurality of first AIR amplicons encoding a first AIR polypeptide, wherein each first AIR amplicon comprises (a) a unique variable (V) region encoding sequence or a unique J region encoding sequence, (b) a barcode sequence, (c) a universal adaptor sequence, and (d) a sequencing platform tag sequence, and
    ii) a plurality of second AIR amplicons encoding a second AIR polypeptide, wherein each second AIR amplicon comprises (a) a unique V region encoding sequence or a unique J region encoding sequence, (b) a barcode sequence, (c) a universal adaptor sequence, and (d) a sequencing platform tag sequence;

C) performing high throughput sequencing of said library of amplicons generated in step B) to obtain a data set of a plurality of first AIR amplicon sequences and second AIR amplicon sequences;

D) determining a container occupancy pattern for each first AIR amplicon sequence and each second AIR amplicon sequence by assigning each first AIR amplicon sequence and each second AIR amplicon sequence to one or more containers, wherein each barcode sequence is associated with a particular container;

E) for each possible pairing of first and second AIR amplicon sequences to form a putative AIR cognate pair, calculating a statistical probability of observing the container occupancy pattern, or a statistical probability of observing any larger proportion of shared containers than expected by chance, given that said first AIR amplicon sequence and second AIR amplicon sequence do not originate from said same clonal population of lymphoid cells, and identifying a plurality of putative AIR cognate pairs based on said statistical probability having a score lower than a predetermined likelihood cutoff;

F) for each identified putative AIR cognate pair, determining a false discovery rate estimation for a possible false pairing of said first AIR amplicon sequence and said second AIR amplicon sequence; and

G) identifying a plurality of putative AIR cognate pairs as true AIR cognate pairs based on said statistical probability and said false discovery rate estimation.

2. The method of claim 1, wherein the plurality of containers comprise wells of a multi-well plate.

3. The method of claim 1, wherein said first polypeptide comprises a TCR$\alpha$ polypeptide and said second polypeptide comprises a TCR$\beta$ polypeptide, wherein said first polypeptide comprises a TCR$\gamma$ polypeptide and said second polypeptide comprises a TCR$\delta$ polypeptide, wherein said first polypeptide comprises an IgH polypeptide and said second polypeptide comprises an Ig$\lambda$ polypeptide, or wherein said first polypeptide comprises an IgH polypeptide and said second polypeptide comprises an Ig$\kappa$ polypeptide.

4. The method of claim 1, wherein said sample comprises a blood sample.

5. The method of claim 1, wherein said sample comprises a tissue sample.

6. The method of claim 1, wherein said sample comprises purified or cultured human lymphoid cells.

7. The method of claim 1, wherein each container comprises at least $10^4$ lymphoid cells.

8. The method of claim 1, wherein calculating said statistical probability comprises determining a p-value for pairing each putative first AIR sequence and second AIR sequence.

9. The method of claim 1, wherein determining a false discovery rate estimation comprises:

a) calculating p-values for possible pairings of each of said plurality of first AIR sequences and second AIR sequences identified in said sample;

b) comparing said p-values for all of said possible pairings of first AIR sequences and second AIR sequences with an expected p-value distribution, wherein said expected p-value distribution is calculated to represent an experiment where no true pairs of first AIR sequences and second AIR sequences are present; and

c) for each putative pair of a first AIR sequence and a second AIR sequence, determining an expected proportion of false positive results, such that all p-values at or below the p-value of said putative pair of first AIR sequence and second AIR sequence are determined to represent a true cognate pairing.

10. The method of claim 9, wherein calculating said expected p-value distribution comprises permuting said containers in which each first AIR sequence and second AIR sequence has been observed in an otherwise-identical experiment with no true first AIR sequence and second AIR sequence pairs, and calculating said distribution of p-values associated with each putative AIR pair.

**Patentansprüche**

1. Verfahren zum Identifizieren einer Vielzahl adaptiver Immunrezeptor (AIR)-kognate Paare, umfassend ein erstes Polypeptid und ein zweites Polypeptid, die ein AIR-Heterodimer bilden aus einem einzigen Klon in einer Probe, wobei die Probe eine Vielzahl Lymphoidzellen eines Säugers umfasst, wobei das Verfahren umfasst:

**EP 2 971 105 B1**

A) Verteilen der Vielzahl Lymphoidzellen auf eine Vielzahl von Behältnissen, sodass jedes Behältnis eine Vielzahl Lymphoidzellen umfasst;

B) Erzeugen einer Bibliothek von Amplikons in der Vielzahl von Behältnissen durch Durchführen von Multiplex-PCR von cDNA-Molekülen, worin die cDNA-Moleküle revers trankribiert wurden von m-RNA-Molekülen, erhalten aus der Vielzahl Lymphoidzellen, und worin die Bibliothek von Amplikons umfasst:

i) eine Vielzahl von ersten AIR-Amplikons, die für ein erstes AIR-Polypeptid codieren, worin jedes erste AIR-Amplikon (a) eine für eine einzigartige variable (V)-Region codierende Sequenz oder eine für eine einzigartige J-Region codierende Sequenz, (b) eine Barcode-Sequenz, (c) eine universelle Adaptorsequenz und (d) eine Sequenzierungs-Plattform-tag-Sequenz umfasst; und

(ii) eine Vielzahl von zweiten AIR-Amplikons, die für ein zweites AIR-Polypeptid codieren, worin jedes zweite AIR-Amplikon (a) eine für eine einzigartige V-Region codierende Sequenz oder eine für eine einzigartige J-Region codierende Sequenz, (b) eine Barcode-Sequenz, (c) eine universelle Adaptorsequenz und (d) eine Sequenzierungs-Plattform-tag-Sequenz umfasst;

(C) Durchführen einer Sequenzierung mit hohem Durchsatz der Bibliothek von Amplikons, die in Schritt B) erzeugt wurde, um einen Datensatz einer Vielzahl von ersten AIR-Amplikonsequenzen und zweiten AIR-Amplikonsequenzen zu erhalten;

(D) Bestimmen eines Behältnisbelegungsmusters für jede erste AIR-Amplikonsequenz und jede zweite AIR-Amplikonsequenz durch Zuordnen jeder ersten AIR-Amplikonsequenz und jeder zweiten AIR-Amplikonsequenz zu einem oder mehreren Behältnissen, worin jede Barcode-Sequenz mit einem bestimmten Behältnis verbunden ist;

E) Berechnen für jede mögliche Paarung von ersten und zweiten AIR-Amplikonsequenzen zur Bildung eines putativen AIR-kognaten Paars einer statistischen Wahrscheinlichkeit der Beobachtung des Behältnisbesetzungsmusters oder einer statistischen Wahrscheinlichkeit der Beobachtung eines größeren Anteils von gemeinsamen Behältnissen, als durch Zufall erwartet, vorausgesetzt, dass die erste AIR-Amplikonsequenz und zweite AIR-Amplikonsequenz nicht von der gleichen Klonpopulation von Lymphoidzellen stammen, und Identifizieren einer Vielzahl von putativen AIR-kognaten Paaren, basierend auf der statistischen Wahrscheinlichkeit mit einem Wert von niedriger als ein vorbestimmter Likelihood-Cutoff;

F) für jedes identifizierte putative AIR-kognate Paar Bestimmen einer False-Discovery-Rate-Abschätzung für eine mögliche Falschpaarung der ersten AIR-Amplikon-Sequenz und der zweiten AIR-Amplikon-Sequenz; und

G) Identifizieren einer Vielzahl von putativen AIR-kognaten Paaren als echte AIR-kognate Paare, basierend auf der statistischen Wahrscheinlichkeit und der False-Discovery-Rate-Abschätzung.

2. Verfahren nach Anspruch 1, worin die Vielzahl von Behältnissen Wells einer Multi-Well-Platte umfasst.

3. Verfahren nach Anspruch 1, worin das erste Polypeptid ein TCR$\alpha$-Polypeptid umfasst und das zweite Polypeptid ein TCR$\beta$-Polypeptid umfasst, worin das erste Polypeptid ein TCR$\gamma$-Polypeptid umfasst und das zweite Polypeptid ein TCR$\delta$-Polypeptid umfasst, worin das erste Polypeptid ein IgH-Polypeptid umfasst und das zweite Polypeptid ein IG$\lambda$-Polypeptid umfasst, oder worin das erste Polypeptid ein IgH-Polypeptid umfasst und das zweite Polypeptid ein Ig$\kappa$-Polypeptid umfasst.

4. Verfahren nach Anspruch 1, worin die Probe eine Blutprobe umfasst.

5. Verfahren nach Anspruch 1, worin die Probe eine Gewebeprobe umfasst.

6. Verfahren nach Anspruch 1, worin die Probe gereinigte oder kultivierte humane Lymphoidzellen umfasst.

7. Verfahren nach Anspruch 1, worin jedes Behältnis mindestens $10^4$ Lymphoidzellen umfasst.

8. Verfahren nach Anspruch 1, worin das Berechnen der statistischen Wahrscheinlichkeit Bestimmen eines p-Werts für Paarung jeder putativen ersten AIR-Sequenz und zweiten AIR-Sequenz umfasst.

9. Verfahren nach Anspruch 1, worin das Bestimmen einer False-Discovery-Rate-Abschätzung umfasst:

a) Berechnen von p-Werten für mögliche Paarungen von jeder aus der Vielzahl von ersten AIR-Sequenzen und zweiten AIR-Sequenzen, die in der Probe identifiziert werden;

b) Vergleichen der p-Werte für alle der möglichen Paarungen von ersten AIR-Sequenzen und zweiten AIR-Sequenzen mit einer erwarteten p-Wert-Verteilung, worin die erwartete p-Wert-Verteilung berechnet ist, um ein Experiment darzustellen, worin keine echten Paare von ersten AIR-Sequenzen und zweiten AIR-Sequenzen vorliegen; und

c) Bestimmen eines erwarteten Anteils von falsch positiven Ergebnissen für jedes putative Paar aus einer ersten AIR-Sequenz und einer zweiten AIR-Sequenz, so dass alle p-Werte bei oder unter dem p-Wert des putativen Paars aus erster AIR-Sequenz und zweiter AIR-Sequenz bestimmt werden, um eine echte kognate Paarung darzustellen.

**10.** Verfahren nach Anspruch 9, worin die Berechnung der erwarteten p-Wert-Verteilung Permutieren der Behältnisse, in welchen jede erste AIR-Sequenz und zweite AIR-Sequenz beobachtet worden ist, in einem ansonsten identischen Experiment mit keinen echten ersten AIR-Sequenz- und zweiten AIR-Sequenzpaaren, und Berechnen der Verteilung von p-Werten, die verbunden sind mit jedem putativen AIR-Paar, umfasst.

**Revendications**

**1.** Procédé d'identification d'une pluralité de paires apparentées de récepteurs de l'immunité adaptative (AIR) comprenant un premier polypeptide et un deuxième polypeptide qui forme un hétérodimère d'AIR à partir d'un seul clone dans un échantillon, ledit échantillon comprenant une pluralité de cellules lymphoïdes issues d'un sujet mammifère, ledit procédé comprenant :

A) la distribution de ladite pluralité de cellules lymphoïdes parmi une pluralité de récipients de sorte que chaque récipient comprenne une pluralité de cellules lymphoïdes ;

B) la génération d'une banque d'amplicons dans ladite pluralité de récipients en réalisant une PCR multiplexe de molécules d'ADNc, où les molécules d'ADNc ont été soumises à une transcription inverse à partir de molécules d'ARNm obtenues à partir de la pluralité de cellules lymphoïdes, et où ladite banque d'amplicons comprend :

i) une pluralité de premiers amplicons d'AIR codant pour un premier polypeptide d'AIR, où chaque premier amplicon d'AIR comprend (a) une séquence codant pour une région variable (V) unique ou une séquence codant pour une région J unique, (b) une séquence code-barres, (c) une séquence d'adaptateur universel, et (d) une séquence de marqueur de plateforme de séquençage, et

ii) une pluralité de deuxièmes amplicons d'AIR codant pour un deuxième polypeptide d'AIR, où chaque deuxième amplicon d'AIR comprend (a) une séquence codant pour une région V unique ou une séquence codant pour une région J unique, (b) une séquence code-barres, (c) une séquence d'adaptateur universel, et (d) une séquence de marqueur de plateforme de séquençage ;

C) la réalisation d'un séquençage à haut débit de ladite banque d'amplicons générée à l'étape B) afin d'obtenir un ensemble de données d'une pluralité de premières séquences d'amplicons d'AIR et de deuxièmes séquences d'amplicons d'AIR ;

D) la détermination d'un profil d'occupation du récipient pour chaque première séquence d'amplicon d'AIR et chaque deuxième séquence d'amplicon d'AIR en attribuant chaque première séquence d'amplicon d'AIR et chaque deuxième séquence d'amplicon d'AIR à un ou plusieurs récipients, où chaque séquence code-barres est associée à un récipient particulier ;

E) pour chaque appariement possible des première et deuxième séquences d'amplicons d'AIR pour former une paire apparentée d'AIR putative, le calcul d'une probabilité statistique d'observer le profil d'occupation du récipient, ou d'une probabilité statistique d'observer une plus grande proportion de récipients partagés que celle due au hasard, à condition que lesdites première séquence d'amplicon d'AIR et deuxième séquence d'amplicon d'AIR ne proviennent pas de ladite même population clonale de cellules lymphoïdes, et l'identification d'une pluralité de paires apparentées d'AIR putatives en se basant sur ladite probabilité statistique présentant un score inférieur à un seuil de probabilité prédéterminé ;

F) pour chaque paire apparentée d'AIR putative identifiée, la détermination d'une estimation d'un taux de fausses découvertes pour un possible faux appariement de ladite première séquence d'amplicon d'AIR et ladite deuxième séquence d'amplicon d'AIR ; et

G) l'identification d'une pluralité de paires apparentées d'AIR putatives en tant que véritables paires apparentées d'AIR en se basant sur ladite probabilité statistique et ladite estimation d'un taux de fausses découvertes.

**2.** Procédé selon la revendication 1, dans lequel la pluralité de récipients comprend les puits d'une plaque multipuits.

**3.** Procédé selon la revendication 1, dans lequel ledit premier polypeptide comprend un polypeptide TCRα et ledit deuxième polypeptide comprend un polypeptide TCRβ, dans lequel ledit premier polypeptide comprend un polypeptide TCRγ et ledit deuxième polypeptide comprend un polypeptide TCRδ, dans lequel ledit premier polypeptide comprend un polypeptide IgH et ledit deuxième polypeptide comprend un polypeptide Igλ, ou dans lequel ledit premier polypeptide comprend un polypeptide IgH et ledit deuxième polypeptide comprend un polypeptide Igκ.

**4.** Procédé selon la revendication 1, dans lequel ledit échantillon comprend un échantillon de sang.

**5.** Procédé selon la revendication 1, dans lequel ledit échantillon comprend un échantillon de tissu.

**6.** Procédé selon la revendication 1, dans lequel ledit échantillon comprend des cellules lymphoïdes humaines purifiées ou en culture.

**7.** Procédé selon la revendication 1, dans lequel chaque récipient comprend au moins $10^4$ cellules lymphoïdes.

**8.** Procédé selon la revendication 1, dans lequel le calcul de ladite probabilité statistique comprend la détermination d'une valeur p pour l'appariement de chaque première séquence d'AIR et deuxième séquence d'AIR putatives.

**9.** Procédé selon la revendication 1, dans lequel la détermination d'une estimation d'un taux de fausses découvertes comprend :

a) le calcul des valeurs p pour les appariements possibles de chacune de ladite pluralité de premières séquences d'AIR et deuxièmes séquences d'AIR identifiées dans ledit échantillon ;
b) la comparaison desdites valeurs p pour l'intégralité desdits appariements possibles de premières séquences d'AIR et deuxièmes séquences d'AIR présentant une distribution de valeurs p attendue, où ladite distribution de valeurs p attendue est calculée pour représenter une expérience dans laquelle aucune véritable paire de premières séquences d'AIR et deuxièmes séquences d'AIR n'est présente ; et
c) pour chaque paire putative d'une première séquence d'AIR et d'une deuxième séquence d'AIR, la détermination d'une proportion attendue de résultats faux-positifs, si bien que toutes les valeurs p égales ou inférieures à la valeur p de ladite paire putative de première séquence d'AIR et deuxième séquence d'AIR sont déterminées pour représenter un véritable appariement apparenté.

**10.** Procédé selon la revendication 9, dans lequel le calcul de ladite distribution de valeurs p attendue comprend la permutation desdits récipients dans lesquels chaque première séquence d'AIR et deuxième séquence d'AIR a été observée dans une expérience autrement identique sans aucune véritable paire de première séquence d'AIR et deuxième séquence d'AIR, et le calcul de ladite distribution des valeurs p associées à chaque paire d'AIR putative.

FIG. 1

FIG. 2

FIG. 3

FIG. 4

FIG. 5

**FIG. 6**

FIG. 7

A

B

TCR α **TCGTATGAGCTTCCATGTG**
TCR β
**AATAGGCAACCTACGTTAG**

TCR α TCGTATGAGCTTCCATGTG
TCR β
AATAGGCAACCTACGTTAG

TCR α **TCGTATGAGCTTCCATGTG**
TCR β
**AATAGGCAACCTACGTTAG**

**Primary Experimental Plate**
**(70,000 T cells per well)**

**Pooled cDNA's**
**from all wells**
**are sequenced**

**Informatic reconstitution of**
**the plate yields cognate**
**paired chains**

FIG. 8

**A**

FDR = 1%

34,763 pairs

Number of called pairs

0    10k    20k    30k    40k

Estimated number of false pairs

0    1k    2k    3k

**B**

Sequenced
Paired

Number of clones

0    5k    10k    15k

0.5k    0.25k    0

0    24    48    72    96

Number of occupied wells

0    24    48    72    96

FIG. 9

FIG. 10

FIG. 11

Subject X    Subject Y

PBMC X    PBMC XY    PBMC Y

Repertoire X    pairSEQ XY    Repertoire Y

$\alpha_x$  $\beta_x$    $\alpha_x\beta_x$  $\alpha_y\beta_y$    $\alpha_x\beta_y$  $\alpha_y\beta_x$    $\alpha_y$  $\beta_y$

**True pairs**    **False pairs**

FIG. 12

FIG. 13

FIG. 14

FIG. 15

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61789408 A
- US 2013045994 W
- US 13217126 B
- US 20120058902 A
- US 12794507 B
- US 20100330571 A
- WO 2010151416 A
- WO 2011106738 A
- US 2011026373 W
- WO 2012027503 A
- US 2011049012 W
- WO 2013134162 A
- WO 2012083225 A

- JP 1997075090 A
- US 5596090 A
- GB 2241703 A
- WO 9640039 A
- EP 668350 A
- US 5683886 A
- US 5571711 A
- US 201070330571 A
- US 4458066 A
- US 20100167353 A
- US 5554516 A
- US 6031091 A, Arnold
- US 20070218490 A

**Non-patent literature cited in the description**

- **ROBINS et al.** *Blood,* 2009, vol. 114, 4099
- **ROBINS et al.** *Sci. Translat. Med.,* 2010, vol. 2, 47ra64
- **ROBINS et al.** *J. Immunol. Meth.,* 2011
- **SHERWOOD et al.** *Sci. Translat. Med.,* 2011, vol. 3, 90ra61
- **KANAGAWA.** *J. Biosci. Bioeng.,* 2003, vol. 96, 317
- **DAY et al.** *Hum. Mol. Genet.,* 1996, vol. 5, 2039
- **OGINO et al.** *J. Mol. Diagnost.,* 2002, vol. 4, 185
- **BARNARD et al.** *Biotechniques,* 1998, vol. 25, 684
- **AIRD et al.** *Genome Biol.,* 2011, vol. 12, R18
- **PEKIN et al.** *Lab Chip,* 2011, vol. 11, 2156
- **MILLER et al.** *Proc. Nat. Acad. Sci. USA,* 2012, vol. 109, 378
- **BROUZES et al.** *Proc. Nat. Acad. Sci. USA,* 2009, vol. 106, 14195
- **JOENSSON et al.** *Angew. Chem. Int. Ed.,* 2009, vol. 81, 4813
- **BARET et al.** *Lab Chip,* 2009, vol. 9, 1850
- **FRENZ et al.** *Lab Chip,* 2009, vol. 9, 1344
- **KISS et al.** *Anal. Chem.,* 2008, vol. 80, 8975
- **LEAMON et al.** *Nat. Meths.,* 2006, vol. 3, 541
- **MARGULIES et al.** *Nature,* 2005, vol. 437, 376-380

- **DIEHL, F. et al.** *Proc. Natl. Acad. Sci. U.S.A.,* 2005, vol. 102, 16368-16373
- **LI, M. et al.** *Nat.Methods,* 2006, vol. 3, 95-97
- **DIEHL, F. et al.** *Nat. Med.,* 2008, vol. 14, 985-990
- **LI, M. et al.** *Nat. Biotechnol.,* 2009, vol. 27, 858-U118
- **ZENG, Y. et al.** *Anal. Chem.,* 2010, vol. 82, 3183-3190
- **A. MANZ ; N. GRABER ; H.M. WIDMER.** Miniaturized total Chemical Analysis systems: A Novel Concept for Chemical Sensing. *Sensors and Actuators, B Chemical,* 1990, 244-248
- **HOLTZE, C. et al.** *Lab Chip,* 2008
- **BROUZES, E. et al.** *PNAS,* 2009, vol. 106 (34), 14195-14200
- **NIU, X. et al.** *Lab Chip,* 2008, vol. 8, 1837-1841
- **ZAGNONI, M. et al.** *Langmuir,* 2010, vol. 26 (18), 14443-14449
- **ABATE, A.R. et al.** *PNAS,* 2010, vol. 107 (45), 19163-19166
- **KWON et al.** *Proc. Nat. Acad. Sci. USA,* 1989, vol. 86, 1963
- **GOODWIN et al.** *Eur. J. Immunol.,* 1993, vol. 23, 2361
- **MELERO et al.** *Eur. J. Immunol.,* 1998, vol. 3, 116
- **JUNE et al.** *Immunol. Today,* 1990, vol. 11, 211

- **FARRAR et al.** *Ann. Rev. Immunol.,* 1993, vol. 11, 571
- **GRAY et al.** *Nature,* 1982, vol. 295, 503
- **RINDERKNECHT et al.** *J. Biol. Chem.,* 1984, vol. 259, 6790
- **DEGRADO et al.** *Nature,* 1982, vol. 300, 379
- 53rd Forum in Immunology. *Research in Immunol.,* 1993, vol. 144, 553-643
- **BANCHEREAU et al.** The Cytokine Handbook. Academic Press, 1994, 99
- **KEEGAN et al.** *J Leukocyt. Biol..,* 1994, vol. 55, 272
- **FREEMAN et al.** *J. Immunol.,* 1989, vol. 43, 2714
- **FREEMAN et al.** *J. Exp. Med.,* 1991, vol. 174, 625
- **FREEMAN et al.** *J. Exp. Med.,* 1993, vol. 178, 2185
- **BORIELLO et al.** *J. Immunol.,* 1995, vol. 155, 5490
- **DONG et al.** *Nat. Med.,* 24 June 2002
- **TSENG et al.** *J. Exp. Med.,* 2001, vol. 193, 839
- **TAMURA et al.** *Blood,* 2001, vol. 97, 1809
- **DONG et al.** *Nat. Med.,* 1999, vol. 5, 1365
- **REITER et al.** *Crit. Rev. Immunol.,* 1993, vol. 13, 1
- **YOSHINO et al.** *J. Immunol.,* 1994, vol. 152, 2393
- **DISIS et al.** *Canc. Res.,* 1994, vol. 54, 16
- **PLOWMAN et al.** *Nature,* 1993, vol. 366, 473
- **TROWBRIDGE ; OMARY.** *Proc. Nat. Acad. USA,* 1981, vol. 78, 3039
- **BARNES et al.** *Proc. Nat. Acad. Sci. USA,* 1989, vol. 86, 7159
- **KAWAKAMI et al.** *Proc. Nat. Acad. Sci. USA,* 1994, vol. 91, 3515
- **TOPALIAN et al.** *Proc. Nat. Acad. Sci. USA,* 1994, vol. 91, 9461
- **WEBER et al.** *J. Clin. Invest,* 1998, vol. 102, 1258
- **ADEMA et al.** *J. Biol. Chem.,* 1994, vol. 269, 20126
- **VAN DEN BRUGGEN et al.** *Science,* 1991, vol. 254, 1643
- **GOLD ; FREEDMAN.** *J. Exp. Med.,* 1985, vol. 121, 439
- When Cells Die: A Comprehensive Evaluation of Apoptosis and Programmed Cell Death. John Wiley & Sons, 1998
- **GREEN et al.** *Science,* 1998, vol. 281, 1309
- **FERREIRA et al.** *Clin. Canc. Res.,* 2002, vol. 8, 2024
- **GURUMURTHY et al.** *Cancer Metastas. Rev.,* 2001, vol. 20, 225
- **KANDUC et al.** *Int. J. Oncol.,* 2002, vol. 21, 165
- **BILLADEAU et al.** *J. Clin. Invest.,* 2002, vol. 109, 161
- *IEEE Rev Biomed Eng.,* 2010, vol. 3, 120-54
- **XU R ; WUNSCH DC 2.** Clustering algorithms in biomedical research: a review. *Mol Biotechnol.,* September 2005, vol. 31 (1), 55-80
- **ZHAO Y ; KARYPIS G.** *Methods Mol Biol.,* 2010, vol. 593, 81-107
- *Proc Natl Acad Sci USA.,* 24 January 2012, vol. 109 (4), 1347-52
- **SHIROGUCHI K ; JIA TZ ; SIMS PA ; XIE XS.** *Proc Natl Acad Sci USA.,* 04 September 2012, vol. 109 (36), 14508-13
- **PEKIN et al.** *Lab. Chip,* 2011, vol. 11 (13), 2156
- **ZHONG et al.** *Lab. Chip,* 2011, vol. 11 (13), 2167
- **TEWHEY et al.** *Nature Biotechnol.,* 2009, vol. 27, 1025
- *Nature Biotechnol.,* 2010, vol. 28, 178
- **BOLOTIN et al.** *Eur. J. Immunol.,* 2012
- **GLANVILLE et al.** *PNAS,* 2011
- **LEFRANC.** *The Immunologist,* 1999, vol. 7, 132
- **KABAT et al.** *In: Sequences of Proteins of Immunological Interest,* 1991
- **CHOTHIA et al.** *J. Mol. Biol.,* 1987, vol. 196, 901
- **CHOTHIA et al.** *Nature,* 1989, vol. 342, 877
- **AL-LAZIKANI et al.** *J. Mol. Biol.,* 1997, vol. 273, 927
- **ROCK et al.** *J. Exp. Med.,* 1994, vol. 179, 323
- **SAADA et al.** *Immunol. Cell Biol.,* 2007, vol. 85, 323
- **ALTSCHUL et al.** *Nucleic Acids Res.,* 1997, vol. 25 (17), 3389-402
- **NARANG et al.** *Meth. Enzymol.,* 1979, vol. 68, 90-99
- **BROWN et al.** *Meth. Enzymol.,* 1979, vol. 68, 109-151
- **BEAUCAGE et al.** *Tetrahedron Lett.,* 1981, vol. 22, 1859-1862
- **GOODCHILD.** *Bioconjugate Chemistry,* 1990, vol. 1 (3), 165-187
- **WILK et al.** *Nucleic Acids Res.,* 1990, vol. 18 (8), 2065
- **DE CAREER et al.** *Adv. Env. Microbiol.,* 2011, vol. 77, 6310
- **PARAMESWARAN et al.** *Nucl. Ac. Res.,* 2007, vol. 35 (19), 330
- **ROH et al.** *Trends Biotechnol.,* 2010, vol. 28, 291
- **DEVITA, HELLMAN ; ROSENBERG'S.** Cancer: Principles and Practice of Oncology. Lippincott, Williams and Wilkins, 2008
- **PIZZO ; POPLACK.** Principles and Practice of Pediatric Oncology. Lippincott, Williams and Wilkins, 2001
- **VOGELSTEIN ; KINZLER.** The Genetic Basis of Human Cancer. McGraw Hill Professional, 2002

- **TALMADGE et al.** *Am. J. Pathol.,* 2007, vol. 170, 793
- **KERBEL.** *Canc. Biol. Therap.,* 2003, vol. 2 (1), S134
- **MAN et al.** *Canc. Met. Rev.,* 2007, vol. 26, 737
- **CESPEDES et al.** *Clin. Transl. Oncol.,* 2006, vol. 8, 318
- **RAMSDEN et al.** *Nucl. Ac. Res.,* 1994, vol. 22, 1785
- **AKAMATSU.** *J. Immunol.,* 1994, vol. 153, 4520
- **HESSE.** *Genes Dev.,* 1989, vol. 3, 1053
- **FANNING.** *Cell. Immunol. Immunopath.,* 1996, vol. 79, 1
- **LARIJANI et al.** *Nucl. Ac. Res.,* 1999, vol. 27, 2304
- **NADEL et al.** *J. Immunol.,* 1998, vol. 161, 6068
- **NADEL et al.** *J. Exp. Med.,* 1998, vol. 187, 1495
- **HESSE et al.** *Genes Dev.,* 1989, vol. 3, 1053
- **LEE et al.** *PLoS,* 2003, vol. 1 (1), E1
- **SAMBROOK et al.** Molecular Cloning: A Laboratory Manual. 2001
- **SAMBROOK et al.** Molecular Cloning: A Laboratory Manual. 1989
- **MANIATIS et al.** Molecular Cloning: A Laboratory Manual. 1982
- **AUSUBEL et al.** Current Protocols in Molecular Biology. John Wiley and Sons, July 2008
- Short Protocols in Molecular Biology: A Compendium of Methods from Current Protocols in Molecular Biology. Greene Pub. Associates and Wiley-Interscience
- **GLOVER.** DNA Cloning: A Practical Approach. IRL Press, Oxford Univ. Press, 1985, vol. I & II
- Current Protocols in Immunology. John Wiley & Sons, 2001
- Real-Time PCR: Current Technology and Applications. Caister Academic Press, 2009
- **ANAND.** Techniques for the Analysis of Complex Genomes. Academic Press, 1992
- **GUTHRIE ; FINK.** Guide to Yeast Genetics and Molecular Biology. Academic Press, 1991
- Oligonucleotide Synthesis. 1984
- Nucleic Acid Hybridization. 1985
- Transcription and Translation. 1984
- Animal Cell Culture. 1986
- **PERBAL.** A Practical Guide to Molecular Cloning. 1984
- **JANITZ.** Next-Generation Genome Sequencing. Wiley-VCH, 2008
- PCR Protocols (Methods in Molecular Biology). Humana Press, 2010
- Immobilized Cells And Enzymes. IRL Press, 1986
- Methods In Enzymology. Academic Press, Inc,
- Gene Transfer Vectors For Mammalian Cells. Cold Spring Harbor Laboratory, 1987
- **HARLOW ; LANE.** Antibodies. Cold Spring Harbor Laboratory Press, 1998
- Immunochemical Methods In Cell And Molecular Biology. Academic Press, 1987
- Handbook Of Experimental Immunology. 1986, vol. I-IV
- **RIOTT.** Essential Immunology. Blackwell Scientific Publications, 1988
- Embryonic Stem Cells: Methods and Protocols. Methods in Molecular Biology. 2002
- Embryonic Stem Cell Protocols: Volume I: Isolation and Characterization. Methods in Molecular Biology. 2006, vol. I
- Embryonic Stem Cell Protocols: Volume II: Differentiation Models. Methods in Molecular Biology. 2006, vol. II
- Human Embryonic Stem Cell Protocols. Methods in Molecular Biology. 2006
- Mesenchymal Stem Cells: Methods and Protocols. Methods in Molecular Biology. 2008
- Hematopoietic Stem Cell Protocols. Methods in Molecular Medicine. 2001
- Hematopoietic Stem Cell Protocols. Methods in Molecular Biology. 2008
- Neural Stem Cells: Methods and Protocols. Methods in Molecular Biology. 2008
- **KOEHL et al.** *Leukemia,* 2003, vol. 17, 232
- *References,* vol. 25, 27-31
- **N. R. GASCOIGNE ; Y.-H. CHIEN ; D. M. BECKER ; J. KAVALER ; M. M. DAVIS.** Genomic organization and sequence of T-cell receptor β-chain constant-and joining-region genes. *Nature,* 1984, vol. 310, 387-391
- **G. SIU ; S. P. CLARK ; Y. YOSHIKAI ; M. MALISSEN ; Y. YANAGI ; E. STRAUSS ; T. W. MAK ; L. HOOD.** The human T cell antigen receptor is encoded by variable, diversity, and joining gene segments that rearrange to generate a complete V gene. *Cell,* 1984, vol. 37, 393-401
- **Y. YOSHIKAI ; S. P. CLARK ; S. TAYLOR ; U. SOHN ; B. I. WILSON ; M. D. MINDEN ; T. W. MAK.** *Organization and sequences of the variable, joining and constant region genes of the human T-cell receptor α-chain,* 1985
- **B. TOYONAGA ; Y. YOSHIKAI ; V. VADASZ ; B. CHIN ; T. W. MAK.** Organization and sequences of the diversity, joining, and constant region genes of the human T-cell receptor beta chain. *Proceedings of the National Academy of Sciences,* 1985, vol. 82, 8624-8628
- **M. M. DAVIS ; P. J. BJORKMAN.** T-cell antigen receptor genes and T-cell recognition. *Nature,* 1988, vol. 334

- J. NIKOLICH-ŽUGICH ; M. K. SLIFKA ; I. MESSAOUDI. The many important facets of T-cell repertoire diversity. *Nature Reviews Immunology,* 2004, vol. 4, 123-132
- H. S. ROBINS ; P. V. CAMPREGHER ; S. K. SRIVASTAVA ; A. WACHER ; C. J. TURTLE ; O. KAHSAI ; S. R. RIDDELL ; E. H. WARREN ; C. S. CARLSON. Comprehensive assessment of T-cell receptor beta-chain diversity in alphabeta T cells. *Blood,* 2009, vol. 114, 4099-4107
- R. L. WARREN ; B. H. NELSON ; R. A. HOLT. Profiling model T-cell metagenomes with short reads. *Bioinformatics,* 2009, vol. 25, 458-464
- J. GLANVILLE ; W. ZHAI ; J. BERKA ; D. TELMAN ; G. HUERTA ; G. R. MEHTA ; I. NI ; L. MEI ; P. D. SUNDAR ; G. M. DAY. Precise determination of the diversity of a combinatorial antibody library gives insight into the human immunoglobulin repertoire. *Proceedings of the National Academy of Sciences of the United States of America,* 2009, vol. 106, 20216-20221
- S. D. BOYD ; E. L. MARSHALL ; J. D. MERKER ; J. M. MANIAR ; L. N. ZHANG ; B. SAHAF ; C. D. JONES ; B. B. SIMEN ; B. HANCZARUK ; K. D. NGUYEN. Measurement and clinical monitoring of human lymphocyte clonality by massively parallel VDJ pyrosequencing. *Science translational medicine,* 2009, vol. 1, 12ra23-12ra23
- H. S. ROBINS ; S. K. SRIVASTAVA ; P. V. CAMPREGHER ; C. J. TURTLE ; J. ANDRIESEN ; S. R. RIDDELL ; C. S. CARLSON ; E. H. WARREN. Overlap and effective size of the human CD8+ T cell receptor repertoire. *Science translational medicine,* 2010, vol. 2, 47ra64
- R. L. WARREN ; J. D. FREEMAN ; T. ZENG ; G. CHOE ; S. MUNRO ; R. MOORE ; J. R. WEBB ; R. A. HOLT. Exhaustive T-cell repertoire sequencing of human peripheral blood samples reveals signatures of antigen selection and a directly measured repertoire size of at least 1 million clonotypes. *Genome research,* 2011, vol. 21, 790-797
- H. ROBINS ; C. DESMARAIS ; J. MATTHIS ; R. LIVINGSTON ; J. ANDRIESEN ; H. REIJONEN ; C. CARLSON ; G. NEPOM ; C. YEE ; K. CEROSALETTI. Ultra-sensitive detection of rare T cell clones. *Journal of Immunological Methods,* 2011
- A. M. SHERWOOD ; C. DESMARAIS ; R. J. LIVINGSTON ; J. ANDRIESEN ; M. HAUSSLER ; C. S. CARLSON ; H. ROBINS. Deep sequencing of the human TCRgamma and TCRbeta repertoires suggests that TCRbeta rearranges after alphabeta and gammadelta T cell commitment. *Science translational medicine,* 2011, vol. 3, 90ra61
- K. LARIMORE ; M. W. MCCORMICK ; H. S. ROBINS ; P. D. GREENBERG. Shaping of human germline IgH repertoires revealed by deep sequencing. *Journal of immunology,* 2012, vol. 189, 3221-3230
- M. FAHAM ; J. ZHENG ; M. MOORHEAD ; V. E. CARLTON ; P. STOW ; E. COUSTAN-SMITH ; C. H. PUI ; D. CAMPANA. Deep-sequencing approach for minimal residual disease detection in acute lymphoblastic leukemia. *Blood,* 2012, vol. 120, 5173-5180
- D. WU ; A. SHERWOOD ; J. R. FROMM ; S. S. WINTER ; K. P. DUNSMORE ; M. L. LOH ; H. A. GREISMAN ; D. E. SABATH ; B. L. WOOD ; H. ROBINS. High-throughput sequencing detects minimal residual disease in acute T lymphoblastic leukemia. *Science translational medicine,* 2012, vol. 4, 134ra163-134ra163
- S. A. GRUPP ; M. KALOS ; D. BARRETT ; R. APLENC ; D. L. PORTER ; S. R. RHEINGOLD ; D. T. TEACHEY ; A. CHEW ; B. HAUCK ; J. F. WRIGHT. Chimeric antigen receptor-Modified T cells for acute lymphoid leukemia. *New England Journal of Medicine,* 2013, vol. 368, 1509-1518
- W.-K. WENG ; R. ARMSTRONG ; S. ARAI ; C. DESMARAIS ; R. HOPPE ; Y. H. KIM. Minimal Residual Disease Monitoring with High-Throughput Sequencing of T Cell Receptors in Cutaneous T Cell Lymphoma. *Science translational medicine,* 2013, vol. 5, 214ra171-214ra171
- M. L. DAVILA ; I. RIVIERE ; X. WANG ; S. BARTIDO ; J. PARK ; K. CURRAN ; S. S. CHUNG ; J. STEFANSKI ; O. BORQUEZ-OJEDA ; M. OLSZEWSKA. Efficacy and Toxicity Management of 19-28z CAR T Cell Therapy in B Cell Acute Lymphoblastic Leukemia. *Science translational medicine,* 2014, vol. 6, 224ra225-224ra225
- M. EMBLETON ; G. GOROCHOV ; P. T. JONES ; G. WINTER. In-cell PCR from mRNA: amplifying and linking the rearranged immunoglobulin heavy and light chain V-genes within single cells. *Nucleic acids research,* 1992, vol. 20, 3831-3837
- P.-J. MEIJER ; P. S. ANDERSEN ; M. HAAHR HANSEN ; L. STEINAA ; A. JENSEN ; J. LANTTO ; M. B. OLEKSIEWICZ ; K. TENGBJERG ; T. R. POULSEN ; V. W. COLJEE. Isolation of human antibody repertoires with preservation of the natural heavy and light chain pairing. *Journal of molecular biology,* 2006, vol. 358, 764-772
- N. CHAPAL ; M. BOUANANI ; M. EMBLETON ; I. NAVARRO-TEULON ; M. BIARD-PIECHACZYK ; B. PAU ; S. PERALDI-ROUX. In-cell assembly of scFv from human thyroid-infiltrating B cells. *Biotechniques,* 1997, vol. 23, 518-524
- S. M. KIM ; L. BHONSLE ; P. BESGEN ; J. NICKEL ; A. BACKES ; K. HELD ; S. VOLLMER ; K. DORNMAIR ; J. C. PRINZ. Analysis of the paired TCR alpha- and beta-chains of single human T cells. *PloS one,* 2012, vol. 7, e37338

- B. J. DEKOSKY ; G. C. IPPOLITO ; R. P. DESCHNER ; J. J. LAVINDER ; Y. WINE ; B. M. RAWLINGS ; N. VARADARAJAN ; C. GIESECKE ; T. DORNER ; S. F. ANDREWS. High-throughput sequencing of the paired human immunoglobulin heavy and light chain repertoire. *Nature biotechnology,* 2013, vol. 31, 166-169
- M. A. TURCHANINOVA ; O. V. BRITANOVA ; D. A. BOLOTIN ; M. SHUGAY ; E. V. PUTINTSEVA ; D. B. STAROVEROV ; G. SHARONOV ; D. SHCHERBO ; I. V. ZVYAGIN ; I. Z. MAMEDOV. Pairing of T-cell receptor chains via emulsion PCR. *European journal of immunology,* 2013, vol. 43, 2507-2515
- X. SUN ; M. SAITO ; Y. SATO ; T. CHIKATA ; T. NARUTO ; T. OZAWA ; E. KOBAYASHI ; H. KISHI ; A. MURAGUCHI ; M. TAKIGUCHI. Unbiased Analysis of TCR$\alpha$/$\beta$ Chains at the Single-Cell Level in Human CD8+ T-Cell Subsets. *PloS one,* 2012, vol. 7, e40386
- S.-M. KIM ; L. BHONSLE ; P. BESGEN ; J. NICKEL ; A. BACKES ; K. HELD ; S. VOLLMER ; K. DORNMAIR ; J. C. PRINZ. Analysis of the paired TCR $\alpha$-and $\beta$-chains of single human T cells. *PloS one,* 2012, vol. 7, e37338
- P. DASH ; J. L. MCCLAREN ; T. H. OGUIN III ; W. ROTHWELL ; B. TODD ; M. Y. MORRIS ; J. BECKSFORT ; C. REYNOLDS ; S. A. BROWN ; P. C. DOHERTY. Paired analysis of TCR$\alpha$ and TCR$\beta$ chains at the single-cell level in mice. *The Journal of clinical investigation,* 2011, vol. 121, 288
- C. E. BUSSE ; I. CZOGIEL ; P. BRAUN ; P. F. ARNDT ; H. WARDEMANN. Single-cell based high-throughput sequencing of full-length immunoglobulin heavy and light chain genes. *European journal of immunology,* 2014, vol. 44, 597-603
- Y.-C. TAN ; L. K. SCALFONE ; S. KONGPACHITH ; C.-H. JU ; X. CAI ; T. M. LINDSTROM ; J. SOKOLOVE ; W. H. ROBINSON. Sequencing Antibody Repertoires Provides Evidence for Original Antigenic Sin Shaping the Antibody Response to Influenza Vaccination. *Clinical Immunology,* 2014
- J. D. ASHWELL ; A. WEISSMAN. Clinical Immunology, Principles and Practice. Mosby International Limited, 2001, 5.1-5.19
- C. S. CARLSON ; R. O. EMERSON ; A. M. SHERWOOD ; C. DESMARAIS ; M. W. CHUNG ; J. M. PARSONS ; M. S. STEEN ; M. A. LAMADRID-HERRMANNSFELDT ; D. W. WILLIAMSON ; R. J. LIVINGSTON. Using synthetic templates to design an unbiased multiplex PCR assay. *Nature communications,* 2013, vol. 4, 2680
- Y. SANDBERG ; B. VERHAAF ; E. J. VAN GASTEL-MOL ; I. L. WOLVERS-TETTERO ; J. DE VOS ; R. A. MACLEOD ; J. G. NOORDZIJ ; W. A. DIK ; J. J. VAN DONGEN ; A. W. LANGERAK. Human T-cell lines with well-defined T-cell receptor gene rearrangements as controls for the BIOMED-2 multiplex polymerase chain reaction tubes. *Leukemia,* 2007, vol. 21, 230-237
- H. P. BONARIUS ; F. BAAS ; E. B. REMMERSWAAL ; R. A. VAN LIER ; I. J. TEN BERGE ; P. P. TAK ; N. DE VRIES. Monitoring the T-cell receptor repertoire at single-clone resolution. *PloS one,* 2006, vol. 1, e55
- M. KLINGER ; K. KONG ; M. MOORHEAD ; L. WENG ; J. ZHENG ; M. FAHAM. Combining next-generation sequencing and immune assays: a novel method for identification of antigen-specific T cells. *PloS one,* 2013, vol. 8, e74231
- H. D. ROYER ; D. RAMARLI ; O. ACUTO ; T. J. CAMPEN ; E. L. REINHERZ. Genes encoding the T-cell receptor alpha and beta subunits are transcribed in an ordered manner during intrathymic ontogeny. *Proceedings of the National Academy of Sciences,* 1985, vol. 82, 5510-5514
- R. N. SANGSTER ; J. MINOWADA ; N. SUCIU-FOCA ; M. MINDEN ; T. W. MAK. Rearrangement and expression of the alpha, beta, and gamma chain T cell receptor genes in human thymic leukemia cells and functional T cells. *The Journal of experimental medicine,* 1986, vol. 163, 1491-1508
- D. CHOWDARY ; J. LATHROP ; J. SKELTON ; K. CURTIN ; T. BRIGGS ; Y. ZHANG ; J. YU ; Y. WANG ; A. MAZUMDER. Prognostic gene expression signatures can be measured in tissues collected in RNAlater preservative. *The journal of molecular diagnostics,* 2006, vol. 8, 31-39
- T. BANCROFT ; C. DU ; D. NETTLETON. Estimation of False Discovery Rate Using Sequential Permutation p-Values. *Biometrics,* 2013, vol. 69, 1-7